(19) Europäisches Patentamt
European Patent Office
Office européen des brevets

(11) Publication number: **0 355 612 B1**

# (12) EUROPEAN PATENT SPECIFICATION

(45) Date of publication of patent specification: **27.07.94**

(51) Int. Cl.[5]: **C07D 417/04**, A61K 31/425, C07D 417/14, A61K 31/44, A61K 31/505

(21) Application number: **89114869.4**

(22) Date of filing: **11.08.89**

The file contains technical information submitted after the application was filed and not included in this specification

## (54) Furylthiazole derivatives, processes for the preparation thereof and pharmaceutical composition comprising the same.

(30) Priority: **15.08.88 GB 8819365**
**14.03.89 GB 8905818**

(43) Date of publication of application:
**28.02.90 Bulletin 90/09**

(45) Publication of the grant of the patent:
**27.07.94 Bulletin 94/30**

(84) Designated Contracting States:
**AT BE CH DE ES FR GB GR IT LI LU NL SE**

(56) References cited:
**EP-A- 0 003 640**
**EP-A- 0 161 841**
**JP-A-84 225 186**
**US-A- 4 814 341**

**CHEMICAL ABSTRACTS, vol. 103, no. 3, July 22, 1985, page 577, abstract no. 22581n, Columbus, Ohio, US**

**CHEMICAL ABSTRACTS, vol. 101, no. 1, July 2, 1984, pages 611-612, abstract no. 7147r, Columbus, Ohio, US**

(73) Proprietor: **FUJISAWA PHARMACEUTICAL CO., LTD.**
**4-7, Doshomachi 3-chome**
**Chuo-ku**
**Osaka-shi Osaka 541(JP)**

(72) Inventor: **Takasugi, Hisashi**
**1-14-33, Hamaguchinishi**
**Suminoe-ku**
**Osaka-shi Osaka 559(JP)**
Inventor: **Katsura, Yousuke**
**3-9-19, Uenonishi**
**Toyonaka-shi Osaka 560(JP)**
Inventor: **Inoue, Yoshikazu**
**233-3, Shimokemaazaminamidai**
**Amagasaki-shi Hyogo 661(JP)**
Inventor: **Tomishi, Tetsuo**
**5-24-11, Niina**
**Minoo-shi Osaka 562(JP)**

**EP 0 355 612 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Türk, Dietmar, Dr. rer. nat. et al**
**Türk, Gille, Hrabal, Leifert**
**Patentanwälte**
**Brucknerstrasse 20**
**D-40593 Düsseldorf (DE)**

## Description

This invention relates to new furylthiazole derivatives and pharmaceutically acceptable salts thereof.

More particularly, it relates to furylthiazole derivatives and pharmaceutically acceptable salts thereof which have antiulcer activity and $H_2$-receptor antagonism, to processes for the preparation thereof, to a pharmaceutical composition comprising the same and to a method for the treatment of ulcer in human being or animals.

Accordingly, one object of this invention is to provide new furylthiazole derivatives and pharmaceutically acceptable salts thereof which possess antiulcer activity and $H_2$-receptor antagonism.

Another object of this invention is to provide processes for the preparation of said furylthiazole derivatives and salt thereof.

A further object of this invention is to provide a pharmaceutical composition comprising, as an active ingredient, said furylthiazole derivatives or pharmaceutically acceptable salts thereof.

Still further object of this invention is to provide a therapeutical method for the treatment of ulcer in human being or animals.

The furylthiazole derivatives of this invention are new and can be represented by the following general formula (I) :

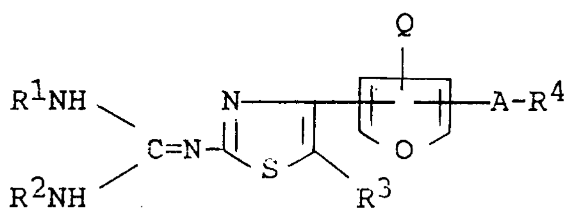

(I)

wherein

$R^1$ and $R^2$ are each hydrogen, furoyl or $C_1$-$C_6$ alkyl which may have halogen; or

$R^1$ and $R^2$ are linked together to form $C_1$-$C_6$ alkylene,

$R^3$ is hydrogen or $C_1$-$C_6$ alkyl,

$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)-alkanoylamino, benzoylamino optionally substituted by nitro,furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)-alkylthio($C_1$-$C_6$)alkanoylamino, furyl($C_1$-$C_6$)alkylsulfinyl($C_1$-$C_6$)alkanoylamino, mono or di-($C_1$-$C_6$)alkylsulfamoylamino, hydroxy($C_1$-$C_6$)alkylureido, $C_1$-$C_6$ alkanoyloxy($C_1$-$C_6$)-alkylureido, $C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of the formula :

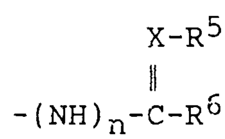

wherein

n is 0 or 1,

X is =CH- or =N-,

$R^5$ is hydrogen, cyano, nitro, carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$

alkyl, $C_1$-$C_6$ alkoxy, halogen and amino, or mono or di($C_1$-$C_6$)alkylsulfamoyl,

$R^6$ is hydrogen, $C_1$-$C_6$ alkyl, $C_1$-$C_6$ alkylthio, $C_1$-$C_6$ alkoxy, amino, mono or di($C_1$-$C_6$)-alkylamino, allylamino, propargylamino, hydroxy($C_1$-$C_6$)alkylamino, $C_1$-$C_6$ alkoxy($C_1$-$C_6$)-alkylamino, or mono or di($C_1$-$C_6$)alkylamino($C_1$-$C_6$)alkylamino;

A is $C_1$-$C_6$ alkylene or -CONH-; or

A-$R^4$ is imidazolyl substituted with $C_1$-$C_6$ alkyl, triazolyl substituted with amino, and

Q is hydrogen, or $C_1$-$C_6$ alkyl.

The object compound (I) or a salt thereof can be prepared by processes as illustrated in the following reaction schemes.

Process 1

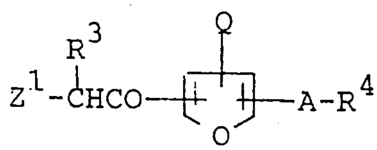

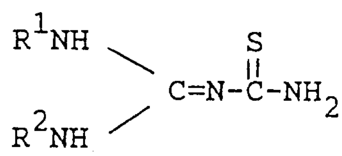

(III)

(II)
or a salt thereof

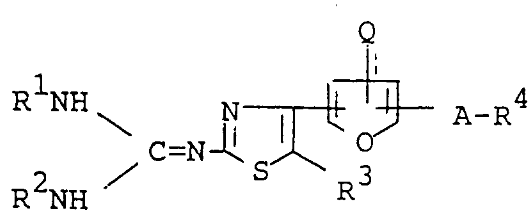

(I)
or a salt thereof

Process 2

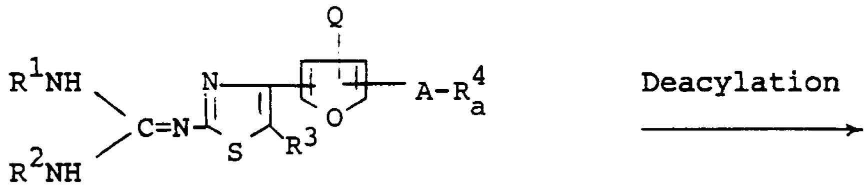


(I-1)
or a salt thereof

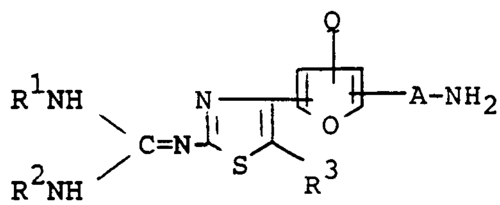

(I-2)
or a salt thereof

Process 3

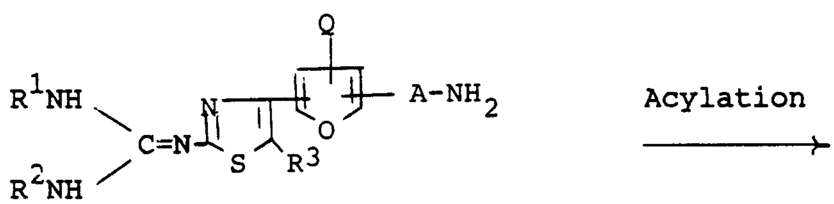


(I-2)
or a salt thereof

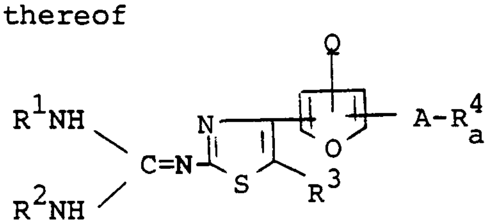

(I-1)
or a salt thereof

Process 4

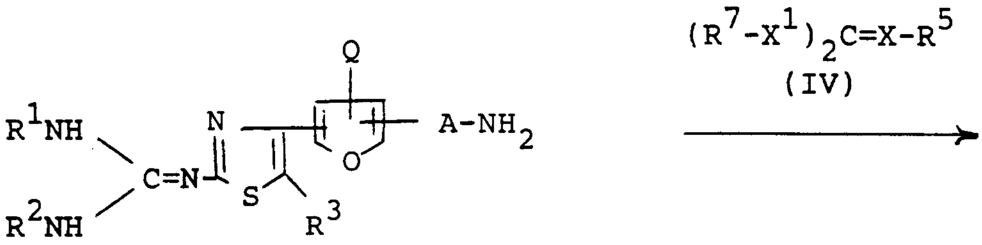


(I-2)
or a salt thereof

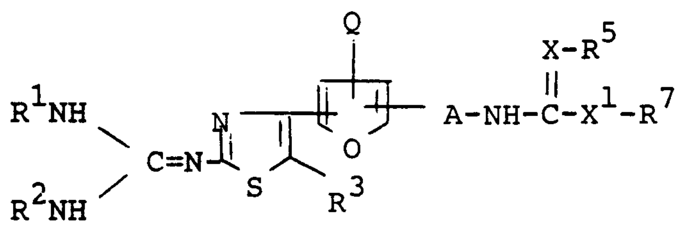

(I-3)
or a salt thereof

Process 5

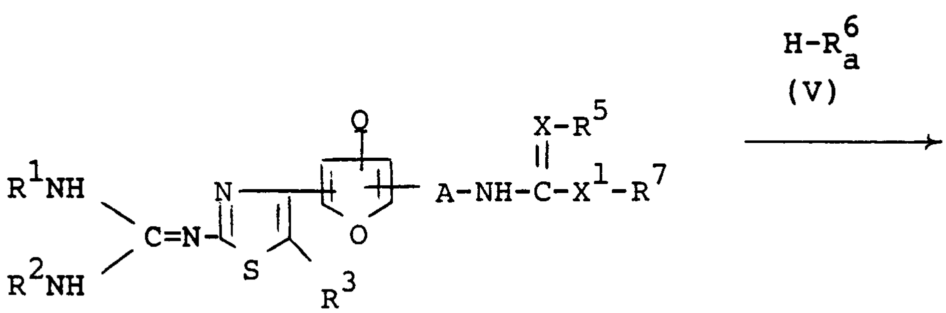


(I-3)
or a salt thereof

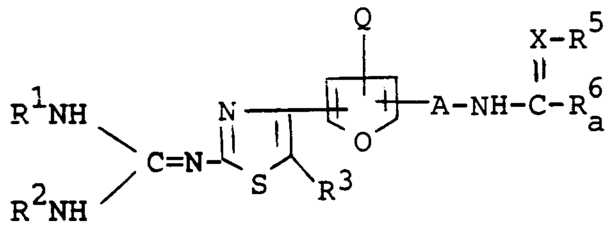

(I-4)
or a salt thereof

Process 6

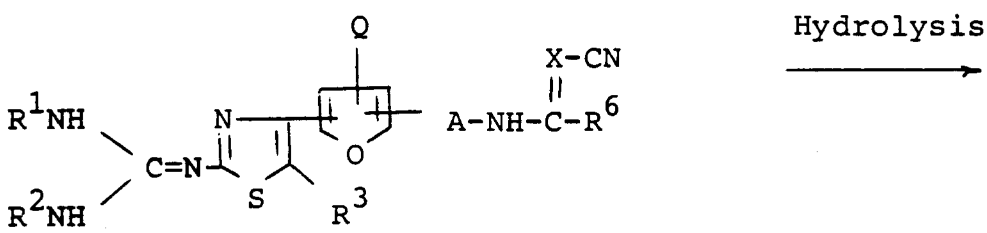


(I-5)
or a salt thereof

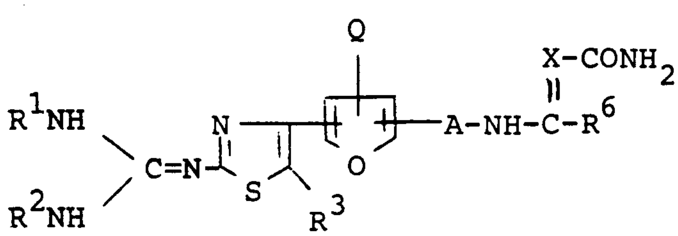

(I-6)
or a salt thereof

Process 7

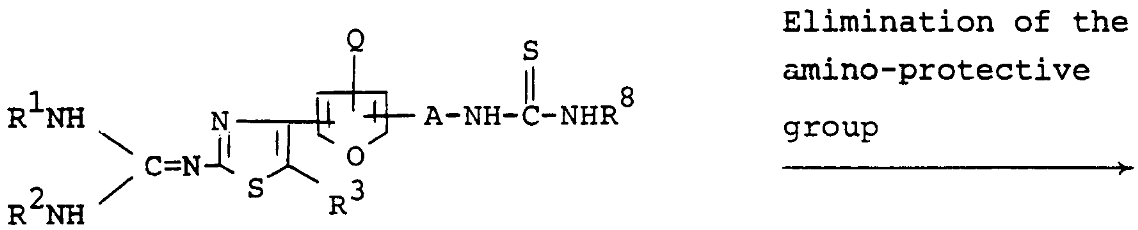


(I-7)
or a salt thereof

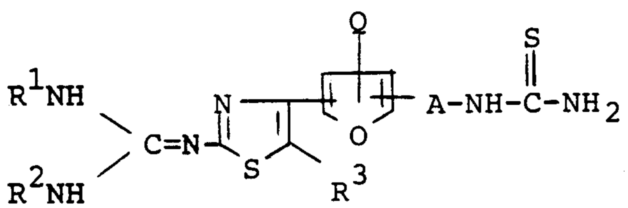

(I-8)
or a salt thereof

Process 8

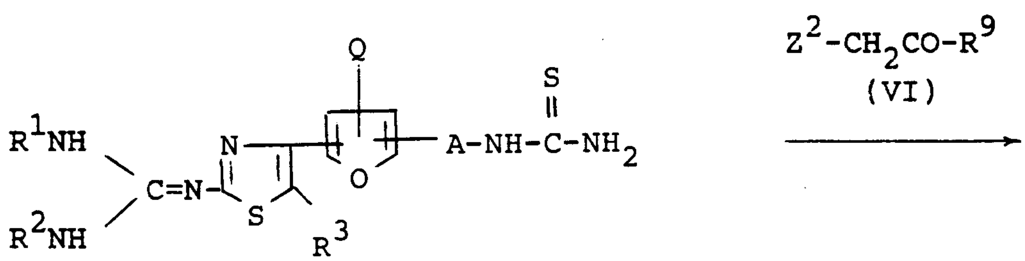


(I-8)
or a salt thereof

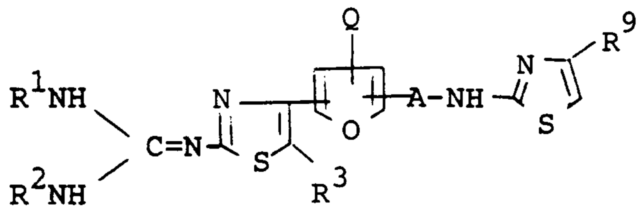

(I-9)
or a salt thereof

<u>Process 9</u>

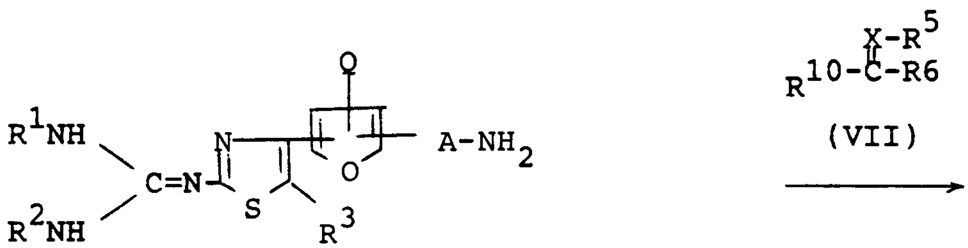


(I-2)
or a salt thereof

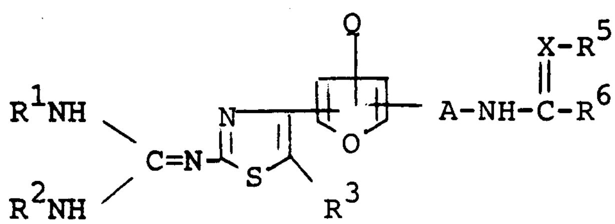

(I-10)
or a salt thereof

Process 10

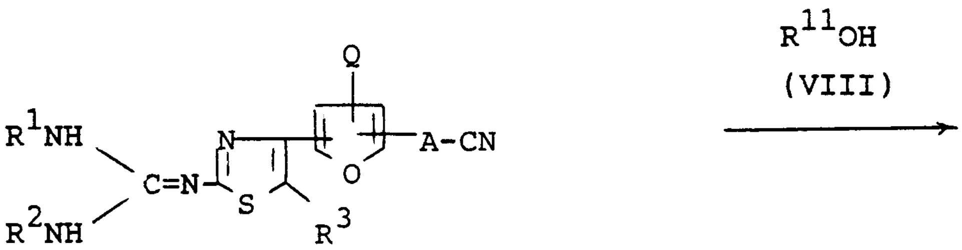


(I-11)
or a salt thereof

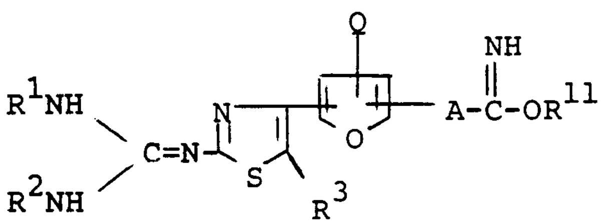

(I-12)
or a salt thereof

Process 11

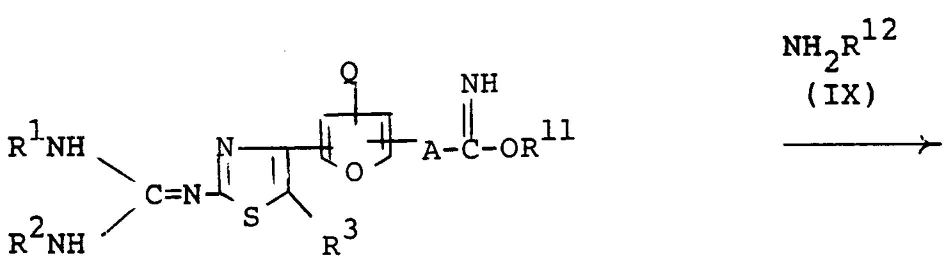


(I-12)
or a salt thereof

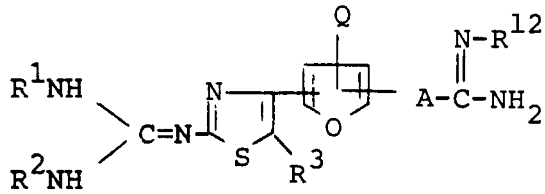

(I-13)

or a salt thereof

Process 12

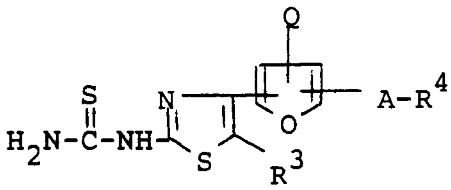

(X)

or a salt thereof

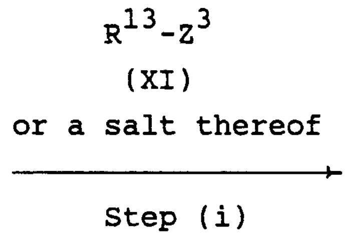


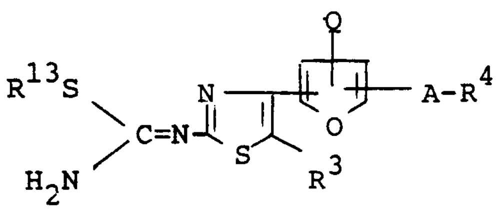

(XII)

or a salt thereof

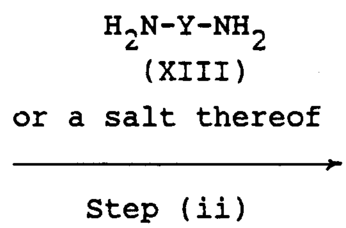


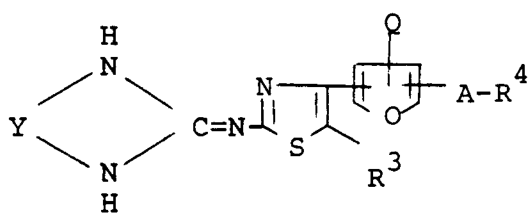

(I-14)

or a salt thereof

Process 13

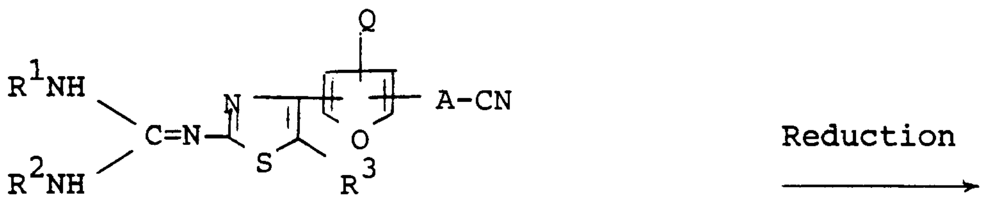


(I-11)
or a salt thereof

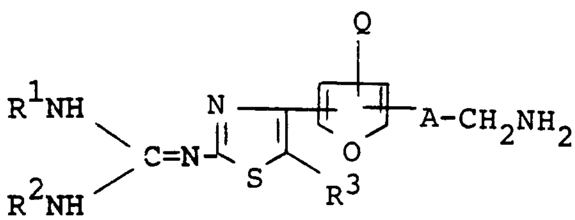

(I-15)
or a salt thereof

Process 14

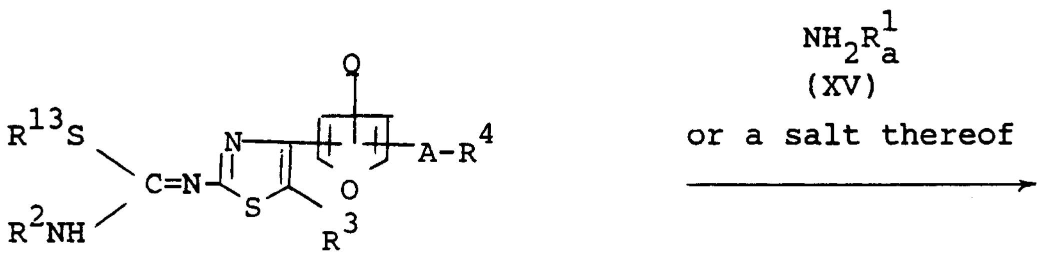


(XIV)
or a salt thereof

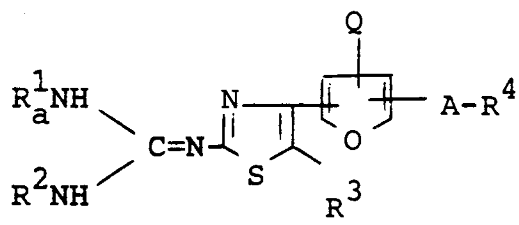

(I-16)
or a salt thereof

Process 15

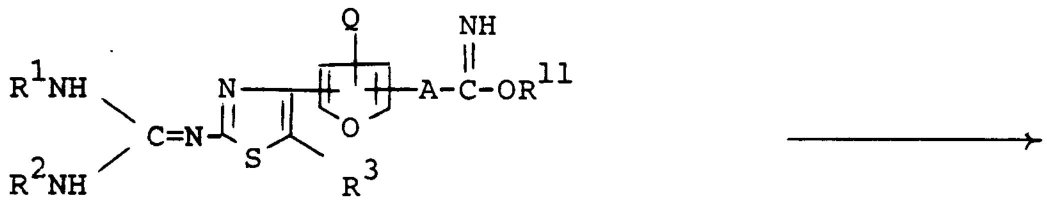

(I-12)
or a salt thereof

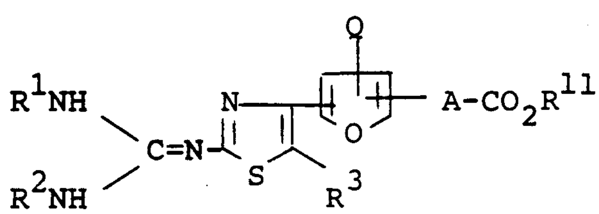

(I-17)
or a salt thereof

Process 16

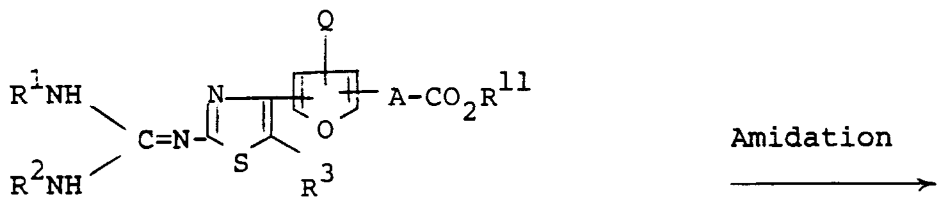


(I-17)
or a salt thereof

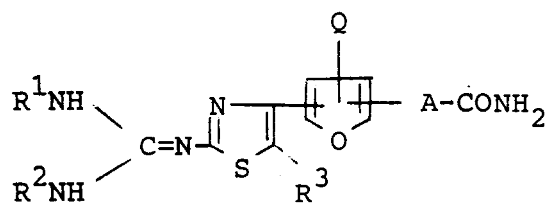

(I-18)
or a salt thereof

Process 17

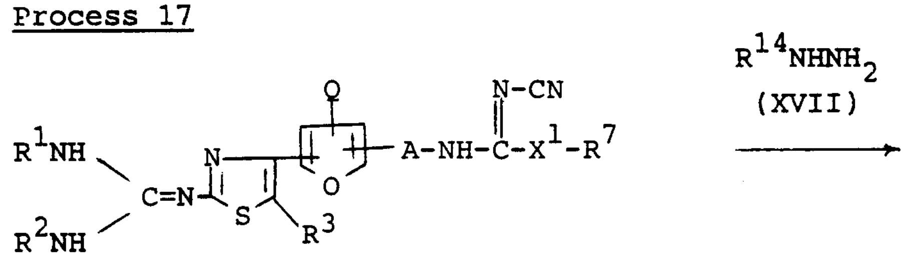


(I-19)
or a salt thereof

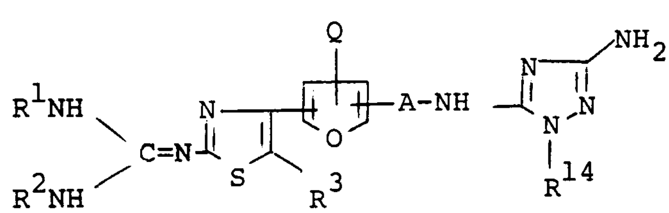

(I-20)
or a salt thereof

Process 18

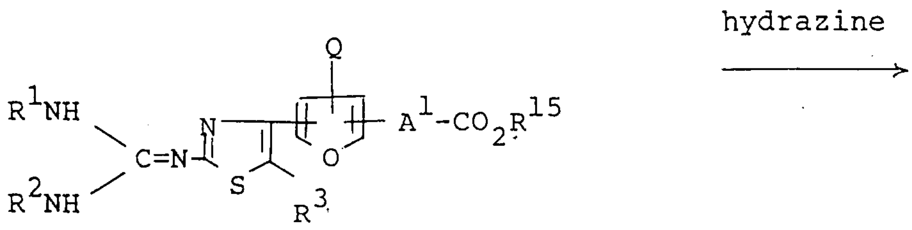


(XVIII)
or a salt thereof

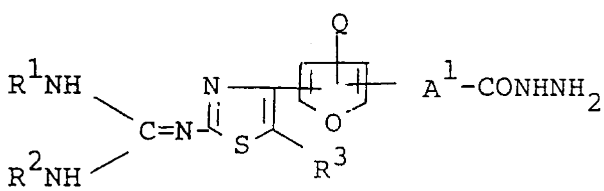

(I-21)
or a salt thereof

Process 19

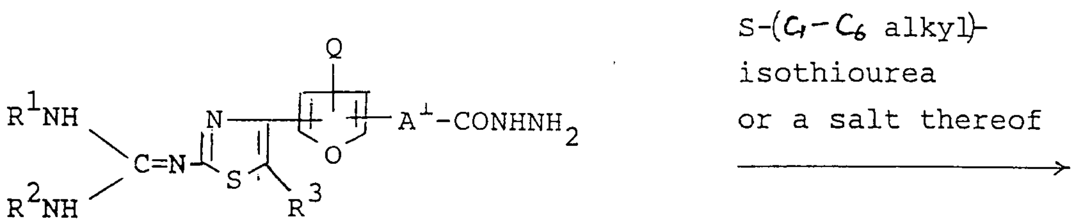


(I-21)
or a salt thereof

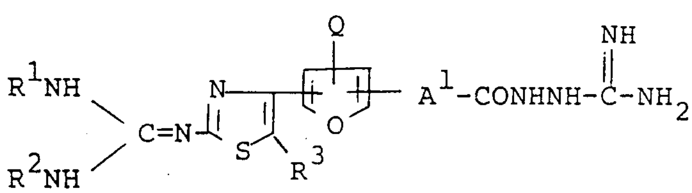

(I-22)
or a salt thereof

Process 20

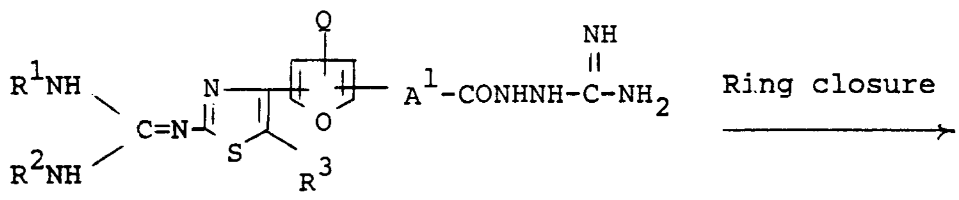


(I-22)
or a salt thereof

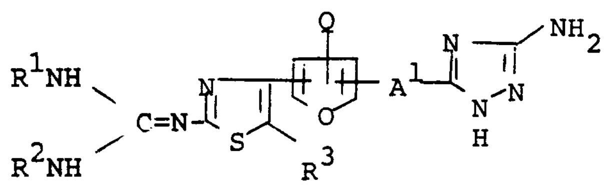

(I-23)
or a salt thereof

Process 21

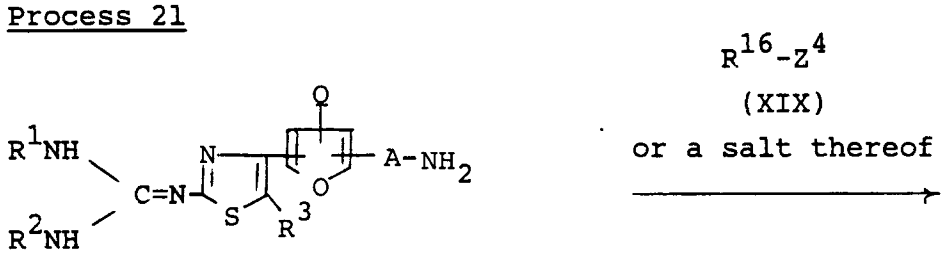


(I-2)
or a salt thereof

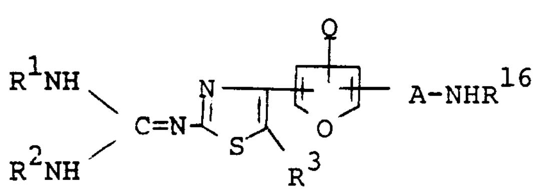

(I-24)
or a salt thereof

Process 22

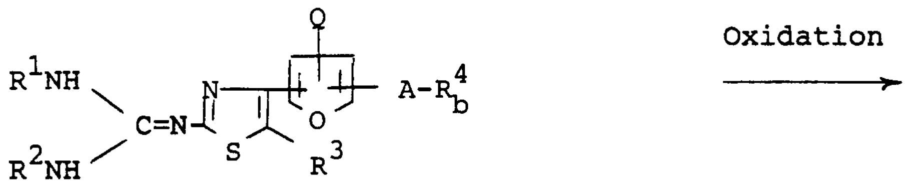


(I-25)
or a salt thereof

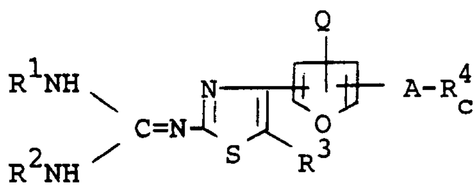

(I-26)
or a salt thereof

Process 23

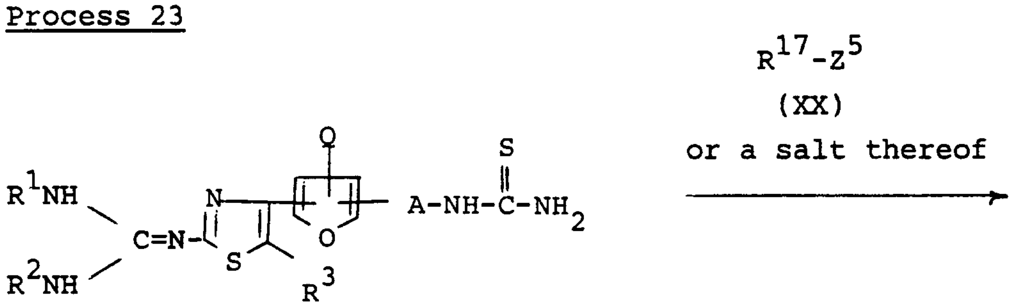


(I-8)
or a salt thereof

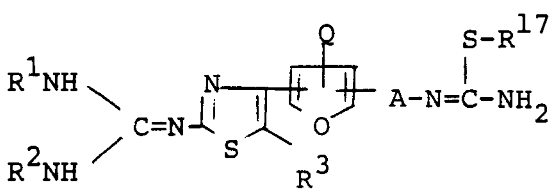

(I-27)
or a salt thereof

Process 24

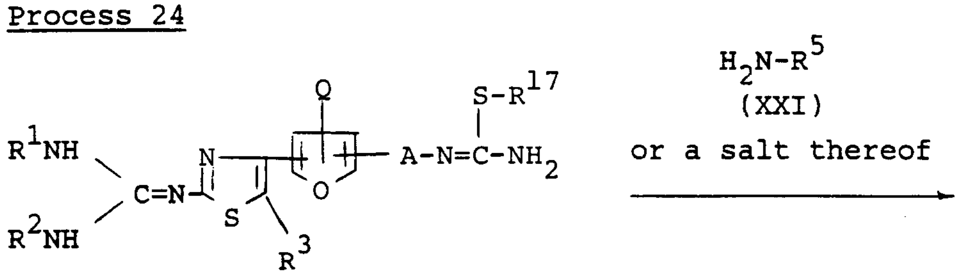


(I-27)
or a salt thereof

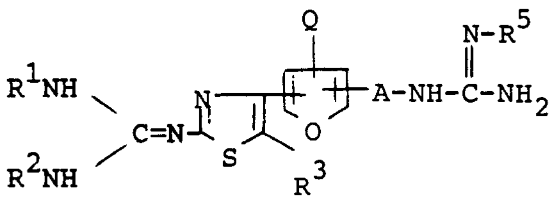

(I-28)
or a salt thereof

Process 25

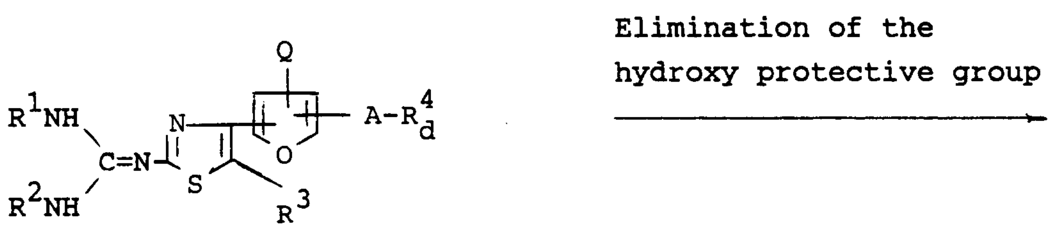


(I-29)
or a salt thereof

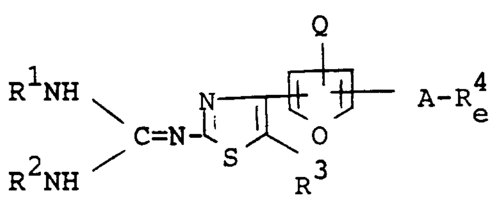

(I-30)
or a salt thereof

Process 26

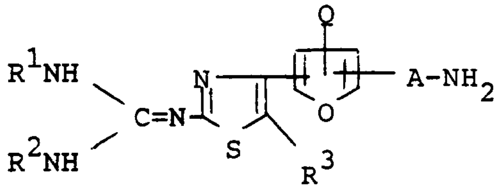

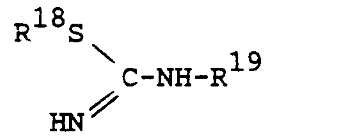

(XXII)
or a salt thereof

(I-2)
or a salt thereof

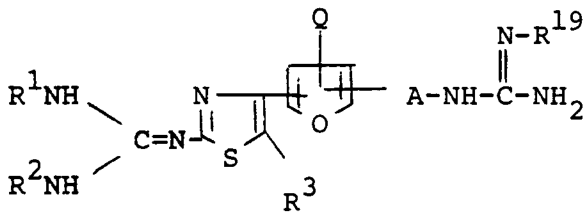

(I-31)
or a salt thereof

Process 27

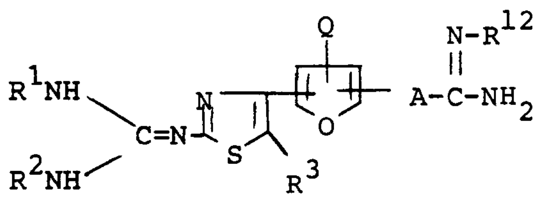

(I-13)
or a salt thereof

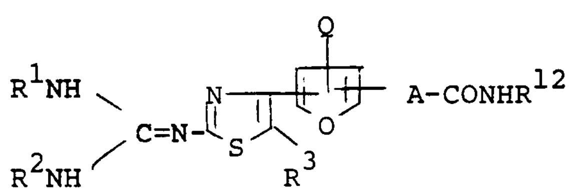

(I-32)
or a salt thereof

wherein

| | |
|---|---|
| $R^1$, $R^2$, $R^3$, $R^4$, $R^5$, $R^6$, X, A and Q | are each as defined above, |
| $R_a^1$ is | $C_1$-$C_6$ alkyl which may have halogen, |
| $R_a^4$ is | ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$- |

$C_6$)alkanoylamino, $C_1$-$C_6$ alkanoyloxy($C_1$-$C_6$)alkanoylamino, $C_1$-$C_6$ alkylthio($C_1$-$C_6$)alkanoylamino, benzoylamino optionally substituted with nitro, furoylamino, thenoylamino, nicotinoylamino, 1-oxonicotionylamino, morpholinocarbonylamino, $C_1$-$c_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)alkanoylamino, mono or di-($C_1$-$C_6$)alkylsulfamoylamino, hydroxy($C_1$-$C_6$)alkylureido, or $C_1$-$C_6$ alkanoyloxy($C_1$-$C_6$)alkylureido,

$R^4_b$ is furyl($C_1$-$C_6$)alkylthio($C_1$-$C_6$)alkanoylamino,

$R^4_c$ is furyl($C_1$-$C_6$)alkylsulfinyl($C_1$-$C_6$)alkanoylamino,

$R^4_d$ is $C_1$-$C_6$ alkanoyloxy($C_1$-$C_6$)alkylureido,

$R^4_e$ is hydroxy($C_1$-$C_6$)alkylureido,

$R^6_a$ is amino which may have mono or di($C_1$-$C_6$)alkyl, allyl, propargyl, hydroxy($C_1$-$C_6$)alkyl or mono or di($C_1$-$C_6$)alkylamino($C_1$-$C_6$)alkyl, or $C_1$-$C_6$ alkoxy,

$R^7$ is $C_1$-$C_6$ alkyl or phenyl,

$R^8$ is amino-protective group consisting of $C_1$-$C_6$ alkanoyl and benzoyl,

$R^9$, $R^{11}$, $R^{13}$, $R^{15}$, $R^{17}$ and $R^{18}$ are each $C_1$-$C_6$ alkyl,

$R^{10}$ is $C_1$-$C_6$ alkanoyloxy, phenyl($C_1$-$C_6$)alkoxy, $C_1$-$C_6$ alkoxy($C_1$-$C_6$)-alkoxy, tetrahydropyranyloxy or $C_1$-$C_6$ alkoxy,

$R^{12}$ and $R^{19}$ are each carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)alkylsulfamoyl,

$R^{14}$ is hydrogen or $C_1$-$C_6$ alkyl,

$R^{16}$ is thiazolyl substituted with $C_1$-$C_6$ alkyl, triazolyl substituted with amino, triazolyl substituted with amino and $C_1$-$C_6$ alkyl, benzoisothiazolyl substituted with oxo, or benzothiadiazinyl substituted with oxo,

$X^1$ is S or O,

Y is $C_1$-$C_6$ alkylene,

$Z^1$, $Z^2$, $Z^3$, $Z^4$ and $Z^5$ are each halogen, and

$A^1$ is $C_1$-$C_6$ alkylene or bond.

Suitable "$C_1$-$C_6$ alkyl" and "($C_1$-$C_6$)alkyl" moiety in the term "($C_1$-$C_6$)alkyl which may have halogen" may be a straight or branched one such as methyl, ethyl, propyl, isopropyl, butyl, isobutyl, tert-butyl, pentyl or hexyl, in which the preferable one is $C_1$-$C_4$ alkyl and the more preferable one is methyl or ethyl.

Suitable "halogen" may be chloro, bromo, fluoro or iodo.

Suitable "($C_1$-$C_6$) alkylene" and $C_1$-$C_6$ alkylene moiety formed by linkage of $R^1$ and $R^2$ may be straight or branched one such as methylene, ethylene, trimethylene, propylene, tetramethylene, pentamethylene or hexamethylene, in which the preferable one is $C_1$-$C_4$ alkylene and the most preferable one is ethylene.

Suitable "$C_1$-$C_6$ alkoxy" may be methoxy , ethoxy, propoxy, isopropoxy, butoxy, isobutoxy, tert-butoxy, pentyloxy or hexyloxy, in which the preferable one is $C_1$-$C_4$ alkoxy, the more preferable one is $C_1$-$C_2$ alkoxy and the most preferable one is methoxy.

Suitable "($C_1$-$C_6$)alkylcarbamoyl" may be the carbamoyl substituted with ($C_1$-$C_6$)alkyl (e.g. methyl, ethyl, propyl, isopropyl, butyl, pentyl or hexyl), and the preferable one is N-($C_1$-$C_6$)alkylcarbamoyl (e.g. N-methylcarbamoyl, N-ethylcarbamoyl or N-isopropylcarbamoyl).

Suitable "($C_1$-$C_6$)alkoxycarbonyl" may be methoxycarbonyl, ethoxycarbonyl, propoxycarbonyl, isopropoxycarbonyl, butoxycarbonyl or tert-butoxycarbonyl.

Suitable "($C_1$-$C_6$)alkanoyl" and "($C_1$-$C_6$)alkanoyl" moiety in the term "($C_1$-$C_6$)alkanoylamino" may be formyl, acetyl, propionyl, butyryl, isobutyryl, valeryl, isovaleryl, pivaloyl or hexanoyl.

Suitable "($C_1$-$C_6$)alkoxycarbonyl" moiety in the term "($C_1$-$C_6$)alkoxycarbonylamino" may be the ones as mentioned before for "$C_1$-$C_6$ alkoxycarbonyl".

Suitable "($C_1$-$C_6$)alkylsulfonyl" moiety in the term "($C_1$-$C_6$)alkylsulfonylamino" may be mesyl, ethanesulfonyl or propanesulfonyl.

Suitable "($C_1$-$C_6$)alkoxy($C_1$-$C_6$)alkanoyl" moiety in the term "($C_1$-$C_6$)alkoxy($C_1$-$C_6$)alkanoylamino" may be ($C_1$-$C_6$)alkanoyl as mentioned before substituted with ($C_1$-$C_6$)alkoxy (e.g. methoxy, ethoxy, propoxy, isopropoxy, butoxy, pentyloxy or hexyloxy) and the preferable one may be methoxyacetyl or ethoxyacetyl.

Suitable "($C_1$-$C_6$)alkanoyloxy" may be formyloxy, acetyloxy, propionyloxy, butyryloxy, isobutyryloxy, valeryloxy, isovaleryloxy, pivaloyloxy or hexanoyloxy.

Suitable "($C_1$-$C_6$)alkylthio may be methylthio, ethylthio, propylthio, isopropylthio, butylthio, pentylthio or hexylthio.

Suitable "($C_1$-$C_6$)alkyl" moiety in the term "($C_1$-$C_6$)alkylureido" and "($C_1$-$C_6$)alkylthioureido" may be ($C_1$-$C_6$)alkyl as mentioned before and the preferable ($C_1$-$C_6$)alkylureido may be 3-($C_1$-$C_6$)alkylureido (e.g. 3-methylureido, 3-ethylureido, 3-propylureido and 3-isopropylureido) and the preferable ($C_1$-$C_6$)alkylthioureido may be 3-($C_1$-$C_6$)alkylthioureido (e.g. 3-methylthioureido),

Suitable "furyl($C_1$-$C_6$)alkylthio($C_1$-$C_6$)alkanoyl" moiety in the term "furyl($C_1$-$C_6$)alkylthio($C_1$-$C_6$)-alkanoylamino" may be ($C_1$-$C_6$)alkylthio($C_1$-$C_6$)alkanoyl as mentioned before substituted with furyl.

Suitable "mono or di($C_1$-$C_6$)alkylamino" may be methylamino, dimethylamino, ethylamino or butylamino.

Suitable "hydroxy($C_1$-$C_6$)alkylamino" may be hydroxymethylamino, hydroxyethylamino, or hydroxypropylamino.

Suitable $C_1$-$C_6$ alkoxy($C_1$-$C_6$)alkylamino" may be methoxymethylamino.

Suitable "mono or di($C_1$-$C_6$)alkylamino($C_1$-$C_6$)alkylamino" may be methylaminomethylamino, or dimethylaminoethylamino.

Suitable "trihalo($C_1$-$C_6$)alkyl" may be trifluoro($C_1$-$C_6$)alkyl (e.g. trifluoromethyl or trifluoroethyl).

Suitable pharmaceutically acceptable salts of the object compound (I) are conventional non-toxic salts and include an organic acid addition salt [e.g. formate, acetate, trifluoroacetate, maleate, tartrate, methanesulfonate, benzenesulfonate, toluenesulfonate], an inorganic acid addition salt [e.g. hydrochloride, hydrobromide, sulfate, phosphate], a salt with an acidic amino acid [e.g. aspartic acid salt, glutamic acid salt].

With respect to the salt of the compound (I-1) to (I-32) in the Processes 1 to 27, it is to be noted that these compounds are included within the scope of the compound (I), and accordingly the suitable examples of the salts of these compounds are to be referred to those as salts of these compounds are to be referred to those as exemplified for the object compound (I).

Particularly, the preferred embodiments of $R^1$, $R^2$, $R^3$, $R^4$, $R^5$, $R^6$, A, X, n and Q are as follows.

| | |
|---|---|
| $R^1$ is | hydrogen, |
| | furoyl; |
| | $C_1$-$C_6$ alkyl which may have halogen such as $C_1$-$C_6$ alkyl (e.g. methyl or ethyl), trihalo($C_1$-$C_6$)alkyl (e.g. trifluoromethyl or trifluoroethyl); |
| $R^2$ is | hydrogen; or |
| $R^1$ and $R^2$ | are linked together to form $C_1$-$C_6$ alkylene (e.g. methylene or ethylene); |
| $R^3$ is | hydrogen; $C_1$-$C_6$ alkyl (e.g. methyl); |
| $R^4$ is | amino; |
| | carbamoyl, aminocarbamoyl, guanidinocarbamoyl, $C_1$-$C_6$ alkylcarbamoyl (e.g. methylcarbamoyl), sulfamoylaminocarbonyl, $C_1$-$C_6$ alkoxycarbonyl (e.g. methoxycarbonyl); |
| | ureido, thioureidosulfamoylamino, $C_1$-$C_6$ alkanoylamino (e.g. formylamino, acetylamino, propionylamino, butyrylamino, isobutyrylamino, or valerylamino), $C_1$-$C_6$ alkoxycarbonylamino (e.g. methoxycarbonylamino), $C_1$-$C_6$ alkylsulfonylamino (e.g. mesylamino), $C_1$-$C_6$ alkoxy($C_1$-$C_6$)alkanoylamino (e.g. methoxyacetylamino), $C_1$-$C_6$ alkanoyloxy($C_1$-$C_6$)-alkanoylamino (e.g. acetoxyacetylamino), $C_1$-$C_6$ alkylthio($C_1$-$C_6$)alkanoylamino (e.g. methylthioacetylamino), benzoylamino optionally substituted by nitro (e.g. nitrobenzoylamino), furoylamino, thenoylamino, nicotionylamino, 1-oxonicotinoylamino, morpholinocarbonylamino, $C_1$-$C_6$ alkylureido such as 3-($C_1$-$C_6$)alkylureido (e.g. 3-methylureido, 3-ethylureido, 3-propylureido, 3-isopropylureido), $C_1$-$C_6$ alkylthioureido such as 3-($C_1$-$C_6$)alkylthioureido (e.g. 3-methylthioureido), $C_1$-$C_6$ alkanoylureido such as 3-($C_1$-$C_6$)alkanoylureido (e.g. 3-acetylureido), allylureido, benzoylthioureido (e.g. 3-benzoylthioureido), furyl($C_1$-$C_6$)alkylthio($C_1$-$C_6$)alkanoylamino (e.g. furylmethylthioacetylamino), furyl($C_1$-$C_6$)alkylsulfinyl($C_1$-$C_6$)alkanoylamino (e.g. furylmethylsulfinylacetylamino), mono or di($C_1$-$C_6$)alkylsulfamoylamino (e.g. methylsulfamoylamino, dimethylsulfsmoylamino), hydroxy($C_1$-$C_6$)alkylureido such as 3-hydroxy($C_1$-$C_6$)alkylureido (e.g. 3-hydroxyethylureido), $C_1$-$C_6$ alkanoyloxy($C_1$-$C_6$)alkylureido such as 3-$C_1$-$C_6$ alkanoyloxy($C_1$-$C_6$)-alkylureido (e.g. 3-acetoxyethylureido); $C_1$-$C_6$ alkylisothioureido such as 2-$C_1$-$C_6$ alkylisothioureido (e.g. 2-methylisothioureido); thiazolylamino substituted with $C_1$-$C_6$ alkyl (e.g. 4-methylthiazolylamino), triazolylamino substituted with amino and/or $C_1$-$C_6$ alkyl (e.g. 3-aminotriazolylamino, 3-amino-1-methyltriazolylamino), benzoisothiazolylamino substituted |

with oxo (e.g. 1,1-dioxobenzoisothiazolylamino), benzothiadiazinylamino substituted with oxo and halogen (e.g. 1,1-dioxochlorobenzothiadiazinylamino); pyrimidinyl substituted with oxo and $C_1$-$C_6$ alkyl (e.g. 6-methyl-5-pentyl-4(1H)-pyrimidinon-2-yl); triazolyl substituted with amino (e.g. 3-aminotriazolyl); a group of the formula :

$$\begin{matrix} & X-R^5 \\ & \| \\ -(NH)_n- & C-R^6 \end{matrix}$$

wherein

n is 0 or 1;

X is =CH- or =N-;

$R^5$ is hydrogen;
cyano;
nitro; or
carbamoyl, $C_1$-$C_6$ alkoxycarbonyl (e.g. methoxycarbonyl), sulfamoyl, $C_1$-$C_6$ alkylsulfonyl (e.g. mesyl, ethylsulfonyl), benzenesulfonyl, which is substituted with one or more substituent(s) selected from $C_1$-$C_6$ alkyl, $C_1$-$C_6$ alkoxy, halogen and amino (e.g. tosyl, methoxyphenylsulfonyl, bromophenylsulfonyl, or aminophenylsulfonyl), mono or di($C_1$-$C_6$)-alkylsulfamoyl (e.g. methylsulfamoyl or dimethylsulfamoyl); and

$R^6$ is hydrogen;
$C_1$-$C_6$ alkyl (e.g. methyl); $C_1$-$C_6$ alkylthio (e.g. methylthio); $C_1$-$C_6$ alkoxy (e.g. methoxy); mono or di($C_1$-$C_6$)alkylamino (e.g. methylamino, dimethylamino, butylamino), allylamino, propargylamino, hydroxy ($C_1$-$C_6$)alkylamino (e.g. hydroxyethylamino, hydroxypropylamino), $C_1$-$C_6$ alkoxy($C_1$-$C_6$)alkylamino (e.g. methoxyethylamino) and mono or di-($C_1$-$C_6$)alkylamino($C_1$-$C_6$)alkylamino (e.g. dimethylaminoethylamino);

A is $C_1$-$C_6$ alkylene (e.g. methylene or ethylene);
-CONH-; or

A-$R^4$ is imidazolyl substituted with $C_1$-$C_6$ alkyl (e.g. 2-methylimidazolyl), triazolyl substituted with amino (e.g. 3-amino-1,2,4-triazolyl); and

Q is hydrogen; or
$C_1$-$C_6$ alkyl (e.g. methyl).

The processes for preparing the object compounds (I) of the present invention are explained in detail in the following.

Process 1 :

The object compound (I) or a salt thereof can be prepared by reacting the compound (II) or a salt thereof with the compound (III).

This reaction is usually conducted in a conventional solvent which does not adversely influence the reaction such as ethyl acetate, dichloromethane, chloroform, carbon tetrachloride, tetrahydrofuran, N,N-dimethylformamide, N,N-dimethylacetamide, dioxane, water, alcohol [e.g. methanol, ethanol. ] acetic acid, formic acid, or a mixture thereof.

The reaction temperature is not critical and the reaction is usually conducted under cooling to heating.

Process 2

The object compound (I-2) or a salt thereof can be prepared by subjecting the compound (I-1) or a salt thereof to deacylation.

Suitable method for this deacylation reaction may include conventional one such as hydrolysis, reduction.

The hydrolysis is preferably carried out in the presence of a base or an acid.

Suitable base may include, for example, an inorganic base such as alkali metal hydroxide (e.g. sodium hydroxide, potassium hydroxide. ), alkaline earth metal hydroxide (e.g. magnesium hydroxide, calcium hydroxide. ), alkali metal carbonate (e.g. sodium carbonate, potassium carbonate. ), alkaline earth metal

carbonate (e.g. magnesium carbonate, calcium carbonate. ), alkali metal bicarbonate (e.g. sodium bicarbonate, potassium bicarbonate. ), alkali metal acetate (e.g. Sodium acetate, potassium acetate. ), alkaline earth metal phosphate (e.g. magnesium phosphate, calcium phosphate. ), alkali metal hydrogen phosphate (e.g. disodium hydrogen phosphate, dipotassium hydrogen phosphate. ), and an organic base such as tri-($C_1$-$C_6$)alkylamine (e.g. trimethylamine, triethylamine. ), picoline, N-methylpyrrolidine, N-methylmorpholine, 1,5-diazabicyclo[4,3,0]non-5-one, 1,4-diazabicyclo[2,2,2]octane, 1,5-diazabicyclo[5,4,0]undecene-5. The hydrolysis using a base is often carried out in water or a hydrophilic organic solvent or a mixed solvent thereof.

Suitable acid may include an organic acid (e.g. formic acid, acetic acid, propionic acid) and an inorganic acid (e.g. hydrochloric acid, hydrobromic acid, sulfuric acid).

The present hydrolysis is usually carried out in an organic solvent, water or a mixed solvent thereof.

The reaction temperature is not critical, and the reaction is usually carried out at ambient temperature or under warming or heating.

Process 3

The object compound (I-1) or a salt thereof can be prepared by reacting the compound (I-2) or a salt thereof with an acylating agent.

The compound (I-2) may be used in the form of its conventional reactive derivative at the amino group.

The acylating agent can be represented by the compound of the formula :

$R^{20}$ - OH

in which $R^{20}$ is carbamoyl, thiocarbamoyl, sulfamoyl, $C_1$-$C_6$ alkanoyl, $C_1$-$C_6$ alkoxycarbonyl, $C_1$-$C_6$ alkylsulfonyl, $C_1$-$C_6$ alkoxy($C_1$-$C_6$)alkanoyl, $C_1$-$C_6$ alkanoyloxy($C_1$-$C_6$)alkanoyl, $C_1$-$C_6$ alkylthio($C_1$-$C_6$)alkanoyl, benzoyl optionally substituted with nitro, furoyl, thenoyl, nicotinoyl, 1-oxonicotinoyl; morpholinocarbonyl, $C_1$-$C_6$ alkylcarbamoyl, $C_1$-$C_6$ alkylthiocarbamoyl, $C_1$-$C_6$ alkanoylcarbamoyl, allylcarbamoyl, benzoylthiocarbamoyl, furyl($C_1$-$C_6$)alkylthio($C_1$-$C_6$)alkanoyl, mono or di($C_1$-$C_6$)alkylsulfamoyl, hydroxy($C_1$-$C_6$)alkylcarbamoyl, or $C_1$-$C_6$ alkanoyloxy($C_1$-$C_6$)alkylcarbamoyl, and its conventional reactive derivative at the hydroxy group.

The suitable example may be an acid halide (e.g. acid chloride), an acid anhydride, an activated amide, an activated ester.

In case the acyl group to be introduced is a carbamoyl type acyl, the acylating agent is usually used in the form of cyanate or isocyanate.

The reaction is usually carried out in a conventional solvent such as water, alcohol [e.g. methanol, ethanol, ] acetone, dioxane, acetonitrile, chloroform, dichloromethane, ethylene chloride, tetrahydrofuran, ethyl acetate, N,N-dimethylformamide, N,N-dimethylacetamide, pyridine, acetic acid or any other organic solvent which does not adversely influence the reaction. These conventional solvents may also be used in a mixture with water.

The reaction temperature is not critical, and the reaction is usually carried out under cooling to warming.

The reaction may also be carried out in the presence of an inorganic or organic base such as an alkali metal bicarbonate, tri($C_1$-$C_6$)alkylamine, pyridine, N-($C_1$-$C_6$)alkylmorpholine, N,N-di($C_1$-$C_6$)alkylbenzylamine.

Process 4

The object compound (I-3) or a salt thereof can be prepared by reacting the compound (I-2) or a salt thereof with the compound (IV).

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 5

The object compound (I-4) or a salt thereof can be prepared by reacting the compound (I-3) or a salt thereof with the compound (V).

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

In case that the compound (V) is liquid, it can be also used as a solvent.

The reaction temperature is not critical, and the reaction is usually carried out at ambient temperature or under warming or heating.

Process 6

The object compound (I-6) or a salt thereof can be prepared by subjecting the compound (I-5) or a salt thereof to hydrolysis reaction.

This reaction is usually carried out in a conventional manner for transforming nitrile to amide.

This reaction is usually carried out in a conventional solvent which does not adversely influence the reaction such as water, alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 7

The object compound (I-8) or a salt thereof can be prepared by subjecting the compound (I-7) or a salt thereof to elimination reaction of the amino-protective group.

This reaction can be carried out in substantially the same manner as Process 2, and therefore the reaction mode and reaction conditions [e.g. solvent, reaction temperature. ] of this reaction are to be referred to those as explained in Process 2.

Process 8

The object compound (I-9) or a salt thereof can be prepared by reacting the compound (I-8) or a salt thereof with the compound (VI).

This reaction can be carried out in substantially the same manner as Process 1, and therefore the reaction mode and reaction conditions [e.g. solvent, reaction temperature. ] of this reaction are to be referred to those as explained in Process 1.

Process 9

The object compound (I-10) or a salt thereof can be prepared by reacting the compound (I-2) or a salt thereof with the compound (VII).

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 10

The object compound (I-12) or a salt thereof can be prepared by reacting the compound (I-11) or a salt thereof with the compound (VIII).

This reaction is usually carried out in the presence of dry hydrogen chloride gas.

This reaction is usually carried out in a conventional solvent such as alcohol [e.g. methanol, ethanol. ], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction.

The reaction temperature is not critical, and the reaction is usually carried out under cooling to heating.

The object compound (I-12) can be used as a starting compound of Process 15 mentioned hereinbelow with or without isolation.

Process 11

The object compound (I-13) or a salt thereof can be prepared by reacting the compound (I-12) or a salt thereof with the compound (IX).

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 12-(i)

The compound (XII) or a salt thereof can be prepared by reacting the compound (X) or a salt thereof with the compound (XI) or a salt thereof.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 12-(ii)

The object compound (I-14) or a salt thereof can be prepared by reacting the compound (XII) or a salt thereof with the compound (XIII) or a salt thereof.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 13

The object compound (I-15) or a salt thereof can be prepared by subjecting the compound (I-11) or a salt thereof to reduction.

The reduction may include, for example, reduction with an alkali metal borohydride (e.g. sodium borohydride.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out under cooling to heating.

Process 14

The object compound (I-16) or a salt thereof can be prepared by reacting the compound (XIV) or a salt thereof with the compound (XV) or a salt thereof.

This reaction can be carried out in substantially the same manner as Process 5, and therefore the reaction mode and reaction conditions [e.g. solvent, reaction temperature. ] of this reaction are to be referred to those as explained in Process 5.

Process 15

The object compound (I-17) or a salt thereof can be prepared by subjecting the compound (I-12) or a salt thereof to hydrolysis.

This reaction is usually carried out in a conventional solvent such as a mixture of water and alcohol [e.g. methanol. ] or any other solvent which does not adversely influence the reaction.

The reaction temperature is not critical, and the reaction is usually carried out under cooling to heating.

Process 16

The object compound (I-18) or a salt thereof can be prepared by subjecting the compound (I-17) or a salt thereof to amidation.

This reaction is usually carried out in the presence of ammonia gas.

This reaction is usually carried out in a conventional solvent such as alcohol [e.g. methanol, ethanol. ], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction.

The reaction temperature is not critical, and the reaction is usually carried out at ambient temperature or under warming or heating.

Process 17

The object compound (I-20) or a salt thereof can be prepared by reacting the compound (I-19) or a salt thereof with the compound (XVII).

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 18

The object compound (I-21) or a salt thereof can be prepared by reacting the compound (XVIII) or a salt thereof with hydrazine.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 19

The object compound (I-22) or a salt thereof can be prepared by reacting the compound (I-21) or a salt thereof with S-($C_1$-$C_6$)alkylisothiourea or a salt thereof.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 20

The object compound (I-23) or a salt thereof can be prepared by subjecting the compound (I-22) or a salt thereof to ring closure.

This reaction is usually carried out in the presence of ammonium hydroxide

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 21

The object compound (I-24) or a salt thereof can be prepared by reacting the compound (I-2) or a salt thereof with the compound (XIX) or a salt thereof.

The reaction is usually carried out in a conventional solvent such as water, acetone, dioxane, acetonitrile, chloroform, dichloromethane, ethylene chloride, tetrahydrofuran, ethyl acetate, N,N-dimethylformamide, N,N-dimethylacetamide, pyridine or any other organic solvent which does not adversely influence the reaction. These conventional solvents may also be used in a mixture with water.

The reaction temperature is not critical, and the reaction is usually carried out under cooling to warming.

The reaction may also be carried out in the presence of an inorganic or organic base such as an alkali metal bicarbonate, tri($C_1$-$C_6$)alkylamine (e.g. triethylamine. ), pyridine, N-($C_1$-$C_6$)alkylmorpholine, N,N-di($C_1$-$C_6$)alkylbenzylamine.

Process 22

The object compound (I-26) or a salt thereof can be prepared by oxidizing the compound (I-25) or a salt thereof.

The oxidizing agent to be used in this reaction may include an inorganic peracid or a salt thereof (e.g. periodic acid, persulfuric acid, or sodium or potassium salt thereof. ), an organic peracid or a salt thereof (e.g. perbenzoic acid, m-chloroperbenzoic acid, performic acid, peracetic acid, chloroperacetic acid, trifluoroperacetic acid, or sodium or potassium salt thereof. ), ozone, hydrogen peroxide, urea-hydrogen peroxide, N-halosuccinimide (e.g. N-bromosuccinimide, N-chlorosuccinimide. ), hypochlorite compound (e.g. tert-butyl hypochlorite. ), permanganate (e.g. potassium permanganate. ), or any other conventional oxidizing agent which can oxidide a sulfide group to a sulfoxide group.

The present reaction can also be carried out in the presence of a compound comprising Group Vb or VIb metal in the Periodic Table of elements, for example, tungstic acid, molybdic acid, vanadic acid, or an alkali or an alkaline earth metal salt thereof.

The present oxidation reaction is usually carried out in a conventional solvent which does not adversely influence the reaction such as water, acetic acid, chloroform, methylene chloride, acetone, methanol, ethanol, tetrahydrofuran, N,N-dimethylformamide or a mixture thereof.

The reaction temperature is not critical and the reaction is preferably carried out under cooling to at ambient temperature.

Process 23

The object compound (I-27) or a salt thereof can be prepared by reacting the compound (I-8) or a salt thereof with the compound (XX) or a salt thereof.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Process 24

The object compound (I-28) or a salt thereof can be prepared by reacting the compound (I-27) or a salt thereof with the compound (XXI) or a salt thereof.

This reaction can be carried out in substantially the same manner as Process 5, and therefore the reaction mode and reaction conditions [e.g. solvent, reaction temperature. ] of this reaction are to be referred to those as explained in Process 5.

Process 25

The object compound (I-30) or a salt thereof can be prepared by subjecting the compound (I-29) or a salt thereof to elimination reaction of the hydroxy-protective group.

This reaction can be carried out in substantially the same manner as Process 2, and therefore the reaction mode and reaction conditions [e.g. solvent, reaction temperature. ] of this reaction are to be referred to those as explained in Process 2.

Process 26

The object compound (I-31) or a salt thereof can be prepared by reacting the compound (I-2) or a salt thereof with the compound (XXII) or a salt thereof.

This reaction is usually carried out in a conventional solvent which does not adversely influence the reaction such as alcohol [e.g. methanol, ethanol, propanol. ] tetrahydrofuran, dioxane, dimethyl sulfoxide, N,N-dimethylformamide or a mixture thereof.

The reaction temperature is not critical, and the reaction is usually carried out at ambient temperature or under warming or heating.

The reaction may also be carried out in the presence of an inorganic or organic base such as tri($C_1$-$C_6$)alkylamine (e.g. triethylamine. ).

Process 27

The object compound (I-32) or a salt thereof can be prepared by subjecting the compound (I-13) or a salt thereof to hydrolysis.

The hydrolysis is preferably carried out in the presence of a base or an acid.

This reaction is usually carried out in a conventional solvent which does not adversely influence the reaction such as alcohol [e.g. methanol, ethanol, propanol. ], tetrahydrofuran, dioxane, dimethyl sulfoxide, N,N-dimethylformamide or a mixture thereof.

The reaction temperature is not critical, and the reaction is usually carried out at ambient temperature or under warming or heating.

Among the starting compounds, some of them are new and such compounds can be prepared by the methods of Preparation mentioned below and by any process known in the art for preparing structurally analogous compounds thereto.

The compounds obtained by the above Processes 1 to 27 can be isolated and purified by a conventional method such as pulverization, recrystallization, column chromatography, reprecipitation.

It is to be noted that each of the object compound (I) may include one or more stereoisomer such as optical isomer(s) and geometrical isomer(s) due to asymmetric carbon atom(s) and double bond(s) and all such isomers and mixture thereof are included within the scope of this invention.

Furthermore, with regard to the compound (I), it is to be noted that the following formula(A) is well known to lie to tautomeric relation with the following formula (B), and accordingly, it is to be understood that both of the isomers are substantially the same.

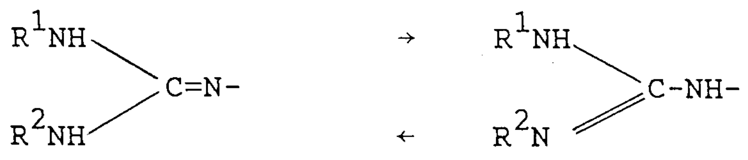

(A) (B)

Accordingly, the both of the tautomeric forms are clearly included within the scope of the present invention. In the present specification, the object and starting compounds including the group of such tautomeric isomers are represented by using one of the expressions.

The new furylthiazole derivatives (I) and pharmaceutically acceptable salts thereof possess antiulcer activity and $H_2$-receptor antagonism, and are useful for a therapeutic treatment of gastritis, ulcer (e.g. gastric ulcer, duodenal ulcer, anastomotic ulcer. ), Zollinger-Ellison syndrome, reflux esophagitis, upper gastrointestinal bleeding.

For therapeutic purpose, the compound (I) and a pharmaceutically acceptable salt thereof of the present invention can be used in a form of pharmaceutical preparation containing one of said compounds, as an active ingredient, inadmixture with a pharmaceutically acceptable carrier such as an organic or inorganic solid or liquid excipient suitable for oral or parenteral administration. The pharmaceutical preparations may be capsules, tablets, dragees, granules, solution, suspension, emulsion.

If desired, there may be included in these preparations, auxiliary substances, stabilizing agents, wetting or emulsifying agents, buffers and other commonly used additives.

While the dosage of the compound (I) will vary depending upon the age and condition of the patient, an average single dose of about 0.1 mg, 1 mg, 10 mg, 50 mg, 100 mg, 250 mg, 500 mg and 1000 mg of the compound (I) may be effective for treating ulcer. In general, amounts between 0.1 mg/body and about 1,000 mg/body may be administered per day.

In order to illustrate the usefulness of the object compound (I), the pharmacological test data of some representative compounds of the compound (I) are shown in the following.

Test Compounds

(a) 4-(5-Acetylaminomethylfuran-2-yl)-2-(diaminomethyleneamino)thiazole
(b) 4-[5-(2-Cyano-3-methylguanidino)methylfuran-2-yl]-2-(diaminomethyleneamino)thiazole
(c) 2-(Diaminomethyleneamino-4-[5-(3-methylureido)methylfuran-2-yl]thiazole
(d) 4-[5-(2-Amino-2-aminosulfonyliminoethyl)furan-2-yl]-2-(diaminomethyleneamino)thiazole

Test A (Gastric secretion in Heidenhain pouch dogs):

Test Method

Beagle dogs, weighing about 8-13 kg, were used for the study on gastric secretion. The animals were surgically provided with a vagally denervated Heidenhain pouch. One month or more later, the dogs were fasted overnight. Gastric secretion was stimulated by an intravenous infusion of tetragastrin (10 µg/kg/hr). Gastric samples were collected at 15 min intervals. After its volume was almost constant, test compound (3.2 mg/kg) suspended on 0.1% methyl cellulose solution was administered orally. Acid concentration was determined by titrating an aliquot to pH 7.0 with 0.1N sodium hydroxide solution using automatic titration (Hiranuma RAT-11 Type). Total acid output was calculated by multiplying total volume of gastric samples by acid concentration, and percentage change of total acid output was calculated by comparing with predosing value of test compound.

Test Result

| Test Compound | Inhibition (%) |
|---|---|
| (a) | 100 |

Test B ($H_2$-receptor antagonism in isolated guinea-pig atrium):

Test Method

The atrial strip isolated from guinea-pig was suspended under an initial tension 0.3 to 0.6 g in an organ bath containing Tyrode solution at 30°C, aerated 95%$O_2$-5%$CO_2$ gas. The beating rate and amplitude of contraction of the atrium were recorded by means of a transducer and a polygraph. Histamine (1 x $10^{-6}$ g/ml) was added to the bathing fluid and the increase in beating rate after dosing was measured. Addition of test compounds (1 x $10^{-6}$ g/ml) was made 30 minutes after washing out histamine. Inhibitory effect of test compound was calculated by comparing histamine-induced increases in beating rate before and 30 minutes after dosing with the test compounds.

Test Results

| Test Compound | $H_2$ Antagonism (%) |
|---|---|
| (b) | 90.7 |
| (c) | 89.1 |

Test C (Inhibition of stress ulcer):

Test Method

Five male Sprague-Dawley rats, aged 7 weeks and weighing about 200 g were used per group for the study on stress ulcer after the fast for 24 hours. Each animal was immobilized in a restrain cage and immersed to a level of the xiphoid in a water bath kept 22 °C. Each of the test compounds (32 mg/kg) suspended in 0.1% methylcellulose solution was administered orally just before the immobilization. Seven hours later, the animals were sacrificed and their stomachs were removed. The stomach was then fixed with 2% formalin. The area of ulcers was measured for each animal, and percentage of inhibition was calculated by comparing the mean area of ulcers ($mm^2$) in the test animals with that in the control animals.

Test Result

| Test Compound | Inhibition (%) |
|---|---|
| (d) | 86.6 |

Test D (Gastric secretion from lumen perfused stomach in anesthetized rats):

Test Method

Male Sprague-Dawley rats weighing about 250 g were used. Rats were deprived of food but allowed free access to water for 24 hours. The animals were anesthetized with 1.25 g/kg urethane intraperitoneally. The abdomen was opened and the gastric lumen was perfused with saline throughout the experiment. The perfusate was titrated by an autotitrator with 25 mM sodium hydroxide as a titrant. Gastric secretion was stimulated by intravenous infusion with histamine (3 mg/kg/hr). After reaching plateau, test compound (1 mg/kg) was given intravenously. Drug effect was expressed as maximal inhibition by acid output.

Test Result

| Test Compound | Inhibition (%) |
|---|---|
| (d) | 99 |

The following Preparations and Examples are given for the purpose of illustrating the present invention in more detail.

Preparation 1

Aluminum chloride (34.1 g) was added to a mixture of 2-furylacetonitrile (11.4 g) and chloroacetyl chloride (12.7 ml) in dichloromethane (170 ml) under ice-cooling and the mixture was stirred for 10 minutes at the same temperature. After the ice bath was removed, the mixture was stirred for 75 minutes.

The reaction mixture was added to a mixture of ice-water (300 ml) and dichloromethane (200 ml) and the separated dichloromethane layer was washed with water.

To this solution was added a water and the mixture was adjusted to pH 8 with 20% aqueous potassium carbonate.

The separated organic layer was washed with water and dried over magnesium sulfate. The solvent was removed by concentration to give [5-(chloroacetyl)furan-2-yl]acetonitrile (16.36 g).

IR (film) : 3120, 2940, 2260, 2210, 1680, 1590, 1510 $cm^{-1}$

NMR (DMSO-$d_6$, $\delta$) : 4.33 (2H, s), 4.83 (2H, s), 6.64 (1H, d, J=4Hz), 7.55 (1H, d, J=4Hz)

Preparation 2

A solution of methyl 3-(furan-2-yl)propanate (4.0 g) in 40% methylamine methanol solution (50 ml) was stirred for 5 hours at room temperature. The solvent was removed under reduced pressure to afford N-methyl-3-(furan-2-yl)proponamide (4.0 g).
IR (Film) : 3300, 3100, 2940, 1660, 1610 $cm^{-1}$
NMR ($CDCl_3$, $\delta$) : 2.48 (2H, t, J = 7.5Hz), 2.76 (3H, d, J = 5Hz), 2.98 (2H, t, J = 7.5Hz), 5.72 (1H, br), 5.99 (1H, dd, J = 3.8 and 0.5Hz), 6.26 (1H, dd, J = 2.0 and 3.8Hz), 7.27 (1H, dd, J = 2.0 and 0.5Hz)

Preparation 3

A solution of methyl isocyanate (34.0 g) in methanol (30 ml) was added dropwise to a solution of furan-2-ylmethylamine (57.9 g) in methanol (300 ml) at 5 to 15 °C. After being stirred at room temperature for three hours, the solvent was evaporated in vacuo. The residue was treated with diisopropyl ether (400 ml) to give N-(furan-2-ylmethyl)-N′-methylurea (81.9 g).
mp : 80 to 81 °C
IR (Nujol) : 3350, 3320, 3150, 3125, 1610 $cm^{-1}$
NMR (DMSO-$d_6$, $\delta$) : 2.60 (3H, d, J = 5Hz), 4.23 (2H, d, J = 5Hz), 5.85 (1H, q, J = 5Hz), 61.8 (1H, d, J = 3Hz), 6.35 (1H, dd, J = 2Hz and 3Hz), 6.38 (1H, t, J = 5Hz) and 7.55 (1H, d, J = 2Hz)

Preparation 4

The following compound was obtained according to a similar manner to that of Example 19.
N-(Furan-2-ylmethyl)urea.
mp : 88-92 °C
IR (Nujol) : 3450, 3300, 3190, 1660, 1610, 1545 $cm^{-1}$
NMR (DMSO-$d_6$, $\delta$) : 4.16 (2H, d, J = 6Hz), 5.55 (2H, s), 6.15-6.22 (1H, m), 6.32-6.42 (2H, m), 7.55 (1H, m)

Preparation 5

A mixture of N-(furan-2-ylmethyl)urea (83.0 g), acetic anhydride (580 ml) and phosphoric acid (10 ml) was stirred for 15 minutes at 80 °C, and then the mixture was evaporated in vacuo. To the residue was added a mixture of ethyl acetate, tetrahydrofuran and water and the mixture was adjusted to pH 7.5 with potassium carbonate. The separated organic layer was washed with brine and the mixture was dried over magnesium sulfate. The solvent was removed by concentration and triturated with a mixture of ethyl acetate and ether. The precipitate was collected by filtration to give 1-acetyl-3-(5-acetylfuran-2-ylmethyl)urea (32.17 g).
IR (Nujol) : 3310, 3230, 3110, 1670 $cm^{-1}$
NMR (DMSO-$d_6$, $\delta$) : 2.02 (3H, s), 2.37 (3H, s), 4.45 (2H, d, J = 6Hz), 6.46 (1H, d, J = 4Hz), 7.39 (1H, d, J = 4Hz), 8.74 (1H, t, J = 6Hz)

The above filtrate was evaporated in vacuo. The residue was purified by column chromatography on silica gel eluting with a mixture of chloroform and methanol (39:1, V/V). The eluted fractions containing the desired product were collected and evaporated in vacuo to give 1-(5-acetylfuran-2-ylmethyl)-1,3-diacetylurea (21.3 g).
IR (Nujol) : 1780, 1670 $cm^{-1}$
NMR (DMSO-$d_6$, $\delta$) : 2.28 (3H, s), 2.38 (3H, s), 2.40 (3H, s), 5.01 (2H, s), 6.60 (1H, d, J = 4Hz), 7.41 (1H, d, J = 4Hz), 11.34 (1H, s)

Preparation 6

A solution of bromine (0.23 ml) in dichloromethane (5 ml) was dropwise added to a mixture of 1-acetyl-3-(5-acetylfuran-2-ylmethyl)urea (1.0 g) in dichloromethane (20 ml) at ambient temperature for 20 minutes and the mixture was stirred at the same temperature for 1.5 hours. The solvent was removed by concentration and residue was triturated with isopropyl ether to give 1-acetyl-3-[5-(bromoacetyl)furan-2-ylmethyl]urea (1.27 g).
mp : 128-134 °C (dec.)
IR (Nujol) : 3290, 3110, 1670, 1550 $cm^{-1}$
NMR (DMSO-$d_6$, $\delta$) : 2.02 (3H, s), 4.47 (2H, d, J = 6Hz), 4.60 (2H, s), 6.53 (1H, d, J = 4Hz), 7.62 (1H, d,

J = 4Hz), 8.77 (1H, t, J = 6Hz), 10.46 (1H, s)

Preparation 7

To a mixture of furan-2-ylacetonitrile (60.0 g) in dry isopropyl alcohol (600 ml) was bubbled with dry hydrogen chloride for 5 hours under ice-cooling and the mixture was stirred for 2 hours at the same temperature. The solvent and excess hydrogen chloride was removed by concentration in vacuo. To the solution of resulting residue in dry isopropyl alcohol (400 ml) was bubbled with an ammonia gas for 1.5 hours under ice-cooling and the mixture was stirred for 1.5 hours at the same temperature. Evaporation of the solvent gave a residue, which was purified by column chromatography on alumina eluting with a methanol. The eluted fractions containing the desired product were collected and evaporated in vacuo to give 2-(furan-2-yl)acetamidine hydrochloride (56.0 g).
IR (film) : 3400-3000 (br), 1690, 1600, 1500 $cm^{-1}$
NMR (DMSO-$d_6$, $\delta$) : 3.91 (2H, s), 6.42 (2H, s), 7.63 (1H, s), 9.08 (2H, br s), 9.38 (2H, br s)

Preparation 8

The mixture of 2-(furan-2-yl)acetamidine hydrochloride (30.0 g), methyl 2-acetylheptanoate (34.8 g) and sodium methoxide (10.1 g) in methanol (300 ml) was stirred for 4.5 hours at ambient temperature, and then the solvent was removed by concentration. To the residue was added a mixture of ethyl acetate and water, and the mixture was adjusted to pH 9 with 6N-hydrochloric acid. The separated organic layer was washed with brine and dried over magnesium sulfate. Evaporation of the solvent gave a residue, which was purified by column chromatography on silica gel eluting with a mixture of chloroform and methanol (19:1, V/V). The eluted fractions containing the desired product were collected and evaporated in vacuo to give 2-(furan-2-ylmethyl)-6-methyl-5-n-pentyl-4(1H)pyrimidinone (18.18 g).
mp : 99-100 ° C
IR (Nujol) : 1660, 1610 $cm^{-1}$
NMR (DMSO-$d_6$, $\delta$) : 0.86 (3H, t, J = 6Hz), 1.20-1.48 (6H, m), 2.17 (3H, s), 2.36 (2H, t, J = 7Hz), 3.86 (2H, s), 6.26 (1H, d, J = 3Hz), 6.39 (1H, m), 7.56 (1H, m), 12.37 (1H, s)

Preparation 9

The following compounds were obtained according to a similar manner to that of Preparation 1.
(1) Methyl N-[5-(chloroacetyl)furan-2-ylmethyl]carbamate.
mp : 86 to 91 ° C
IR (Nujol) : 3330, 1600 (br), 1510 $cm^{-1}$
NMR (DMSO-$d_6$, $\delta$) : 3.59 (3H, s), 4.30 (2H, d, J = 6Hz), 4.85 (2H, s), 6.56 (1H, d, J = 3Hz), 7.59 (1H, d, J = 3Hz)
(2) N-Methyl-3-[5-(chloroacetyl)furan-2-yl]propanamide.
mp : 110 to 115 ° C
IR (Nujol) : 3300, 1640 $cm^{-1}$
NMR ($CDCl_3$, $\delta$) : 2.79 (3H, d, J = 4.7Hz), 4.47 (2H, s), 5.75 (1H, br), 6.26 (1H, d, J = 3.6Hz), 7.23 (1H, d, J = 3.6Hz)
(3) N-[5-(Chloroacetyl)furan-2-ylmethyl]-N′-methylurea.
mp : 125 to 126 ° C
IR (Nujol) : 3370, 3330, 1680, 1635 $cm^{-1}$
NMR (DMSO-$d_6$, $\delta$) : 2.55 (3H, d, J = 4.5Hz), 4.27 (2H, d, J = 6Hz), 4.84 (2H, s), 5.92 (1H, q, J = 4.5Hz), 6.45 (1H, d, J = 3Hz), 6.50 (1H, t, J = 6Hz) and 7.55 (1H, d, J = 3Hz)
(4) 2-[5-(Chloroacetyl)furan-2-ylmethyl]-6-methyl-5-n-pentyl-4(1H)-pyrimidinone.
mp : 136-139 ° C
IR (Nujol) : 1685, 1645, 1610 $cm^{-1}$
NMR (DMSO-$d_6$, $\delta$) : 0.80-0.94 (3H, m), 1.15-1.50 (6H, m), 2.16 (3H, s), 2.27-2.42 (2H, m), 4.01 (2H, s), 4.85 (2H, s), 6.59 (1H, d, J = 4Hz), 7.59 (1H, d, J = 4Hz), 12.49 (1H, s)
(5) 2-Acetamidomethyl-5-chloroacetyl-3-methylfuran.
mp : 94-100 ° C
IR (Nujol) : 3340, 1690, 1650, 1550, 1520 $cm^{-1}$
NMR (DMSO-$d_6$, $\delta$) : 1.82 (3H, s), 2.04 (3H, s), 4.27 (2H, d, J = 6Hz), 4.80 (2H, s), 7.45 (1H, s), 8.43 (1H, t, J = 6Hz)

Preparation 10

The following compounds were obtained according to a similar manner to that of Example 1.

(1) 2-Amino-4-[5-(3-methylureidomethyl)furan-2-yl]thiazole.

mp : 196 ° C

IR (Nujol) : 3400, 3300, 1610, 1575, 1515 $cm^{-1}$

NMR ($DMSO-d_6$, δ) : 2.56 (3H, d, J=5Hz), 4.20 (2H, d, J=6Hz), 5.83 (1H, q, J=5Hz), 6.22 (1H, d, J=3Hz), 6.34 (1H, t, J=6Hz), 6.43 (1H, d, J=3Hz), 6.63 (1H, s), 7.11 (4H, s)

(2) 2-Amino-4-(5-cyanomethylfuran-2-yl)thiazole.

mp : 215-216 ° C

IR (Nujol) : 3390, 3310, 3150, 2270, 1640, 1530 $cm^{-1}$

NMR ($DMSO-d_6$, δ) : 4.21 (2H, s), 6.43 (1H, d, J=3Hz), 6.51 (1H, d, J=3Hz), 6.73 (1H, s), 7.15 (2H, s)

Preparation 11

Benzoyl chloride (2.67 ml) was dropped to a refluxing solution of ammonium thiocyanate (1.92 g) in acetone (50 ml) and the mixture was refluxed for 15 minutes. 4-(5-Acetylaminomethylfuran-2-yl)-2-aminothiazole (5.20 g) was added portionwise to the refluxing mixture. After the mixture was refluxed for further two hours, the solvent was evaporated in vacuo and the residue was mixed with water and ethyl acetate. The resulting precipitate was collected by filtration and washed with ethyl acetate to afford 4-(5-acetylaminomethylfuran-2-yl)-2-(3-benzoylthioureido)thiazole (5.50 g).

mp : 213 to 214 ° C

IR (Nujol) : 3270, 1675, 1630 $cm^{-1}$

NMR ($DMSO-d_6$, δ) : 1.90 (3H, s), 4.37 (2H, d, J=6Hz), 6.40 (1H, d, J=3Hz), 6.80 (1H, d, J=3Hz), 7.42 (1H, s), 7.58-8.17 (5H, m), 8.40 (1H, d, J=6Hz), 12.00 (1H, s) and 14.08 (1H, s)

Preparation 12

The following compounds were obtained according to a similar manner to that of Preparation 11.

(1) 2-(3-Benzoylthioureido)-4-(5-cyanomethylfuran-2-yl)thiazole.

mp : 207-210 ° C (dec.)

IR (Nujol) : 3280, 2270, 1660, 1550, 1535 $cm^{-1}$

NMR ($DMSO-d_6$, δ) : 4.27 (2H, s), 6.52 (1H, d, J=3Hz), 6.81 (1H, d, J=3Hz), 7.40 (1H, s), 7.51-7.71 (3H, m), 8.00-8.04 (2H, m), 13.06 (1H, br s)

(2) 2-(3-Benzoylthioureido)-4-[5-(3-methylureidomethyl)furan-2-yl)thiazole.

mp : 213-214 ° C

IR (Nujol) : 3340, 1675, 1630, 1590, 1545, 1520 $cm^{-1}$

NMR ($DMSO-d_6$, δ) : 2.58 (3H, d, J=5Hz), 4.26 (2H, d, J=6Hz), 5.89 (1H, q, J=5Hz), 6.31 (1H, d, J=3Hz), 6.44 (1H, t, J=6Hz), 6.74 (1H, d, J=3Hz), 7.37 (1H, s), 7.50-7.65 (2H, m), 7.65-7.78 (1H, m), 7.98-8.10 (2H, m)

Preparation 13

A solution of sodium hydroxide (0.55 g) in water (5 ml) was added to a suspension of 4-(5-acetylaminomethylfuran-2-yl)-2-(3-benzoylthioureido)thiazole (5.40 g) in methanol (50 ml) and the mixture was stirred at 60 ° C for two hours. Following evaporation in vacuo, the residue was mixed with water (50 ml) and ethyl acetate (15 ml) and stirred for several minutes. The resulting precipitate was collected by filtration and washed with water and ethyl acetate to afford 4-(5-acetylaminomethylfuran-2-yl)-2-thioureidothiazole (2.95 g).

mp : 231 to 232 ° C

IR (Nujol) : 3310, 3270, 3190, 3140, 1635, 1620 $cm^{-1}$

NMR ($DMSO-d_6$, δ) : 1.86 (3H, s), 4.27 (2H, d, J=6Hz), 6.30 (1H, d, J=3Hz), 6.61 (1H, d, J=3Hz), 7.10 (1H, s), 8.28 (1H, t, J=6Hz), 8.33 (2H, br s) and 11.81 (1H, s)

Preparation 14

The following compounds were obtained according to a similar manner to that of Preparation 13.

(1) 4-(5-Cyanomethylfuran-2-yl)-2-thioureidothiazole.

mp : 216-220 °C

IR (Nujol) : 3290, 3180, 3130, 2250, 1620, 1560, 1520 $cm^{-1}$

NMR (DMSO-$d_6$, δ) : 4.25 (2H, s), 6.50 (1H, d, J=3Hz), 6.72 (1H, d, J=3Hz), 7.23 (1H, s), 8.10 (1H, br s), 8.74 (1H, br s), 11.84 (1H, s)

(2) 4-[5-(3-Methylureidomethyl)furan-2-yl]-2-thioureidothiazole.

mp : 229 °C

IR (Nujol) : 3305, 1615, 1560 $cm^{-1}$

NMR (DMSO-$d_6$, δ) : 2.57 (3H, d, J=5Hz), 4.22 (2H, d, J=6Hz), 5.83 (1H, q, J=5Hz), 6.28 (1H, d, J=3Hz), 6.36 (1H, t, J=6Hz), 6.63 (1H, d, J=3Hz), 7.13 (1H, s)

Preparation 15

A mixture of 4-(5-acetylaminomethylfuran-2-yl)-2-(3-benzoylthioureido)thiazole (39.5 g) in ethanol (400 ml) and conc. hydrochloric acid (87.1 ml) was heated under reflux for 15 hours and then the mixture was evaporated in vacuo. To the residue was added to a mixture of ethyl acetate and water and the mixture was adjusted to pH 9.5 with a potassium carbonate. The isolated precipitate was collected by filtration to give 4-(5-aminomethylfuran-2-yl)-2-thioureidothiazole (10.1 g).

mp : 154-158 °C

IR (Nujol) : 3300, 3190, 3140, 1620, 1580, 1525 $cm^{-1}$

NMR (DMSO-$d_6$, δ) : 3.74 (2H, s), 6.31 (1H, d, J=3Hz), 6.61 (1H, d, J= 3Hz), 7.11 (1H, s)

Preparation 16

A solution of potassium cyanate (9.6 g) in water (50 ml) was added to a mixture of 4-(5-aminomethylfuran-2-yl)-2-thioureidothiazole (10.0 g) in N,N-dimethylformamide (80 ml) and 1N-hydrochloric acid (78.6 ml), and the mixture was stirred for 16 hours at ambient temperature. To the reaction mixture was added a water (100 ml) and isolated precipitate was collected by filtration to give 2-thioureido-4-(5-ureidomethylfuran-2-yl)thiazole (10.74 g).

mp : 227-229 °C

IR (Nujol) : 3480, 3300, 1650, 1620, 1600, 1560, 1525 $cm^{-1}$

NMR (DMSO-$d_6$, δ) : 4.22 (2H, d, J=6Hz), 5.58 (2H, s), 6.29 (1H, d, J=3Hz), 6.41 (1H, t, J=6Hz), 6.66 (1H, d, J=3Hz), 7.13 (1H, s)

Preparation 17

A mixture of 4-(5-cyanomethylfuran-2-yl)-2-thioureidothiazole (11.4 g) and methyl iodide (2.7 ml) in methanol (110 ml) and tetrahydrofuran (60 ml) was refluxed for 1.5 hours under stirring. Evaporation of the solvent gave a residue, which was triturated with ethyl acetate to give 2-[(amino)(methylthio)-methyleneamino]-4-(5-cyanomethylfuran-2-yl)thiazole hydriodide (14.50 g).

mp : 116-118 °C

IR (Nujol) : 3390, 3210, 2280, 1650, 1605, 1570 $cm^{-1}$

NMR (DMSO-$d_6$, δ) : 2.70 (3H, s), 4.30 (2H, s), 6.55 (1H, d, J=3Hz), 7.01 (1H, d, J=3Hz), 7.47 (1H, s)

Preparation 18

The following compounds were obtained according to a similar manner to that of Preparation 17.

(1) 2-[(Amino)(methylthio)methyleneamino]-4-[5-(3-methylureido)methylfuran-2-yl)thiazole hydriodide.

mp : 164-167 °C

IR (Nujol) : 3320, 3100, 1620, 1590, 1570 $cm^{-1}$

NMR (DMSO-$d_6$, δ) : 2.58 (3H, s), 2.64 (3H, s), 4.25 (2H, s), 6.32 (1H, d, J=3Hz), 6.90 (1H, d, J=3Hz), 7.33 (1H, s), 9.79 (1H, br s)

(2) 2-[(Amino)(methylthio)methyleneamino]-4-(5-ureidomethylfuran-2-yl)thiazole hydriodide.

mp : 161-165 °C

IR (Nujol) : 3430, 3180-3310 (br), 1625, 1570, 1530 $cm^{-1}$

NMR (DMSO-$d_6$, δ) : 2.61 (3H, s), 4.23 (2H, s), 6.33 (1H, d, J=3Hz), 6.88 (1H, d, J=3Hz), 7.32 (1H, s), 9.50-10.00 (1H, br s)

Preparation 19

A solution of (furan-2-yl)glyoxal (5.68 g), acetaldehyde (3.10 ml) and concentrated aqueous ammonia (30.9 ml) in ethanol (60 ml) was stirred for two hours at room temperature. The solvent was evaporated in vacuo and the residue was chromatographed on alumina by eluting with a mixture of chloroform and methanol (50:1, V/V) to give 4-(furan-2-yl)-2-methylimidazole (2.90 g).
mp : 109 to 114°C
IR (Nujol) : 3240, 1605 $cm^{-1}$
NMR (DMSO-$d_6$, δ) : 2.29 (3H, s), 6.44 (1H, dd, J=1Hz and 3Hz), 6.48 (1H, dd, J=2Hz and 3Hz), 7.18 (1H, s) and 7.56 (1H, dd, J=1Hz and 2Hz)

Preparation 20

The following compound was obtained according to a similar manner to that of Preparation 1.
4-[5-Chloroacetylfuran-2-yl]-2-methylimidazole.
mp : >300°C
IR (Nujol) : 1670 $cm^{-1}$
NMR (DMSO-$d_6$, δ) : 2.32 (3H, s), 4.85 (2H, s), 6.75 (1H, d, J=3.5Hz), 7.58 (1H, s), 7.68 (1H, d, J=3.5Hz) and 14.65 (1H, br s)

Example 1

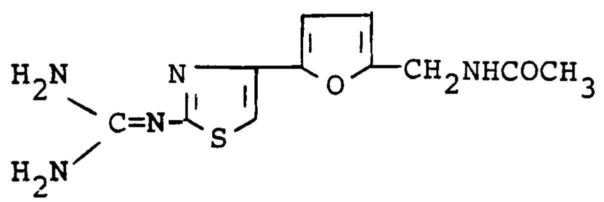

A solution of 5-acetylaminomethyl-2-chloroacetylfuran (39.9 g) and diaminomethylenethiourea (21.9 g) in ethanol (400 ml) was refluxed for two hours with stirring. The solvent was evaporated in vacuo and the residue was dissolved in water (300 ml). The solution was basified with an aqueous potassium carbonate. The resulting precipitate was collected by filtration, washed with water and recrystallized from a mixture of methanol, tetrahydrofuran and diisopropyl ether to afford 4-(5-acetylaminomethylfuran-2-yl)-2-diaminomethyleneamino)thiazole (24.1 g).
mp : 230 to 231°C
IR (Nujol) : 3430, 3200, 3110, 3060, 1650 $cm^{-1}$
NMR (DMSO-$d_6$, δ) : 1.88 (3H, s), 4.26 (2H, d, J=6Hz), 6.25 (1H, d, J=3Hz), 6.56 (1H, d, J=3Hz), 6.73 (1H, s), 6.83 (4H, s), 8.24 (1H, t, J=6Hz)

| Anal. Calcd. for $C_{11}H_{13}N_5O_2S$ : | | | |
|---|---|---|---|
| | C 47.30, | H 4.69, | N 25.07 |
| Found | C 47.31, | H 4.71, | N 24.65 |

Example 2

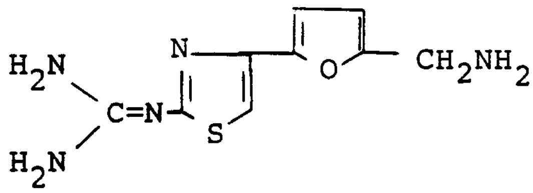

A solution of 4-(5-acetylaminomethylfuran-2-yl)-2-(diaminomethyleneamino)thiazole (4.76 g) in 1N-hydrochloric acid (51.1 ml) was refluxed for 8 hours with stirring. The solution was made basic to pH 10 with an aqueous potassium carbonate. The resulting precipitate was collected by filtration and washed with water to afford 4-(5-aminomethylfuran-2-yl)-2-(diaminomethyleneamino)thiazole (4.03 g).
mp : 160 to 163 °C
IR (Nujol) : 3300 (broad), 1710, 1630 $cm^{-1}$
NMR (DMSO-$d_6$, $\delta$) : 4.17 (2H, br s), 4.35 (2H, br s), 6.30 (1H, d, J = 3Hz), 6.62 (1H, d, J = 3Hz), 6.78 (1H, s), 7.00 (4H, br s)

Example 3

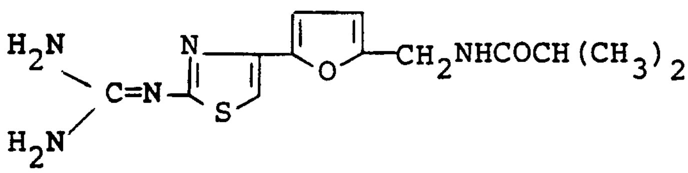

Isobutyryl chloride (0.4 ml) was added to a mixture of 4-(5-aminomethylfuran-2-yl)-2-(diaminomethyleneamino)thiazole (0.8 g) and triethylamine (1.3 ml) in dichloromethane (16 ml) under ice-cooling and the mixture was stirred for 2 hours at the same temperature. The solvent was removed by concentration in vacuo and the residue was dissolved in a mixture of tetrahydrofuran, ethyl acetate and water. The mixture was adjusted to pH 10 with 4N sodium hydroxide under solution of aqueous layer in sodium chloride.

The separated organic layer was washed with a brine and dried over magnesium sulfate. Evaporation of a solvent gave a residue, which was purified by column chromatography on silica gel eluting with a mixture of chloroform and methanol (9:1 to 4:1 V/V).

The eluted fraction containing the desired product were collected and evaporated to give 2-(diaminomethyleneamino)-4-(5-isobutyrylaminomethylfuran-2-yl)thiazole (0.14 g).
mp : 211-213 °C
IR (Nujol) : 3390, 3270, 3180, 1660, 1645, 1610, 1545 $cm^{-1}$
NMR (DMSO-$d_6$, $\delta$) : 1.05 (6H, d, J = 7Hz), 2.15-2.61 (1H, m), 4.26 (2H, d, J = 5Hz), 6.22 (1H, d, J = 3Hz), 6.56 (1H, d, J = 3Hz), 6.72 (1H, s), 6.86 (4H, s), 8.15 (1H, t, J = 5Hz)

Example 4

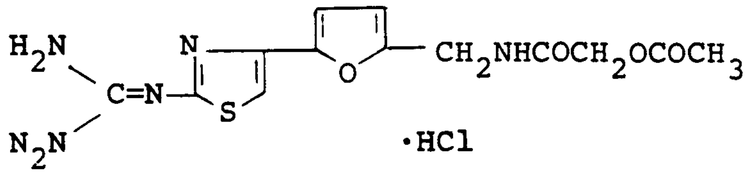

Acetoxyacetyl chloride (0.5 g) was added to a mixture of 4-(5-aminomethylfuran-2-yl)-2-(diaminomethyleneamino)thiazole (0.8 g) and triethylamine (1.3 ml) in dichloromethane (16 ml) under ice-cooling and the mixture was stirred for 20 hours at ambient temperature. Evaporation of the solvent gave a residue, which was purified by column chromatography on silica gel eluting with a mixture of chloroform and methanol (9:1, V/V).

The eluted fractions containing the desired product were collected and evaporated to give 4-(5-acetoxyacetylaminomethylfuran-2-yl)-2-(diaminomethyleneamino)thiazole hydrochloride (0.41 g).

mp : 250-253 ° C (dec.)

IR (Nujol) : 3300, 1750, 1685, 1655, 1590 $cm^{-1}$

NMR (DMSO-$d_6$, $\delta$) : 2.14 (3H, s), 4.36 (2H, d, J = 5Hz), 4.53 (2H, s), 6.35 (1H, d, J = 3Hz), 6.93 (1H, d, J = 3Hz), 7.27 (1H, s), 8.24 (4H, s), 8.56 (1H, t, J = 5Hz)

Example 5

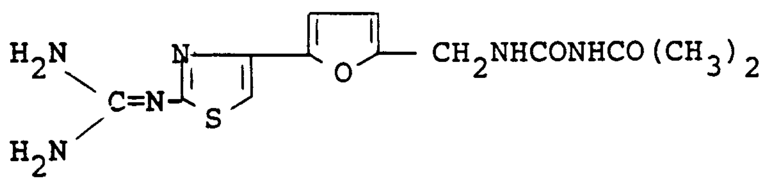

A mixture of 4-(5-aminomethylfuran-2-yl)-2-(diaminomethyleneamino)thiazole (0.8 g) and isopropylisocyanate (0.46 ml) in tetrahydrofuran (16 ml) and methanol (6.6 ml) was stirred for 8 hours at ambient temperature. Evaporation of a solvent gave a residue, which was purified by column chromatography on silica gel eluting with a mixture of chloroform and methanol (4:1, V/V).

The eluted fraction containing the desired product were collected and evaporated to give 2-(diaminomethyleneamino)-4-[5-(3-isopropylureido)methylfuran-2-yl]thiazole (0.41 g)

mp : 222-224 ° C (dec.)

IR (Nujol) : 3440, 3320, 1665, 1615, 1585, 1570, 1550 $cm^{-1}$

NMR (DMSO-$d_6$, $\delta$) : 1.06 (6H, d, J = 7Hz), 3.40-3.89 (1H, m), 4.20 (2H, d, J = 5Hz), 5.64 (1H, d, J = 8Hz), 6.08 (1H, t, J = 5Hz), 6.21 (1H, d, J = 4Hz), 6.56 (1H, d, J = 4Hz), 6.73 (1H, s), 6.86 (4H, s)

Example 6

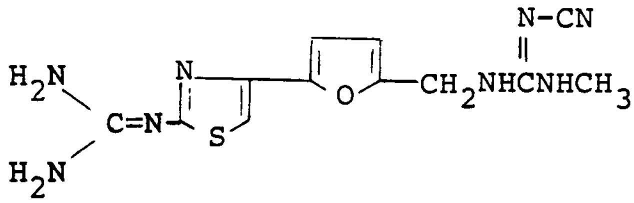

A solution of 4-(5-aminomethylfuran-2-yl)-2-(diaminomethyleneamino)thiazole (7.00 g) and dimethyl N-cyanodithioiminocarbonate (4.31 g) in N,N-dimethylformamide (70 ml) was stirred for 70 °C for two hours. 40% Aqueous methylamine by weight (14 ml) was added to the warmed solution and the mixture was stirred for an additional one hour at the same temperature. After the solvent was evaporated in vacuo, the residue was chromatographed on alumina, eluting with a mixture of chloroform and methanol (9:1, V/V), followed by recrystallization from a mixture of methanol, tetrahydrofuran and diisopropyl ether to afford 2-(diaminomethyleneamino)-4-[5-(2-cyano-3-methylguanidino)methylfuran-2-yl]thiazole (4.05 g).

mp : 231 to 232 °C

IR (Nujol) : 3440, 3310, 3200, 2160, 1630 $cm^{-1}$

NMR (DMSO-$d_6$, $\delta$) : 2.70 (3H, d, J=5Hz), 4.32 (2H, d, J=6Hz), 6.23 (1H, d, J=3Hz), 6.53 (1H, d, J=3Hz), 6.70 (1H, s), 6.81 (4H, s), 6.98 (1H, q, J=5Hz), 7.35 (1H, t, J=6Hz)

Example 7

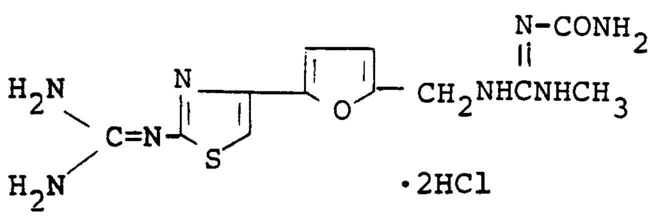

A mixture of 2-(diaminomethyleneamino)-4-[5-(2-cyano-3-methylguanidino)methylfuran-2-yl]thiazole (2.28 g) and 4M-dioxanic hydrogen chloride (10 ml) in methanol (20 ml) was stirred at ambient temperature for 19 hours and further at 50 °C for 5 hours. The resulting precipitate was collected by filtration, washed with methanol, and recrystallized from a mixture of methanol and water to afford 2-(diaminomethyleneamino)-4-[5-(2-carbamoyl-3-methylguanidino)methylfuran-2-yl]thiazole dihydrochloride (1.65 g).

mp : 241 to 242 °C

IR (Nujol) : 3440-3100, 1715, 1685, 1665 $cm^{-1}$

NMR (DMSO-$d_6$, $\delta$) : 2.93 (3H, d, J=5Hz), 4.73 (2H, d, J=6Hz), 6.60 (1H, d, J=3Hz), 6.97 (1H, d, J=3Hz), 7.33 (1H, s), 7.60 (2H, br s), 8.30 (4H, s), 9.08 (2H, br s), 10.43 (1H, br s), 12.63 (1H, br s)

| Anal. Calcd. for $C_{12}H_{16}N_8O_2S \cdot 2HCl$ : | | | | |
|---|---|---|---|---|
| | C 35.21, | H 4.43, | N 27.38, | Cl 17.22 |
| Found | C 35.06, | H 4.29, | N 27.25, | Cl 17.43 |

Example 8

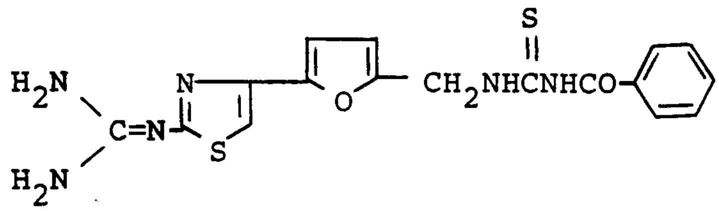

Benzoyl chloride (2.06 ml) was added dropwise to a refluxing suspension of ammonium isothiazonate (1.48 g) in acetone (40 ml). After the mixture was refluxed for 15 minutes, 4-(5-aminomethylfuran-2-yl)-2-(diaminomethyleneamino)thiazole (4.00 g) was added by portions and the mixture was refluxed further for 3.5 hours. The solvent was evaporated in vacuo and the residue was mixed with ethyl acetate (20 ml) and water (50 ml) and stirred for one hour. The resulting precipitate was collected, washed with water and then ethyl acetate, followed by drying under reduced pressure to afford 2-(diaminomethyleneamino)-4-[5-{3-(benzoyl)thioureido}methylfuran-2-yl]thiazole (3.31 g).
mp : 220 to 221 ° C (dec.)
IR (Nujol) : 3430-3120, 1720 (shoulder), 1670 $cm^{-1}$
NMR (DMSO-$d_6$, $\delta$) : 4.90 (2H, d, J = 5Hz), 6.47 (1H, d, J = 3Hz), 6.72 (1H, d, J = 3Hz), 6.93 (1H, s), 7.23 (4H, s), 7.36-7.97 (5H, m), 11.19 (1H, t, J = 5Hz), 11.43 (1H, br s)

Example 9

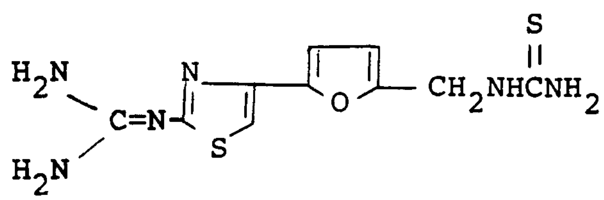

A suspension of 2-(diaminomethyleneamino)-4-[5-{3-(benzoyl)thioureido}methylfuran-2-yl]thiazole (3.20 g) and sodium hydroxide (0.32 g) in methanol (30 ml) was stirred for 60 ° C for one hour. The reaction mixture was cooled in an ice bath and the resulting precipitate was collected by filtration and washed with methanol to afford 2-(diaminomethyleneamino)-4-(5-thioureidomethylfuran-2-yl)thiazole (1.73 g).
mp : 177 to 179 ° C
IR (Nujol) : 3370, 3180, 1650, 1630 $cm^{-1}$
NMR (DMSO-$d_6$, $\delta$) : 4.56 (2H, d, J = 5Hz), 6.30 (1H, d, J = 3Hz), 6.59 (1H, d, J = 3Hz), 6.76 (1H, s), 6.84 (4H, s), 7.12 (2H, s), 7.93 (1H, t, J = 5Hz)

Example 10

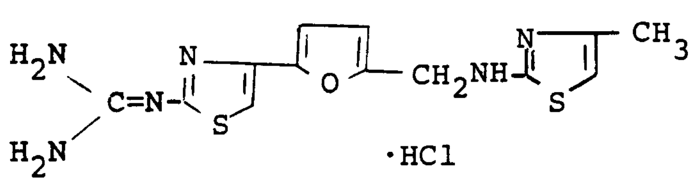

A suspension of 2-(diaminomethyleneamino)-4-(5-thioureidomethylfuran-2-yl)thiazole (0.70 g) and chloroacetone (0.22 g) in ethanol (15 ml) was refluxed for 4 hours with stirring. The solvent was evaporated in vacuo and the residue was recrystallized from a mixture of methanol and diisopropyl ether to afford 2-(diaminomethyleneamino)-4-[5-(4-methylthiazol-2-yl)aminomethylfuran-2-yl]thiazole (0.66 g).

mp : 193 to 194 ° C
IR (Nujol) : 3300, 3180, 3120, 1685 $cm^{-1}$
NMR (DMSO-$d_6$, $\delta$) : 2.20 (3H, s), 4.52 (2H, s), 6.25 (1H, s), 6.47 (1H, d, J = 3Hz), 7.00 (1H, d, J = 3Hz), 7.30 (1H, s), 8.33 (4H, s)

| Anal. Calcd. for $C_{13}H_{14}N_6OS_2$ • HCl : | | | |
|---|---|---|---|
| | C 42.10, | H 4.08, | N 22.66 |
| Found | C 41.82, | H 4.23, | N 22.53 |

Example 11

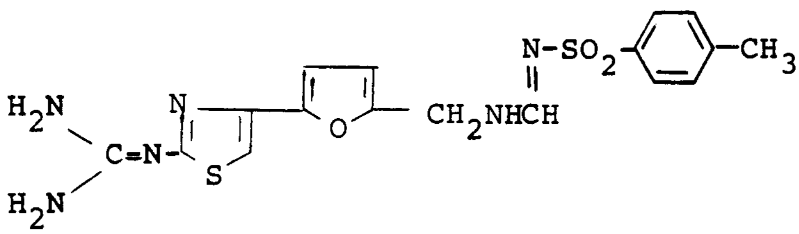

A mixture of 4-(5-aminomethylfuran-2-yl)-2-(diaminomethyleneamino)thiazole (0.80 g) and ethyl tosylformimidate

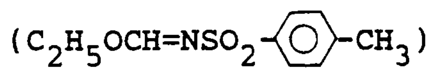

(0.77g) in methanol (10 ml) was stirred for 5 hours at ambient temperature. The reaction mixture was diluted with diisopropyl ether (10 ml) and the resulting precipitate was collected by filtration. The product was purified by column chromatography on silica gel eluting with chloroform-methanol (9:1, V/V), followed by recrystallization from a mixture of methanol, tetrahydrofuran and diisopropyl ether to afford 2-(diaminomethyleneamino)-4-(5-tosyliminomethylaminomethylfuran-2-yl)thiazole (0.46 g).
mp : 141 to 142 ° C
IR (Nujol) : 3370, 1620 (shoulder), 1605, 1330, 1145 $cm^{-1}$
NMR (DMSO-$d_6$, $\delta$) : 2.39 (3H, s), 4.46 (2H, d, J = 5Hz), 6.33 (1H, d, J = 3Hz), 6.56 (1H, s), 6.58 (1H, d, J = 3Hz), 6.86 (4H, s), 7.28 (2H, d, J = 8Hz), 7.65 (2H, d, J = 8Hz), 8.14 (1H, d, J = 5Hz), 9.10-9.27 (1H, m)

| Anal. Calcd. for $C_{17}H_{18}N_6O_3S_2$ • $H_2O$ : | | | | |
|---|---|---|---|---|
| | C 46.78, | H 4.82, | N 19.25, | $H_2O$ 4.13 |
| Found | C 46.86, | H 4.59, | N 18.78, | $H_2O$ 3.39 |

Example 12

The following compound was obtained according to a similar manner to that of Example 4.

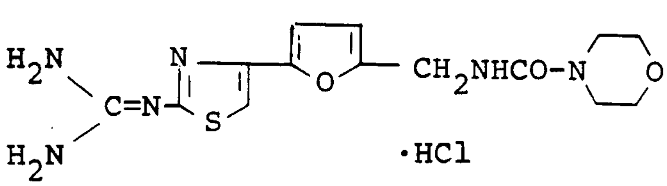

•HCl

2-(Diaminomethyleneamino)-4-(5-morpholinocarbonylaminomethylfuran-2-yl)thiazole hydrochloride.
mp : 214-215 °C
IR (Nujol) : 3370, 3320, 3200, 1680, 1615, 1600 $cm^{-1}$
NMR (DMSO-$d_6$, δ) : 3.19-3.47 (4H, m), 3.47-3.76 (4H, m), 4.31 (2H, d, J = 5Hz), 6.33 (1H, d, J = 3Hz), 6.94 (1H, d, J = 3Hz), 7.12 (1H, t, J = 5Hz), 7.27 (1H, s), 8.21 (4H, s)

Example 13

The following compound was obtained according to a similar manner to that of Example 6.

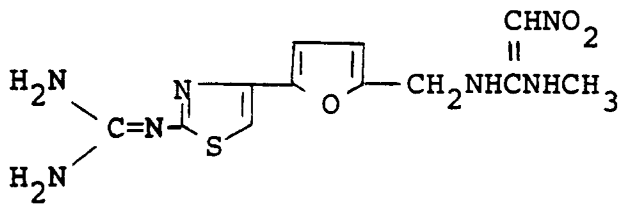

2-(Diaminomethyleneamino)-4-[5-{N-(1-methylamino-2-nitrovinyl)aminomethyl}furan-2-yl]thiazole.
mp : 238-239 °C
IR (Nujol) : 3350, 1610, 1550, 1380 $cm^{-1}$
NMR (DMSO-$d_6$, δ) : 2.85 (3H, d, J = 4Hz), 4.45 (2H, d, J = 5Hz), 6.38 (1H, d, J = 3Hz), 6.55 (1H, s), 6.61 (1H, d, J = 3Hz), 6.76 (1H, s), 6.84 (4H, s)

| | | | |
|---|---|---|---|
| Anal. Calcd. | C 42.72, | H 4.48, | N 29.06 |
| Found | C 42.77, | H 4.53, | N 28.96 |

Example 14

The following compound was obtained according to a similar manner to that of Example 5.

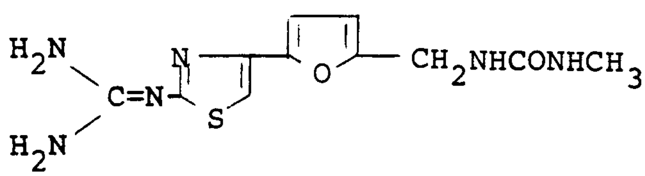

2-(Diaminomethyleneamino)-4-[5-(3-methylureido)methylfuran-2-yl]thiazole.
mp : 218-219 °C (dec.)
IR (Nujol) : 3400, 3330, 1630, 1590, 1540 $cm^{-1}$
NMR (DMSO-$d_6$, δ) : 2.54 (3H, d, J = 5Hz), 4.17 (2H, d, J = 5Hz), 5.56-5.85 (1H, m), 6.06-6.34 (1H, m), 6.17 (1H, d, J = 3Hz), 6.51 (1H, d, J = 3Hz), 6.68 (1H, s), 6.79 (4H, s)

Example 15

The following compounds were obtained according to a similar manner to that of Example 3.

(1)

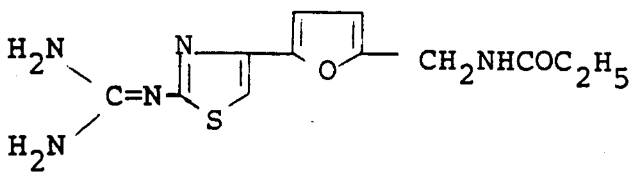

2-(Diaminomethyleneamino)-4-(5-propionylaminomethylfuran-2-yl)thiazole.
mp : 192-194 °C (dec.)
IR (Nujol) : 3380, 3290, 3110, 1660, 1615, 1540 $cm^{-1}$
NMR (DMSO-$d_6$, δ) : 1.06 (3H, t, J = 7Hz), 2.16 (2H, q, J = 7Hz), 4.28 (2H, d, J = 5Hz), 6.25 (1H, d, J = 3Hz), 6.58 (1H, d, J = 3Hz), 6.74 (1H, s), 6.86 (4H, s), 7.18 (1H, t, J = 5Hz)

(2)

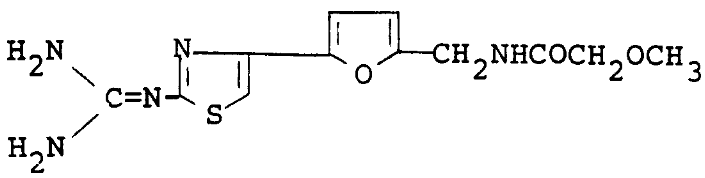

2-(Diaminomethyleneamino)-4-[5-(2-methoxyacetylaminomethyl)furan-2-yl]thiazole.
mp : 201-202 °C (dec.)
IR (Nujol) : 3430, 3370, 3110, 1670, 1640, 1600, 1555 $cm^{-1}$
NMR (DMSO-$d_6$, δ) : 3.33 (3H, s), 3.86 (2H, s), 4.33 (2H, d, J = 5Hz), 6.24 (1H, d, J = 3Hz), 6.56 (1H, d, J = 3Hz), 6.73 (1H, s), 6.86 (4H, s), 8.20 (1H, t, J = 5Hz)

(3)

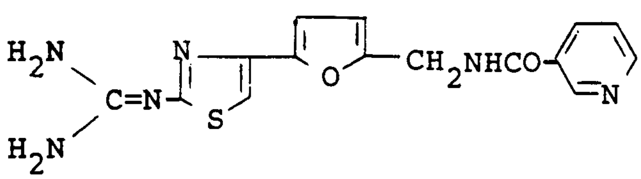

2-(Diaminomethyleneamino)-4-(5-nicotinoylaminomethylfuran-2-yl]thiazole.
mp : 224-225 °C
IR (Nujol) : 3480, 3400, 3350, 1610 $cm^{-1}$
NMR (DMSO-$d_6$, δ) : 4.51 (2H, d, J = 5Hz), 6.33 (1H, d, J = 3Hz), 6.57 (1H, d, J = 3Hz), 6.73 (1H, s), 6.82 (4H, s), 7.46 (1H, dd, J = 4 and 8Hz), 8.17 (1H, dt, J = 2 and 8Hz), 8.65 (1H, dd, J = 2 and 4Hz), 8.98 (1H, d, J = 2Hz), 9.13 (1H, t, J-5Hz)

| | | | |
|---|---|---|---|
| Anal. Calcd. | C 49.99, | H 4.48, | N 23.32 |
| Found | C 50.00, | H 4.50, | N 23.39 |

Example 16

The following compounds were obtained according to a similar manner to that of Example 1.

(1)

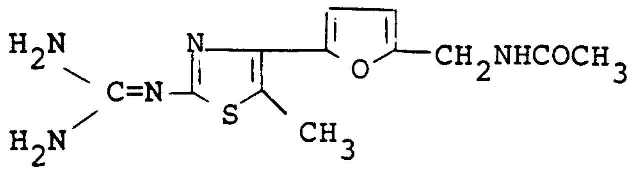

4-(5-Acetylaminomethylfuran-2-yl)-2-(diaminomethyleneamino)-5-methylthiazole.
mp : 247-248 °C
IR (Nujol) : 3420, 3350, 1660, 1605, 1550 $cm^{-1}$
NMR (DMSO-$d_6$, $\delta$) : 1.85 (3H, s), 2.40 (3H, s), 4.24 (2H, d, J=5Hz), 6.22 (1H, d, J=3Hz) 6.44 (1H, d, J=3Hz), 6.72 (4H, s), 8.20 (1H, t, J=5Hz)

(2)

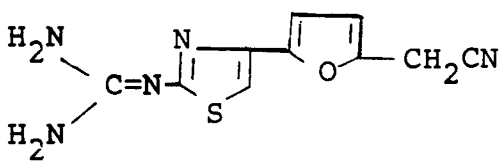

2-(Diaminomethyleneamino)-4-(5-cyanomethylfuran-2-yl)thiazole.
mp : 215-218 °C (dec.)
IR (Nujol) : 3450, 3420, 3370, 3110, 2250, 2210, 1650, 1610, 1590, 1510 $cm^{-1}$
NMR (DMSO-$d_6$, $\delta$) : 4.20 (2H, s), 6.42 (1H, d, J=3Hz), 6.65 (1H, d, J=3Hz), 6.83 (1H, s), 6.87 (4H, s)

Example 17

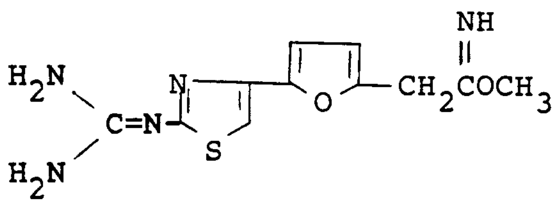

To a solution of 2-(diaminomethyleneamino)-4-(5-cyanomethylfuran-2-yl)thiazole (4.0 g) in dry chloroform (50 ml) and dry methanol (50 ml) was bubbled with dry hydrogen chloride for 100 minutes under ice-cooling and the mixture was stirred for 3.5 hours at the same temperature. The excess hydrogen chloride was removed by reduced pressure under stirring.

The resultant mixture was made basic to pH 9.5 with an aqueous potassium carbonate under ice-cooling. The mixture was extracted with the mixture of tetrahydrofuran and ethyl acetate. The extract layer was washed with a brine and dried over magnesium sulfate. Evaporation of the solvent gave a residue, which was triturated with ether to give 2-(diaminomethyleneamino)-4-[5-(2-imino-2-methoxyethyl)furan-2-yl]-thiazole (4.28 g).
mp : 156-162 °C (dec.)
IR (Nujol) : 3420, 3110, 1655, 1610, 1590, 1550, 1510 $cm^{-1}$
NMR (DMSO-$d_6$, $\delta$) : 3.60 (3H, s), 3.65 (2H, s), 6.26 (1H, d, J=3Hz), 6.58 (1H, d, J=3Hz), 6.73 (1H, s), 6.84 (4H, s), 7.81 (1H, s)

Example 18

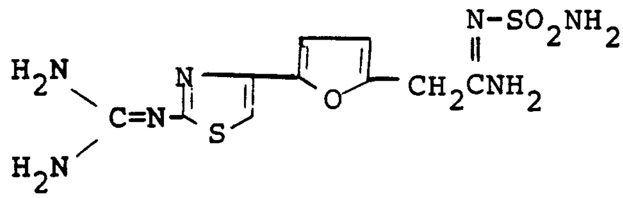

A mixture of 2-(diaminomethyleneamino)-4-[5-(2-imino-2-methoxyethyl)furan-2-yl]thiazole (4.2 g) and sulfamide (2.9 g) in methanol (100 ml) was heated under reflux for 3 hours. The solvent was removed by concentration in vacuo and to the residue was added a mixture of ethyl acetate and water. The mixture was adjusted to pH 1.0 with 6N-hydrochloric acid and the separated aqueous layer was adjusted to pH 10.0 with 20% aqueous potassium carbonate. The aqueous solution was extracted with a mixture of ethyl acetate and tetrahydrofuran under solution of potassium chloride in aqueous layer. The extract layer was washed with a brine and dried over magnesium sulfate.

Evaporation of the solvent gave a residue, which was purified by column chromatography on alumina eluting with the mixture of chloroform and methanol (9:1, V/V). The eluted fractions containing the desired product were evaporated to give 4-[5-[2-amino-2-(aminosulfonylimino)ethyl]furan-2-yl]-2-(diaminomethyleneamino)thiazole (0.73 g).
mp : 222-225 °C (dec.)
IR (Nujol) : 3470, 3450, 3400, 3350, 3320, 3230, 1620, 1530 $cm^{-1}$
NMR (DMSO-$d_6$, $\delta$) : 3.58 (2H, s), 6.28 (1H, d, J=3Hz), 6.52 (2H, s), 6.56 (1H, d, J=3Hz), 6.73 (1H, s), 6.81 (4H, s), 7.35 (1H, s), 8.18 (1H, s)

Example 19

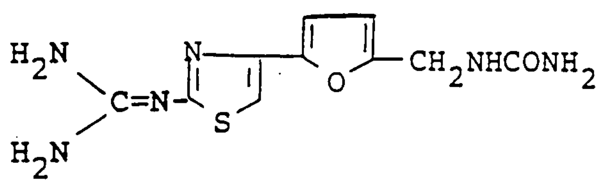

A solution of potassium cyanate (1.0 g) in water (9 ml) was dropwise added to a mixture of 4-(5-aminomethylfuran-2-yl)-2-(diaminomethyleneamino)thiazole (1.5 g) in water (9 ml) and acetic acid (4.5 ml) at 35 °C for 2 minutes and the mixture was stirred at the same temperature for 2 hours. To the reaction mixture was added a mixture of ethyl acetate,tetrahydrofuran and water and the resultant mixture was adjusted to pH 9.0 with 20% aqueous potassium carbonate. The separated organic layer was washed with a brine and dried over magnesium sulfate. Evaporation of the solvent gave a residue, which was purified by column chromatography on silica gel eluting with a mixture of ethyl acetate and methanol (85:15, V/V). The eluted fractions containing the desired product were collected and evaporated in vacuo. The residue was recrystallized from a aqueous N,N-dimethylformamide to give 2-(diaminomethyleneamino)-4-(5-ureidomethylfuran-2-yl)thiazole (0.31 g).
mp : 214-215 °C
IR (Nujol) : 3400, 3320, 3130, 1650, 1590 $cm^{-1}$
NMR (DMSO-$d_6$, $\delta$) : 4.19 (2H, d, J=5Hz), 5.53 (2H, s), 6.24 (1H, d, J=3Hz), 6.36 (1H, t, J=5Hz), 6.59 (1H, d, J=3Hz), 6.76 (1H, s), 6.87 (4H, s)

| Anal. Calcd. for $C_{10}H_{12}N_6O_2S \cdot 1/3\ H_2O$ : | | | | |
|---|---|---|---|---|
| | C 41.95, | H 4.46, | N 29.35, | $H_2O$ 2.10 |
| Found | C 42.04, | H 4.53, | N 29.04, | $H_2O$ 2.33 |

Example 20

The following compounds were obtained according to a similar manner to that of Example 1.

(1)

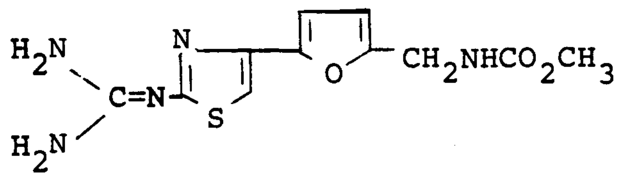

2-(Diaminomethyleneamino)-4-(5-methoxycarbonylaminomethylfuran-2-yl)thiazole
mp : 196 °C
IR (Nujol) : 3450, 3400, 1710, 1650, 1610 $cm^{-1}$
NMR (DMSO-$d_6$, $\delta$) : 3.57 (3H, s), 4.19 (2H, d, J=5Hz), 6.26 (1H, d, J=3Hz), 6.58 (1H, d, J=3Hz), 6.75 (1H, s), 6.85 (4H, s), 7.58 (1H, br s)

| Anal. Calcd. for $C_{11}H_{13}N_5O_3S$ : | | | |
|---|---|---|---|
| | C 44.74, | H 4.44, | N 23.71 |
| Found | C 44.71, | H 4.63, | N 23.83 |

(2)

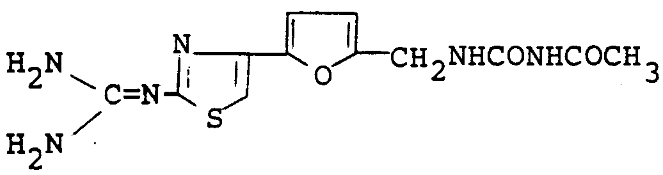

4-[5-(3-Acetylureidomethyl)furan-2-yl]-2-(diaminomethyleneamino)thiazole.
mp : 245 °C (dec.)
IR (Nujol) : 3400, 3300, 1680, 1650, 1540 $cm^{-1}$
NMR (DMSO-$d_6$, $\delta$) : 2.02 (3H, s), 4.41 (2H, d, J=6Hz), 6.31 (1H, d, J=3Hz), 6.62 (1H, d, J=3Hz), 6.78 (1H, s), 6.90 (4H, s), 8.68 (1H, t, J=6Hz), 10.43 (1H, s)

| Anal. Calcd. for $C_{12}H_{14}N_6O_3S$ : | | | |
|---|---|---|---|
| | C 44.71, | H 4.38, | N 26.07 |
| Found | C 44.53, | H 4.24, | N 26.24 |

(3)

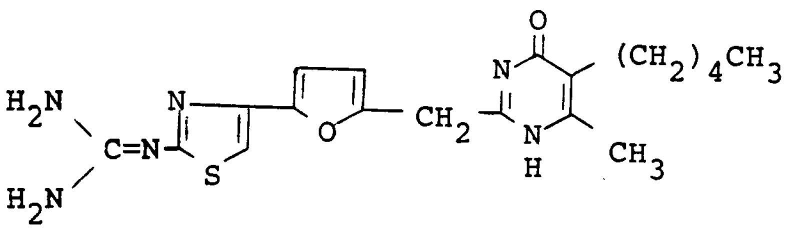

2-(Diaminomethyleneamino)-4-[5-(6-methyl-5-pentyl-4(1H)-pyrimidinon-2-ylmethyl)furan-2-yl]thiazole.
mp : 212-213 °C
IR (Nujol) : 3450, 1640, 1605, 1540 $cm^{-1}$
NMR (DMSO-$d_6$, $\delta$) : 0.75-0.95 (3H, m), 1.20-1.50 (6H, m), 2.18 (3H, s), 2.28-2.48 (2H, m), 3.90 (2H, s), 6.31 (1H, d, J=3Hz), 6.61 (1H, d, J=3Hz), 6.76 (1H, s), 6.90 (1H, s), 12.40 (1H, s)

| Anal. Calcd. for $C_{19}H_{24}N_6O_2S$ : | | | |
|---|---|---|---|
| | C 56.98, | H 6.04, | N 20.98 |
| Found | C 56.72, | H 5.95, | N 20.76 |

(4)

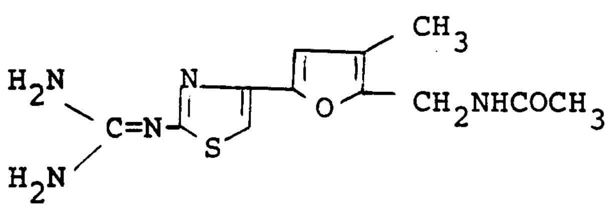

4-(5-Acetylaminomethyl-4-methylfuran-2-yl)-2-(diaminomethyleneamino)thiazole.
mp : 237 °C
IR (Nujol) : 3400, 3280, 3140, 3070, 1640, 1600, 1540 $cm^{-1}$
NMR (DMSO-$d_6$, $\delta$) : 1.82 (3H, s), 1.99 (3H, s), 4.22 (2H, d, J=5Hz), 6.51 (1H, s), 6.72 (1H, s), 6.88 (4H, s), 8.25 (1H, t, J=5Hz)

| Anal. Calcd. for $C_{12}H_{15}N_5O_2S$ : | | | |
|---|---|---|---|
| | C 49.13, | H 5.15, | N 23.87 |
| Found | C 49.48, | H 5.15, | N 23.93 |

(5)

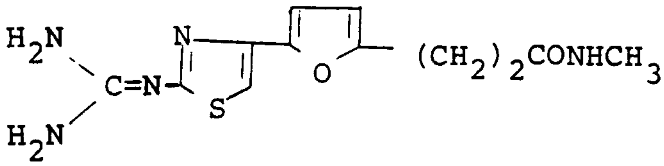

2-(Diaminomethyleneamino)-4-[5-{2-(methylcarbamoyl)ethyl}furan-2-yl]thiazole.
mp : 229 to 230 °C
IR (Nujol) : 3400, 3340, 1640, 1610 $cm^{-1}$
NMR (DMSO-$d_6$, δ) : 2.42 (2H, t, J=7.5Hz), 2.59 (3H, d, J=4.5Hz), 2.88 (2H, t, J=7.5Hz), 6.11 (1H, d, J=3.0Hz), 6.52 (1H, d, J=3.0Hz), 6.70 (1H, s), 6.85 (4H, s), 7.79 (1H, d, J=4.5Hz)

| Anal. Calcd. for $C_{12}H_{15}N_5O_2S$ : | | | |
|---|---|---|---|
| | C 49.13, | H 5.15, | N 23.87 |
| Found | C 49.08, | H 5.32, | N 23.67 |

(6)

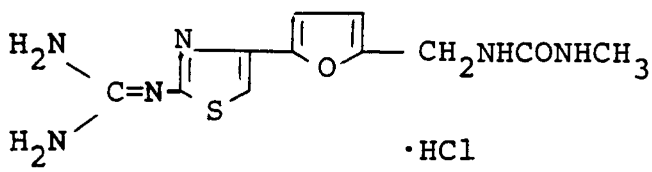

2-(Diaminomethyleneamino)-4-[5-(3-methylureido)methylfuran-2-yl]thiazole hydrochloride.
mp : 220 to 221 °C (dec.)
IR (Nujol) : 3280, 3140, 1685 $cm^{-1}$
NMR (DMSO-$d_6$, δ) : 2.63 (3H, s), 4.30 (2H, d, J=5Hz), 6.00 (1H, br s), 6.32 (1H, d, J=3Hz), 6.45 (1H, t, J=5Hz), 6.80 (1H, d, J=3Hz), 7.07 (1H, s), 8.00 (4H, s) and 12.67 (1H, br s)

Example 21

The following compounds were obtained from 4-(5-aminomethylfuran-2-yl)-2-(diaminomethyleneamino)-thiazole according to a similar manner to that of Example 3.

(1)

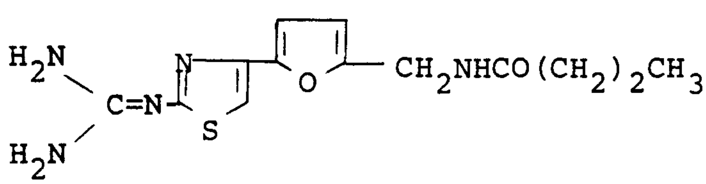

4-(5-Butyrylaminomethylfuran-2-yl)-2-(diaminomethyleneamino)thiazole.
mp : 182 to 183 ° C
IR (Nujol) : 3520, 1610 $cm^{-1}$
NMR (DMSO-$d_6$, $\delta$) : 0.85 (3H, t, J = 7.0Hz), 1.75-1.28 (2H, m), 2.09 (2H, t, J = 7Hz), 4.25 (2H, d, J = 5.0Hz), 6.24 (1H, d, J = 3.3Hz), 6.55 (1H, d, J = 3.3Hz), 6.72 (1H, s), 6.83 (4H, s), 8.21 (1H, t, J = 5.0Hz)

| Anal. Calcd. for $C_{13}H_{17}N_5O_2S \cdot H_2O$ : | | | |
|---|---|---|---|
| | C 47.99, | H 5.86, | N 21.52 |
| Found | C 48.26, | H 5.97, | N 21.70 |

(2)

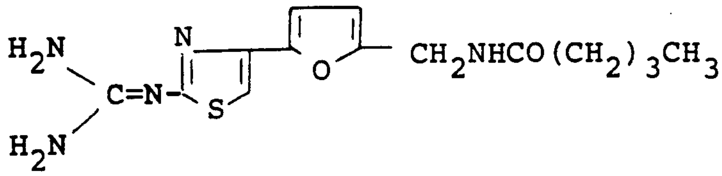

2-(Diaminomethyleneamino)-4-(5-valerylaminomethylfuran-2-yl)thiazole.
mp : 197 ° C
IR (Nujol) : 3390, 3290, 1650, 1610 $cm^{-1}$
NMR (DMSO-$d_6$, $\delta$) : 0.80 (3H, t, J = 6.3Hz), 1.68-1.00 (4H, m), 2.08 (2H, t, J = 6.9Hz), 4.21 (2H, d, J = 5.7Hz), 6.18 (1H, d, J = 3.0Hz), 6.51 (1H, d, J = 3.0Hz), 6.65 (1H, s), 6.79 (4H, s), 8.17 (1H, t, J = 5.7Hz)

| Anal. Calcd. for $C_{14}H_{19}N_5O_2S$ : | | | |
|---|---|---|---|
| | C 52.30, | H 5.96, | N 21.79 |
| Found | C 52.90, | H 6.05, | N 22.01 |

(3)

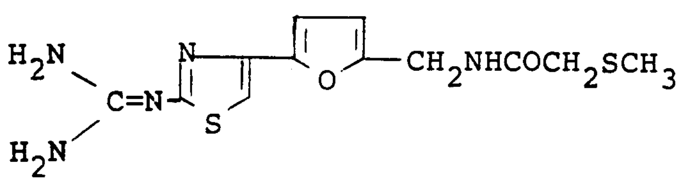

2-(Diaminomethyleneamino)-4-(5-methylthioacetylaminomethylfuran-2-yl)thiazole.
mp : 195-197 ° C (dec.)
IR (Nujol) : 3440, 3250, 1620, 1595, 1540 $cm^{-1}$
NMR (DMSO-$d_6$, $\delta$) : 2.10 (3H, s), 3.10 (2H, s), 4.27 (2H, d, J = 5Hz), 6.25 (1H, d, J = 3Hz), 6.55 (1H, d, J = 3Hz), 6.70 (1H, s), 6.81 (4H, s), 8.37 (1H, t, J = 5Hz)

| Anal. Calcd. for $C_{12}H_{15}N_5O_2S$ : | | | |
|---|---|---|---|
| | C 44.29, | H 4.65, | N 21.52 |
| Found | C 44.17, | H 5.09, | N 21.14 |

(4)

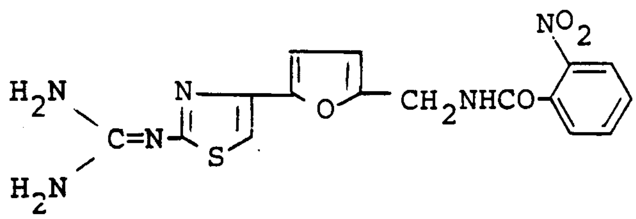

2-(Diaminomethyleneamino)-4-[5-(2-nitrobenzoyl)aminomethylfuran-2-yl]thiazole.
mp : 218 to 220 °C
IR (Nujol) : 3420, 3350, 1650, 1610 $cm^{-1}$
NMR (DMSO-$d_6$, $\delta$) : 4.43 (2H, d, J=5.4Hz), 6.32 (1H, d, J=3.0Hz), 6.55 (1H, d, J=3.0Hz), 6.70 (1H, s), 6.78 (4H, s), 7.99-7.48 (3H, m), 7.97 (1H, dd, J=6.6 and 1.8Hz), 9.09 (1H, t, J=5.4Hz)

| Anal. Calcd. for $C_{16}H_{14}N_6O_4S$ : | | | |
|---|---|---|---|
| | C 49.74, | H 3.65, | N 21.75 |
| Found | C 49.37, | H 3.76, | N 21.57 |

(5)

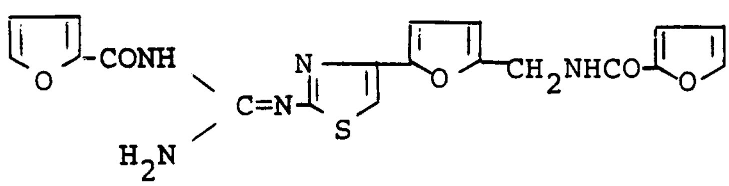

2-[(Amino) (2-furoylamino)methyleneamino]-4-[5-(2-furoyl)aminomethylfuran-2-yl]thiazole.
mp : 199 to 200 °C
IR (Nujol) : 3450, 3375, 1660, 1630 $cm^{-1}$
NMR (DMSO-$d_6$, $\delta$) : =4.47 (2H, d, J=5.3Hz), 6.36 (1H, d, J=3.2Hz), 6.65-6.55 (2H, m), 6.71 (1H, d, J=3.2Hz), 7.12 (1H, s), 7.20-7.05 (1H, m), 7.52 (1H, br), 7.81 (1H, t, J=0.7Hz), 7.94 (1H, s), 8.82 (2H, m)

| Anal. Calcd. for $C_{19}H_{15}N_5O_5S$ : | | | |
|---|---|---|---|
| | C 53.64, | H 3.55, | N 16.46 |
| Found | C 53.95, | H 3.64, | N 16.35 |

(6)

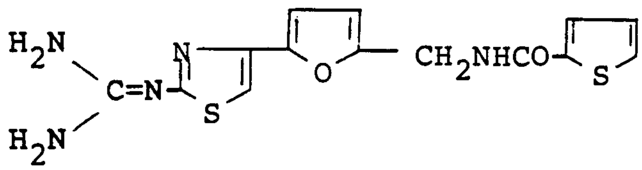

2-(Diaminomethyleneamino)-4-[5-(2-thenoyl)aminomethylfuran-2-yl]thiazole.
mp : 217 to 220 °C
IR (Nujol) : 3400, 1610 $cm^{-1}$
NMR (DMSO-$d_6$, $\delta$) : 4.41 (2H, d, J = 5.4 Hz), 6.26 (1H, d, J = 3.0Hz), 6.53 (1H, d, J = 3.0Hz), 6.69 (1H, s), 6.78 (4H, s), 7.07 (1H, t, J = 4.5Hz), 7.80-7.64 (2H, m), 8.92 (1H, t, J = 5.4Hz)

| Anal. Calcd. for $C_{14}H_{13}N_5O_2S_2$ : | | | |
|---|---|---|---|
| | C 48.40, | H 3.77, | N 20.16 |
| Found | C 47.98, | H 4.13, | N 20.22 |

(7)

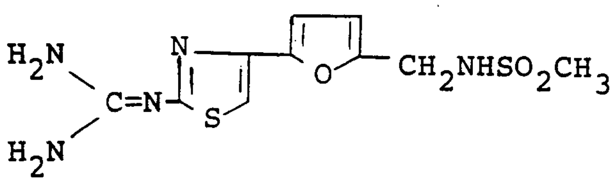

2-(Diaminomethyleneamino)-4-(5-mesylaminomethylfuran-2-yl)thiazole.
mp : 198 °C
IR (Nujol) : 3420, 3270, 1650, 1305, 1145 $cm^{-1}$
NMR (DMSO-$d_6$, $\delta$) : 2.91 (3H, s), 4.19 (2H, d, J = 5.5Hz), 6.37 (1H, d, J = 3.5Hz), 6.60 (1H, d, J = 3.5Hz), 6.78 (1H, s), 6.85 (4H, s), 7.54 (1H, t, J = 5.5Hz)

| Anal. Calcd. for $C_{10}H_{13}N_5O_3S_2$ : | | | |
|---|---|---|---|
| | C 38.08, | H 4.16, | N 22.21 |
| Found | C 37.54, | H 4.12, | N 21.53 |

(8)

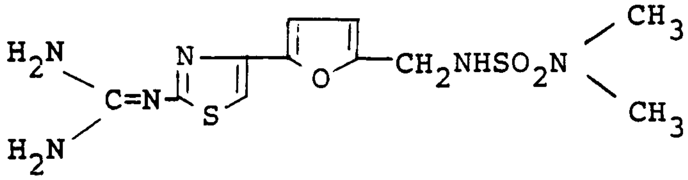

2-(Diaminomethyleneamino)-4-[5-(N,N-dimethylamino)sulfonylaminomethylfuran-2-yl]thiazole.
mp : 169 °C
IR (Nujol) : 3400, 1605, 1520, 1140 $cm^{-1}$
NMR (DMSO-$d_6$, δ) : 2.64 (6H, s), 4.13 (2H, d, J=5.8Hz), 6.37 (1H, d, J=3.2Hz), 6.62 (1H, d, J=3.2Hz), 6.79 (1H, s), 6.89 (4H, s), 7.70 (1H, t, J=5.8Hz)

| Anal. Calcd. for $C_{11}H_{16}N_6O_3S_2$ : | | | |
|---|---|---|---|
| | C 38.36, | H 4.68, | N 24.40 |
| Found | C 37.97, | H 4.36, | N 24.31 |

Example 22

The following compounds were obtained according to a similar manner to that of Example 5.

(1)

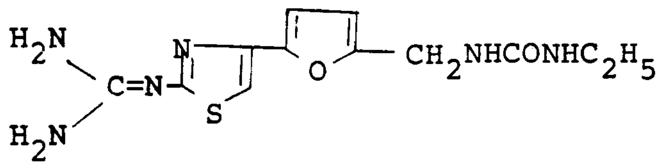

2-(Diaminomethyleneamino)-4-[5-(3-ethylureido)methylfuran-2-yl]thiazole.
mp : 221-223 °C (dec.)
IR (Nujol) : 3380, 3300, 1640, 1610, 1540 $cm^{-1}$
NMR (DMSO-$d_6$, δ) : 1.00 (3H, t, J=7Hz), 2.82-3.20 (2H, m), 4.18 (2H, d, J=6Hz), 5.84 (1H, t, J=6Hz), 6.02-6.33 (1H, m), 6.19 (1H, d, J=3Hz), 6.53 (1H, d, J=3Hz), 6.70 (1H, s), 6.82 (4H, s)

| Anal. Calcd. for $C_{12}H_{16}N_6O_2S$ : | | | |
|---|---|---|---|
| | C 46.74, | H 5.23, | N 27.25 |
| Found | C 46.96, | H 5.50, | N 27.33 |

(2)

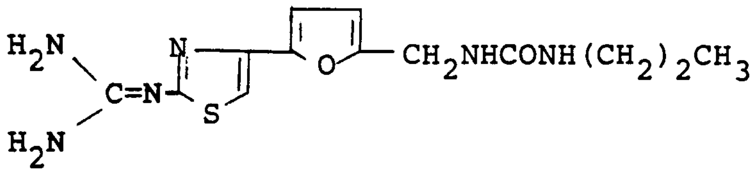

2-(Diaminomethyleneamino)-4-[5-(3-n-propylureido)methylfuran-2-yl]thiazole.
mp : 223 °C
IR (Nujol) : 3400, 3330, 3130, 1625, 1595, 1545 $cm^{-1}$
NMR (DMSO-$d_6$, δ) : 0.83 (3H, t, J=7Hz), 1.06-1.64 (2H, m), 3.81-3.12 (2H, m), 4.21 (2H, d, J=5Hz), 5.91 (1H, t, J=5Hz), 6.07-6.34 (1H, m), 6.22 (1H, d, J=3Hz), 6.58 (1H, d, J=3Hz), 6.75 (1H, s), 6.86 (4H, s)

| Anal. Calcd. for $C_{13}H_{18}N_6O_2S$ : | | | |
|---|---|---|---|
| | C 48.43, | H 5.63, | N 26.07 |
| Found | C 48.03, | H 5.53, | N 25.90 |

(3)

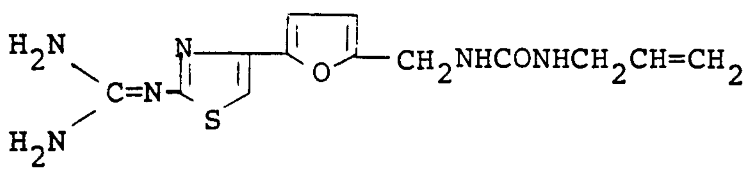

4-[5-(3-Allylureido)methylfuran-2-yl]-2-(diaminomethyleneamino)thiazole.
mp : 219-220 °C (dec)
IR (Nujol) : 3440, 3400, 3325, 1620, 1590 $cm^{-1}$
NMR (DMSO-$d_6$, δ) : 3.55-3.90 (2H, m), 4.27 (2H, d, J=6Hz), 4.90-5.40 (2H, m), 5.57-6.10 (1H, m), 5.90-6.55 (2H, m), 6.28 (1H, d, J=3Hz), 6.61 (1H, d, J=3Hz), 6.78 (1H, s), 6.91 (4H, s)

| Anal. Calcd. for $C_{13}H_{16}N_6O_2S$ : | | | |
|---|---|---|---|
| | C 48.74, | H 5.03, | N 26.23 |
| Found | C 48.51, | H 5.30, | N 25.99 |

(4)

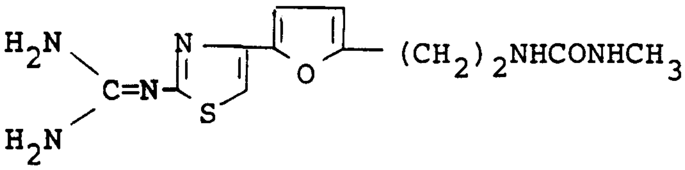

2-(Diaminomethyleneamino)-4-[5-{2-(3-methylureido)ethyl}furan-2-yl]thiazole.
mp : 224-225 ° C
IR (Nujol) : 3440, 3320, 1660, 1630, 1590, 1540 $cm^{-1}$
NMR (DSMO-$d_6$, δ) : 2.52 (3H, d, J = 5Hz), 2.70 (2H, t, J = 7Hz), 3.05-3.38 (2H, m), 5.57-5.98 (2H, m), 6.13 (1H, d, J = 3Hz), 6.51 (1H, d, J = 3Hz), 6.70 (1H, s), 6.80 (4H, m)

| Anal. Calcd. for $C_{12}H_{16}N_6O_2S$ : | | | |
|---|---|---|---|
| | C 46.74, | H 5.23, | N 27.25 |
| Found | C 46.50, | H 4.82, | N 27.03 |

(5)

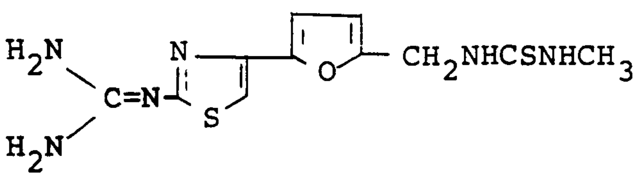

2-(Diaminomethyleneamino)-4-[5-(3-methylthioureido)methylfuran-2-yl]thiazole.
mp : 220 ° C
IR (Nujol) : 3400, 3340, 1610 $cm^{-1}$
NMR (DMSO-$d_6$, δ) : 2.85 (3H, d, J = 3.5Hz), 4.64 (2H, d, J = 4.5Hz), 6.33 (1H, d, J = 3.0Hz), 6.61 (1H, d, J = 3.0Hz),6.78 (1H, s), 6.89 (4H, s), 7.50 (1H, br), 7.85 (1H, t, J = 4.5Hz)

Example 23

The following compounds were obtained according to a similar manner to that of Example 6.

(1)

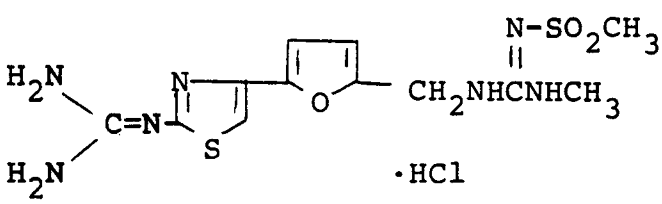

2-(Diaminomethyleneamino)-4-[5-(2-mesyl-3-methylguanidino)methylfuran-2-yl]thiazole hydrochloride.
mp : 233 to 234 ° C
IR (Nujol) : 3350, 3230, 1675, 1370, 1100 $cm^{-1}$
NMR (DMSO-$d_6$, δ) : 2.76 (3H, d, J=5Hz), 2.80 (3H, s), 4.42 (2H, d, J=6Hz), 6.40 (1H, d, J=3Hz), 6.96 (1H, d, J=3Hz), 7.12 (1H, q, J=5Hz), 7.32 (1H, s), 7.45 (1H, t, J=6Hz) and 8.34 (4H, s)

| Anal. Calcd. for $C_{12}H_{17}N_7O_3S_2$ • HCl : | C 35.34, | H 4.45, | N 24.04, | Cl 8.69 |
|---|---|---|---|---|
| found | C 35.11, | H 4.59, | N 23.88, | Cl 8.83 |

(2)

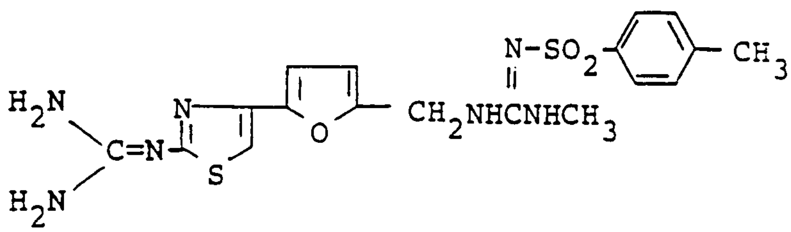

2-(Diaminomethyleneamino)-4-[5-(3-methyl-2-tosylguanidino)methylfuran-2-yl]thiazole.
mp : 208 to 209 ° C
IR (Nujol) : 3420, 3380, 3330, 1650, 1375, 1125 $cm^{-1}$
NMR (DMSO-$d_6$, δ) : 2.31 (3H, s), 2.71 (3H, d, J=4.5Hz), 4.38 (2H, d, J=5.5Hz), 6.18 (1H, d, J=3Hz), 6.58 (1H, d, J=3Hz), 6.67 (1H, s), 6.90 (4H, br s), 7.21 (2H, d, J=8Hz), 7.23 (1H, t, J=4.5Hz), 7.54 (1H, t, J=5.5Hz) and 7.61 (2H, d, J=8Hz)

| Anal. Calcd. for $C_{18}H_{21}N_7O_3S_2$ : | C 48.31, | H 4.73, | N 21.91 |
|---|---|---|---|
| Found | C 48.21, | H 5.00, | N 21.66 |

Example 24

The following compounds were obtained according to a similar manner to that of Example 11.

(1)

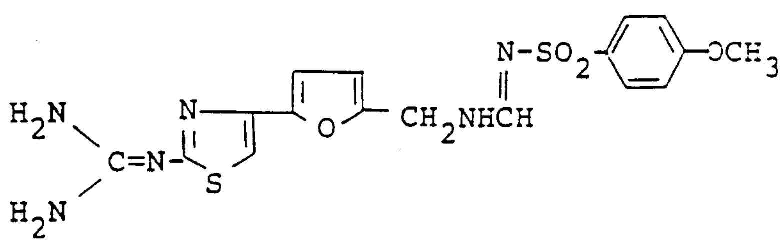

2-(Diaminomethyleneamino)-4-[5-(p-methoxyphenylsulfonyl)iminomethylaminomethylfuran-2-yl]thiazole
mp : 185 to 186 ° C
IR (Nujol) : 3370, 1620, 1330, 1145 $cm^{-1}$
NMR (DMSO-$d_6$, δ) : 3.85 (3H, s), 4.50 (2H, d, J=5Hz), 6.38 (1H, d, J=3Hz), 6.63 (1H, s), 6.65 (1H, d, J=3Hz), 6.97 (4H, s), 7.08 (2H, d, J=9Hz), 7.76 (2H, d, J=9Hz), 8.21 (1H, d, J=5Hz) and 9.08-9.38 (1H, m)

| Anal. Calcd. for $C_{17}H_{18}N_6O_4S_2 \cdot H_2O$ : | | | | |
|---|---|---|---|---|
| | C 45.12, | H 4.45, | N 18.57, | $H_2O$ 3.98 |
| Found | C 45.25, | H 4.81, | N 18.07, | $H_2O$ 2.56 |

(2)

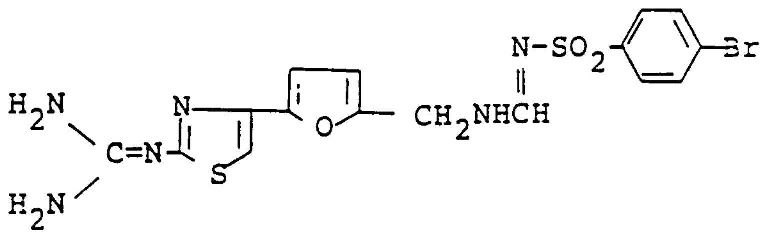

2- Diaminoethyleneamino -4-[5-(p-bromophenylsulfonyl)iminomethylaminomethylfuran-2-yl]thiazole
mp : 210 to 211 °C
IR (Nujol) : 3520, 3400, 1610, 1330, 1140 $cm^{-1}$
NMR (DMSO-$d_6$, $\delta$) : 4.55 (2H, d, J = 5Hz), 6.42 (1H, d, J = 3Hz), 6.65 (1H, s), 6.68 (1H, d, J = 3Hz), 6.97 (4H, s), 7.75 (4H, s), 8.25 (1H, d, J = 4Hz) and 9.35-9.67 (1H, m)

| Anal. Calcd. for $C_{16}H_{15}BrN_6O_3S_2$ : | | | |
|---|---|---|---|
| | C 39.76, | H 3.13, | N 17.39 |
| Found | C 40.17, | H 3.41, | N 17.12 |

(3)

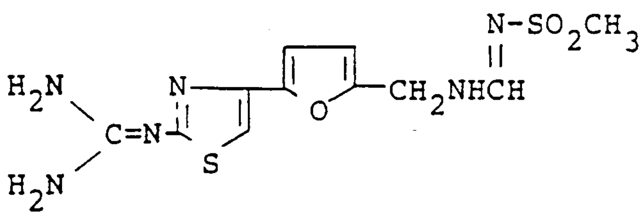

2-(Diaminomethyleneamino)-4-(5-mesyliminomethylaminomethylfuran-2-yl)thiazole.
mp : 222 to 223 °C (dec.)
IR (Nujol) : 3440, 3360, 3290, 1600, 1320, 1120 $cm^{-1}$
NMR (DMSO-$d_6$, $\delta$) : 2.90 (3H, s), 4.48 (2H, d, J = 5.4Hz), 6.41 (1H, d, J = 3.0Hz), 6.62 (1H, d, J = 3.0Hz), 6.80 (1H, s), 6.85 (4H, s), 8.03 (1H, d, J = 4.5Hz) and 9.09 (1H, br s)

| Anal. Calcd. for $C_{11}H_{14}N_6O_3S_2$ | | | |
|---|---|---|---|
| | C 38.59, | H 4.12, | N 24.54 |
| Found | C 38.66, | H 4.24, | N 24.85 |

(4)

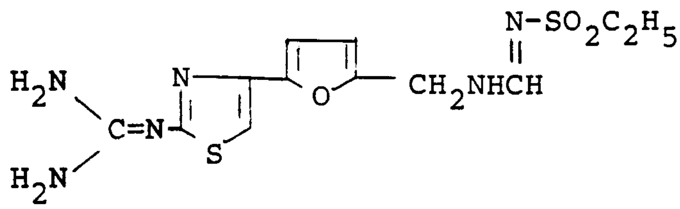

2-(Diaminomethyleneamino)-4-(5-ethylsulfonyliminomethylaminomethylfuran-2-yl)thiazole.
mp : 188 ° C
IR (Nujol) : 3460, 3400, 3350, 1630, 1320, 1130 $cm^{-1}$
NMR (DMSO-$d_6$, δ) : 1.13 (2.64 H, t, J = 7.5Hz), 1.14 (0.36H, t, J = 7.5Hz), 2.96 (2H, q, J = 7.5Hz), 4.48 (1.76H, d, J = 5.0 Hz), 4.54 (0.24 H, d, J = 5.5Hz), 6.42 (1H, d, J = 3.0Hz), 6.65 (1H, d, J = 3.0Hz), 6.78 (0.12H, s), 6.80 (0.88H, s), 6.89 (4H, s), 8.00 (0.88H, d, J = 4.5Hz), 8.18 (0.12H, d, J = 13.0Hz), 9.14 (0.88H, dd, J = 5.0 and 4.5Hz), 9.4-9.2 (0.12H, br)

| Anal. Calcd. for $C_{12}H_{16}N_6O_3S_2$ : | | | |
|---|---|---|---|
| | C 40.44, | H 4.52, | N 23.58 |
| Found | C 40.49, | H 4.53, | N 23.46 |

(5)

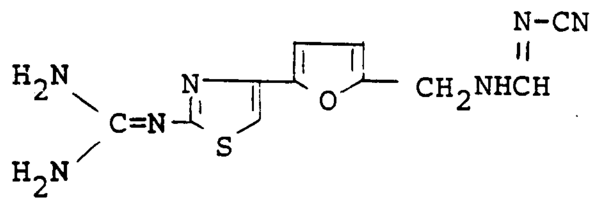

4-(5-Cyanoiminomethylaminomethylfuran-2-yl)-2-(diaminomethyleneamino)thiazole.
mp : 193 to 194 ° C
IR (Nujol) : 3400, 3190, 2170, 1630 $cm^{-1}$
NMR (DMSO-$d_6$, δ) : 4.52 (2H, s), 6.59 (1H, d, J = 3Hz), 6.68 (1H, d, J = 3Hz), 6.83 (1H, s), 6.92 (4H, s), 8.42 (1H, s) and 9.50 (1H, br s)

| Anal. Calcd. for $C_{11}H_{11}N_7OS$ : | | | |
|---|---|---|---|
| | C 45.67, | H 3.83, | N 33.89 |
| Found | C 45.61, | H 4.53, | N 33.49 |

(6)

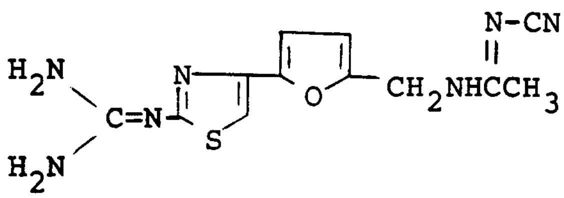

4-[5-(1-Cyanoiminoethyl)aminomethylfuran-2-yl)-2-(diaminomethyleneamino)thiazole
mp : 215 to 217 °C
IR (Nujol) : 3320, 3180, 2180, 1600, 1570, 1530 $cm^{-1}$
NMR (DMSO-$d_6$, $\delta$) : 2.25 (3H, s), 4.42 (2H, d, J = 4Hz), 6.43 (1H, s), 6.64 (1H, s), 6.81 (1H, s), 6.90 (4H, s), 9.28 (1H, s)

| Anal. Calcd. for $C_{12}H_{13}N_7OS$ : | | | |
|---|---|---|---|
| | C 47.53, | H 4.32, | N 32.32 |
| Found | C 47.48, | H 4.33, | N 31.95 |

Example 25

The following compounds were obtained according to a similar manner to that of Example 18.

(1)

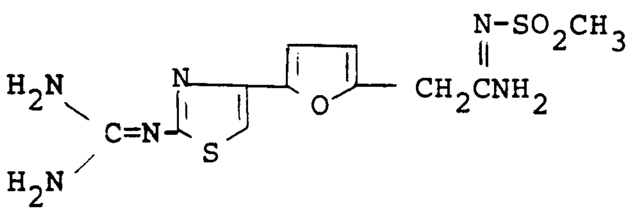

4-[5-(2-Amino-2-mesyliminoethyl)furan-2-yl]-2-(diaminomethyleneamino)thiazole.
mp : 168 to 169 °C (dec.)
IR (Nujol) : 3460, 3400, 3350, 3240, 3170, 3120, 1660, 1640, 1620, 1540 $cm^{-1}$
NMR (DMSO-$d_6$, $\delta$) : 2.88 (3H, s), 3.69 (2H, s), 6.32 (1H, d, J = 3Hz), 6.61 (1H, d, J = 3Hz), 6.77 (1H, s), 6.86 (4H, s), 7.83 (1H, s), 8.50 (1H, s)

| Anal. Calcd. for $C_{11}H_{14}N_6O_3S_2 \cdot H_2O$ | | | | |
|---|---|---|---|---|
| | C 36.66, | H 4.47, | N 23.32, | $H_2O$ 5.00 |
| Found | C 36.80, | H 4.31, | N 23.44, | $H_2O$ 5.22 |

(2)

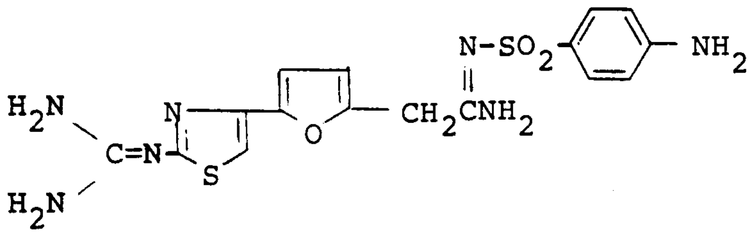

4-[5-{2-Amino-2-(p-aminophenylsulfonyl)iminoethyl}furan-2-yl]-2-(diaminomethyleneamino)thiazole.
mp : 190-191 °C (dec.)
IR (Nujol) : 3400, 3340, 1635, 1600, 1550 $cm^{-1}$
NMR (DMSO-$d_6$, $\delta$) : 3.63 (2H, s), 5.81 (2H, s), 6.19 (1H, d, J=3Hz), 6.55 (2H, d, J=8Hz), 6.58 (1H, d, J=3Hz), 6.70 (1H, s), 6.87 (4H, s), 7.45 (2H, d, J=8Hz), 7.85 (1H, s), 8.47 (1H, s)

| Anal. Calcd. for $C_{16}H_{17}N_7O_3S_2$ : | | | |
|---|---|---|---|
| | C 45.81, | H 4.08, | N 23,37 |
| Found | C 45.92, | H 4.21, | N 22.95 |

(3)

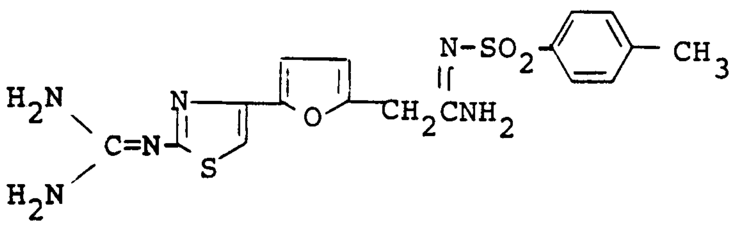

4-[5-(2-Amino-2-tosyliminoethyl)furan-2-yl]-2-(diaminomethyleneamino)thiazole.
mp : 207-209 °C (dec.)
IR (Nujol) : 3450, 3420, 3380, 3320, 1670, 1630, 1605, 1550 $cm^{-1}$
NMR (DMSO-$d_6$, $\delta$) : 2.33 (3H, s), 3.68 (2H, s), 6.20 (1H, d, J=3Hz), 6.57 (1H, d, J=3Hz), 6.64 (1H, s), 6.90 (4H, s), 7.26 (2H, d, J=8Hz), 7.67 (2H, d, J=8Hz), 8.10 (1H, s), 8.76 (1H, s)

| Anal. Calcd. for $C_{17}H_{18}N_6O_3S_2$ : | | | |
|---|---|---|---|
| Found | C 48.79,<br>C 48.44, | H 4.34,<br>H 4.19, | N 20.08<br>N 19.77 |

(4)

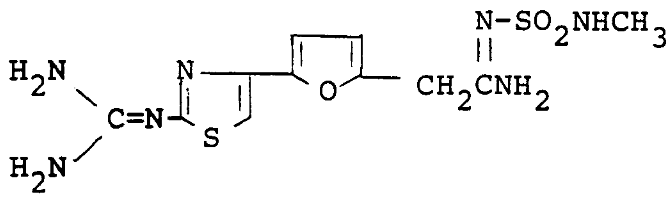

4-[5-(2-Amino-2-methylaminosulfonyliminoethyl)furan-2-yl]-2-(diaminomethyleneamino)thiazole.
mp : 219 to 220 °C (dec.)
IR (Nujol) : 3480, 3430, 3380, 3330, 1610, 1530 $cm^{-1}$
NMR (DMSO-$d_6$, δ) : 2.42 (3H, d, J=5Hz), 3.65 (2H, s), 6.32 (1H, d, J=3Hz), 6.59 (1H, q, J=5Hz), 6.62 (1H, d, J=3Hz), 6.75 (1H, s), 6.89 (4H, s), 7.63 (1H, s), 8.42 (1H, s)

| Anal. Calcd. for $C_{11}H_{15}N_7O_3S_2$ : | | | |
|---|---|---|---|
| Found | C 36.97,<br>C 36.93, | H 4.23,<br>H 4.15, | N 27.43<br>N 27.41 |

(5)

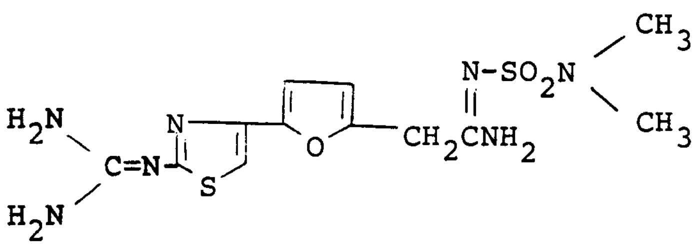

4-[5-{2-Amino-2-(N,N-dimethylamino)sulfonyliminoethyl}furan-2-yl]-2-(diaminomethyleneamino)thiazole.
mp : 161 °C
IR (Nujol) : 3460, 3340, 1660, 1630, 1565, 1515 $cm^{-1}$
NMR (DMSO-$d_6$, δ) : 2.51 (6H, s), 3.69 (2H, s), 6.33 (1H, d, J=3Hz), 6.61 (1H, d, J=3Hz), 6.75 (1H, s), 6.89 (4H, s), 7.81 (1H, s), 8.51 (1H, s)

| Anal. Calcd. for $C_{12}H_{17}N_7O_3S_2$ : | | | |
|---|---|---|---|
| Found | C 38.80,<br>C 38.62, | H 4.61,<br>H 4.39, | N 26.40<br>N 26.42 |

Example 26

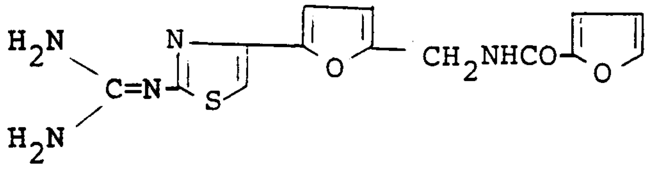

2-Furoyl chloride (0.48 g) was added slowly to a solution of 4-(5-aminomethylfuran-2-yl)-2-(diaminomethyleneamino)thiazole (0.80 g) in N,N-dimethylformamide (20 ml) with cooling on an ice bath and the mixture was stirred for an hour with cooling on an ice bath. The solvent was removed under reduced pressure and the residue was chromatographed on a silica gel column eluting with a mixture of chloroform and methanol (30:1, V/V). Recrystallization from methanol afforded 2-(diaminomethyleneamino)-4-[5-(2-furoyl)aminomethylfuran-2-yl]thiazole (0.20 g).
mp : 246 ° C
IR (Nujol) : 3400, 1655, 1625, 1600 $cm^{-1}$
NMR (DMSO-$d_6$, $\delta$) : 4.42 (2H, d, J = 5.5Hz), 6.26 (1H, d, J = 3.8Hz), 6.64-6.40 (2H, m), 6.72 (1H, s), 6.83 (4H, s), 7.11 (1H, dd, J = 1.0 and 3.8Hz), 7.77 (1H, m), 8.78 (1H, t, J = 5.5Hz)

| Anal. Calcd. for $C_{14}H_{13}N_5O_3S$ : | | | |
|---|---|---|---|
| | C 50.75, | H 3.95, | N 21.14 |
| Found | C 50.69, | H 3.81, | N 21.01 |

Example 27

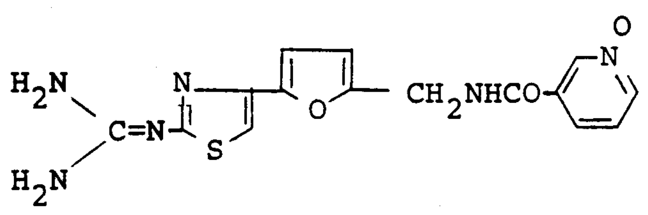

Dicyclohexylcarbodiimide (2.3 g) was added slowly to a suspension of nicotinic acidN-oxide (1.5 g) in N,N-dimethylformamide (40 ml) with cooling on an ice bath and the mixture was stirred for 15 minutes with cooling on an ice bath. To the mixture 4-(5-aminomethylfuran-2-yl)-2-(diaminomethyleneamino)thiazole (2.0 g) was added with cooling on an ice bath. The mixture was stirred for an hour in cooling on an ice bath and then stirred for 41 hours at room temperature. The solvent was removed under reduced pressure and the residue was suspended in water. 6N-Hydrochloric acid solution was added to the suspension and the resulting precipitate was removed by filtration. The solvent was removed under reduced pressure and the residue was dissolved in methanol. The insoluble material was removed by filtration and the solvent was removed under reduced pressure. The residue was chromatographed on a silica gel column eluting with a mixture of chloroform and methanol (10:1, V/V). Recrystallization from a mixture of methanol, dioxane and diisopropyl ether afforded 2-(diaminomethyleneamino)-4-[5-(1-oxonicotinoylaminomethyl)furan-2-yl]thiazole (0.30 g).
mp : 217 ° C
IR (Nujol) : 3420, 1670, 1550 $cm^{-1}$
NMR (DMSO-$d_6$, $\delta$) : 4.51 (2H, d, J = 5.5Hz), 6.39 (1H, d, J = 3.2Hz), 6.63 (1H, d, J = 3.2Hz), 6.78 (1H, s), 6.90 (4H, s), 7.52 (1H, dd, J = 6.4 and 8.0Hz), 7.76 (1H, d, J = 8.0Hz), 8.37 (1H, d, J = 6.4Hz), 8.62 (1H, s), (9.31 (1H, t, J = 5.5Hz),

| Anal. Calcd. for $C_{15}H_{14}N_6O_3S \bullet 3/4H_2O$ : | | | |
|---|---|---|---|
| | C 48.45, | H 4.20, | N 22.59 |
| Found | C 48.79, | H 4.03, | N 22.22 |

Example 28

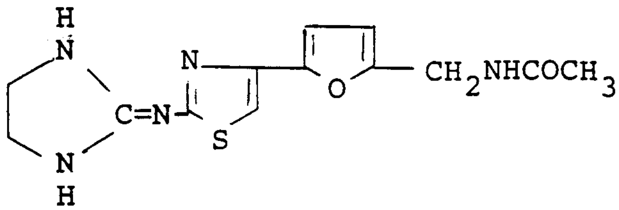

A mixture of 4-(5-acetylaminomethylfuran-2-yl)-2-thioureidothiazole(1.50 g) and methyl iodide (0.32 ml) in methanol (30 ml) and tetrahydrofuran (15 ml) was refluxed for 5 hours with stirring. After the solvent was evaporated in vacuo to give 2-[(amino)(methylthio)methyleneamino]-4-(5-acetylaminomethylfuran-2-yl)-thiazole, the residue was mixed with ethylenediamine (0.91 g) and ethanol (30 ml) and the mixture was refluxed for 18 hours. After cooling, the resulting precipitate was collected by filtration and mixed with water (30 ml). The suspension was acidified to pH 2 with 6N hydrochloric acid, and then made basic to pH 11 with an aqueous potassium carbonate. The resulting precipitate was collected, washed with water and recrystallized from a mixture of methanol and tetrahydrofuran to afford 4-(5-acetylaminomethylfuran-2-yl)-2-(imidazolidin-2-ylideneamino)thiazole (0.51 g).
mp : 239 to 240 °C
IR (Nujol) : 3290, 3105, 1630 $cm^{-1}$
NMR (DMSO-$d_6$, $\delta$) : 1.88 (3H, s), 3.57 (4H, s), 4.33 (2H, d, J = 6Hz), 6.33 (1H, d, J = 3Hz), 6.82 (1H, s), 6.85 (1H, d, J = 3Hz), 7.68 (2H, s) and 8.33 (1H, t, J = 6Hz)

| Anal. Calcd. for $C_{13}H_{15}N_5O_2S$ : | | | |
|---|---|---|---|
| | C 51.13, | H 4.95, | N 22.93 |
| Found | C 50.91, | H 4.62, | N 22.85 |

Example 29

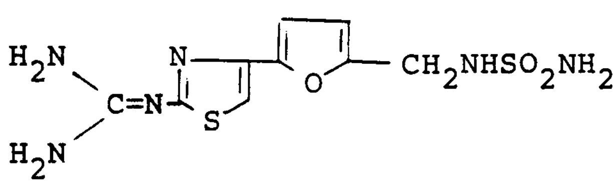

A suspension of 4-(5-aminomethylfuran-2-yl)-2-(diaminomethyleneamino)thiazole (1.6 g) and sulfamide (1.0 g) in ethylene glycol dimethyl ether (30 ml) was refluxed for 24 hours. The solvent was removed under reduced pressure and the residue was chromatographed on an alumina column eluting with a mixture of chloroform and methanol (4:1, V/V). Recrystallization from a mixture of methanol, ethyl acetate and diisopropyl ether afforded 2-(diaminomethyleneamino)-4-(5-sulfamoylaminomethylfuran-2-yl)thiazole (1.16 g).
mp : 175 to 177 °C (dec.)
IR (Nujol) : 3400, 3360, 1650, 1530, 1140 $cm^{-1}$

NMR (DMSO-$d_6$, δ) : 4.01 (2H, d, J=6.1Hz), 6.38 (1H, d, J=3.2z), 6.62 (1H, d, J=3.2Hz), 6.67 (2H, s), 6.83 (1H, s), 6.96 (4H, s), 7.07 (1H, t, J=6.1Hz)

| Anal. Calcd. for $C_9H_{12}N_6O_3S_2$ : | | | |
|---|---|---|---|
| | C 34.17, | H 3.82, | N 26.57 |
| Found | C 33.92, | H 3.79, | N 26.55 |

Example 30

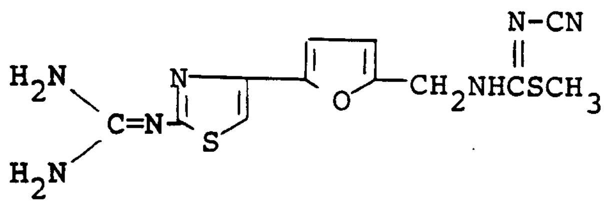

A mixture of 4-(5-aminomethylfuran-2-yl)-2-(diaminomethyleneamino)thiazole (2.50 g) and dimethyl N-cyanodithioiminocarbonate (1.54 g) in ethanol (25 ml) was refluxed for two hours with stirring. After the solvent was evaporated in vacuo, the residue was chromatographed on alumina eluting with a mixture of chloroform and methanol (20:1, V/V), followed by recrystallization from a mixture of methanol, tetrahydrofuran and diisopropyl ether to afford 2-(diaminomethyleneamino)-4-[5-(3-cyano-2-methylisothioureido)methylfuran-2-yl]thiazole (1.48 g).
mp : 210°C (dec.)
IR (Nujol) : 3450, 3300, 3270, 2165, 1655 $cm^{-1}$
NMR (DMS0-$d_6$, δ) : 2.59 (3H, s), 4.48 (3H, s), 6.33 (1H, d, J=3Hz), 6.58 (1H, d, J=3Hz), 6.73 (1H, s), 6.83 (4H, s) and 8.82 (1H, br s)

| Anal. Calcd. for $C_{12}H_{13}N_7OS_2$ : | | | |
|---|---|---|---|
| | C 42.97, | H 3.91, | N 29.23 |
| Found | C 43.08, | H 3.65, | N 29.38 |

Example 31

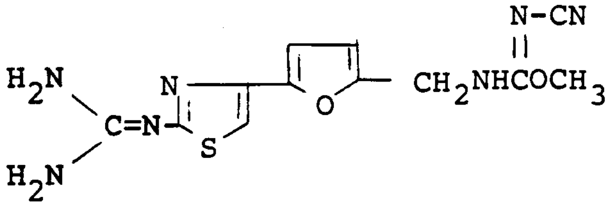

A mixture of 2-(diaminomethyleneamino)-4-[5-(3-cyano-2-methyl-1-isothioureido)methylfuran-2-yl]-thiazole (1.35 g) and 28% methanolic sodium methoxide by weight (0.78 g) in methanol was refluxed for one hour with stirring. After the solvent was evaporated in vacuo, the residue was mixed with water (5 ml) and the medium was adjusted to pH 9 with 6N-hydrochloric acid.

The insoluble material was collected by filtration, washed with water and recrystallized from aqueous N,N-dimethylformamide to afford 4-[5-(3-cyano-2-methyl-1-isoureido)methylfuran-2-yl)]-2-(diaminomethyleneamino)thiazole (0.82 g).

mp : 205 to 206 ° C
IR (Nujol) : 3460, 3345, 2180, 1630 $cm^{-1}$
NMR (DMSO-$d_6$, δ) : 3.83 (3H, s), 4.33 (2H, s), 6.33 (1H, d, J = 3Hz), 6.61 (1H, d, J = 3Hz), 6.74 (1H, s), 6.87 (4H, s) and 8.50 (1H, br s)

| Anal. Calcd. for $C_{12}H_{13}N_7O_2S$ : | | | |
|---|---|---|---|
| | C 45.13, | H 4.10, | N 30.70 |
| Found | C 44.93, | H 4.33, | N 30.42 |

Example 32

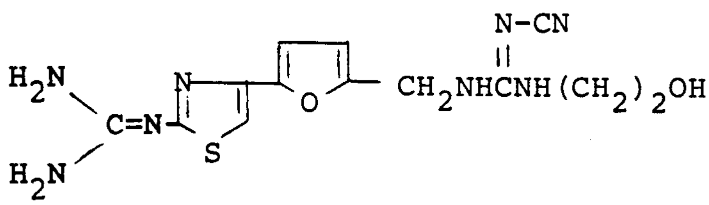

A mixture of 2-(diaminomethyleneamino)-4-[5-(3-cyano-2-methyl-1-isothioureido)methylfuran-2-yl]-thiazole (1.10 g) and monoethanolamine (1 ml) in N,N-dimethylformamide (10 ml) was stirred at 70 ° C for 7 hours. The solvent was evaporated in vacuo and the residue was mixed with water. The resulting precipitate was collected by filtration, washed with water and recrystallized from a mixture of dioxane, methanol and diisopropyl ether to afford 4-[5-{2-cyano-3-(2-hydroxyethyl)guanidino}methylfuran-2-yl]-2-(diaminomethyleneamino)thiazole (0.80 g).
mp : 208 to 209 ° C
IR (Nujol) : 3500, 3390, 3350, 3300, 2160, 1610 $cm^{-1}$
NMR (DMSO-$d_6$, δ) : 3.23-3.52 (4H, m), 4.40 (2H, d, J = 5Hz), 4.92 (1H, t, J = 4Hz), 6.34 (1H, d, (J = 3Hz), 6.62 (1H, d, J = 3Hz), 6.80 (1H, s), 6.90 (4H, s) and 7.52 (1H, t, J = 5Hz)

| Anal. Calcd. for $C_{13}H_{16}N_8O_2S$ : | | | |
|---|---|---|---|
| | C 44.82, | H 4.63, | N 32.16 |
| Found | C 45.12, | H 4.91, | N 32.10 |

Example 33

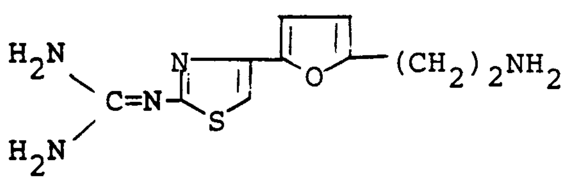

A solution of trifluoroacetic acid (24.9 ml) in tetrahydrofuran (50 ml) was dropwise added to a mixture of sodium borohydride (12.2 g) in tetrahydrofuran (100 ml) for an hour under ice-cooling and the mixture was stirred for 30 minutes at the same temperature.

To this mixture was dropwise added a mixture of 4-(5-cyanomethylfuran-2-yl)-2-(diaminomethyleneamino) thiazole (16.0 g) in tetrahydrofuran (30 ml) for 30 minutes under ice cooling and the mixture was stirred for an hour at ambient temperature. The reaction mixture was added to a mixture of

ethyl acetate and water and the mixture was adjusted to pH 1 with 6N-hydrochloric acid. The separated aqueous layer was adjusted to pH 10 with 10% aqueous sodium hydroxide and the mixture was extracted with a mixture of tetrahydrofuran and ethyl acetate. The extract layer was washed with brine and dried over magnesium sulfate. The solvent was concentrated to give 4-[5-(2-aminoethyl)furan-2-yl]-2-(diaminomethyleneamino)thiazole (4.71 g).
mp : 156-158 °C
IR (Nujol) : 3400, 1660, 1590, 1550 $cm^{-1}$
NMR (DMSO-$d_6$, δ) : 2.57-3.00 (4H, m), 6.16 (1H, d, J=3Hz), 6.53 (1H, d, J=3Hz), 6.71 (1H, s), 6.88 (4H, s)

Example 34

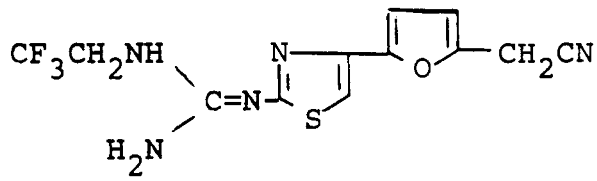

A mixture of 2-[(amino)(methylthio)methyleneamino]-[4-(5-cyanomethylfuran-2-yl)thiazole hydriodide (6.0 g) and 2,2,2-trifluoroethylamine (3.5 ml) in ethanol (120 ml) was refluxed for 18 hours. The solvent was removed by concentration and a residue was added to a mixture of water, ethyl acetate and tetrahydrofuran. The mixture was adjusted to pH 10.0 with potassium carbonate and a separated organic layer was washed with brine, dried over magnesium sulfate.

Evaporation of a solvent gave a residue, which was purified by column chromatography on silica gel eluting with a mixture of chloroform and methanol (19:1, V/V). The eluted fractions containing the desired product were collected and evaporated in vacuo to give 2-[(amino){(2,2,2-trifluoroethyl)-amino}methyleneamino]-4-(5-cyanomethylfuran-2-yl)thiazole (2.41 g).
mp : 152 to 153 °C
IR (Nujol) : 3450, 3400, 2260, 1630, 1540 $cm^{-1}$
NMR (DMSO-$d_6$, δ) : 4.02-4.15 (2H, m), 4.23 (2H, s), 6.47 (1H, d, J=3Hz), 6.72 (1H, d, J=7Hz), 6.87 (1H, s), 7.14 (1H, br s), 7.77 (2H, br s)

Example 35

The following compounds were obtained according to a similar manner to that of Example 34.

(1)

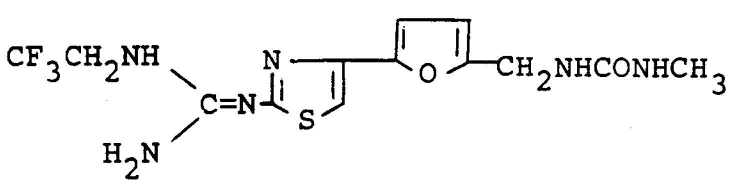

2-[(Amino){(2,2,2-trifluoroethyl)amino}methyleneamino]-4-[5-(3-methylureido)methylfuran-2-yl]thiazole.
mp : 170 to 171 °C
IR (Nujol) : 3375, 3330, 1630, 1570, 1545 $cm^{-1}$
NMR (DMSO-$d_6$, δ) : 2.57 (3H, d, J=5Hz), 4.00-4.23 (2H, m) 4.22 (2H, d, J=6Hz), 5.82 (1H, q, J=5Hz), 6.25 (1H, d, J=3Hz), 6.35 (1H, t, J=6Hz), 6.63 (1H, d, J=3Hz), 6.87 (1H, s), 7.12 (1H, br s), 7.75 (1H, br s)

| Anal. Calcd. for $C_{13}H_{15}N_6O_2SF_3 \cdot 1/5H_2O$ : | | | |
|---|---|---|---|
| | C 41.00, | H 4.10, | N 22.07 |
| Found | C 41.11, | H 3.95, | N 21.67 |

(2)

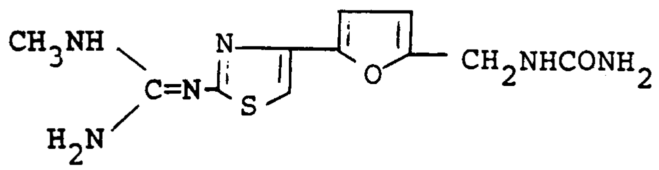

2-[(Amino)(methylamino)methyleneamino]-4-(5-ureidomethylfuran-2-yl)thiazole.
mp : 210 to 211 °C
IR (Nujol) : 3490, 3400, 3340, 3300, 3110, 1640, 1595 $cm^{-1}$
NMR (DMSO-$d_6$, δ) : 2.73 (3H, d, J=5Hz), 4.18 (2H, d, J=5Hz), 5.52 (2H, s), 6.23 (1H, d, J=3Hz), 6.35 (1H, t, J=5Hz), 6.57 (1H, d, J=3Hz), 6.87-7.18 (1H, m) 7.39 (2H, s)

| Anal. Calcd. for $C_{11}H_{14}N_6O_2S$ : | | | |
|---|---|---|---|
| | C 44.89, | H 4.79, | N 28.55 |
| Found | C 45.05, | H 4.79, | N 28.20 |

(3)

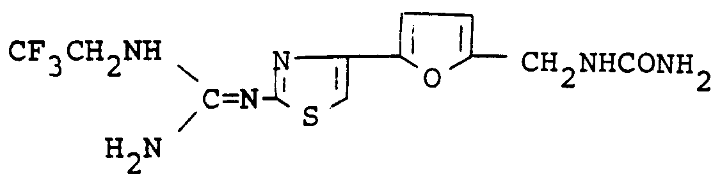

2-[(Amino){(2,2,2-trifluoroethyl)amino}methyleneamino]-4-(5-ureidomethylfuran-2-yl)thiazole.
mp : 198 to 200 °C
IR (Nujol) : 3400, 3300, 1635, 1570, 1550 $cm^{-1}$
NMR (DMSO-$d_6$, δ) : 4.01-4.20 (2H, m), 4.19 (2H, d, J=6Hz), 5.56 (2H, s), 6.26 (1H, d, J=3Hz), 6.38 (1H, t, J=6Hz), 6.64 (1H, d, J=3Hz), 6.88 (1H, s), 7.13 (1H, s), 7.75 (2H, s)

| Anal. Calcd. for $C_{12}H_{13}N_6O_2SF_3$ : | | | |
|---|---|---|---|
| | C 39.78, | H 3.62, | N 23.19 |
| Found | C 39.93, | H 3.71, | N 23.15 |

Example 36

The following compound was obtained according to a similar manner to that of the latter half of Example 28.

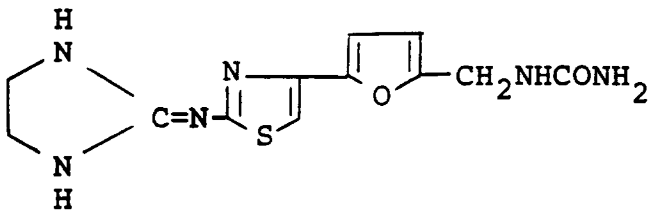

2-(Imidazolidin-2-ylideneamino)-4-(5-ureidomethylfuran-2-yl)thiazole.
mp : 245 to 247 ° C (dec.)
IR (Nujol : 3430, 3375, 3310, 1650, 1625, 1590 $cm^{-1}$
NMR (DMSO-$d_6$, δ) : 3.53 (4H, s), 4.21 (2H, d, J = 5Hz), 5.60 (2H, s), 6.27 (1H, s), 6.41 (1H, t, J = 5Hz), 6.78 (1H, s), 6.82 (1H, s), 7.57 (2H, s)

| Anal. Calcd. for $C_{12}H_{14}N_6O_2S$ : | | | |
|---|---|---|---|
| | C 47.05, | H 4.61, | N 27.43 |
| Found | C 46.91, | H 4.69, | N 26.99 |

Example 37

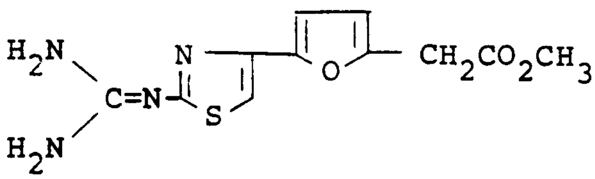

An ice-cooled mixture of 4-(5-cyanomethylfuran-2-yl)-2-(diaminomethyleneamino)thiazole (30.0 g) in dry methanol (250 ml) and chloroform (250 ml) was bubbled with hydrogen chloride for 2 hours and the mixture was stirred for 3 hours under ice-cooling. To a mixture was added a diisopropyl ether (300 ml) and the isolated precipitate was collected by filtration. To a precipitate was added a solution of methanol (100 ml) and water (200 ml) and a mixture was stirred for 30 minutes at ambient temperature.

The mixture was adjusted to pH 9.5 with potassium carbonate and the mixture was extracted with a mixture of tetrahydrofuran and ethyl acetate. The extract layer was washed with a brine and dried over magnesium sulfate. Evaporation of a solvent gave a residue, which was triturated with diisopropyl ether to give 2-(diaminomethyleneamino)-4-(5-methoxycarbonylmethylfuran-2-yl)thiazole (24.4 g).
mp : 187 to 193 ° C
IR (Nujol) : 3450, 3350, 3120, 1730, 1660, 1600, 1550 $cm^{-1}$
NMR (DMSO-$d_6$, δ) : 3.65 (3H, s), 3.83 (2H, s), 6.35 (1H, d, J = 3Hz), 6.62 (1H, d, J = 3Hz), 6.77 (1H, s), 6.91 (4H, s)

Example 38

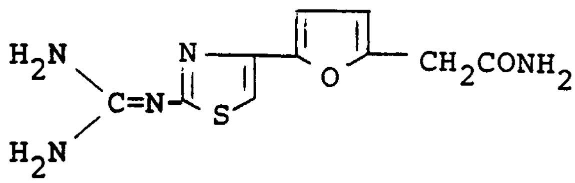

An ice-cooled solution of 2-(diaminomethyleneamino)-4-(5-methoxycarbonylmethylfuran-2-yl)thiazole (1.0 g) in methanol (20 ml) was bubbled with ammonia for 30 minutes and a mixture was stirred at ambient temperature for 25 hours. Evaporation of a solvent gave a residue, which was purified by column chromatography on silica gel eluting with a mixture of chloroform and methanol (9:1, V/V). The eluted fractions containing the desired product were collected and evaporated in vacuo. The residue was recrystallized from a mixture of ethanol, dioxane and diisopropyl ether to give 4-(5-carbamoylmethylfuran-2-yl)-2-diaminomethyleneamino)thiazole (0.44 g).
mp : 219 to 220 °C
IR (Nujol) : 3360, 3160, 3110, 1650, 1600, 1530 $cm^{-1}$
NMR (DMSO-$d_6$, $\delta$) : 3.51 (2H, s), 6.25 (1H, d, J=3Hz), 6.58 (1H, d, J=3Hz), 6.73 (1H, s), 6.87 (4H, s), 6.95 (1H, br s), 7.40 (1H, br s) Mass : m/e 265 ($M^+$)

| Anal. Calcd. for $C_{10}H_{11}N_5O_2S$ | | | |
|---|---|---|---|
| | C 45.28, | H 4.18, | N 26.40 |
| Found | C 44.77, | H 4.13, | N 26.23 |

Example 39

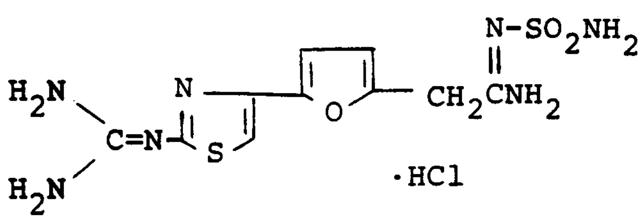

A solution of 4N-hydrogenchloride in 1,4-dioxane (2.2 ml) was added to a mixture of 4-[5-(2-amino-2-aminosulfonyliminoethyl)furan-2-yl]-2-(diaminomethyleneamino)thiazole (3.0 g) in methanol (60 ml) and the mixture was stirred at ambient temperature for 4 hours. To a mixture was added a diisopropyl ether (30 ml) and the isolated precipitate was collected by filtration. The precipitate was recrystallized from an aqueous ethanol to give 4-[5-(2-amino-2-aminosulfonyliminoethyl)furan-2-yl]-2-(diaminomethyleneamino)thiazole hydrochloride (2.17 g).
mp : 219 to 221 °C
IR (Nujol) : 3380, 3340, 1680, 1640, 1615, 1550, 1505 $cm^{-1}$
NMR (DMSO-$d_6$, $\delta$) : 3.63 (2H, s), 6.38 (1H, d, J=3Hz), 6.54 (1H, br s), 6.94 (1H, d, J=3Hz), 7.20 (1H, s), 7.31 (1H, s), 8.32 (5H, br s)

| Anal. Calcd. for $C_{10}H_{13}N_7O_3S_2 \cdot HCl$ | | | | |
|---|---|---|---|---|
| | C 31.62, | H 3.71, | N 25.81, | Cl 9.33 |
| Found | C 31.02, | H 3.71, | N 25.53, | Cl 9.43 |

Example 40

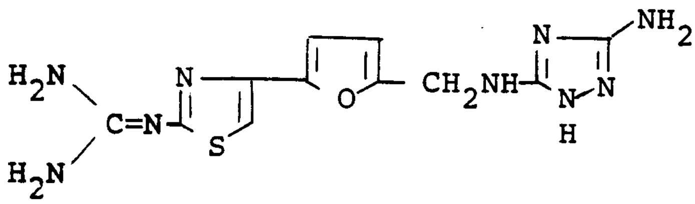

A mixture of 2-(diaminomethyleneamino)-4-[5-(3-cyano-2-methyl-1-isothioureido)methylfuran-2-yl]-thiazole (0.80 g) and hydrazine hydrate (1.2 g) in ethanol (10 ml) was stirred at ambient temperature for two hours. After cooling, the resulting precipitate was collected by filtration and recrystallized from aqueous N,N-dimethylformamide to afford 4-[5-{(3-amino-1H-1,2,4-triazol-5-yl)aminomethyl}furan-2-yl]-2-(diaminomethyleneamino)thiazole (0.52 g).

mp : 241 to 242 °C

IR (Nujol) : 3570, 3470, 3360, 3295, 1630 $cm^{-1}$

NMR (DMSO-$d_6$, $\delta$) : 4.23 (2H, d, J=6Hz), 5.34 (2H, br s), 5.93 (1H, br s), 6.23 (1H, d, J=3Hz), 6.55 (1H, d, J=3Hz), 6.73 (1H, s), and 6.85 (4H, br s)

| Anal. Calcd. for $C_{11}H_{13}N_9OS \cdot H_2O$ : | | | | |
|---|---|---|---|---|
| | C 39.16, | H 4.48, | N 37.37, | $H_2O$ 5.34 |
| Found | C 38.94, | H 4.30, | N 37.30, | $H_2O$ 5.70 |

Example 41

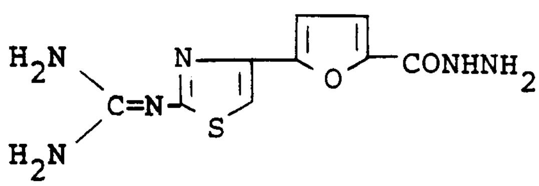

A solution of 2-(diaminomethyleneamino)-4-(5-methoxycarbonylfuran-2-yl)thiazole (5.00 g) and hydrazine hydrate (9.40 g) in ethanol (50 ml) was refluxed for 4 hours with stirring. The mixture was cooled and the resulting precipitate was collected by filtration to afford 2-(diaminomethyleneamino)-4-(5-hydrazinocarbonylfuran-2-yl)thiazole (4.86 g).

mp : 279 to 280 °C

IR (Nujol) : 3400, 3360, 3320, 3250, 3175, 3125, 1660, 1630 $cm^{-1}$

NMR (DMSO-$d_6$, $\delta$) : 4.53 (2H, br s), 6.85 (1H, d, J=3Hz), 6.98 (4H, br s), 7.18 (1H, d, J=3Hz), 7.27 (1H, s) and 9.77 (1H, br s)

Example 42

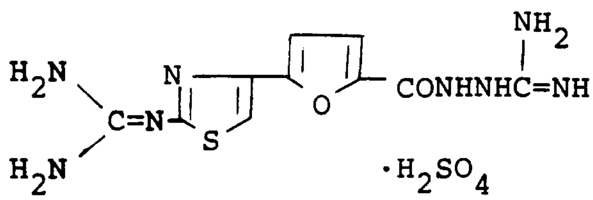

A solution of 2-(diaminomethyleneamino)-4-(5-hydrazinocarbonylfuran-2-yl)thiazole (1.50 g) and S-methylisothiourea hemisulfate (1.57 g) in dimethyl sulfoxide (12 ml) was heated at 180 °C for two hours with stirring. After cooling, the resulting precipitate was collected by filtration and washed with dimethyl sulfoxide followed by washing with isopropyl alcohol and diisopropyl ether to afford 2-(diaminomethyleneamino)-4-(5-guanidinocarbamoylfuran-2-yl)thiazole sulfate (1.83 g).
mp : >300 °C
IR (Nujol) : 3325, 3150, 3110, 1690, 1670 $cm^{-1}$

| Anal. Calcd. for $C_{10}H_{12}N_8O_2S \cdot H_2SO_4$ : | | | |
|---|---|---|---|
| | C 29.55, | H 3.47, | N 27.57 |
| Found | C 28.07, | H 3.72, | N 28.22 |

Example 43

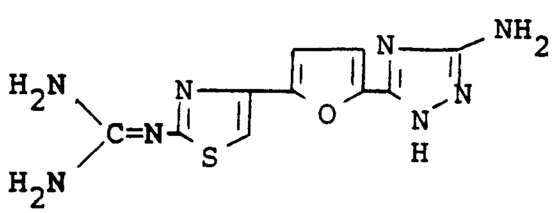

A suspension of 2-(diaminomethyleneamino)-4-(5-guanidinocarbamoylfuran-2-yl)thiazole sulfate (1.70 g) in concentrated ammonium hydroxide (17 ml) was refluxed with stirring. Additional ammonium hydroxide (8.5 ml) was added twice. After refluxing for 8 hours, water was added to the mixture and the resulting precipitate was collected by filtration, followed by recrystallization from aqueous N,N-dimethylformamide to afford 4-[5-(3-amino-1H-1,2,4-triazol-5-yl)furan-2-yl]-2-(diaminomethyleneamino)thiazole (0.73 g).
mp : >300 °C
IR (Nujol) : 3440, 3400, 3330, 3120, 1670, 1640 $cm^{-1}$
NMR (DMSO-$d_6$, δ) : 6.00 (2H, s), 6.76 (2H, s), 6.87 (1H, s), 6.92 (4H, s)

| Anal. Calcd. for $C_{10}H_{10}N_8OS \cdot 1/4H_2O$ | | | | |
|---|---|---|---|---|
| | C 40.74, | H 3.59, | N 38.17, | $H_2O$ 1.53 |
| Found | C 40.48, | H 3.55, | N 37.61, | $H_2O$ 1.25 |

Example 44

The following compounds were obtained according to a similar manner to that of Example 30.

(1)

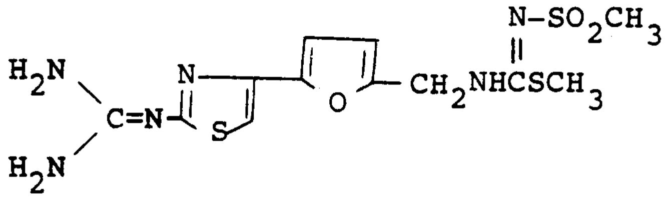

2-(Diaminomethyleneamino)-4-[5-(3-mesyl-2-methyl-1-isothioureido)methylfuran-2-yl]thiazole.
mp : 210 to 211 °C
IR (Nujol) : 3420, 3320, 1655, 1350, 1120 $cm^{-1}$
NMR (DMSO-$d_6$, $\delta$) : 2.53 (3H, s), 3.00 (3H, s), 4.58 (2H, s), 6.45 (1H, d, J=3Hz), 6.72 (1H, d, J=3Hz), 6.85 (1H, s), 6.95 (4H, s) and 8.50 (1H, br s)

| Anal. Calcd. for $C_{12}H_{16}N_6O_3S_2$ | | | |
|---|---|---|---|
| | C 37.10, | H 4.15, | N 21.63 |
| Found | C 37.42, | H 4.36, | N 21.76 |

(2)

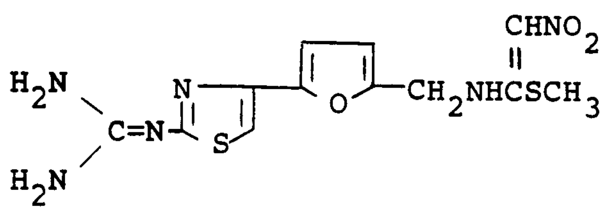

2-(Diaminomethyleneamino)-4-[5-{N-(1-methylthio-2-nitrovinyl)aminomethyl}furan-2-yl]thiazole.
mp: 214 °C (dec.)
IR (Nujol) : 3350, 1660, 1610, 1550 $cm^{-1}$
NMR (DMSO-$d_6$, $\delta$) : 2.45 (3H, s), 4.65 (2H, d, J=5.4Hz), 6.38 (1H, d, J=3.6Hz), 6.65-6.57 (2H, m), 6.72 (1H, s), 6.76 (4H, s), 10.55 (1H, br)

Example 45

The following compounds were obtained according to a similar manner to that of Example 32.

(1)

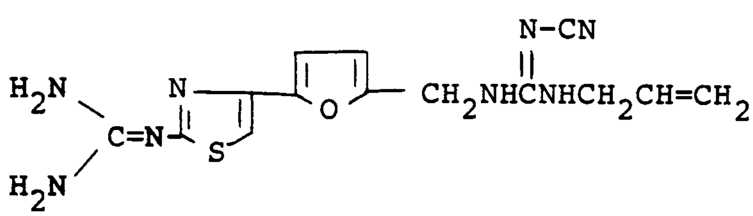

4-[5-(3-Allyl-2-cyanoguanidino)methylfuran-2-yl]-2-(diaminomethyleneamino)thiazole.
mp : 164 to 165 °C
IR (Nujol) : 3425, 3240, 2160, 1645 $cm^{-1}$
NMR (DMSO-$d_6$, $\delta$) : 3.78 (2H, t, J=6Hz), 4.36 (2H, d, J=6Hz), 5.01 (1H, d, J=1.5Hz), 5.16 (1H, dd, J=1.5Hz and 8Hz), 5.62-6.03 (1H, m), 6.28 (1H, d, J=3Hz), 6.61 (1H, d, J=3Hz), 6.74 (1H, s), 6.86 (4H, s) and 7.14-7.46 (2H, m)

| Anal. Calcd. for $C_{14}H_{16}N_8OS$ : | | | |
|---|---|---|---|
| | C 48.83, | H 4.68, | N 32.54 |
| Found | C 48.86, | H 4.41, | N 32.58 |

(2)

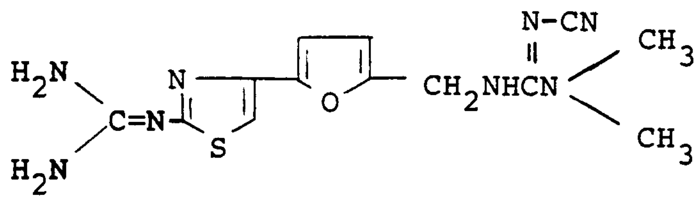

4-[5-(2-Cyano-3,3-dimethylguanidino)methylfuran-2-yl]-2-(diaminomethyleneamino)thiazole.
mp : 205 to 206 °C
IR (Nujol) : 3350, 2160, 1650 $cm^{-1}$
NMR (DMSO-$d_6$, $\delta$) : 2.99 (6H, s), 4.51 (2H, d, J=5.6Hz), 6.36 (1H, d, J=3.2Hz), 6.63 (1H, d, J=3.2Hz), 6.78 (1H, s), 6.91 (4H, s), 7.54 (1H, t, J=5.6Hz)

| Anal. Calcd. for $C_{13}H_{16}N_8OS$ | | | |
|---|---|---|---|
| | C 46.98, | H 4.85, | N 33.71 |
| Found | C 47.14, | H 4.92, | N 33.66 |

(3)

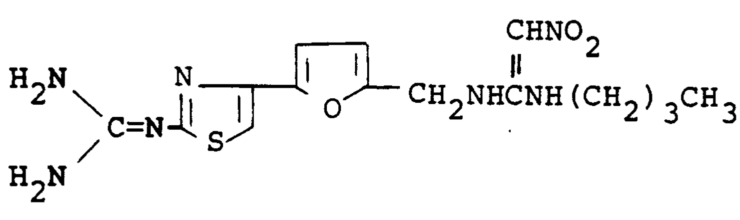

4-[5-{N-(1-n-Butylamino-2-nitrovinyl)aminomethyl}furan-2-yl]-2-(diaminomethyleneamino)thiazole.
mp : 215 to 217 °C
IR (Nujol) : 3360, 1610, 1550, 1370 $cm^{-1}$
NMR (DMSO-$d_6$, $\delta$) : 0.89 (3H, t, J=7.2Hz), 1.53-1.27 (2H, m), 2.52-2.48 (2H, m), 3.22 (2H, br), 4.43 (2H, br), 6.41 (1H, d, J=3.2Hz), 6.65-6.64 (2H, m), 6.76 (1H, s), 6.89 (4H, s), 7.75 (1H, br), 10.17 (1H, br)

| Anal. Calcd. for $C_{15}H_{21}N_7O_3S$ | C 47.48, | H 5.58, | N 25.84 |
|---|---|---|---|
| Found | C 47.44, | H 5.71, | N 25.97 |

(4)

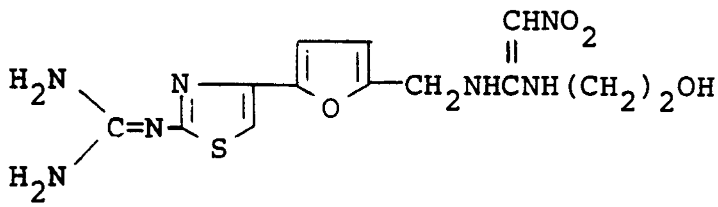

2-(Diaminomethyleneamino)-4-[5-[N-{1-(2-hydroxyethyl)amino-2-nitrovinyl}aminomethyl]furan-2-yl]-thiazole.
mp : 182 to 183 ° C (dec.)
IR (Nujol) : 3330, 1610, 1550, 1380 $cm^{-1}$
NMR (DMSO-$d_6$, δ) : 3.65-3.00 (4H, m), 4.34 (2H, d, J = 4.8Hz), 5.10-4.60 (1H, br), 6.29 (1H, d, J = 3.0Hz), 6.52 (2H, s), 6.67 (1H, s), 6.75 (4H, s), 7.90-7.30 (1H, br), 10.30-9.30 (1H, br)

| Anal. Calcd. for $C_{13}H_{17}N_7O_4S \cdot 3/4H_2O$ : | C 40.99, | H 4.90, | N 25.74 |
|---|---|---|---|
| Found | C 41.04, | H 5.13, | N 26.04 |

(5)

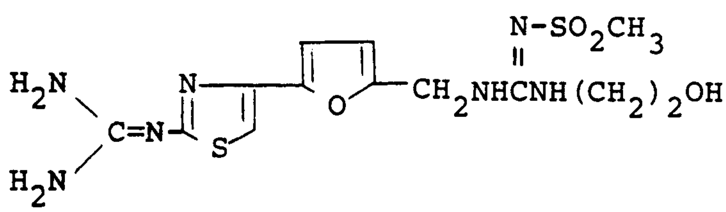

2-(Diaminomethyleneamino)-4-[5-{3-(2-hydroxyethyl)-2-mesylguanidino}methylfuran-2-yl]thiazole.
mp : 202 to 203 ° C
IR (Nujol) : 3570, 3410, 3340, 3220, 3120, 1610, 1375, 1360, 1340, 1100 $cm^{-1}$
NMR (DMSO-$d_6$, δ) : 2.80 (3H, s), 3.14-3.26 (2H, m), 3.43-3.60 (2H, m), 4.39 (2H, d, J = 6Hz) 4.91 (1H, t, J = 5Hz), 6.34 (1H, d, J = 3Hz), 6.62 (1H, d, J = 3Hz), 6.78 (1H, s), 6.86 (4H, s), 7.21 (1H, t, J = 5Hz) and 7.49 (1H, t, J = 6Hz)

| Anal. Calcd. for $C_{13}H_{19}N_7O_4S_2$ : | | | |
|---|---|---|---|
| | C 38.89, | H 4.77, | N 24.42 |
| Found | C 38.89, | H 5.20, | N 24.22 |

(6)

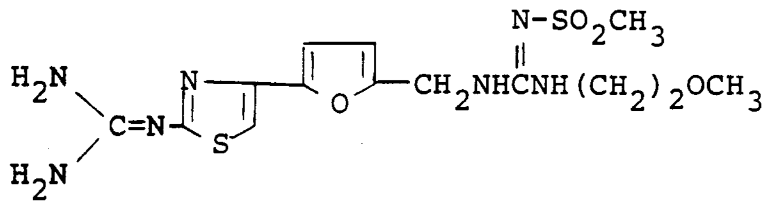

2-(Diaminomethyleneamino)-4-[5-{2-mesyl-3-(2-methoxyethyl)guanidino}methylfuran-2-yl]thiazole.
mp : 173 to 174 °C
IR (Nujol) : 3430, 3400, 3340, 1360, 1110 $cm^{-1}$
NMR (DMSO-$d_6$, δ) : 2.77 (3H, s), 3.23 (3H, s), 3.30-3.47 (4H, m), 4.39 (2H, d, J=6Hz), 6.31 (1H, d, J=3Hz), 6.60 (1H, d, J=3Hz), 6.75 (1H, s), 6.87 (4H, s), 7.20 (1H, t, J=6Hz) and 7.49 (1H, t, J=6Hz)

| Anal. Calcd. for $C_{14}H_{21}N_7O_4S_2$ : | | | |
|---|---|---|---|
| | C 40.47, | H 5.09, | N 23.60 |
| Found | C 40.56, | H 4.94, | N 23.64 |

(7)

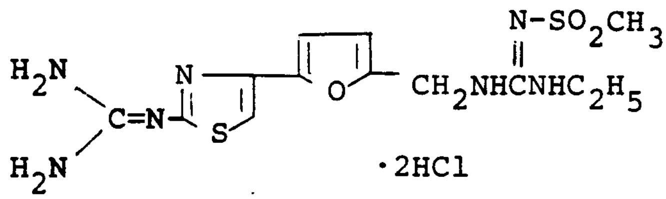

2-(Diaminomethyleneamino)-4-[5-(3-ethyl-2-mesylguanidino)methylfuran-2-yl]thiazole.
mp : 151 to 152 °C
IR (Nujol) : 3380, 3230, 1680, 1650, 1340, 1160 $cm^{-1}$
NMR (DMSO-$d_6$, δ) : 1.09 (3H, t, J=7Hz), 2.78 (3H, s), 3.13-3.26 (2H, m), 4.43 (2H, d, J=5Hz), 6.40 (1H, d, J=3Hz), 7.00 (1H, d, J=3Hz), 7.18 (1H, br s), 7.30 (1H, s), 7.75 (1H, br s), 8.37 (4H, s) and 12.80 (1H, br s)

| Anal. Calcd. for $C_{13}H_{19}N_7O_3S_2 \bullet 2HCl \bullet 1/3H_2O$ | C 33.62, | H 4.70, | N 21.11, | Cl 15.27, | $H_2O$ 1.29 |
|---|---|---|---|---|---|
| Found | C 33.75, | H 4.53, | N 21.05, | Cl 15.06, | $H_2O$ 1.18 |

(8)

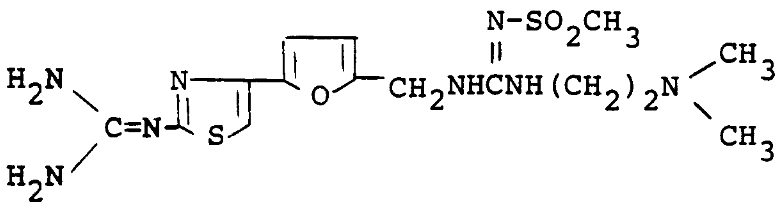

2-(Diaminomethyleneamino)-4-[5-{3-(2-dimethylaminoethyl)-2-mesylguanidino}methylfuran-2-yl]thiazole.
mp : 183 to 184 °C
IR (Nujol) : 3450, 3410, 3340, 1650, 1650, 1330, 1110 $cm^{-1}$
NMR (DMSO-$d_6$, $\delta$) : 2.13 (6H, s), 2.37 (2H, t, J = 6Hz), 2.78 (3H, s), 3.19-3.27 (2H, m), 4.37 (2H, d, J = 5Hz), 6.36 (1H, d, J = 3Hz), 6.63 (1H, d, J = 3Hz), 6.78 (1H, s), 6.90 (4H, br s), 6.90 (1H, t, J = 5Hz) and 8.14 (1H, br s)

| Anal. Calcd. For $C_{15}H_{24}N_8O_3S_2$ : | C 42.04, | H 5.64, | N 26.15 |
|---|---|---|---|
| Found | C 42.32, | H 5.61, | N 26.31 |

(9)

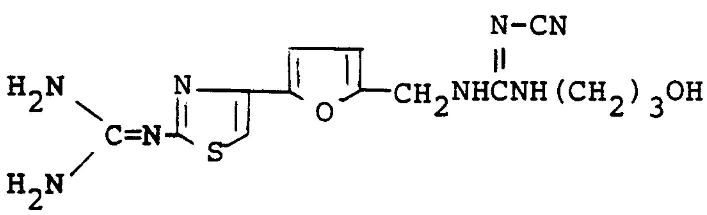

4-[5-{2-Cyano-3-(3-hydroxypropyl)guanidino}methylfuran-2-yl]-2-(diaminomethyleneamino)thiazole.
mp : 160 to 161 °C
IR (Nujol) : 3350, 2040, 1650 $cm^{-1}$
NMR (DMSO-$d_6$, $\delta$) : 1.56-1.69 (2H, m), 3.20 (2H, q, J = 6Hz), 3.43 (2H, q, J = 5Hz), 4.35 (2H, d, J = 5.5Hz), 4.53 (1H, t, J = 5Hz), 6.30 (1H, d, J = 3Hz), 6.62 (1H, d, J = 3Hz), 6.76 (1H, s), 6.90 (4H, br s), 7.09 (1H, t, J = 5.5Hz) and 7.47 (1H, t, J = 6Hz)

Example 46

The following compound was obtained according to similar manners to those of Example 30 and Example 32, continuously.

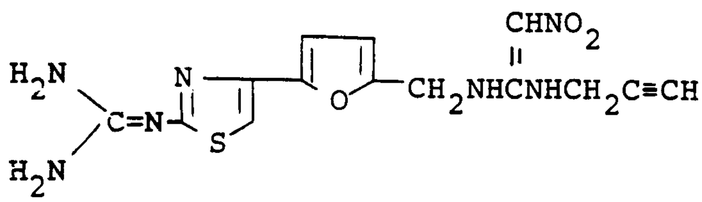

2-(Diaminomethyleneamino)-4-[5-{N-(2-nitro-1-propargylaminovinyl)aminomethyl}furan-2-yl]thiazole.
mp : 199 to 200 °C (dec.)
IR (Nujol) : 3430, 1620, 1560, 1370 $cm^{-1}$
NMR (DMSO-$d_6$, $\delta$) : 3.38-3.30 (1H, m), 4.20-3.80 (2H, m), 4.45 (2H, d, J=5.4Hz), 6.35 (1H, d, J=3.3Hz), 6.55 (1H, s), 6.58 (1H, d, J=3.3Hz), 6.71 (1H, s), 6.78 (4H, s), 8.00 (1H, br), 10.00 (1H, br)

| Anal. Calcd. for $C_{14}H_{15}N_7O_3S$ : | | | |
|---|---|---|---|
| Found | C 46.53,<br>C 46.18, | H 4.18,<br>H 4.92, | N 27.13<br>N 26.83 |

Example 47

The following compound was obtained according to a similar manner to that of Example 37.

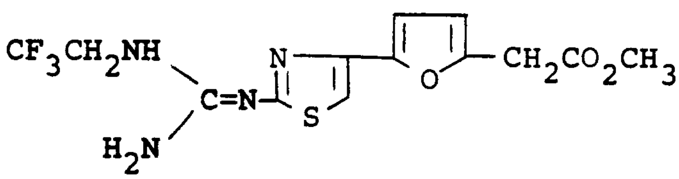

2-[(Amino){(2,2,2-trifluoroethyl)amino}methyleneamino]-4-(5-methoxycarbonylmethylfuran-2-yl)thiazole.
mp : 126 to 127 °C
IR (Nujol) : 3410, 1715, 1630, 1550 $cm^{-1}$
NMR (DMSO-$d_6$, $\delta$) : 3.66 (3H, s), 3.84 (2H, s), 4.01-4.22 (2H, m), 6.36 (1H, d, J=3Hz), 6.66 (1H, d, J=3Hz), 6.89 (1H, s), 7.14 (1H, br s), 7.76 (2H, s)

Example 48

The following compounds were obtained according to a similar manner to that of Example 38.

(1)

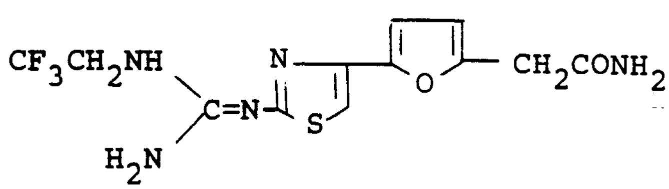

2-[(Amino){(2,2,2-trifluoroethyl)amino}methyleneamino]-4-(5-carbamoylmethylfuran-2-yl)thiazole.
mp : 163 to 165 °C
IR (Nujol) : 3350, 3190, 1660, 1620, 1540 $cm^{-1}$
NMR (DMSO-$d_6$, $\delta$) : 3.50 (2H, s), 3.90-4.27 (2H, m), 6.27 (1H, d, J=3Hz), 6.63 (1H, d, J=3Hz), 6.86 (1H, s), 6.94-7.30 (2H, m), 7.47 (1H, s), 7.75 (2H, br s)

| Anal. Calcd. for $C_{12}H_{12}N_5O_2SF_3$ : | | | |
|---|---|---|---|
| | C 41.50, | H 3.48, | N 20.16 |
| Found | C 41.10, | H 3.45, | N 19.51 |

(2)

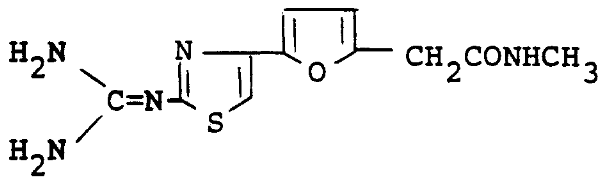

2-(Diaminoethyleneamino)-4-(5-methylcarbamoylmethylfuran-2-yl)thiazole.

mp : 212 to 213 ° C

IR (Nujol) : 3420, 3350, 1660, 1610, 1540 $cm^{-1}$

NMR (DMSO-$d_6$, $\delta$) : 2.61 (3H, d, J=5Hz), 3.51 (2H, s), 6.22 (1H, d, J=3Hz), 6.56 (1H, d, J=3Hz), 6.71 (1H, s), 6.84 (4H, s), 7.75 (1H, br s)

Mass : m/e 279 ($M^+$)

| Anal. Calcd. for $C_{11}H_{13}N_5O_2S$ : | | | |
|---|---|---|---|
| | C 47.30, | H 4.69, | N 25.07 |
| Found | C 47.54, | H 4.37, | N 25.43 |

Example 49

The following compound was obtained according to a similar manner to that of Example 39.

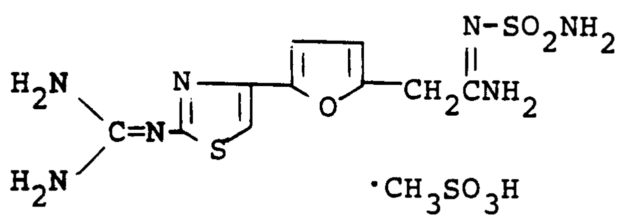

4-[5-(2-Amino-2-aminosulfonyliminoethyl)furan-2-yl]-2-(diaminomethyleneamino)thiazole methanesulfonate.

mp : 221 to 223 ° C (dec.)

IR (Nujol) : 3400, 3300, 3180, 3110, 1690, 1630, 1575 $cm^{-1}$

NMR (DMSO-$d_6$, $\delta$) : 2.53 (3H, s), 3.65 (2H, s), 6.40 (1H, d, J=3Hz), 6.56 (1H, br s), 6.95 (1H, d, J=3Hz), 7.31 (1H, s), 7.41 (1H, br s), 8.31 (4H, s)

| Anal. Calcd. for $C_{10}H_{13}N_7O_3S_2 \cdot CH_3SO_3H$ : | | | | |
|---|---|---|---|---|
| | C 30.06, | H 3.90, | N 22.31, | S 21.89 |
| Found | C 30.03, | H 3.91, | N 22.54, | S 21.87 |

Example 50

The following compound was obtained according to a similar manner to that of Example 29.

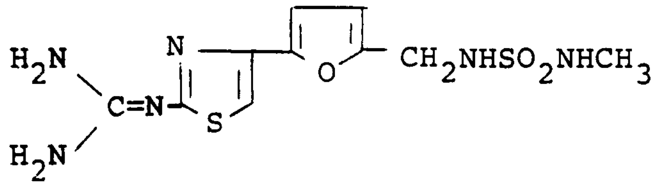

2-(Diaminomethyleneamino)-4-(5-methylsulfamoylaminomethylfuran-2-yl)thiazole.
mp : 197 °C
IR (Nujol) : 3420, 3275, 1600 $cm^{-1}$
NMR (DMSO-$d_6$, δ) : 2.41 (3H, d, J=5.5Hz), 3.98 (2H, d, J=6.0Hz), 6.32 (1H, d, J=3.0Hz), 6.55 (1H, d, J=3.0Hz), 6.62-6.72 (1H, m), 6.73 (1H, s), 6.82 (4H, s), 7.30 (1H, t, J=6.0Hz)

| Anal. Calcd. for $C_{10}H_{14}N_6O_3S_2$ : | | | |
|---|---|---|---|
| | C 36.35, | H 4.27, | N 25.44 |
| Found | C 36.38, | H 4.26, | N 25.24 |

Example 51

The following compound was obtained according to a similar manner to that of Example 41.

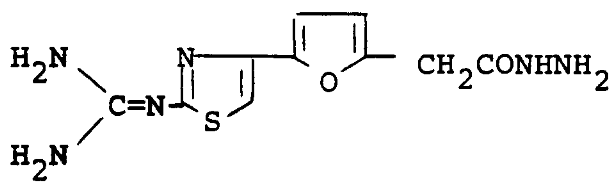

2-(Diaminomethyleneamino)-4-(5-hydrazinocarbonylmethylfuran-2-yl)thiazole.
mp : 222 to 223 °C
IR (Nujol) : 3450, 3350, 3200, 1645, 1605, 1540 $cm^{-1}$
NMR (DMSO-$d_6$, δ) : 3.53 (2H, s), 4.35 (2H, br s), 6.31 (1H, d, J=3Hz), 6.64 (1H, d, J=3Hz), 6.77 (1H, s), 6.94 (4H, s), 9.25 (1H, s)

Example 52

The following compound was obtained according to a similar manner to that of Example 42.

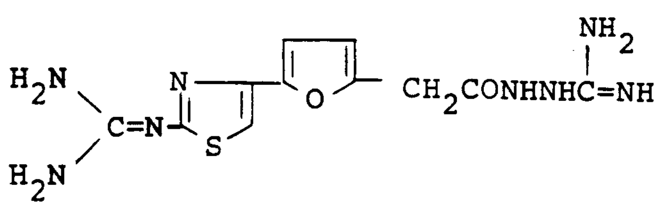

2-(Diaminomethyleneamino)-4-(5-guanidinocarbamoylmethylfuran-2-yl)thiazole.
mp : 250 to 251 °C
IR (Nujol) : 3300, 3160, 1650, 1600, 1540 $cm^{-1}$

NMR (DMSO-$d_6$, δ) : 3.43 (2H, s), 6.22 (1H, d, J=3Hz), 6.60 (1H, d, J=3Hz), 6.73 (1H, s), 6.93 (4H, s)

Example 53

The following compound was obtained according to a similar manner to that of Example 43.

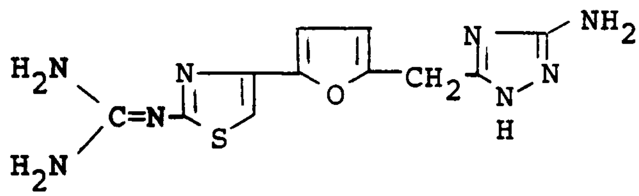

4-[5-(3-Amino-1H-1,2,4-triazol-5-yl)methylfuran-2-yl]-2-(diaminomethyleneamino)thiazole.
mp : 259 to 261 °C (dec.)
IR (Nujol) : 3410, 3120, 1660, 1640, 1600, 1550 $cm^{-1}$
NMR (DMSO-$d_6$, δ) : 3.83 (2H, s), 5.71 (2H, br s), 6.18 (1H, d, J=3Hz), 6.56 (1H, d, J=3Hz), 6.70 (1H, s), 6.86 (4H, s)

| Anal. Calcd. for $C_{11}H_{12}N_8OS \cdot 1/2H_2O$ : | | | | |
|---|---|---|---|---|
| | C 42.17, | H 4.18, | N 35.76, | $H_2O$ 2.87 |
| Found | C 42.12, | H 4.12, | N 35.96, | $H_2O$ 3.00 |

Example 54

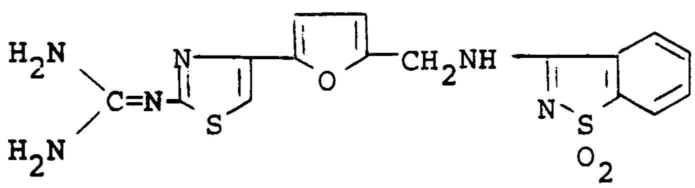

An ice-cooling mixture of 4-(5-aminomethylfuran-2-yl)-2-(diaminomethyleneamino)thiazole (0.8 g), 3-chlorobenzoisothiazol-1,1-dioxide (0.8 g) and triethylamine (0.5 ml) in N,N-dimethylformamide (15 ml) was stirred for 3 hours. To a reaction mixture was added a mixture of ethyl acetate, tetrahydrofuran and water and the mixture was adjusted to pH 9.5 with 20% aqueous potassium carbonate. The separated organic layer was washed with brine and dried over magnesium sulfate. The solvent was removed by concentration and a residue was recrystallized from a mixture of methanol, dioxane and diisopropyl ether to give 2-(diaminomethyleneamino)-4-[5-(1,1-dioxobenzoisothiazol-3-yl)aminomethylfuran-2-yl]thiazole (0.49 g).
mp : 255 to 256 °C (dec.)
IR (Nujol) : 3430, 3320, 1660, 1620, 1590, 1535 $cm^{-1}$
NMR (DMSO-$d_6$, δ) : 4.73 (2H, s), 6.51 (1H, d, J=3Hz), 6.67 (1H, d, J=3Hz), 6.82 (1H, s), 6.88 (4H s), 7.63-8.06 (3H, m), 8.09-8.40 (1H, m), 9.89 (1H, br s)

| Anal. Calcd. for $C_{16}H_{14}N_6O_3S_2 \cdot 1/3H_2O$ : | | | | |
|---|---|---|---|---|
| | C 47.05, | H 3.62, | N 20.49, | $H_2O$ 1.47 |
| Found | C 47.04, | H 4.28, | N 20.49, | $H_2O$ 1.71 |

Example 55

The following compound was obtained according to a similar manner to that of Example 54.

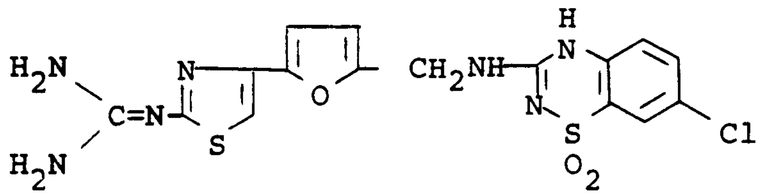

4-[5-(7-Chloro-1,1-dioxo-4H-1,2,4-benzothiadiazin-3-yl)aminomethylfuran-2-yl]-2-(diaminomethyleneamino)thiazole.
mp : 220 to 221 °C
IR (Nujol) : 3325, 1690, 1630 $cm^{-1}$
NMR ($DMSO-d_6$, δ) : 4.49 (2H, d, J=4.5Hz), 6.42 (1H, d, J=3Hz), 6.71 (1H, d, J=3Hz), 6.94 (1H, s), 7.24 (4H, br s), 7.26 (1H, d, J=8.5Hz), 7.63 (1H, dd, J=2Hz and 8.5Hz), 7.69 (1H, d, J=2Hz), 7.95 (1H, br s) and 11.07 (1H, br s)

| Anal. Calcd. for $C_{16}H_{14}ClN_7O_3S_2 \cdot 1.5H_2O$ : | | | |
|---|---|---|---|
| | C 40.13, | H 3.58, | N 20.47 |
| Found | C 40.10, | H 3.56, | N 20.61 |

Example 56

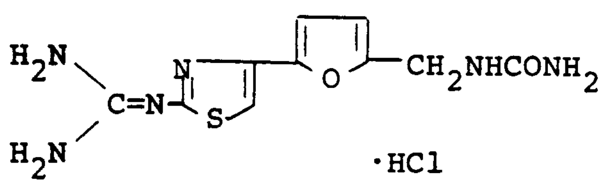

To a mixture of 2-(diaminomethyleneamino)-4-(5-ureidomethylfuran-2-yl)thiazole (15.4 g) in methanol (160 ml) was added a solution of 19% (W/V) hydrogen chloride in methanol (31.6 ml) and the mixture was stirred for an hour at ambient temperature. To a reaction mixture was added a isopropyl ether and the isolated precipitate was collected by filtration. The precipitate was recrystallized from an aqueous ethanol to give 2-(diaminomethyleneamino)-4-(5-ureidomethylfuran-2-yl)thiazole hydrochloride (6.82 g).
mp : 221 °C
IR (Nujol) : 3400, 3300, 1695, 1655, 1620, 1590, 1565 $cm^{-1}$
NMR ($DMSO-d_6$, δ) : 4.23 (2H, d, J=5Hz), 5.67 (2H, s), 6.32 (1H, d, J=3Hz), 6.55 (1H, t, J=5Hz), 6.97 (1H, d, J=3Hz), 7.31 (1H, s), 8.35 (4H, s)

| Anal. Calcd. for $C_{10}H_{12}N_6O_2S \cdot HCl \cdot H_2O$ : | | | | | |
|---|---|---|---|---|---|
| | C 35.88, | H 4.22, | N 25.10, | Cl 10.58, | $H_2O$ 5.38 |
| Found | C 35.77, | H 4.47, | N 25.08, | Cl 10.79, | $H_2O$ 5.63 |

Example 57

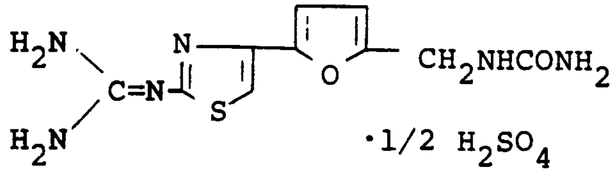

A solution of conc. sulfuric acid (0.7 ml) in methanol (10 ml) was added to a mixture of 2-(diaminomethyleneamino)-4-(5-ureidomethylfuran-2-yl)thiazole (4.0 g) in methanol (60 ml) and the mixture was stirred for an hour at ambient temperature. The isolated precipitate was collected by filtration and the precipitate was recrystallized from an aqueous ethanol to give 2-(diaminomethyleneamino)-4-(5-ureidomethylfuran-2-yl)thiazole hemisulfate (4.26 g).

mp : 170 to 172 °C

IR (Nujol) : 3400, 3290, 1690, 1650, 1620, 1580, 1565, 1510 $cm^{-1}$

NMR (DMSO-$d_6$, $\delta$) : 4.21 (2H, d, J = 6Hz), 5.61 (2H, s), 6.29 (1H, d, J = 3Hz), 6.45 (1H, t, J = 6Hz), 6.80 (1H, d, J = 3Hz), 7.09 (1H, s), 7.86 (4H, s)

Example 58

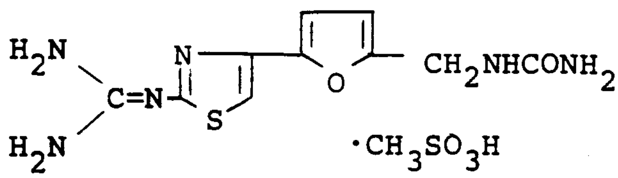

Methanesulfonic acid (0.93 ml) was added to a mixture of 2-(diaminomethyleneamino)-4-(5-ureidomethylfuran-2-yl)thiazole (4.0 g) in methanol (60 ml) and the mixture was stirred for an hour at ambient temperature. The isolated precipitate was collected by filtration and the precipitate was recrystallized from a solution of an aqueous ethanol to give 2-(diaminomethyleneamino)-4-(5-ureidomethylfuran-2-yl)-thiazole methanesulfonate (2.77 g) .

mp : 185 to 186 °C

IR (Nujol) : 3325, 3125, 1690, 1640 (br), 1545, 1510, 1045 $cm^{-1}$

NMR (DMSO-$d_6$, $\delta$) : 2.48 (3H, s), 4.22 (2H, d, J = 5Hz), 5.62 (2H, s), 6.32 (1H, d, J = 3Hz), 6.48 (1H, t, J = 5Hz), 6.95 (1H, d, J = 3Hz), 7.32 (1H, s), 8.34 (4H, s), 12.12 (1H, s)

| Anal. Calcd. for $C_{10}H_{12}N_6O_2S \cdot CH_3SO_3H$ : | | | |
|---|---|---|---|
| | C 35.10, | H 4.28, | N 22.33 |
| Found | C 34.96, | H 4.22, | N 22.35 |

Example 59

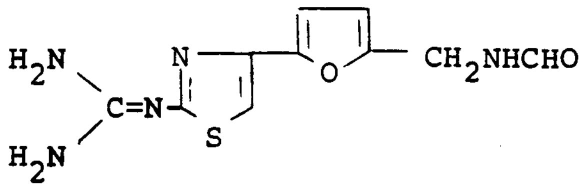

A mixture of acetic anhydride (2.4 ml) and formic acid (1.0 ml) was stirred for 30 minutes at 50 to 60 °C. The above mixture was added to a mixture of 4-(5-aminomethylfuran-2-yl)-2-(diaminomethyleneamino)thiazole (1.5 g) in tetrahydrofuran (15 ml) and N,N-dimethylformamide (15 ml) at ambient temperature and the mixture was stirred for 2.5 hours at ambient temperature. To the reaction mixture was added a mixture of ethyl acetate, tetrahydrofuran and water and the mixture was adjusted to pH 9.5 with 20% aqueous potassium carbonate. The separated organic layer was washed with a brine and dried over magnesium sulfate. The solvent was removed by concentration in vacuo and the residue was recrystallized from a mixture of methanol, dioxane and tetrahydrofuran to give 2-(diaminomethyleneamino)-4-(5-formamidomethylfuran-2-yl)thiazole (0.80 g).

mp : 179 to 181 °C

IR (Nujol) : 3350, 3310, 3130, 1650, 1530 $cm^{-1}$

NMR (DMSO-$d_6$, δ) : 4.36 (2H, d, J=6Hz), 6.37 (1H, d, J=3Hz), 6.78 (1H, d, J=3Hz), 7.11 (1H, s), 8.11 (1H, s), 8.56 (4H, s)

Example 60

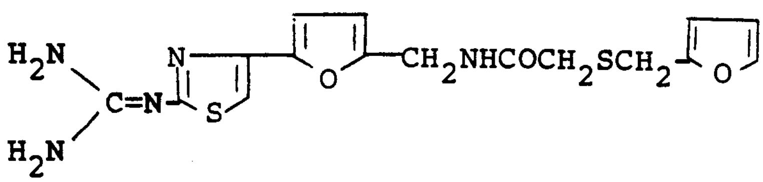

The mixture of 4-(5-aminomethylfuran-2-yl)-2-(diaminomethyleneamino)thiazole (4.0 g), furfurylthioacetic acid (3.4 g) and 1-(3-dimethylaminopropyl)-3-ethylcarbodiimide hydrochloride (4.2 g) in N,N-dimethylformamide (40 ml) was stirred for 15 hours at ambient temperature. The mixture was added a mixture of ethyl acetate, tetrahydrofuran and water and the mixture was adjusted to pH 9.5 with potassium carbonate. The separated organic layer was washed with a brine and dried over magnesium sulfate. Evaporation of a solvent gave a residue, which was purified by column chromatography on silica gel eluting with a mixture of ethyl acetate and methanol (9:1, V/V). The eluted fractions containing the desired product were collected and evaporated in vacuo. The residue was recrystallized from a mixture of methanol, dioxane and isopropyl ether to give 2-(diaminomethyleneamino)-4-[5-{(furan-2-yl)methylthio}acetamidomethylfuran-2-yl]thiazole (3.50 g).

mp : 180 to 181 °C

IR (Nujol) : 3400, 3190, 1655, 1630, 1610, 1530 $cm^{-1}$

NMR (DMSO-$d_6$, δ) : 3.14 (2H, s), 3.85 (2H, s), 4.30 (2H, d, J=5Hz), 6.19-6.44 (3H, m), 6.59 (1H, d, J=3Hz), 6.74 (1H, s), 6.87 (4H, s), 7.55 (1H, s), 8.48 (1H, t, J=5Hz)

| Anal. Calcd. for $C_{16}H_{17}N_5O_3S_2$ : | | | |
|---|---|---|---|
| | C 49.09, | H 4.38, | N 17.89 |
| Found | C 49.25, | H 4.40, | N 17.98 |

Example 61

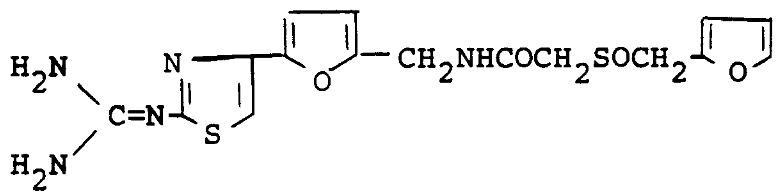

A mixture of 2-(diaminomethyleneamino)-4-[5-{(furan-2-yl)methylthio}acetamidomethylfuran-2-yl]thiazole (1.5 g) and 3-chloroperbenzoic acid (0.9 g) in a mixture of tetrahydrofuran (30 ml) and N,N-dimethylformamide (10 ml) was stirred for 1.5 hours at ambient temperature. To the reaction mixture was added a solution of water and ethyl acetate and the mixture was adjusted to pH 9.5 with 20% aqueous potassium carbonate. The separated organic layer was washed with brine and dried over magnesium sulfate. Evaporation of a solvent gave a residue, which was purified by column chromatography on silica gel eluting with a mixture of ethyl acetate and methanol (9:1, V/V). The eluted fractions containing the desired product were collected and evaporated in vacuo. The residue was recrystallized from a mixture of methanol, dioxane and isopropyl ether to give 2-(diaminomethyleneamino)-4-[5-{(furan-2-yl)-methylsulfinyl}acetamidomethylfuran-2-yl]thiazole (1.5 g).
mp : 208 to 209 °C
IR (Nujol) : 3425, 3260, 3070, 1650, 1595, 1540 $cm^{-1}$
NMR (DMSO-$d_6$, δ) : 3.16 (1H, d, J=13Hz), 3.79 (1H, d, J=13Hz), 4.16 (1H, d, J=14Hz), 4.34 (2H, d, J=5Hz), 4.36 (1H, d, J=14Hz), 6.35 (1H, d, J=3Hz), 6.44-6.48 (2H, m), 6.61 (1H, d, J=3Hz), 6.78 (1H, s), 6.89 (4H, s), 7.69 (1H, s), 8.83 (1H, t, J=5Hz)

| Anal. Calcd. for $C_{16}H_{17}N_5O_4S_2$ : | | | |
|---|---|---|---|
| Found | C 47.16,<br>C 47.15, | H 4.21,<br>H 4.15, | N 17.19<br>N 17.02 |

Example 62

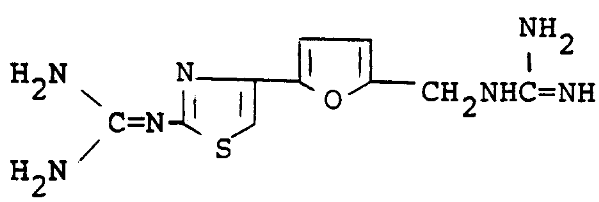

A suspension of 4-(5-aminomethylfuran-2-yl)-2-(diaminomethyleneamino)thiazole (8.0 g) and S-methylisothiourea hemisulfate (11.08 g in dimethyl sulfoxide (250 ml) was heated at 100 °C for 6 hours. The resulting precipitate was collected by filtration and then suspended in water (50 ml). The suspension was alkalized to pH 13 with a 4N-sodium hydroxide solution. The resulting precipitate was collected by filtration to afford 2-(diaminomethyleneamino)-4-(5-guanidinomethylfuran-2-yl)thiazole (8.26 g).
NMR (DMSO-$d_6$, δ) : 4.30 (2H, s), 6.35 (1H, d, J=3.0Hz), 6.58 (1H, d, J=3.0Hz), 6.77 (4H,s), 7.6-6.7 (4H, br)

Example 63

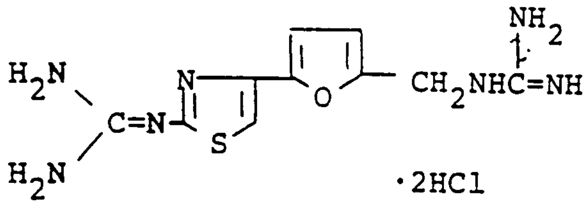

A solution of hydrogen chloride in methanol (0.19 g/ml, 3 ml) was added slowly to a suspension of 2-(diaminomethyleneamino)-4-(5-guanidinomethylfuran-2-yl)thiazole (500 mg) in methanol (10 ml) with cooling on an ice-water bath. The mixture was stirred with cooling on an ice-water bath for 2 hours. The mixture was diluted with diisopropyl ether (20 ml) and the resulting precipitate was collected by filtration. Recrystallization from a mixture of methanol, tetrahydrofuran and diisopropyl ether afforded 2-(diaminomethyleneamino)-4-(5-guanidinomethylfuran-2-yl)thiazole dihydrochloride (340 mg).
mp : 255 °C
IR (Nujol) : 3230, 1680, 1620 $cm^{-1}$
NMR ($DMSO-d_6$, $\delta$) : 4.48 (2H, d, J=6.0Hz), 6.52 (1H, d, J=3.5Hz), 7.02 (1H, d, J=3.5Hz), 7.35 (1H, s), 7.45 (4H, s), 8.24 (1H, t, J=6.0Hz), 8.36 (4H, s), 12.80 (1H, s)

| Anal. Calcd. for $C_{10}H_{13}N_7OS \cdot 2HCl$ : | | | | |
|---|---|---|---|---|
| | C 34.10, | H 4.29, | N 27.84, | Cl 20.13 |
| Found | C 34.08, | H 4.29, | N 27.41, | Cl 19.85 |

Example 64

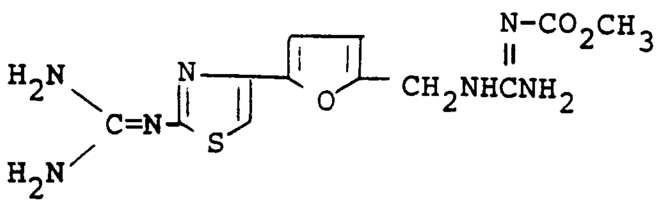

A suspension of 4-(5-aminomethylfuran-2-yl)-2-(diaminomethyleneamino)thiazole (1.6 g), imino(methylthio)methylcarbamic acid methylester (1.2 g) and triethylamine (1.5 g) was stirred at room temperature for 26 hours and then refluxed for 2.5 hours. Imino(methylthio)methylcarbamic acid methylester (1.0 g) was added to the reaction mixture and the mixture was refluxed for 10 hours. The solvent was removed under reduced pressure. The residue was chromatographed on a silica gel column eluting with a mixture of chloroform and methanol (10:1, V/V). Recrystallization from a mixture of methanol and diisopropyl ether afforded 2-(diaminomethyleneamino)-4-[5-(2-methoxycarbonylguanidino)methylfuran-2-yl]thiazole (530 mg).
mp : 199 to 200 °C (dec.)
IR (Nujol) : 3450, 3375, 1630 $cm^{-1}$
NMR ($DMSO-d_6$, $\delta$) : 3.48 (3H, s), 4.39 (2H, s), 6.33 (1H, d, J=3.0Hz), 6.62 (1H, d, J=3.0Hz), 6.79 (1H, s), 6.90 (4H, s), 7.37 (1H, br)

| Anal. Calcd. for $C_{12}H_{15}N_7O_3S$ : | | | |
|---|---|---|---|
| | C 42.72, | H 4.48, | N 29.06 |
| Found | C 42.92, | H 4.62, | N 28.82 |

Example 65

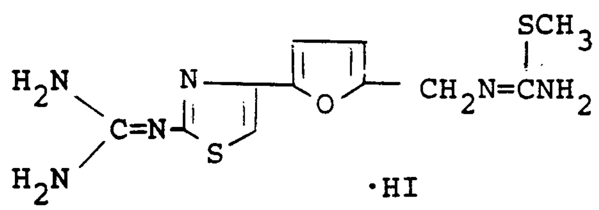

A suspension of 2-(diaminomethyleneamino)-4-(5-thioureidomethylfuran-2-yl)thiazole (1.0 g) and methyl iodide (600 mg) in methanol was stirred at room temperature for 34 hours. The solvent was removed under reduced pressure to afford 2-(diaminomethyleneamino)-4-[5-(2-methyl-3-isothioureido)methylfuran-2-yl]-thiazole hydriodide (1.3 g).
NMR (DMSO-$d_6$, δ) : 2.63 (3H, s), 4.61 (2H, s), 6.53 (1H, d, J = 3.0Hz), 6.69 (1H, d, J = 3.0Hz), 6.83 (1H, s), 6.80-7.40 (7H, br), 9.42 (1H, br)

Example 66

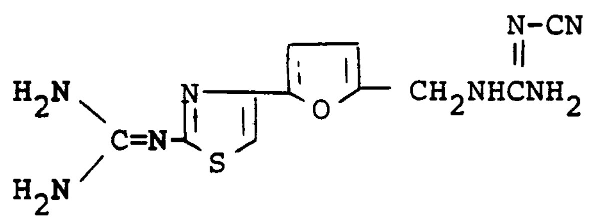

A solution of 2-(diaminomethyleneamino)-4-[5-(2-methyl-3-isothioureido)methylfuran-2-yl]thiazole hydriodide (1.0 g), cyanamide (120 g) and triethylamine (1.2 g) in N,N-dimethylformamide (20 ml) was heated at 100 °C for 3 hours. The solvent was removed under reduced pressure. The residue was chromatographed on a silica gel column eluting with a mixture of chloroform and methanol (20:1, V/V). Recrystallization from 20% methanol afforded 2-(diaminomethyleneamino)-4-[5-(2-cyanoguanidino)-methylfuran-2-yl]thiazole (170 mg).
mp : 224 to 225 °C
IR (Nujol) : 3310, 3200, 2180, 1630 $cm^{-1}$
NMR (DMSO-$d_6$, δ) : 4.30 (2H, d, J = 5.5Hz), 6.33 (1H, d, J = 3.0Hz), 6.62 (1H, d, J = 3.0Hz), 6.78 (1H, s), 6.80 (2H, br), 6.88 (4H, s), 7.25 (1H, br)

| Anal. Calcd. for $C_{11}H_{12}N_8OS \cdot 7/10\ H_2O$ : | | | | |
|---|---|---|---|---|
| | C 41.69, | H 4.26, | N 35.35, | $H_2O$ 3.98 |
| Found | C 41.61, | H 4.33, | N 35.00, | $H_2O$ 3.70 |

Example 67

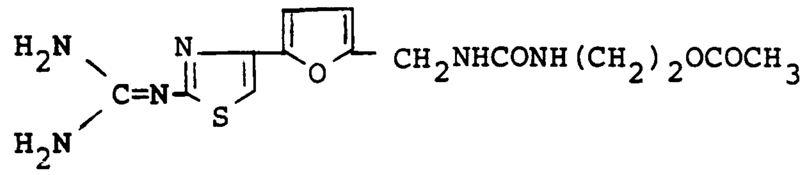

A mixture of 3-acetoxypropionic acid (2.5 g), diphenylphosphorylazide (4.3 ml) and triethylamine (2.6 ml) in dry benzene (50 ml) was stirred for an hour at 75 to 80 °C. To the mixture was added to a mixture of 4-(5-aminomethylfuran-2-yl)-2-(diaminomethyleneamino)thiazole (3.0 g) in a solution of tetrahydrofuran (45 ml) and methanol (45 ml) at ambient temperature, and the mixture was stirred for 2.5 hours at the same temperature. Evaporation of a solvent gave a residue, which was purified by column chromatography on silica gel eluting with a mixture of ethyl acetate and methanol (9:1, V/V). The eluted fractions containing the desired product were collected and evaporated in vacuo. The residue was recrystallized from a mixture of methanol, dioxane and isopropyl ether to give 4-[5-{3-(2-acetoxyethyl)ureido}methylfuran-2-yl]-2-(diaminomethyleneamino)thiazole (1.82 g).

mp : 200 to 202 °C

IR (Nujol) : 3380, 1725, 1655, 1600, 1550 (br) $cm^{-1}$

NMR (DMSO-$d_6$, $\delta$) : 2.00 (3H, s), 3.07-3.43 (2H, m), 3.97 (2H, t, J = 5Hz), 4.20 (2H, d, J = 5Hz), 6.04 (1H, t, J = 5Hz), 6.19 (1H, d, J = 3Hz), 6.33 (1H, t, J = 5Hz), 6.53 (1H, d, J = 3Hz), 6.70 (1H, s), 6.82 (4H, s)

| Anal. Calcd. for $C_{14}H_{18}N_6O_4S$ : | | | |
|---|---|---|---|
| | C 45.89, | H 4.95, | N 22.94 |
| Found | C 45.67, | H 4.87, | N 22.72 |

Example 68

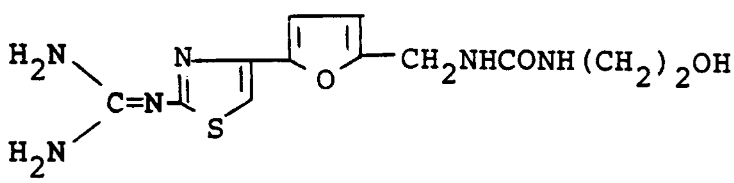

A mixture of 4-[5-{3-(2-acetoxyethyl)ureido}methylfuran-2-yl]-2-(diaminomethyleneamino)thiazole (1.0 g) in methanol (30 ml) and 1N-sodium hydroxide (8.2 ml) was stirred for 2 hours at ambient temperature. The solvent was removed by concentration in vacuo and to the residue was added a mixture of ethyl acetate, tetrahydrofuran and water. The separated organic layer was washed with brine and dried over magnesium sulfate. Evaporation of a solvent gave a residue, which was purified by column chromatography on alumina eluting with a mixture of chloroform and methanol (9:1, V/V). The eluted fractions containing the desired product were collected and evaporated in vacuo. The residue was recrystallized from a mixture of methanol, dioxane and isopropyl ether to give 2-(diaminomethyleneamino)-4-[5-{3-(2-hydroxyethyl)ureido}methylfuran-2-yl]thiazole (0.42 g).

mp : 201 to 203 °C

IR (Nujol) : 3380, 3340, 1635, 1605, 1590 $cm^{-1}$

NMR (DMSO-$d_6$, $\delta$) : 3.00-3.15 (2H, m), 3.28-3.48 (2H, m), 4.22 (2H, d, J = 6Hz), 4.68 (1H, t, J = 5Hz), 6.00 (1H, t, J = 6Hz), 6.24 (1H, d, J = 3Hz), 6.41 (1H, t, J = 6Hz), 6.59 (1H, d, J = 3Hz), 6.76 (1H, s), 6.90 (4H, s)

| Anal. Calcd. for $C_{12}H_{16}N_6O_3S \cdot 1/5H_2O$ : | | | |
|---|---|---|---|
| | C 43.95, | H 5.04, | N 25.63 |
| Found | C 43.96, | H 4.84, | N 25.51 |

Example 69

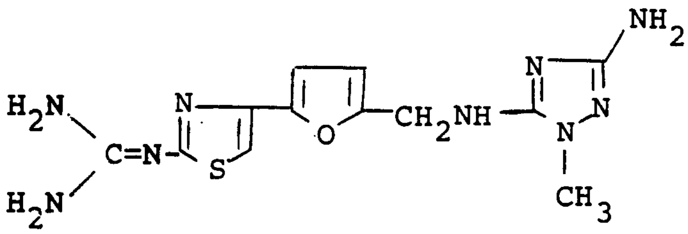

A solution of 4-(5-aminomethylfuran-2-yl)-2-(diaminomethyleneamino)thiazole (1.50 g) and diphenyl cyanocarbonimidate (1.51 g) in methanol (20 ml) was stirred for 4 hours at room temperature. After the solvent was evaporated in vacuo, acetonitrile (20 ml) and methylhydrazine (1.46 g) was added to the residue and the mixture was stirred for three hours at room temperature. The solvent was evaporated in vacuo and the residue was mixed with water. The mixture was made acidic with 6N-hydrochloric acid and washed with diethyl ether. The acidic solution was made basic to pH 11 with aqueous potassium carbonate and the resulting precipitate was collected by filtration. The product was converted to the hydrochloric acid in an usual manner, and which was recrystallized from a mixture of methanol, water and diisopropyl ether to give 4-[5-(3-amino-1-methyl-1H-1,2,4-triazol-5-yl)aminomethylfuran-2-yl]-2-(diaminomethyleneamino)thiazole dihydrochloride (0.61 g).
mp : 184 to 185 °C
IR (Nujol) : 3300, 3175, 1675, 1650 $cm^{-1}$
NMR (DMSO-$d_6$, $\delta$) : 3.48 (3H, s), 4.58 (2H, d, J = 5Hz), 6.61 (1H, d, J = 3Hz), 7.04 (1H, d, J = 3Hz), 7.36 (1H, s), 8.43 (4H, s) and 8.91 (1H, t, J = 5Hz)

| Anal. Calcd for $C_{12}H_{15}N_9OS \cdot 2HCl \cdot H_2O$ : | | | | | |
|---|---|---|---|---|---|
| | C 33.97, | H 4.51, | N 29.71, | Cl 16.71, | $H_2O$ 4.25 |
| Found | C 34.17, | H 4.54, | N 29.17, | Cl 16.30, | $H_2O$ 3.77 |

Example 70

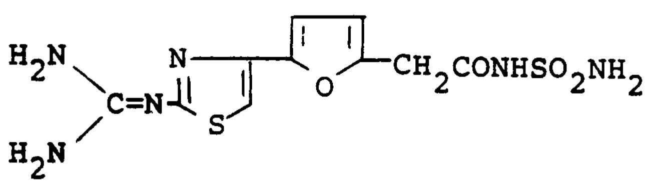

A mixture of 4-[5-(2-amino-2-aminosulfonyliminoethyl)furan-2-yl]-2-(diaminomethyleneamino)thiazole (2.0 g) in N,N-dimethylformamide (15 ml) and 1N-hydrochloric acid (17.5 ml) was stirred for 87 hours at ambient temperature. To a water (50 ml) was added a reaction mixture and the mixture was adjusted to pH 9.5 with 20% aqueous potassium carbonate. The isolated precipitate was collected by filtration and the precipitate was recrystallized from aqueous N,N-dimethylformamide to give 2-(diaminomethyleneamino)-4-(5-sulfamoylaminocarbonylmethylfuran-2-yl)thiazole (1.1 g).

mp : 179 to 181 ° C
IR (Nujol) : 3350, 3280, 3210, 3100, 1690, 1620, 1580, 1570, 1510 $cm^{-1}$
NMR (DMSO-$d_6$, δ) : 3.68 (2H, s), 6.33 (1H, d, J=3Hz), 6.63 (1H, d, J=3Hz), 6.77 (1H, s), 6.92 (4H, s), 7.42 (2H, s)

Example 71

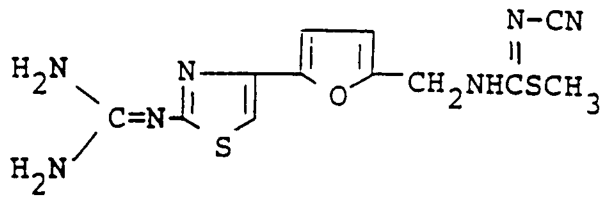

A mixture of 4-(5-aminomethylfuran-2-yl)-2-(diaminomethyleneamino)thiazole (8.00 g) and dimethyl N-cyanodithioiminocarbonate (1.54 g) in ethanol (80 ml) was refluxed for two hours with stirring. After cooling to room temperature, diisopropyl ether (80 ml) was added and the resulting precipitate was collected filtration. Recrystallization from a mixture of N,N-dimethylformamide and ethyl acetate to give 2-(diaminomethyleneamino)-4-[5-(3-cyano-2-methyl-1-isothioureido)methylfuran-2-yl]thiazole (10.2 g).
IR (Nujol) : 3450, 3300, 3270, 2165, 1655 $cm^{-1}$

Example 72

The following compound was obtained according to a similar manner to that of Example 1.

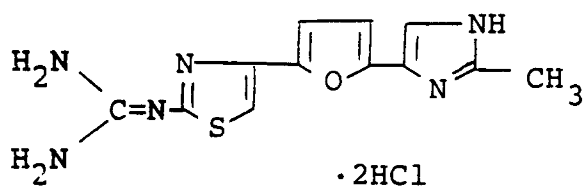

2-(Diaminomethyleneamino)-4-[5-(2-methylimidazol-4-yl)furan-2-yl]thiazole dihydrochloride.
mp : >300 ° C
IR (KBr) : 3000, 1680, 1590 $cm^{-1}$
NMR (DMSO-$d_6$, δ) : 2.64 (3H, s), 7.14 (1H, d, J=3.5Hz), 7.23 (1H, d, J=3.5Hz), 7.60 (1H, s), 7.90 (1H, s) and 8.38 (4H, s)

Example 73

The following compounds were obtained according to a similar manner to that of Example 1.

(1)

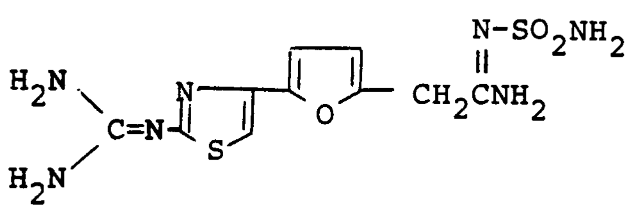

4-[5-(2-Amino-2-aminosulfonyliminoethyl)furan-2-yl]-2-(diaminomethyleneamino)thiazole.
mp : 222-225 °C (dec.)
IR (Nujol) : 3470, 3450, 3400, 3350, 3320, 3230, 1620, 1530 $cm^{-1}$
NMR (DMSO-$d_6$, δ) : 3.58 (2H, s), 6.28 (1H, d, J=3Hz), 6.52 (2H, s), 6.56 (1H, d, J=3Hz), 6.73 (1H, s), 6.81 (4H, s), 7.35 (1H, s), 8.18 (1H, s)

(2)

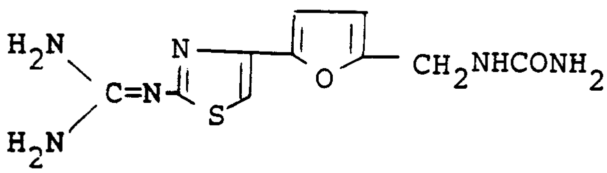

2-(Diaminomethyleneamino)-4-(5-ureidomethylfuran-2-yl)thiazole.
mp : 214-215 °C
IR (Nujol) : 3400, 3320, 3130, 1650, 1590 $cm^{-1}$
NMR (DMSO-$d_6$, δ) : 4.19 (2H, d, J=5Hz), 5.53 (2H, s), 6.24 (1H, d, J=3Hz), 6.36 (1H, t, J=5Hz), 6.59 (1H, d, J=3Hz), 6.76 (1H, s), 6.87 (4H, s)

| Anal. Calcd. for $C_{10}H_{12}N_6O_2S \cdot 1/3\ H_2O$ | | | | |
|---|---|---|---|---|
| | C 41.95, | H 4.46, | N 29.35, | $H_2O$ 2.10 |
| Found | C 42.04, | H 4.53, | N 29.04, | $H_2O$ 2.33 |

**Claims**
**Claims for the following Contracting States : AT, BE, CH, DE, FR, GB, IT, LI, LU, NL, SE**

**1.** A compound of the formula :

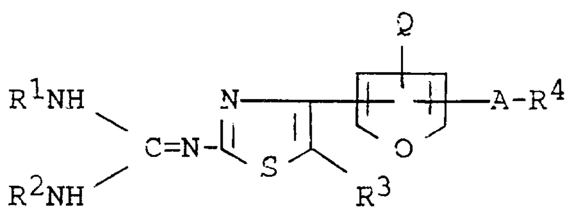

wherein
$R^1$ and $R^2$ are each hydrogen, furoyl or $C_1$-$C_6$ alkyl which may have halogen; or
$R^1$ and $R^2$ are linked together to form $C_1$-$C_6$ alkylene,
$R^3$ is hydrogen or $C_1$-$C_6$ alkyl,
$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio-($C_1$-$C_6$)alkanoylamino, benzoylamino optionally substituted by nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)alkanoylamino, furyl($C_1$-$C_6$)alkylsulfinyl($C_1$-$C_6$)-alkanoylamino, mono or dl($C_1$-$C_6$)alkylsulfamoylamino, hydroxy($C_1$-$C_6$)alkylureido, $C_1$-$C_6$ alkanoyloxy($C_1$-$C_6$)alkylureido, $C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of the formula :

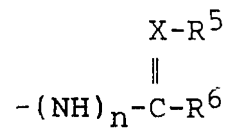

wherein

n is 0 or 1,

X is =CH- or =N-,

$R^5$ is hydrogen, cyano, nitro, carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)-alkylsulfamoyl,

$R^6$ is hydrogen, $C_1$-$C_6$ alkyl, $C_1$-$C_6$ alkylthio, $C_1$-$C_6$ alkoxy, amino, mono or di($C_1$-$C_6$)-alkylamino, allylamino, propargylamino, hydroxy($C_1$-$C_6$)alkylamino, $C_1$-$C_6$ alkoxy($C_1$-$C_6$)alkylamino, or mono or di($C_1$-$C_6$)alkylamino($C_1$-$C_6$)alkylamino;

A is $C_1$-$C_6$ alkylene or -CONH- ; or

A-$R^4$ is imidazolyl substituted with $C_1$-$C_6$ alkyl, triazolyl substituted with amino, and

Q is hydrogen, or $C_1$-$C_6$ alkyl,

and pharmaceutically acceptable salts thereof.

**2.** A compound of claim 1, wherein

$R^1$ and $R^2$ are each hydrogen, $C_1$-$C_6$ alkyl or trihalo($C_1$-$C_6$)alkyl; or

$R^1$ and $R^2$ are linked together to form $C_1$-$C_6$ alkylene,

$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)alkanoylamino, nitrobenzoylamino, furoylamino, thenoylamino, nicotinoylamino, 1-oxonicotinoylamino, morpholinocarbonylamino, 3-$C_1$-$C_6$ alkylureido, 3-$C_1$-$C_6$ alkylthioureido, 3-$C_1$-$C_6$ alkanoylureido, 3-allylureido, 3-benzoylthioureido, furyl($C_1$-$C_6$)alkylthio($C_1$-$C_6$)alkanoylamino, furyl($C_1$-$C_6$)alkylsulfinyl($C_1$-$C_6$)alkanoylamino, mono or di($C_1$-$C_6$)alkylsulfamoylamino, 3-hydroxy($C_1$-$C_6$)-alkylureido, 3-$C_1$-$C_6$ alkanoyloxy($C_1$-$C_6$)alkylureido, 2-$C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of the formula :

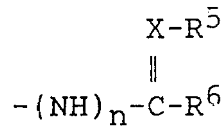

wherein

n, X and $R^6$ are as defined in claim 1,

$R^5$ is hydrogen, cyano, nitro, carbamoyl, $C_1$-$C_6$ alkoxycarbonyl, sulfamoyl, $C_1$-$C_6$ alkylsulfonyl, phenylsulfonyl substituted with $C_1$-$C_6$ alkyl, phenylsulfonyl substituted with $C_1$-$C_6$ alkoxy, phenylsulfonyl substituted with halogen, phenylsulfonyl substituted with amino, mono or di($C_1$-$C_6$)alkylsulfamoyl,

A is as defined in claim 1 ; or

A-$R^4$ is as defined in claim 1, and

$R^3$ and Q are each as defined in claim 1.

**3.** A compound of claim 2, wherein
$R^1$ and $R^2$ are each hydrogen,
$R^3$ is hydrogen,
$R^4$ is a group of the formula :

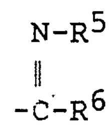

wherein
$R^5$ is sulfamoyl, and
$R^6$ is amino,
A is $C_1$-$C_6$ alkylene, and
Q is hydrogen.

**4.** A compound of claim 3, which is 4-[5-(2-amino-2-sulfamoyliminoethyl)furan-2-yl]-2-(diaminomethyleneamino)thiazole or its hydrochloride or its methanesulfonate.

**5.** A compound of claim 2, wherein
$R^1$ and $R^2$ are each hydrogen,
$R^3$ is hydrogen,
$R^4$ is ureido or 3-$C_1$-$C_6$ alkylureido,
A is $C_1$-$C_6$ alkylene, and
Q is hydrogen.

**6.** A compound of claim 5, which is selected from the group consisting of 2-(diaminomethyleneamino)-4-(5-ureidomethylfuran-2-yl)thiazole or its hydrochloride, and 2-(diaminomethyleneamino)-4-[5-(3-methylureido)methylfuran-2-yl]thiazole or its hydrochloride.

**7.** A process for preparing a compound of the formula :

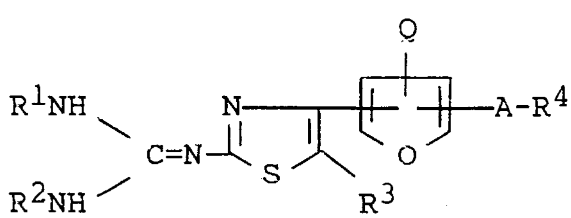

wherein
$R^1$, $R^2$, $R^3$, $R^4$, $R^5$, $R^6$, A, A-$R^4$, Q, X and n are each as defined in claim 1,
or a salt thereof,
which comprises,
(1) reacting a compound of the formula :

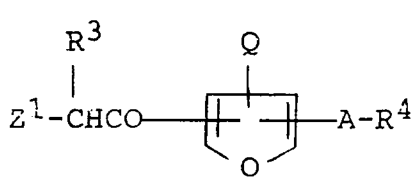

wherein
$R^3$, $R^4$, A and Q are each as defined above, and

$Z^1$ is halogen,

or a salt thereof, with a compound of the formula :

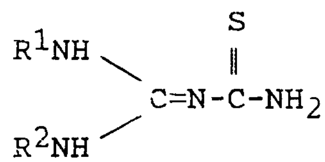

wherein
$R^1$ and $R^2$ are each as defined above,

to give a compound of the formula :

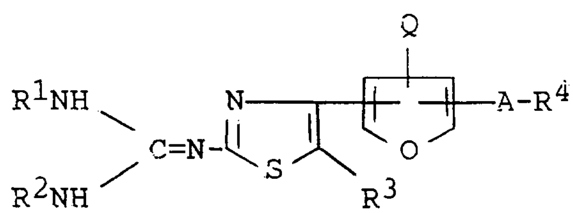

wherein
$R^1$, $R^2$, $R^3$, $R^4$, A and Q are each as defined above,

or a salt thereof, or

(2) subjecting a compound of the formula :

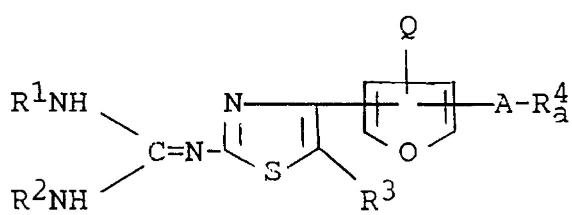

wherein
$R^1$, $R^2$, $R^3$, A and Q are each as defined above, and
$R^4_a$ is ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)-alkanoylamino, benzoylamino optionally substituted with nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)-alkanoylamino, mono or di($C_1$-$C_6$)alkylsulfamoylamino, hydroxy($C_1$-$C_6$)-alkylureido, or $C_1$-$C_6$ alkanoyloxy($C_1$-$C_6$)alkylureido,

or a salt thereof, to deacylation reaction, to give a compound of the formula :

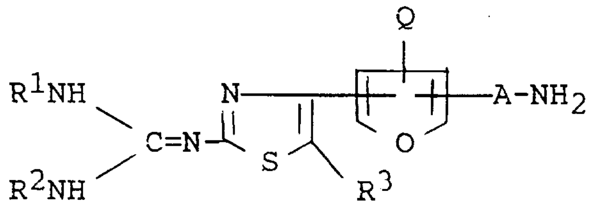

wherein
$R^1$, $R^2$, $R^3$, A and Q are each as defined above,
or a salt thereof, or
(3) subjecting a compound of the formula :

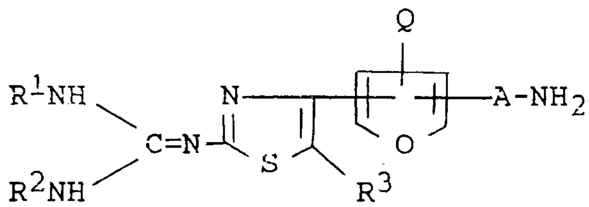

wherein
$R^1$, $R^2$, $R^3$, A and Q are each as defined above,
or a salt thereof, to acylation reaction, to give a compound of the formula :

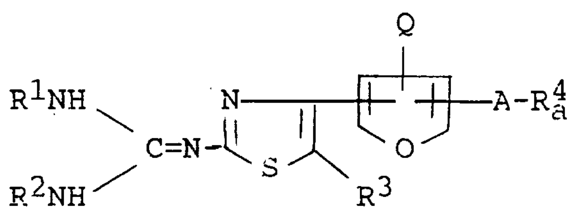

wherein
$R^1$, $R^2$, $R^3$, $R_a^4$ , A and Q are each as defined above,
or a salt thereof, or
(4) reacting a compound of the formula :

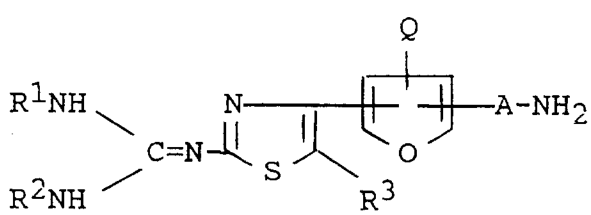

wherein
$R^1$, $R^2$, $R^3$, A and Q are each as defined above,
or a salt thereof, with a compound of the formula :

$(R^7\text{-}X^1)_2C=X\text{-}R^5$

wherein
$R^5$ and X are each as defined above,

$R^7$ is C1-C6 alkyl or phenyl, and
$X^1$ is S or O,
to give a compound of the formula :

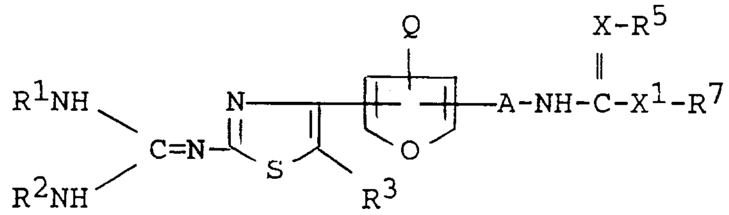

wherein
$R^1$, $R^2$, $R^3$, $R^5$, $R^7$, X, $X^1$, A and Q are each as defined above,
or a salt thereof, or
(5) reacting a compound of the formula :

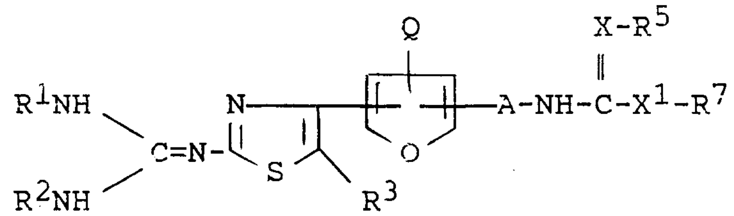

wherein
$R^1$, $R^2$, $R^3$, $R^5$, $R^7$, X, $X^1$, A and Q are each as defined above,
or a salt thereof, with a compound of the formula :

$H\text{-}R^6_a$

wherein
$R^6_a$ is amino which may have mono or di($C_1$-$C_6$)alkyl, allyl, propargyl, hydroxy($C_1$-$C_6$)alkyl or mono or di($C_1$-$C_6$)alkylamino($C_1$-$C_6$)alkyl, or $C_1$-$C_6$ alkoxy,
to give a compound of the formula :

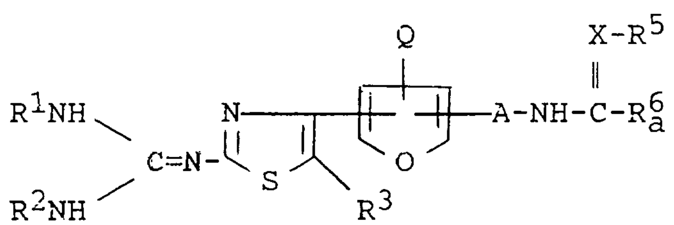

wherein
$R^1$, $R^2$, $R^3$, $R^5$, $R^6_a$ , X, A and Q are each as defined above,
or a salt thereof, or

(6) subjecting a compound of the formula :

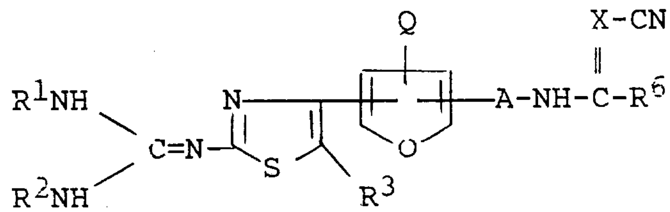

wherein
R¹, R², R³, R⁶, X, A and Q are each as defined above,
or a salt thereof, to hydrolysis, to give a compound of the formula :

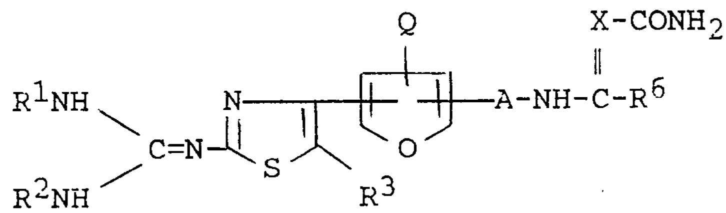

wherein
$R^1$, $R^2$, $R^3$, $R^6$, X, A and Q are each as defined above,
or a salt thereof, or
(7) subjecting a compound of the formula :

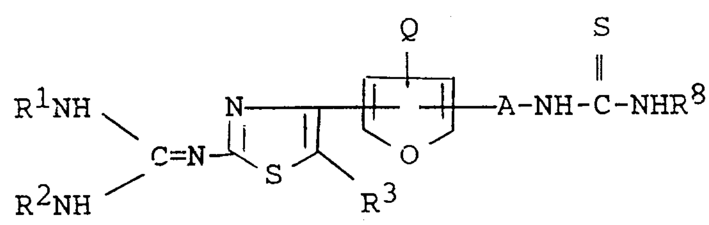

wherein
$R^1$, $R^2$, $R^3$, A and Q are each as defined above, and
$R^8$ is amino-protective group consisting of $C_1$-$C_6$ alkanoyl and benzoyl,
or a salt thereof, to elimination reaction of the amino-protective group, to give a compound of the formula :

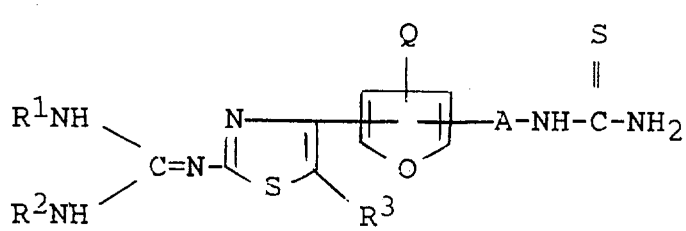

wherein
$R^1$, $R^2$, $R^3$, A and Q are each as defined above,

or a salt thereof, or

(8) reacting a compound of the formula :

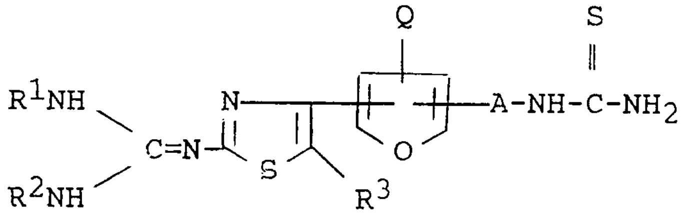

wherein

$R^1$, $R^2$, $R^3$, A and Q are each as defined above,

or a salt thereof, with a compound of the formula :

$Z^2$-$CH_2$-CO-$R^9$

wherein

$R^9$ is $C_1$-$C_6$ alkyl, and

$Z^2$ is halogen,

to give a compound of the formula :

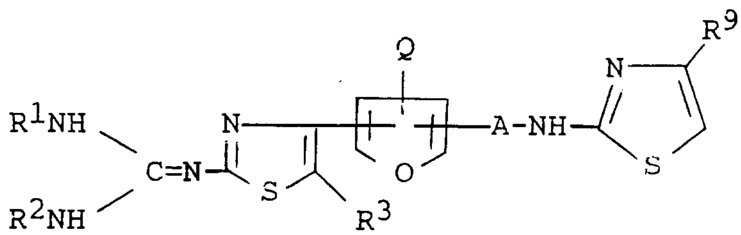

wherein

$R^1$, $R^2$, $R^3$, $R^9$, A and Q are each as defined above,

or a salt thereof, or

(9) reacting a compound of the formula :

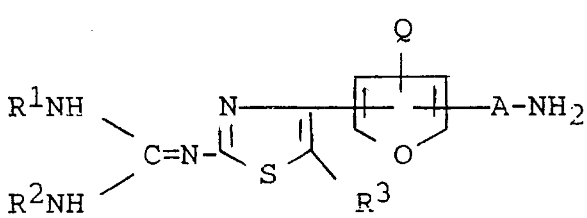

wherein

$R^1$, $R^2$, $R^3$, A and Q are each as defined above,

or a salt thereof, with a compound of the formula :

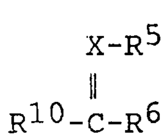

wherein

$R^5$, $R^6$ and X are each as defined above, and

$R^{10}$ is $C_1$-$C_6$ alkanoyloxy, phenyl($C_1$-$C_6$)alkoxy, $C_1$-$C_6$ alkoxy($C_1$-$C_6$)alkoxy, tetrahydropyranyloxy or $C_1$-$C_6$ alkoxy,

to give a compound of the formula :

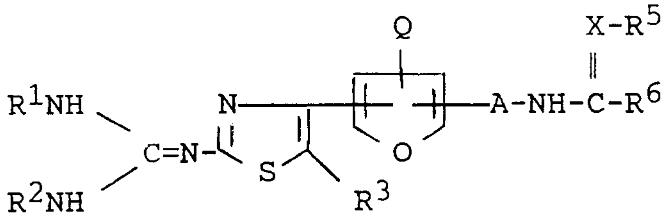

wherein

$R^1$, $R^2$, $R^3$, $R^5$, $R^6$, X, A and Q are each as defined above,

or a salt thereof, or

(10) reacting a compound of the formula :

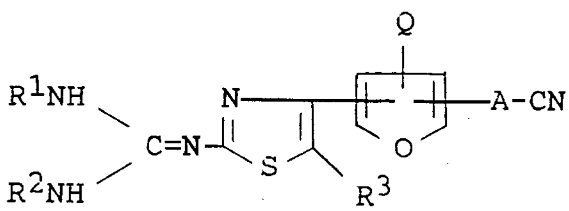

wherein

$R^1$, $R^2$, $R^3$, A and Q are each as defined above,

or a salt thereof, with a compound of the formula :

$R^{11}OH$

wherein

$R^{11}$ is $C_1$-$C_6$ alkyl,

to give a compound of the formula :

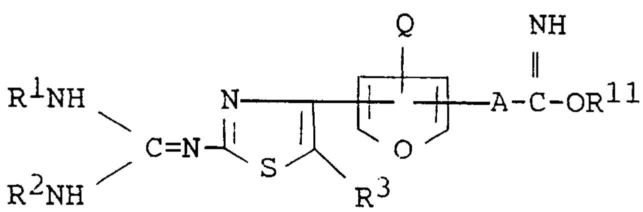

wherein

$R^1$, $R^2$, $R^3$, $R^{11}$, A and Q are each as defined above,

or a salt thereof, or

(11) reacting a compound of the formula :

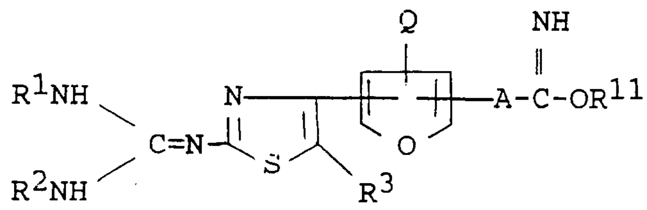

wherein
$R^1$, $R^2$, $R^3$, $R^{11}$, A and Q are each as defined above,
or a salt thereof, with a compound of the formula :

$NH_2R^{12}$

wherein
$R^{12}$ is acyl,
to give a compound of the formula :

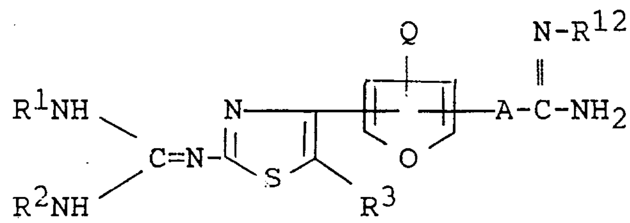

wherein
$R^1$, $R^2$, $R^3$, $R^{12}$, A and Q are each as defined above,
or a salt thereof, or
(12) reacting a compound of the formula :

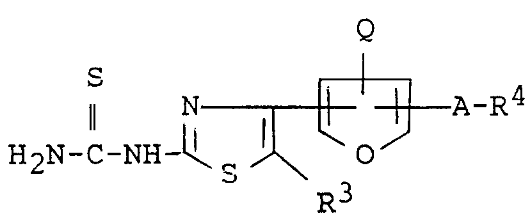

wherein
$R^3$, $R^4$, A and Q are each as defined above,
or a salt thereof, with a compound of the formula :

$R^{13}$-$Z^3$

wherein
$R^{13}$ is $C_1$-$C_6$ alkyl, and
$Z^3$ is halogen,
or a salt thereof, to give a compound of the formula:

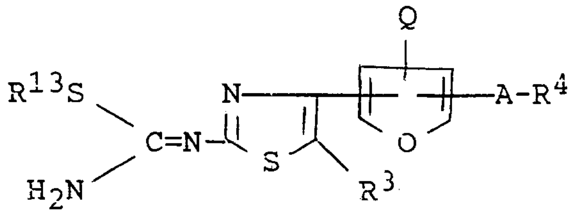

wherein

$R^3$, $R^4$, $R^{13}$, A and Q are each as defined above,

or a salt thereof, and continuously reacting it with a compound of the formula :

$H_2N\text{-}Y\text{-}NH_2$

wherein

Y is $C_1$-$C_6$ alkylene,

or a salt thereof,

to give a compound of the formula :

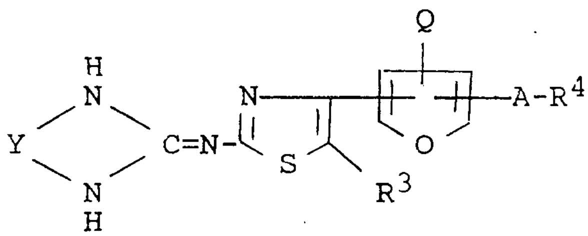

wherein

$R^3$, $R^4$, A, Q and Y are each as defined above,

or a salt thereof, or

(13) subjecting a compound of the formula :

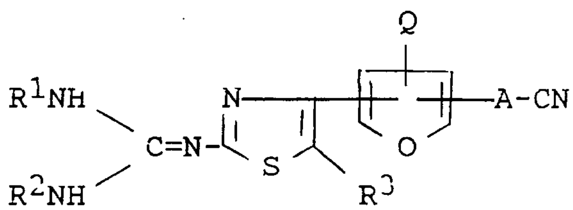

wherein

$R^1$, $R^2$, $R^3$, A and Q are each as defined above,

or a salt thereof, to reduction, to give a compound of the formula :

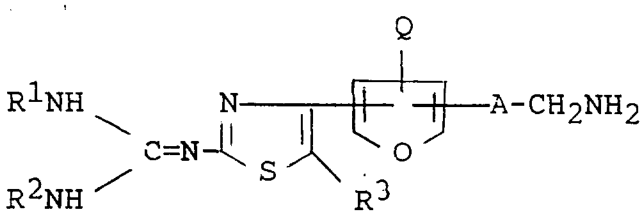

wherein

$R^1$, $R^2$, $R^3$, A and Q are each as defined above,
or a salt thereof, or
(14) reacting a compound of the formula :

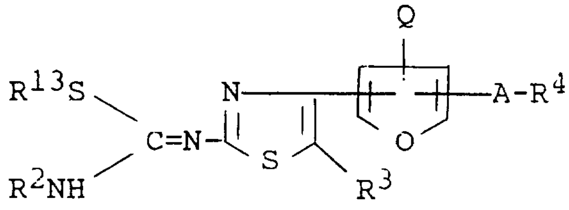

wherein
$R^2$, $R^3$, $R^4$, $R^{13}$, A and Q are each as defined above,
or a salt thereof, with a compound of the formula :

$NH_2R_a^1$

wherein
$R_a^1$ is $C_1$-$C_6$ alkyl which may have halogen,
or a salt thereof, to give a compound of the formula:

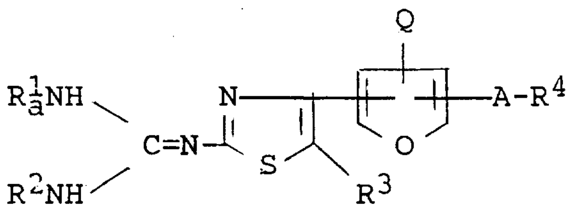

wherein
$R_a^1$, $R^2$, $R^3$, $R^4$, A and Q are each as defined above,
or a salt thereof, or
(15) subjecting a compound of the formula :

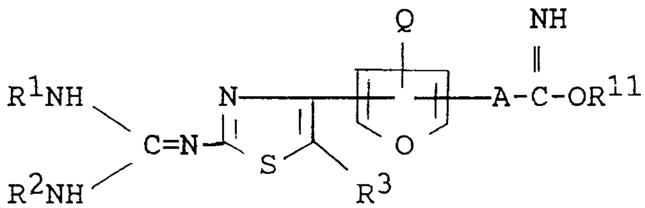

wherein
$R^1$, $R^2$, $R^3$, $R^{11}$, A and Q are each as defined above,
or a salt thereof, to hydrolysis, to give a compound of the formula :

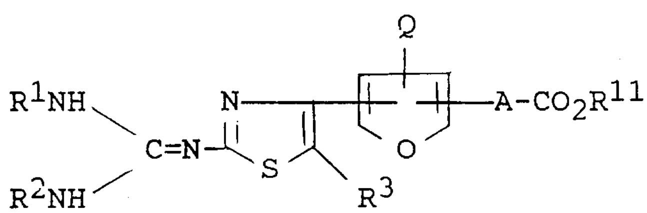

wherein

$R^1$, $R^2$, $R^3$, $R^{11}$, A and Q are each as defined above,

or a salt thereof, or

(16) subjecting a compound of the formula :

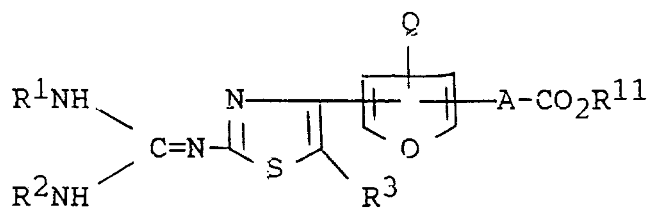

wherein

$R^1$, $R^2$, $R^3$, $R^{11}$, A and Q are each as defined above,

or a salt thereof, to amidation reaction, to give a compound of the formula :

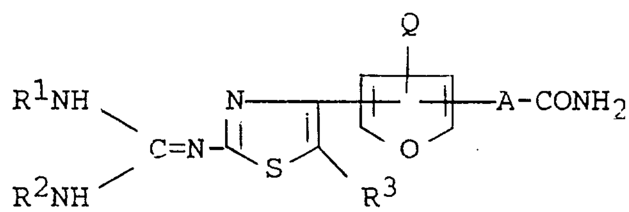

wherein

$R^1$, $R^2$, $R^3$, A and Q are each as defined above,

or a salt thereof, or

(17) reacting a compound of the formula :

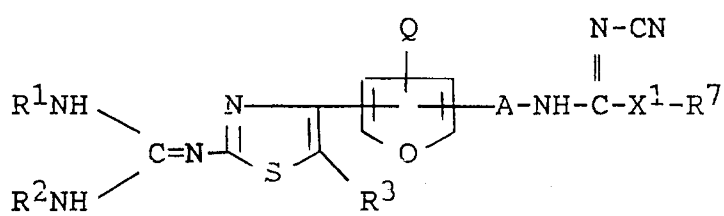

wherein

$R^1$, $R^2$, $R^3$, $R^7$, $X^1$, A and Q are each as defined above,

or a salt thereof, with a compound of the formula :

$R^{14}NHNH_2$

wherein

$R^{14}$ is hydrogen or $C_1$-$C_6$ alkyl,

to give a compound of the formula :

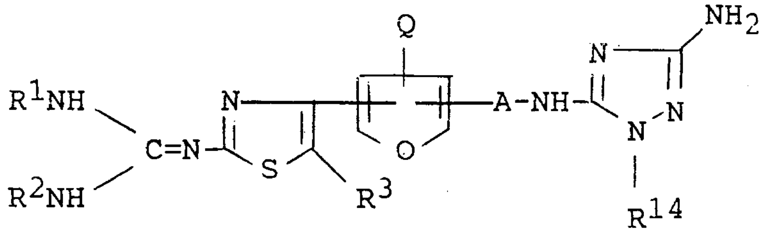

wherein

$R^1$, $R^2$, $R^3$, $R^{14}$, A and Q are each as defined above,

or a salt thereof, or

(18) reacting a compound of the formula :

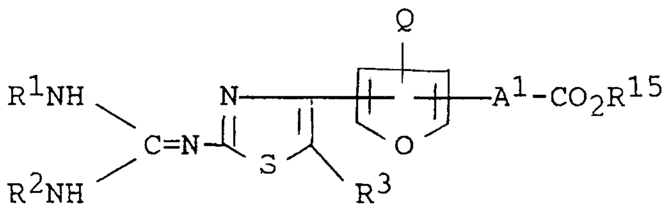

wherein

$R^1$, $R^2$, $R^3$ and Q are each as defined above,

$R^{15}$ is $C_1$-$C_6$ alkyl, and

$A^1$ is $C_1$-$C_6$ alkylene or bond,

or a salt thereof, with hydrazine, to give a compound of the formula :

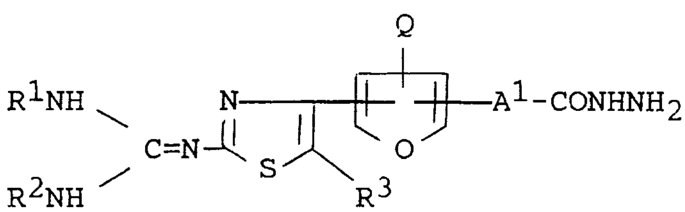

wherein

$R^1$, $R^2$, $R^3$, $A^1$ and Q are each as defined above,

or a salt thereof, or

(19) reacting a compound of the formula :

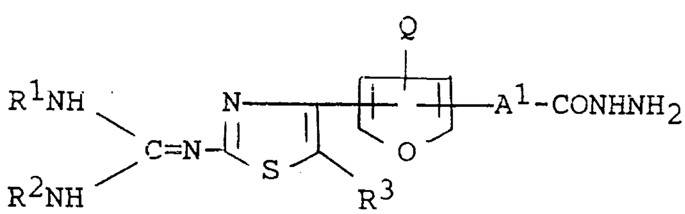

wherein

$R^1$, $R^2$, $R^3$, $A^1$ and Q are each as defined above,

or a salt thereof, with S-$C_1$-$C_6$ alkylisothiourea

or a salt thereof, to give a compound of the formula:

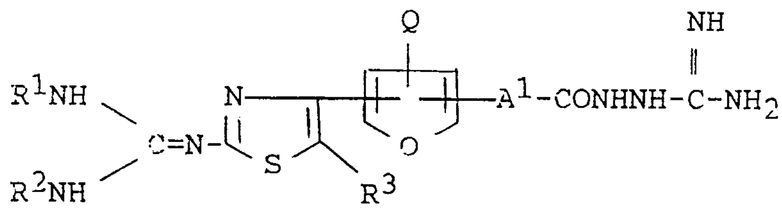

wherein

$R^1$, $R^2$, $R^3$, $A^1$ and Q are each as defined above,

or a salt thereof, or

(20) subjecting a compound of the formula :

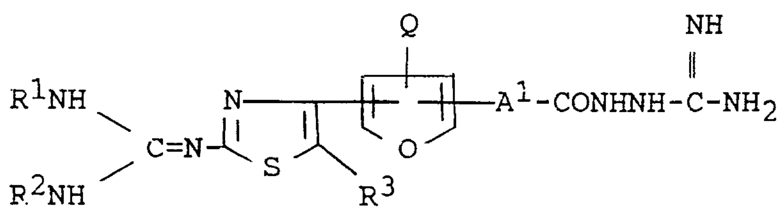

wherein

$R^1$, $R^2$, $R^3$, $A^1$ and Q are each as defined above,

or a salt thereof, to ring closure, to give a compound of the formula :

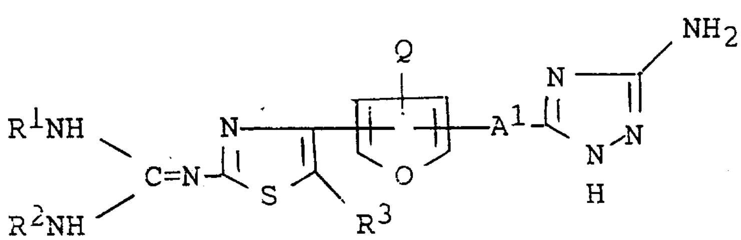

wherein

$R^1$, $R^2$, $R^3$, $A^1$ and Q are each as defined above,

or a salt thereof, or

(21) reacting a compound of the formula :

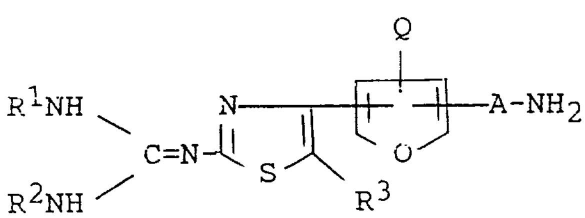

wherein

$R^1$, $R^2$, $R^3$, A and Q are each as defined above,

or a salt thereof, with a compound of the formula :

$R^{16}$-$Z^4$

wherein

$R^{16}$ is thiazolyl substituted with $C_1$-$C_6$ alkyl, triazolyl substituted with amino, triazolyl substituted with amino and $C_1$-$C_6$ alkyl, benzoisothiazolyl substituted with oxo, or benzothiadiazinyl substituted with oxo, and

$Z^4$ is halogen,

or a salt thereof, to give a compound of the formula:

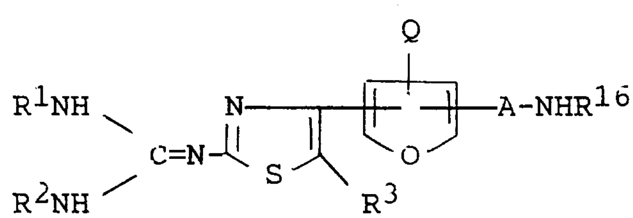

wherein

$R^1$, $R^2$, $R^3$, $R^{16}$, A and Q are each as defined above,

or a salt thereof, or

(22) subjecting a compound of the formula :

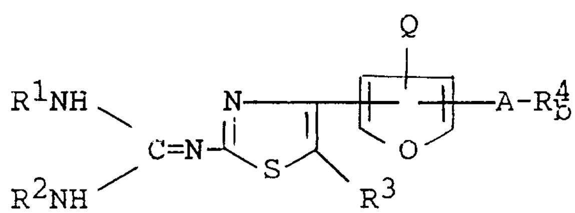

wherein

$R^1$, $R^2$, $R^3$, A and Q are each as defined above, and

$R^4_b$ is furyl($C_1$-$C_6$)alkylthio($C_1$-$C_6$)alkanoylamino,

or a salt thereof, to oxidation reaction, to give a compound of the formula :

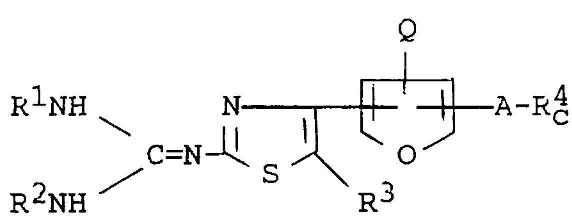

wherein

$R^1$, $R^2$, $R^3$, A and Q are each as defined above, and

$R^4_c$ is furyl($C_1$-$C_6$)alkylsulfinyl($C_1$-$C_6$)alkanoylamino,

or a salt thereof, or

(23) reacting a compound of the formula :

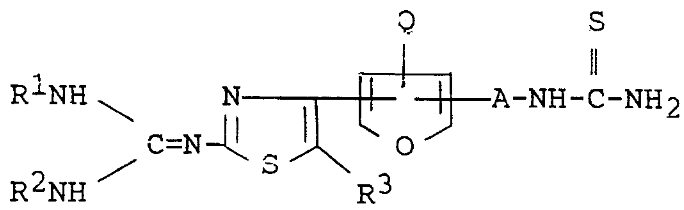

wherein
$R^1$, $R^2$, $R^3$, A and Q are each as defined above,
or a salt thereof, with a compound of the formula :

$R^{17}$-$Z^5$

wherein
$R^{17}$ is $C_1$-$C_6$ alkyl, and
$Z^5$ is halogen,
or a salt thereof, to give a compound of the formula:

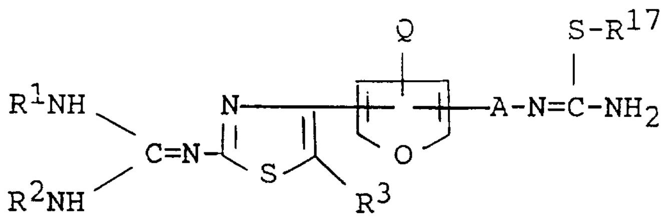

wherein
$R^1$, $R^2$, $R^3$, $R^{17}$, A and Q are each as defined above,
or a salt thereof, or
(24) reacting a compound of the formula :

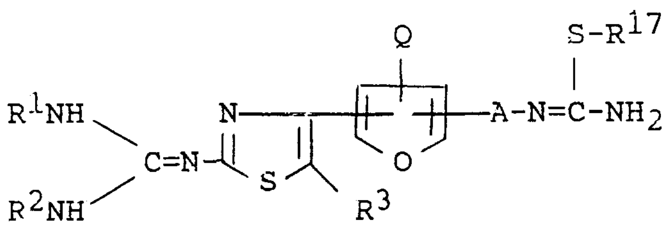

wherein
$R^1$, $R^2$, $R^3$, $R^{17}$, A and Q are each as defined above,
or a salt thereof, with a compound of the formula :

$H_2N$-$R^5$

wherein
$R^5$ is as defined above,
or a salt thereof, to give a compound of the formula:

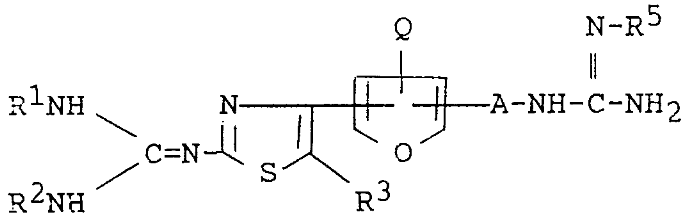

wherein
$R^1$, $R^2$, $R^3$, $R^5$, A and Q are each as defined above,
or a salt thereof, or
(25) subjecting a compound of the formula :

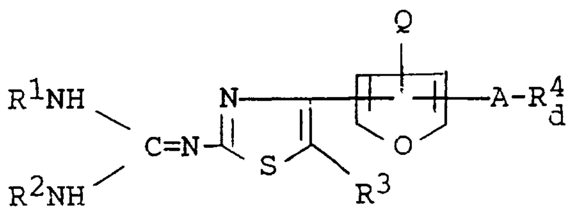

wherein
$R^1$, $R^2$, $R^3$, A and Q are each as defined above, and
$R^4_d$ is $C_1$-$C_6$ alkanoyloxy($C_1$-$C_6$)alkylureido,
or a salt thereof, to elimination reaction of $C_1$-$C_6$ alkanoyl, to give a compound of the formula :

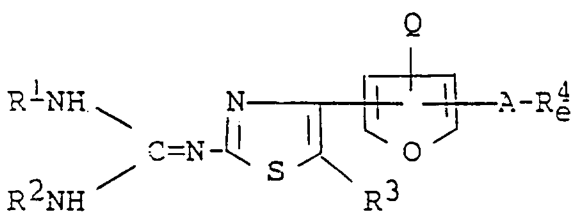

wherein
$R^1$, $R^2$, $R^3$, A and Q are each as defined above, and
$R^4_e$ is hydroxy($C_1$-$C_6$)alkylureido,
or a salt thereof, or
(26) reacting a compound of the formula :

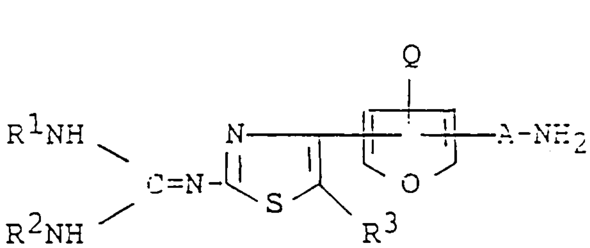

wherein
$R^1$, $R^2$, $R^3$, A and Q are each as defined above,
or a salt thereof, with a compound of the formula :

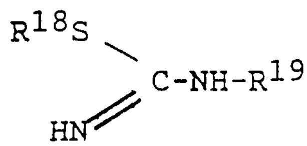

wherein

$R^{18}$ is $C_1$-$C_6$ alkyl, and

$R^{19}$ is carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)alkylsulfamoyl,

or a salt thereof, to give a compound of the formula:

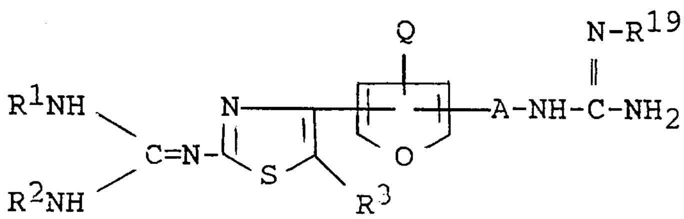

wherein

$R^1$, $R^2$, $R^3$, $R^{19}$, A and Q are each as defined above,

or a salt thereof, or

(27) subjecting a compound of the formula :

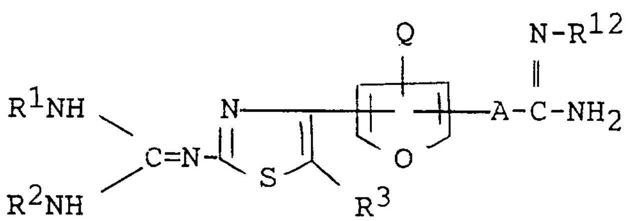

wherein

$R^1$, $R^2$, $R^3$, $R^{12}$, A and Q are each as defined above,

or a salt thereof, to hydrolysis, to give a compound of the formula :

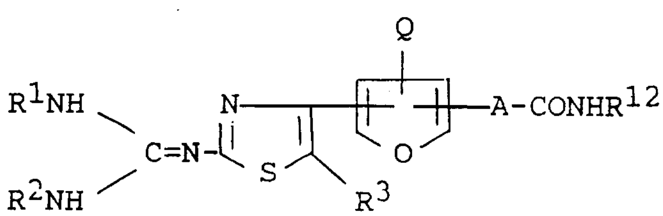

wherein

$R^1$, $R^2$, $R^3$, $R^{12}$, A and Q are each as defined above,

or a salt thereof.

**8.** A pharmaceutical composition which comprises, as an active ingredient, a compound of claim 1 or a pharmaceutically acceptable salt thereof in admixture with pharmaceutically acceptable carriers.

9. Use of a compound of claim 1 or a pharmaceutically acceptable salt thereof for the manufacture of a medicament for the therapeutic treatment of ulcer in human beings or animals.

10. A compound of claim 1 or a pharmaceutically acceptable salt thereof for use as a medicament.

**Claims for the following Contracting State : ES**

1. A process for preparing a compound of the formula :

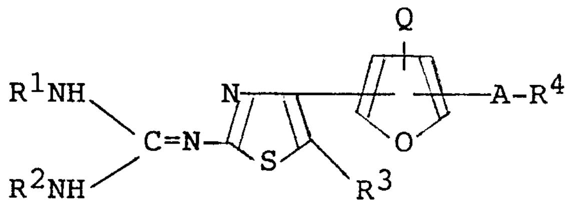

wherein

| | |
|---|---|
| $R^1$ and $R^2$ | are each hydrogen, furoyl or $C_1$-$C_6$ alkyl which may have halogen; or |
| $R^1$ and $R^2$ | are linked together to form $C_1$-$C_6$ alkylene, |
| $R^3$ is | hydrogen or $C_1$-$C_6$ alkyl, |
| $R^4$ is | amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio-($C_1$-$C_6$)alkanoylamino, benzoylamino optionally substituted by nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)alkanoylamino, furyl($C_1$-$C_6$)alkylsulfinyl($C_1$-$C_6$)-alkanoylamino, mono or di($C_1$-$C_6$)alkylsulfamoylamino, hydroxy($C_1$-$C_6$)alkylureido, $C_1$-$C_6$ alkanoyloxy($C_1$-$C_6$)alkylureido, $C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of the formula : 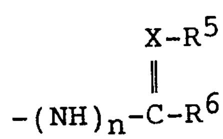 wherein |
| n is | 0 or 1, |
| X is | =CH- or =N-, |
| $R^5$ is | hydrogen, cyano, nitro, carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)-alkylsulfamoyl, |
| $R^6$ is | hydrogen, $C_1$-$C_6$ alkyl, $C_1$-$C_6$ alkylthio, $C_1$-$C_6$ alkoxy, amino, mono or di($C_1$-$C_6$)-alkylamino, allylamino, propargylamino, hydroxy($C_1$-$C_6$)alkylamino, $C_1$-$C_6$ alkoxy($C_1$-$C_6$)alkylamino, or mono or di($C_1$-$C_6$)alkylamino($C_1$-$C_6$)alkylamino; |
| A is | $C_1$-$C_6$ alkylene or -CONH- ; or |
| A-$R^4$ is | imidazolyl substituted with $C_1$-$C_6$ alkyl, triazolyl substituted with amino, and |
| Q is | hydrogen, or $C_1$-$C_6$ alkyl, |

or a salt thereof,
which comprises

(1) reacting a compound of the formula :

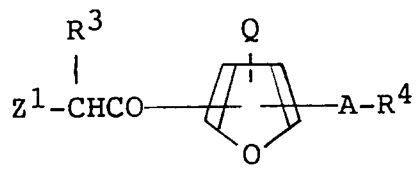

wherein

$R^3$, $R^4$, A and Q are each as defined above, and

$Z^1$ is halogen,

or a salt thereof, with a compound of the formula :

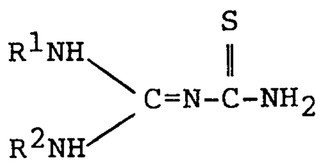

wherein

$R^1$ and $R^2$ are each as defined above,

to give a compound of the formula :

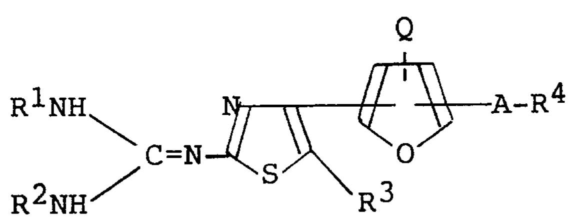

wherein

$R^1$, $R^2$, $R^3$, $R^4$, A and Q are each as defined above,

or a salt thereof, or

(2) subjecting a compound of the formula :

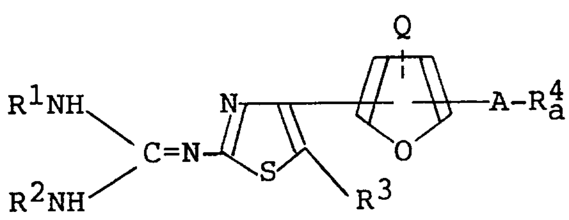

wherein

$R^1$, $R^2$, $R^3$, A and Q are each as defined above, and

$R^4_a$ is ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)-alkanoylamino, benzoylamino optionally substituted with nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ al-

kanoylureido, allylureido, benzoylthioureido, furyl($C_1$-$C_6$)alkylthio($C_1$-$C_6$)-alkanoylamino, mono or di($C_1$-$C_6$)alkylsulfamoylamino, hydroxy($C_1$-$C_6$)-alkylureido, or $C_1$-$C_6$ alkanoyloxy($C_1$-$C_6$)alkylureido,

or a salt thereof, to deacylation reaction, to give a compound of the formula :

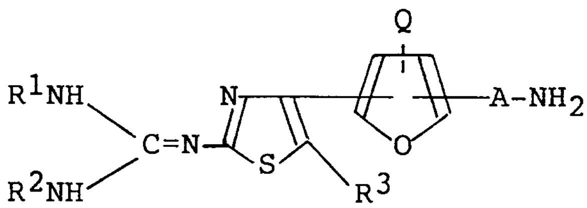

wherein

$R^1$, $R^2$, $R^3$, A and Q are each as defined above,

or a salt thereof, or

(3) subjecting a compound of the formula :

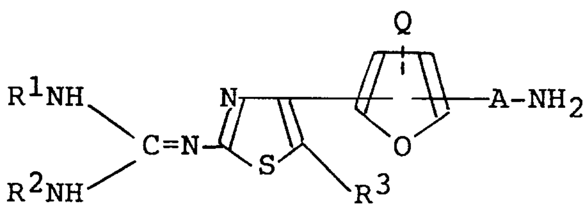

wherein

$R^1$, $R^2$, $R^3$, A and Q are each as defined above,

or a salt thereof, to acylation reaction, to give a compound of the formula :

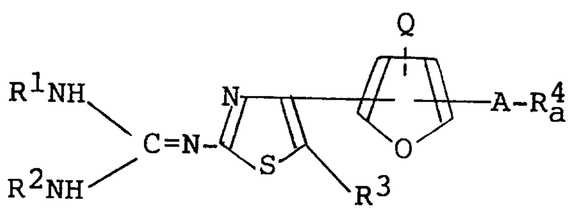

wherein

$R^1$, $R^2$, $R^3$, $R^4_a$, A and Q are each as defined above,

or a salt thereof, or

(4) reacting a compound of the formula :

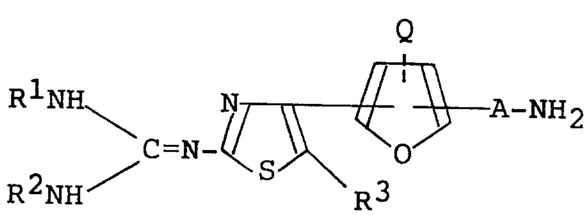

wherein

$R^1$, $R^2$, $R^3$, A and Q are each as defined above,

or a salt thereof, with a compound of the formula :

$(R^7\text{-}X^1)_2C=X\text{-}R^5$

wherein
$R^5$ and X are each as defined above,
$R^7$ is $C_1$-$C_6$ alkyl or phenyl, and
$X^1$ is S or O,
to give a compound of the formula :

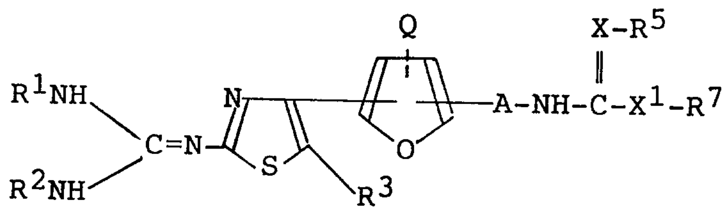

wherein
R1, $R^2$, $R^3$, $R^5$, $R^7$, X, $X^1$, A and Q are each as defined above,
or a salt thereof, or
(5) reacting a compound of the formula :

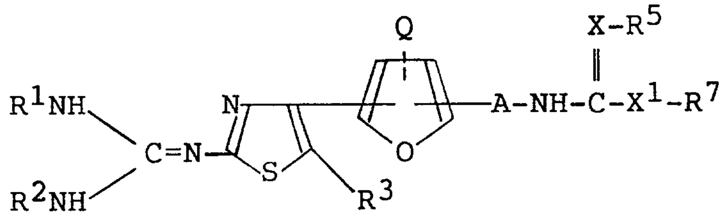

wherein
$R^1$, $R^2$, $R^3$, $R^5$, $R^7$, X, $X^1$, A and Q are each as defined above,
or a salt thereof, with a compound of the formula :

$H\text{-}R^6_a$

wherein
$R^6_a$ is amino which may have mono or di($C_1$-$C_6$)alkyl, allyl, propargyl, hydroxy($C_1$-$C_6$)alkyl or mono or di($C_1$-$C_6$)alkylamino($C_1$-$C_6$)alkyl, or $C_1$-$C_6$ alkoxy,
to give a compound of the formula :

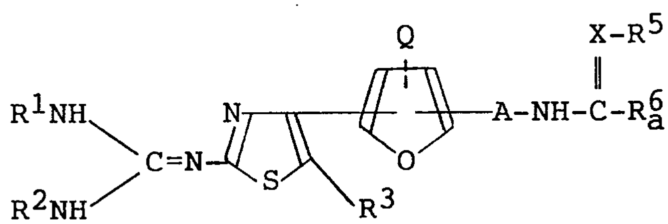

wherein
$R^1$, $R^2$, $R^3$, $R^5$, $R^6_a$, X, A and Q are each as defined above,
or a salt thereof, or

(6) subjecting a compound of the formula :

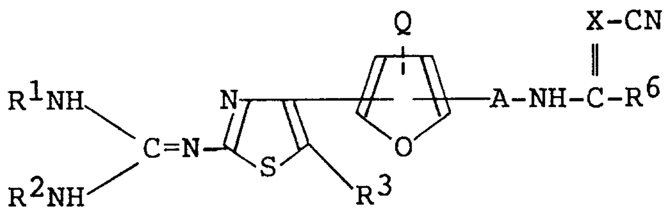

wherein
$R^1$, $R^2$, $R^3$, $R^6$, X, A and Q are each as defined above,
or a salt thereof, to hydrolysis, to give a compound of the formula :

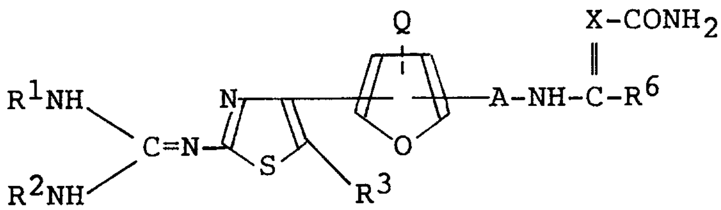

wherein
$R^1$, $R^2$, $R^3$, $R^6$, X, A and Q are each as defined above,
or a salt thereof, or
(7) subjecting a compound of the formula :

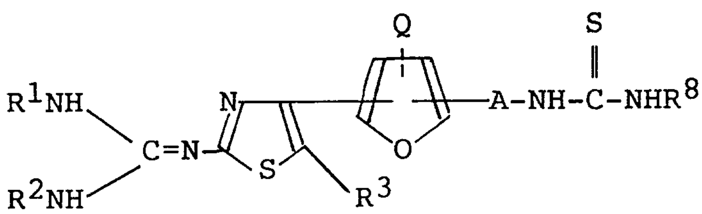

wherein
R1, $R^2$, $R^3$, A and Q are each as defined above, and
$R^8$ is amino-protective group consisting of $C_1$-$C_6$ alkanoyl and benzoyl,
or a salt thereof, to elimination reaction of the amino-protective group, to give a compound of the formula :

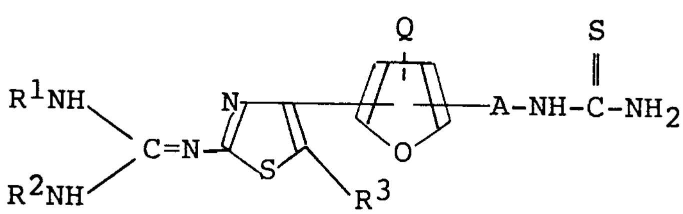

wherein
$R^1$, $R^2$, $R^3$, A and Q are each as defined above,
or a salt thereof, or

(8) reacting a compound of the formula :

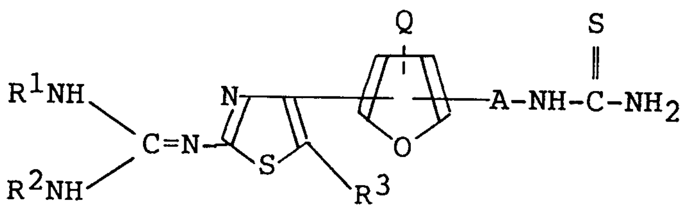

wherein
$R^1$, $R^2$, $R^3$, A and Q are each as defined above,
or a salt thereof, with a compound of the formula :

$Z^2$-$CH_2$-CO-$R^9$

wherein
$R^9$ is $C_1$-$C_6$ alkyl, and
$Z^2$ is halogen,
to give a compound of the formula :

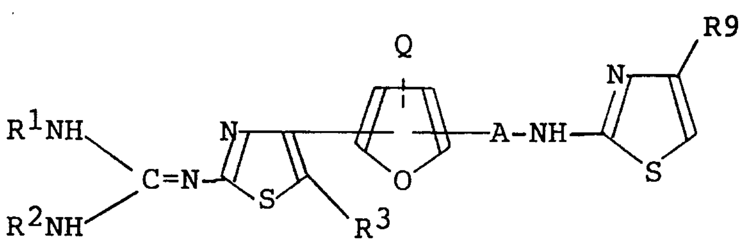

wherein
$R^1$, $R^2$, $R^3$, $R^9$, A and Q are each as defined above,
or a salt thereof, or
(9) reacting a compound of the formula :

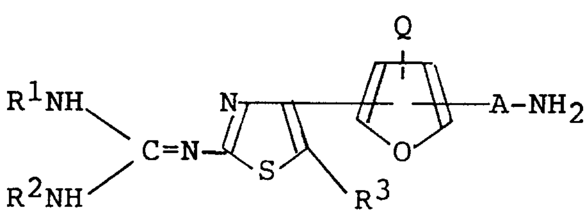

wherein
$R^1$, $R^2$, $R^3$, A and Q are each as defined above,
or a salt thereof, with a compound of the formula :

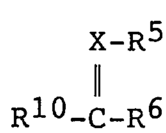

wherein
$R^5$, $R^6$ and X are each as defined above, and
$R^{10}$ is $C_1$-$C_6$ alkanoyloxy, phenyl($C_1$-$C_6$)alkoxy, $C_1$-$C_6$ alkoxy($C_1$-$C_6$)alkoxy,

tetrahydropyranyloxy or $C_1$-$C_6$ alkoxy,
to give a compound of the formula :

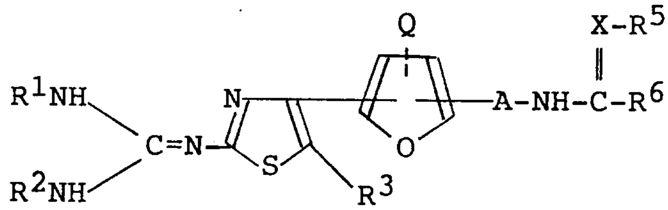

wherein
$R^1$, $R^2$, $R^3$, $R^5$, $R^6$, X, A and Q are each as defined above,
or a salt thereof, or
(10) reacting a compound of the formula :

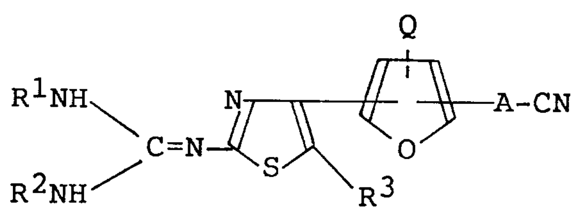

wherein
$R^1$, $R^2$, $R^3$, A and Q are each as defined above,
or a salt thereof, with a compound of the formula :

$R^{11}OH$

wherein
$R^{11}$ is $C_1$-$C_6$ alkyl,
to give a compound of the formula :

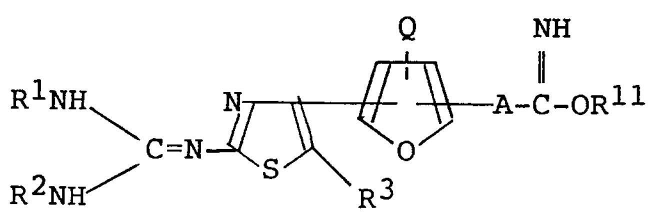

wherein
$R^1$, $R^2$, $R^3$, $R^{11}$, A and Q are each as defined above,
or a salt thereof, or
(11) reacting a compound of the formula :

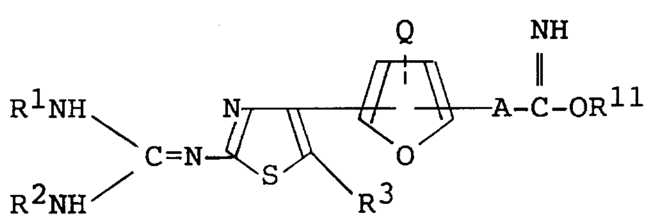

wherein
$R^1$, $R^2$, $R^3$, $R^{11}$, A and Q are each as defined above,
or a salt thereof, with a compound of the formula :

$NH_2R^{12}$

wherein
$R^{12}$ is acyl,
to give a compound of the formula :

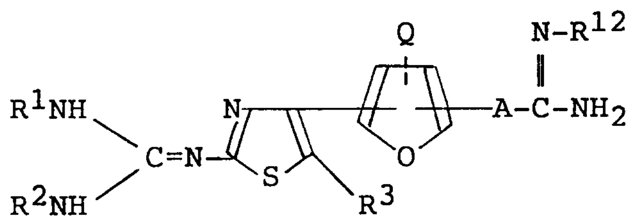

wherein
$R^1$, $R^2$, $R^3$, $R^{12}$, A and Q are each as defined above,
or a salt thereof, or
(12) reacting a compound of the formula :

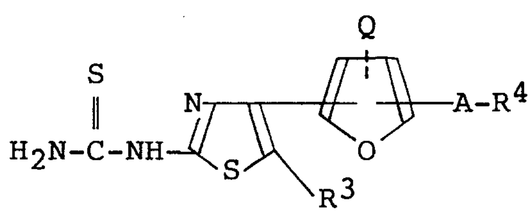

wherein
$R^3$, $R^4$, A and Q are each as defined above,
or a salt thereof, with a compound of the formula :

$R^{13}$-$Z^3$

wherein
$R^{13}$ is $C_1$-$C_6$ alkyl, and
$Z^3$ is halogen,
or a salt thereof, to give a compound of the formula:

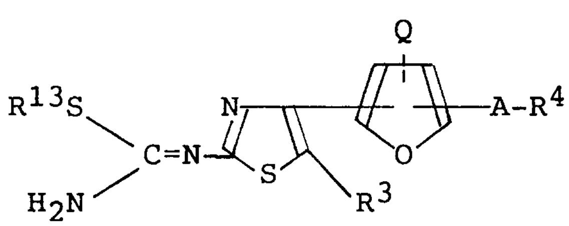

wherein
$R^3$, $R^4$, $R^{13}$, A and Q are each as defined above,
or a salt thereof, and continuously reacting it with a compound of the formula :

$H_2N$-Y-$NH_2$

wherein

Y is $C_1$-$C_6$ alkylene,

or a salt thereof,

to give a compound of the formula :

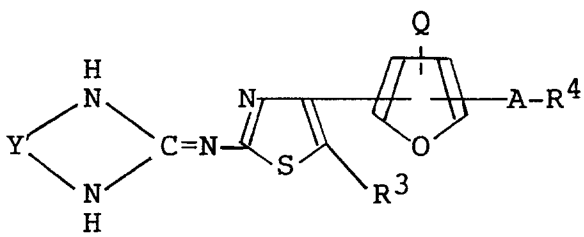

wherein

$R^3$, $R^4$, A, Q and Y are each as defined above,

or a salt thereof, or

(13) subjecting a compound of the formula :

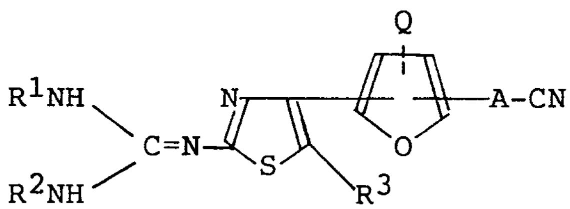

wherein

$R^1$, $R^2$, $R^3$, A and Q are each as defined above,

or a salt thereof, to reduction, to give a compound of the formula :

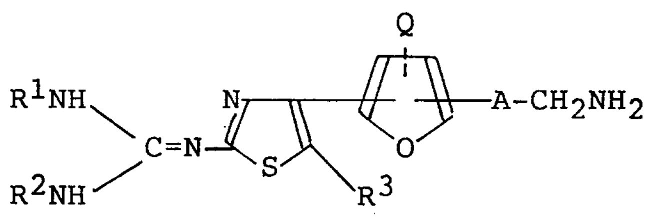

wherein

$R^1$, $R^2$, $R^3$, A and Q are each as defined above,

or a salt thereof, or

(14) reacting a compound of the formula :

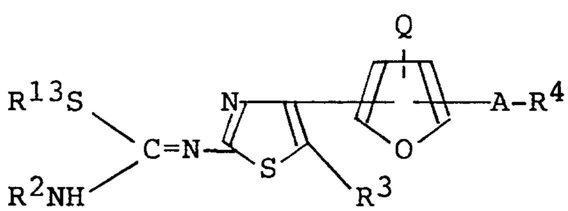

wherein

$R^2$, $R^3$, $R^4$, $R^{13}$, A and Q are each as defined above,
or a salt thereof, with a compound of the formula :

$NH_2R^1_a$

wherein
$R^1_a$ is $C_1$-$C_6$ alkyl which may have halogen,
or a salt thereof, to give a compound of the formula:

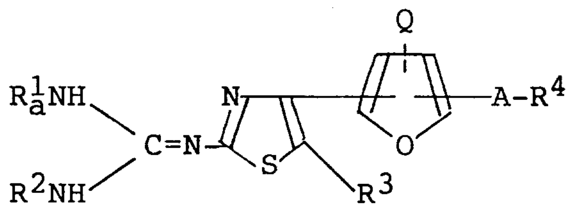

wherein
$R^1_a$, $R^2$, $R^3$, $R^4$, A and Q are each as defined above,
or a salt thereof, or
(15) subjecting a compound of the formula :

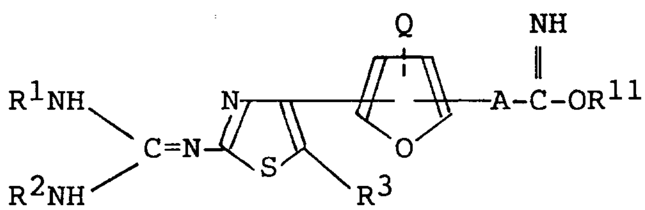

wherein
$R^1$, $R^2$, $R^3$, $R^{11}$, A and Q are each as defined above,
or a salt thereof, to hydrolysis, to give a compound of the formula :

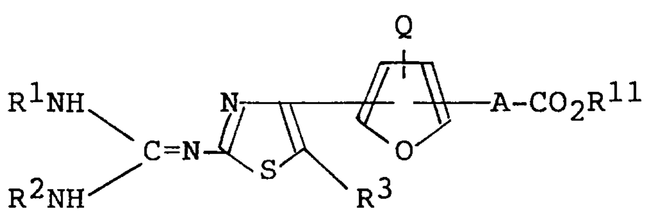

wherein
$R^1$, $R^2$, $R^3$, $R^{11}$, A and Q are each as defined above,
or a salt thereof, or

(16) subjecting a compound of the formula :

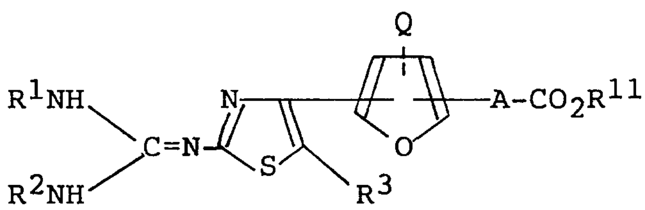

wherein

$R^1$, $R^2$, $R^3$, $R^{11}$, A and Q are each as defined above,

or a salt thereof, to amidation reaction, to give a compound of the formula :

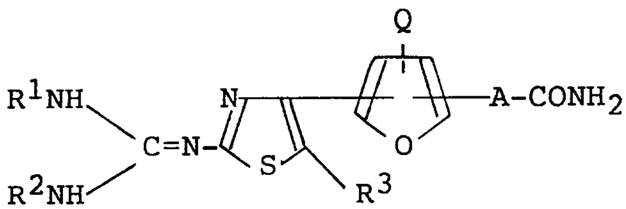

wherein

$R^1$, $R^2$, $R^3$, A and Q are each as defined above,

or a salt thereof, or

(17) reacting a compound of the formula :

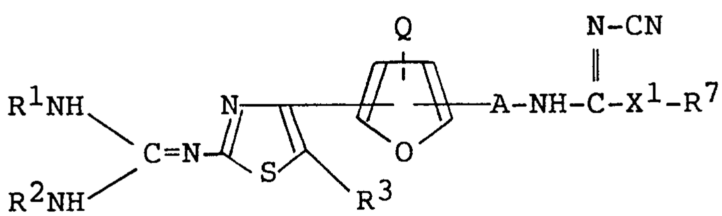

wherein

$R^1$, $R^2$, $R^3$, $R^7$, $X^1$, A and Q are each as defined above,

or a salt thereof, with a compound of the formula :

$R^{14}NHNH_2$

wherein

$R^{14}$ is hydrogen or $C_1$-$C_6$ alkyl,

to give a compound of the formula :

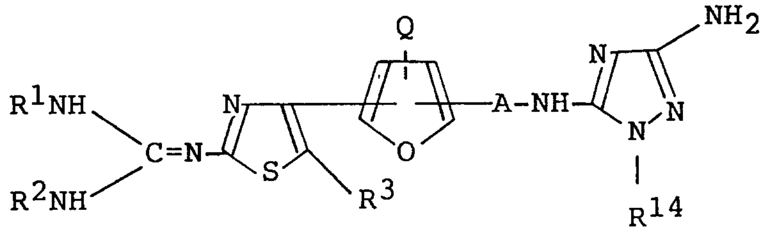

wherein

R1, $R^2$, $R^3$, $R^{14}$, A and Q are each as defined above,

or a salt thereof, or

(18) reacting a compound of the formula :

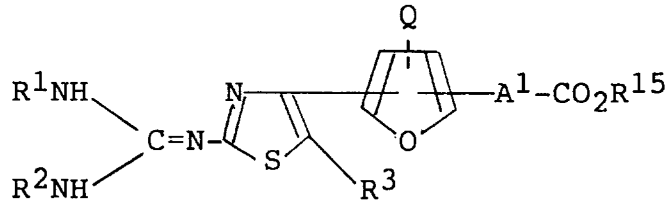

wherein

$R^1$, $R^2$, $R^3$ and Q are each as defined above,

$R^{15}$ is $C_1$-$C_6$ alkyl, and

$A^1$ is $C_1$-$C_6$ alkylene or bond,

or a salt thereof, with hydrazine, to give a compound of the formula :

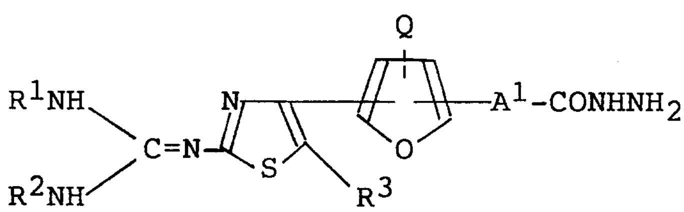

wherein

$R^1$, $R^2$, $R^3$, $A^1$ and Q are each as defined above,

or a salt thereof, or

(19) reacting a compound of the formula :

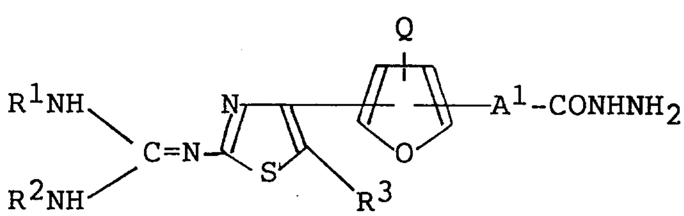

wherein

R1, $R^2$, $R^3$, $A^1$ and Q are each as defined above,

or a salt thereof, with S-$C_1$-$C_6$ alkylisothiourea

or a salt thereof, to give a compound of the formula:

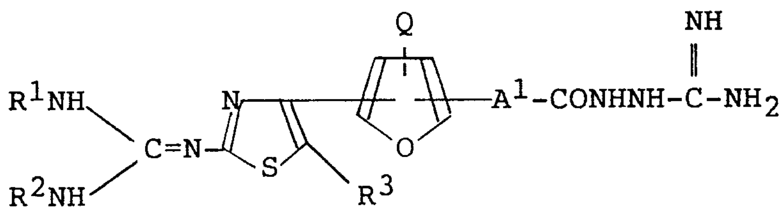

wherein

$R^1$, $R^2$, $R^3$, $A^1$ and Q are each as defined above,

or a salt thereof, or

(20) subjecting a compound of the formula :

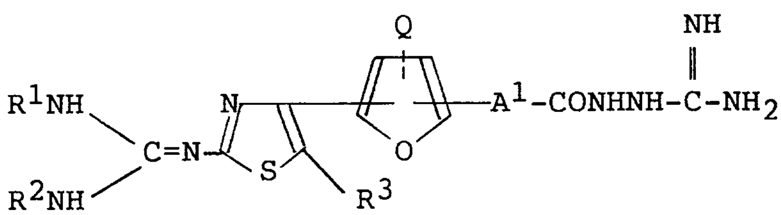

wherein

$R^1$, $R^2$, $R^3$, $A^1$ and Q are each as defined above,

or a salt thereof, to ring closure, to give a compound of the formula :

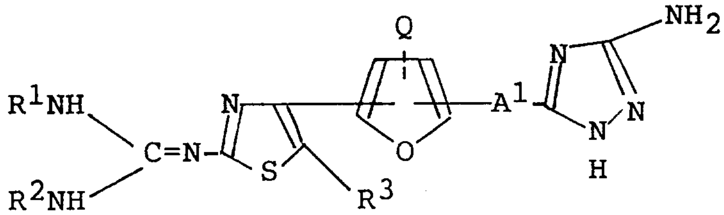

wherein

$R^1$, $R^2$, $R^3$, $A^1$ and Q are each as defined above,

or a salt thereof, or

(21) reacting a compound of the formula :

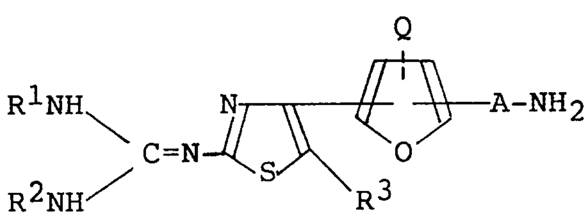

wherein

$R^1$, $R^2$, $R^3$, A and Q are each as defined above,

or a salt thereof, with a compound of the formula :

$R^{16}$-$Z^4$

wherein

$R^{16}$ is thiazolyl substituted with $C_1$-$C_6$ alkyl, triazolyl substituted with amino, triazolyl substituted with amino and $C_1$-$C_6$ alkyl, benzoisothiazolyl substituted with oxo, or benzothiadiazinyl substituted with oxo, and

$Z^4$ is halogen,

or a salt thereof, to give a compound of the formula:

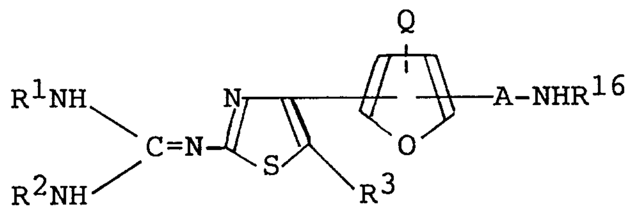

wherein

$R^1$, $R^2$, $R^3$, $R^{16}$, A and Q are each as defined above,

or a salt thereof, or

(22) subjecting a compound of the formula :

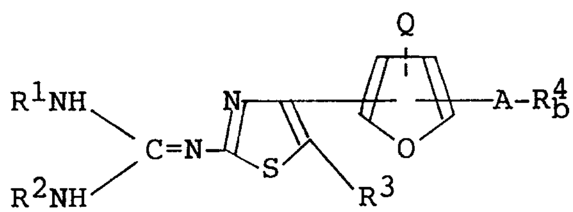

wherein

$R^1$, $R^2$, $R^3$, A and Q are each as defined above, and

$R^4_b$ is furyl($C_1$-$C_6$)alkylthio($C_1$-$C_6$)alkanoylamino,

or a salt thereof, to oxidation reaction, to give a compound of the formula :

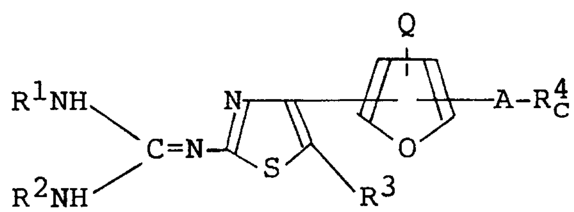

wherein

$R^1$, $R^2$, $R^3$, A and Q are each as defined above, and

$R^4_c$ is furyl($C_1$-$C_6$)alkylsulfinyl($C_1$-$C_6$)alkanoylamino,

or a salt thereof, or

(23) reacting a compound of the formula :

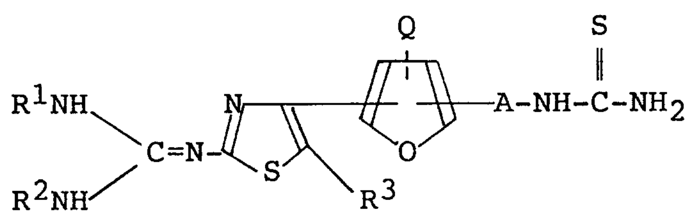

wherein

R1, $R^2$, $R^3$, A and Q are each as defined above,

or a salt thereof, with a compound of the formula :

$R^{17}$-$Z^5$

wherein

$R^{17}$ is $C_1$-$C_6$ alkyl, and

$Z^5$ is halogen,

or a salt thereof, to give a compound of the formula:

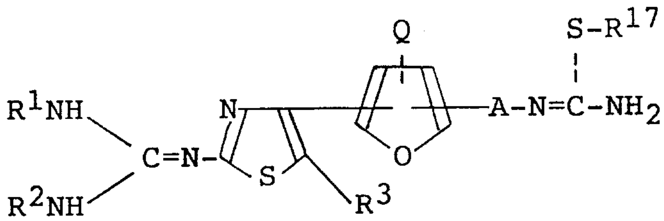

wherein

$R^1$, $R^2$, $R^3$, $R^{17}$, A and Q are each as defined above,

or a salt thereof, or

(24) reacting a compound of the formula :

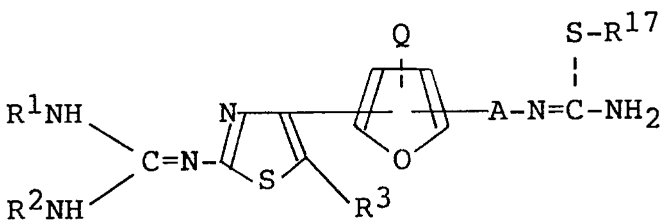

wherein

$R^1$, $R^2$, $R^3$, $R^{17}$, A and Q are each as defined above,

or a salt thereof, with a compound of the formula :

$H_2N$-R5

wherein

$R^5$ is as defined above,

or a salt thereof, to give a compound of the formula:

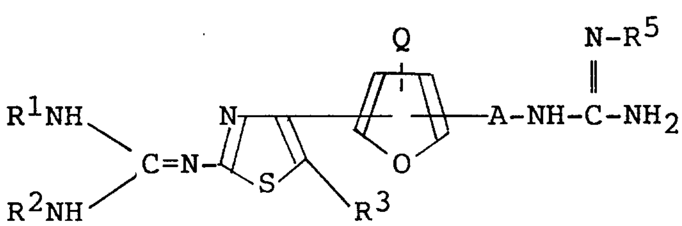

wherein

$R^1$, $R^2$, $R^3$, $R^5$, A and Q are each as defined above,

or a salt thereof, or

(25) subjecting a compound of the formula :

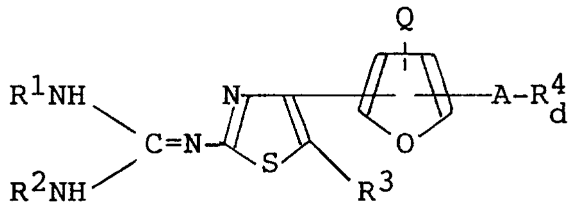

wherein

$R^1$, $R^2$, $R^3$, A and Q are each as defined above, and

$R^4_d$ is $C_1$-$C_6$ alkanoyloxy($C_1$-$C_6$)alkylureido,

or a salt thereof, to elimination reaction of $C_1$-$C_6$ alkanoyl, to give a compound of the formula :

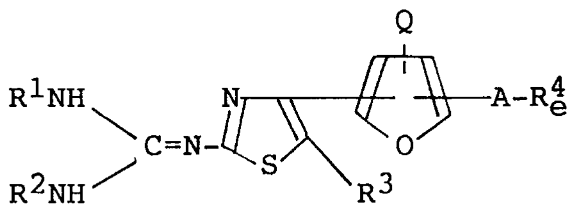

wherein

R1, $R^2$, $R^3$, A and Q are each as defined above; and

$R^4_e$ is hydroxy($C_1$-$C_6$)alkylureido,

or a salt thereof, or

(26) reacting a compound of the formula :

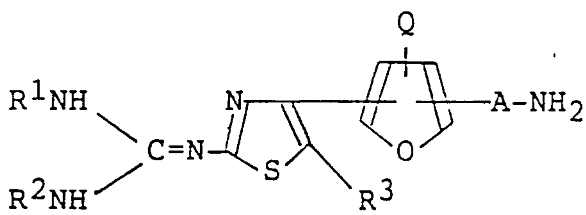

wherein

$R^1$, $R^2$, $R^3$, A and Q are each as defined above,

or a salt thereof, with a compound of the formula :

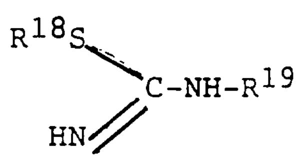

wherein

$R^{18}$ is $C_1$-$C_6$ alkyl, and

$R^{19}$ is carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)alkylsulfamoyl,

or a salt thereof, to give a compound of the formula:

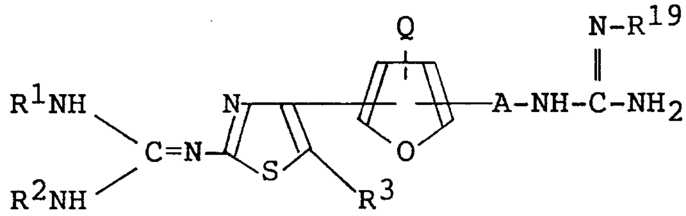

wherein

$R^1$, $R^2$, $R^3$, $R^{19}$, A and Q are each as defined above,

or a salt thereof, or

(27) subjecting a compound of the formula :

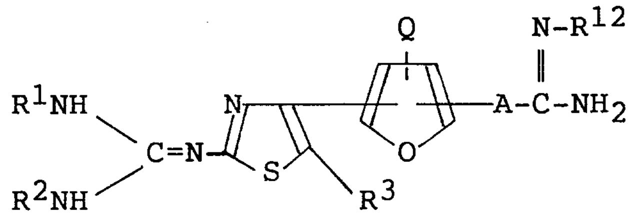

wherein

$R^1$, $R^2$, $R^3$, $R^{12}$, A and Q are each as defined above,

or a salt thereof, to hydrolysis, to give a compound of the formula :

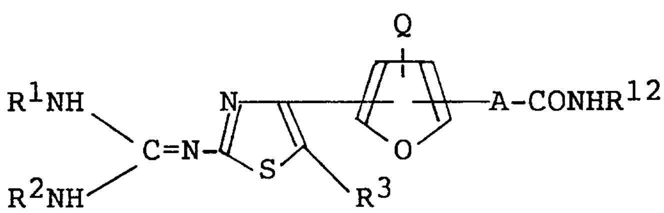

wherein

$R^1$, $R^2$, $R^3$, $R^{12}$, A and Q are each as defined above,

or a salt thereof.

**2.** A process according to claim 1, wherein $R^1$ and $R^2$ are each hydrogen, $C_1$-$C_6$ alkyl or trihalo($C_1$-$C_6$)-alkyl; or

$R^1$ and $R^2$ are linked together to form $C_1$-$C_6$ alkylene,

$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)alkanoylamino, nitrobenzoylamino, furoylamino, thenoylamino, nicotinoylamino, 1-oxonicotinoylamino, morpholinocarbonylamino, 3-$C_1$-$C_6$ alkylureido, 3-$C_1$-$C_6$ alkylthioureido, 3-$C_1$-$C_6$ alkanoylureido, 3-allylureido, 3-benzoylthioureido, furyl($C_1$-$C_6$)alkylthio($C_1$-$C_6$)alkanoylamino, furyl($C_1$-$C_6$)alkylsulfinyl($C_1$-$C_6$)alkanoylamino, mono or di($C_1$-$C_6$)alkylsulfamoylamino, 3-hydroxy($C_1$-$C_6$)-alkylureido, 3-$C_1$-$C_6$ alkanoyloxy($C_1$-$C_6$)alkylureido, 2-$C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of

the formula :

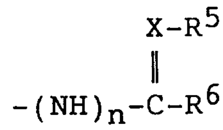

wherein

n, X and $R^6$ are as defined in claim 1,

$R^5$ is hydrogen, cyano, nitro, carbamoyl, $C_1$-$C_6$ alkoxycarbonyl, sulfamoyl, $C_1$-$C_6$ alkylsulfonyl, phenylsulfonyl substituted with $C_1$-$C_6$ alkyl, phenylsulfonyl substituted with $C_1$-$C_6$ alkoxy, phenylsulfonyl substituted with halogen, phenylsulfonyl substituted with amino, mono or di($C_1$-$C_6$)alkylsulfamoyl,

A is as defined in claim 1 ; or

A-$R^4$ is as defined in claim 1, and

$R^3$ and Q are each as defined in claim 1.

**3.** A process according to claim 2, wherein

$R^1$ and $R^2$ are each hydrogen,

$R^3$ is hydrogen,

$R^4$ is a group of the formula :

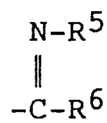

wherein

$R^5$ is sulfamoyl, and

$R^6$ is amino,

A is $C_1$-$C_6$ alkylene, and

Q is hydrogen.

**4.** A process according to claim 2, wherein

$R^1$ and $R^2$ are each hydrogen,

$R^3$ is hydrogen,

$R^4$ is ureido or 3-$C_1$-$C_6$ alkylureido,

A is $C_1$-$C_6$ alkylene, and

Q is hydrogen.

**5.** A process for the manufacture of a pharmaceutical composition which comprises, as an active ingredient, a compound of claim 1 or a pharmaceutically acceptable salt thereof characterized by mixing a compound of claim 1 with at least one pharmaceutically acceptable carrier or excipient.

**6.** A process for the manufacture of a composition according to claim 5 for the therapeutic treatment of ulcer in human beings or animals.

**Claims for the following Contracting State : GR**

**1.** A process for preparing a compound of the formula :

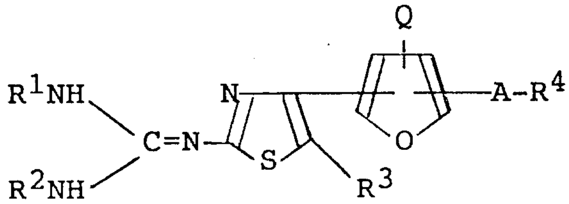

(I)

wherein

$R^1$ and $R^2$ are each hydrogen, furoyl or $C_1$-$C_6$ alkyl which may have halogen; or

$R^1$ and $R^2$ are linked together to form $C_1$-$C_6$ alkylene,

$R^3$ is hydrogen or $C_1$-$C_6$ alkyl,

$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio-($C_1$-$C_6$)alkanoylamino, benzoylamino optionally substituted by nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)alkanoylamino, furyl($C_1$-$C_6$)alkylsulfinyl($C_1$-$C_6$)-alkanoylamino, mono or di($C_1$-$C_6$)alkylsulfamoylamino, hydroxy($C_1$-$C_6$)alkylureido, $C_1$-$C_6$ alkanoyloxy($C_1$-$C_6$)alkylureido, $C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl substituted with oxo and $C_1$-$C_6$ alkyl, triazolyl substituted with amino, or a group of the formula :

$$-(NH)_n-\underset{\displaystyle}{C}(=X-R^5)-R^6$$

wherein

n is 0 or 1,

X is =CH- or =N-,

$R^5$ is hydrogen, cyano, nitro, carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)-alkylsulfamoyl,

$R^6$ is hydrogen, $C_1$-$C_6$ alkyl, $C_1$-$C_6$ alkylthio, $C_1$-$C_6$ alkoxy, amino, mono or di($C_1$-$C_6$)-alkylamino, allylamino, propargylamino, hydroxy($C_1$-$C_6$)alkylamino, $C_1$-$C_6$ alkoxy($C_1$-$C_6$)alkylamino, or mono or di($C_1$-$C_6$)alkylamino($C_1$-$C_6$)alkylamino;

A is $C_1$-$C_6$ alkylene or -CONH- ; or

A-$R^4$ is imidazolyl substituted with $C_1$-$C_6$ alkyl, triazolyl substituted with amino, and

Q is hydrogen, or $C_1$-$C_6$ alkyl,

or a salt thereof,
which comprises

(1) reacting a compound of the formula :

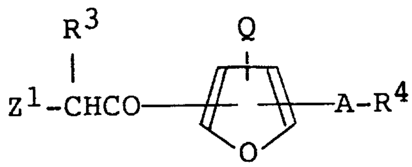

wherein

$R^3$, $R^4$, A and Q are each as defined above, and

$Z^1$ is halogen,

or a salt thereof, with a compound of the formula :

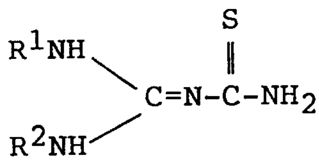

wherein

$R^1$ and $R^2$ are each as defined above,

to give a compound of the formula :

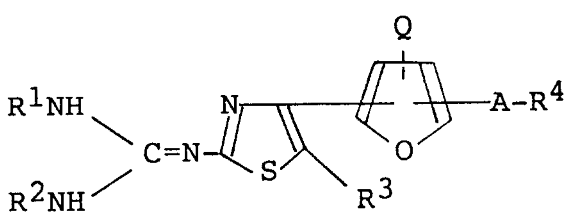

wherein

$R^1$, $R^2$, $R^3$, $R^4$, A and Q are each as defined above,

or a salt thereof, or

(2) subjecting a compound of the formula :

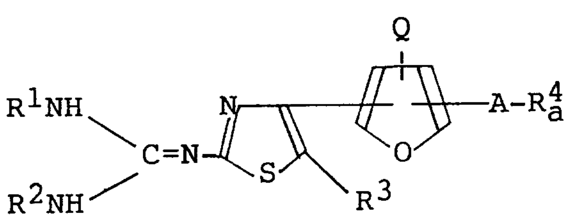

wherein

$R^1$, $R^2$, $R^3$, A and Q are each as defined above, and

$R^4_a$ is ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)-alkanoylamino, benzoylamino optionally substituted with nitro, furoylamino, thenoylamino, nicotinoylamino, 1-oxonicotinoylamino, morpholinocarbonylamino, $C_1$-$C_6$ alkylureido, $C_1$-$C_6$ alkylthioureido, $C_1$-$C_6$ alkanoylureido, allylureido, benzoylthioureido, furyl($C_1$-$C_6$)alkylthio($C_1$-$C_6$)-

alkanoylamino, mono or di($C_1$-$C_6$)alkylsulfamoylamino, hydroxy($C_1$-$C_6$)-alkylureido, or $C_1$-$C_6$ alkanoyloxy($C_1$-$C_6$)alkylureido,

or a salt thereof, to deacylation reaction, to give a compound of the formula :

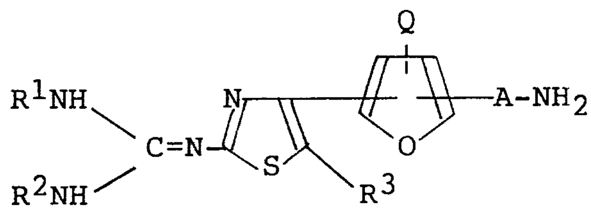

wherein

$R^1$, $R^2$, $R^3$, A and Q are each as defined above,

or a salt thereof, or

(3) subjecting a compound of the formula :

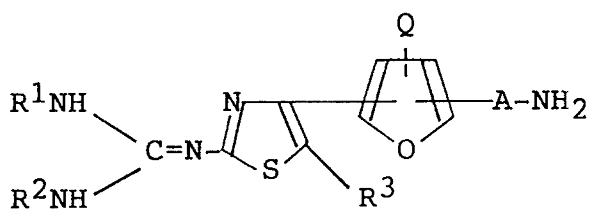

wherein

$R^1$, $R^2$, $R^3$, A and Q are each as defined above,

or a salt thereof, to acylation reaction, to give a compound of the formula :

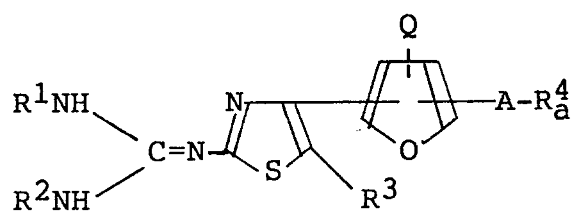

wherein

$R^1$, $R^2$, $R^3$, $R^4_a$ , A and Q are each as defined above,

or a salt thereof, or

(4) reacting a compound of the formula :

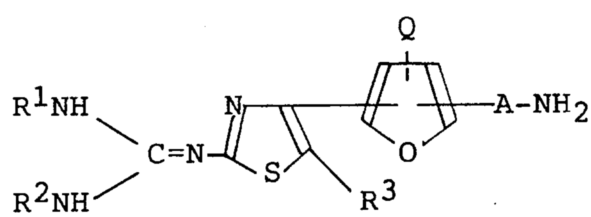

wherein

$R^1$, $R^2$, $R^3$, A and Q are each as defined above,

or a salt thereof, with a compound of the formula :

$(R^7\text{-}X^1)_2C=X\text{-}R^5$

wherein

| | |
|---|---|
| $R^5$ and X | are each as defined above, |
| $R^7$ is | $C_1$-$C_6$ alkyl or phenyl, and |
| $X^1$ is | S or O, |

to give a compound of the formula :

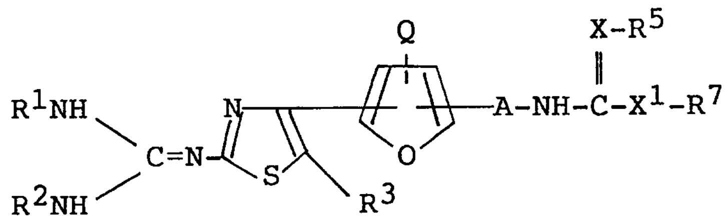

wherein

$R1$, $R^2$, $R^3$, $R^5$, $R^7$, X, $X^1$, A and Q  are each as defined above,

or a salt thereof, or

(5) reacting a compound of the formula :

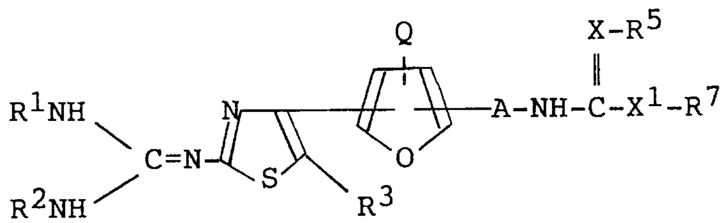

wherein

$R^1$, $R^2$, $R^3$, $R^5$, $R^7$, X, $X^1$, A and Q  are each as defined above,

or a salt thereof, with a compound of the formula :

$H\text{-}R^6_a$

wherein

$R^6_a$ is  amino which may have mono or di($C_1$-$C_6$)alkyl, allyl, propargyl, hydroxy($C_1$-$C_6$)alkyl or mono or di($C_1$-$C_6$)alkylamino($C_1$-$C_6$)alkyl, or $C_1$-$C_6$ alkoxy,

to give a compound of the formula :

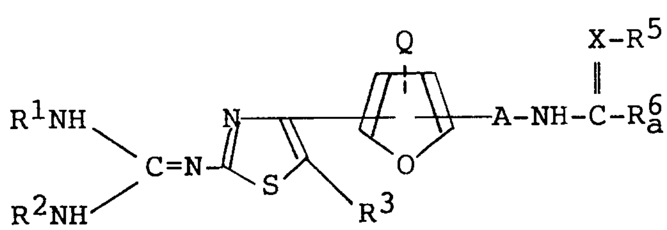

wherein

$R^1$, $R^2$, $R^3$, $R^5$, $R^6_a$ , X, A and Q  are each as defined above,

or a salt thereof, or

(6) subjecting a compound of the formula :

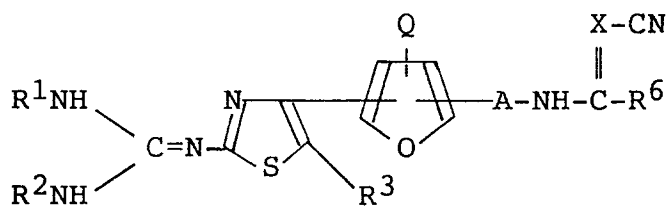

wherein

$R^1$, $R^2$, $R^3$, $R^6$, X, A and Q are each as defined above,

or a salt thereof, to hydrolysis, to give a compound of the formula :

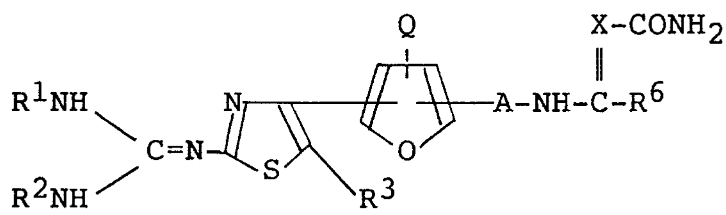

wherein

$R^1$, $R^2$, $R^3$, $R^6$, X, A and Q are each as defined above,

or a salt thereof, or

(7) subjecting a compound of the formula :

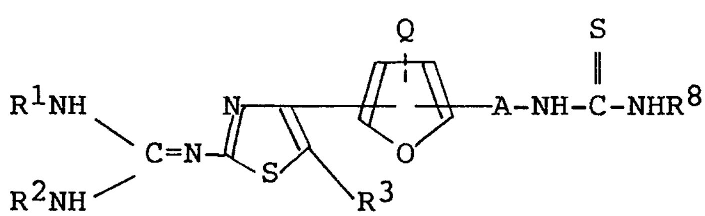

wherein

R1, $R^2$, $R^3$, A and Q are each as defined above, and

$R^8$ is amino-protective group consisting of $C_1$-$C_6$ alkanoyl and benzoyl,

or a salt thereof, to elimination reaction of the amino-protective group, to give a compound of the formula :

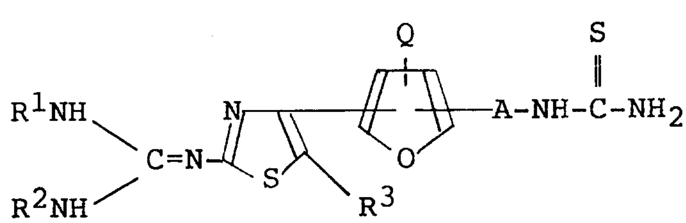

wherein

$R^1$, $R^2$, $R^3$, A and Q are each as defined above,

or a salt thereof, or

(8) reacting a compound of the formula :

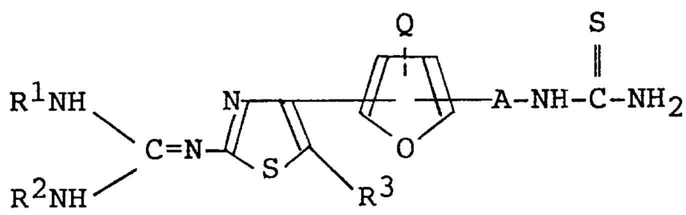

wherein
$R^1$, $R^2$, $R^3$, A and Q are each as defined above,
or a salt thereof, with a compound of the formula :

$Z^2$-$CH_2$-CO-$R^9$

wherein
$R^9$ is $C_1$-$C_6$ alkyl, and
$Z^2$ is halogen,
to give a compound of the formula :

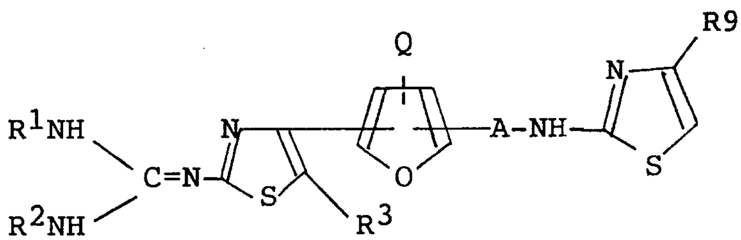

wherein
$R^1$, $R^2$, $R^3$, $R^9$, A and Q are each as defined above,
or a salt thereof, or
(9) reacting a compound of the formula :

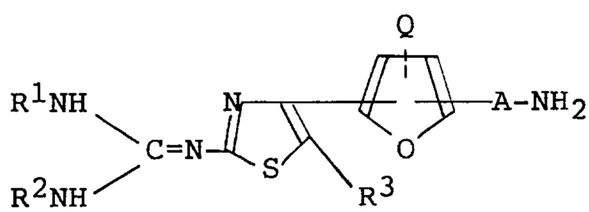

wherein
$R^1$, $R^2$, $R^3$, A and Q are each as defined above,
or a salt thereof, with a compound of the formula :

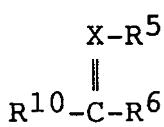

wherein
$R^5$, $R^6$ and X are each as defined above, and

$R^{10}$ is $C_1$-$C_6$ alkanoyloxy, phenyl($C_1$-$C_6$)alkoxy, $C_1$-$C_6$ alkoxy($C_1$-$C_6$)alkoxy, tetrahydropyranyloxy or $C_1$-$C_6$ alkoxy,

to give a compound of the formula :

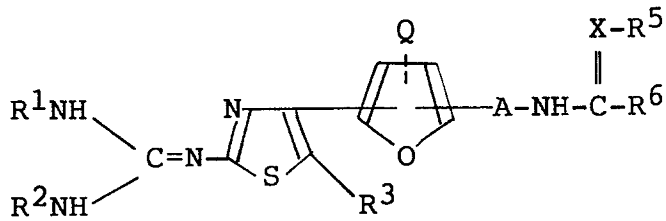

wherein

$R^1$, $R^2$, $R^3$, $R^5$, $R^6$, X, A and Q are each as defined above,

or a salt thereof, or

(10) reacting a compound of the formula :

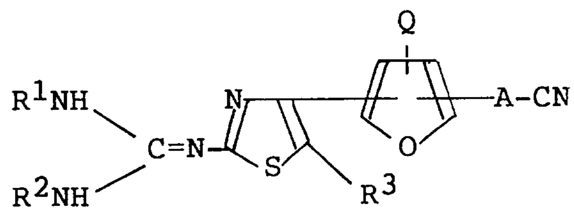

wherein

$R^1$, $R^2$, $R^3$, A and Q are each as defined above,

or a salt thereof, with a compound of the formula :

$R^{11}OH$

wherein

$R^{11}$ is $C_1$-$C_6$ alkyl,

to give a compound of the formula :

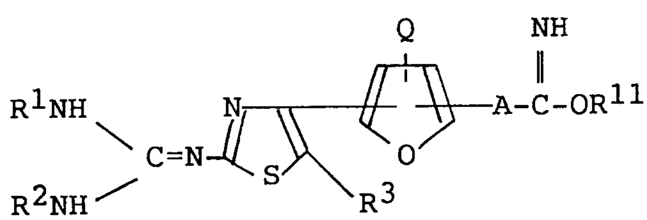

wherein

$R^1$, $R^2$, $R^3$, $R^{11}$, A and Q are each as defined above,

or a salt thereof, or

(11) reacting a compound of the formula :

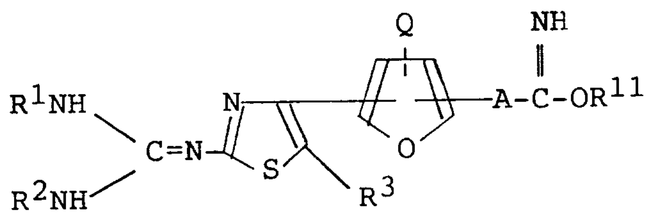

wherein
$R^1$, $R^2$, $R^3$, $R^{11}$, A and Q are each as defined above,
or a salt thereof, with a compound of the formula :

$NH_2R^{12}$

wherein
$R^{12}$ is acyl,
to give a compound of the formula :

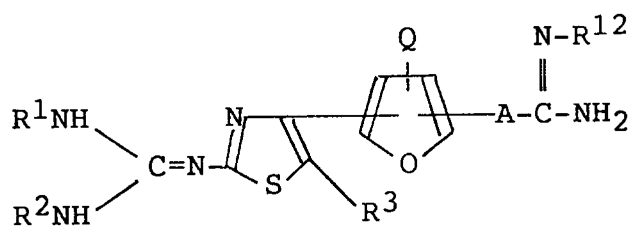

wherein
$R^1$, $R^2$, $R^3$, $R^{12}$, A and Q are each as defined above,
or a salt thereof, or
(12) reacting a compound of the formula :

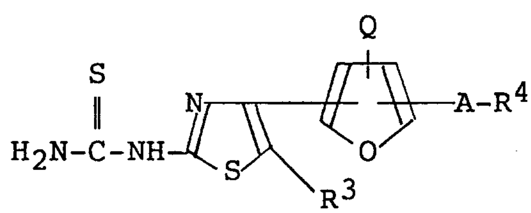

wherein
$R^3$, $R^4$, A and Q are each as defined above,
or a salt thereof, with a compound of the formula :

$R^{13}$-$Z^3$

wherein
$R^{13}$ is $C_1$-$C_6$ alkyl, and
$Z^3$ is halogen,
or a salt thereof, to give a compound of the formula:

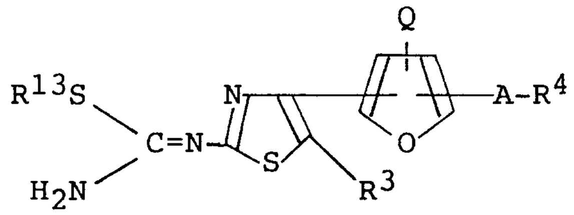

wherein

$R^3$, $R^4$, $R^{13}$, A and Q are each as defined above,

or a salt thereof, and continuously reacting it with a compound of the formula :

$H_2N$-Y-$NH_2$

wherein

Y is $C_1$-$C_6$ alkylene,

or a salt thereof,

to give a compound of the formula :

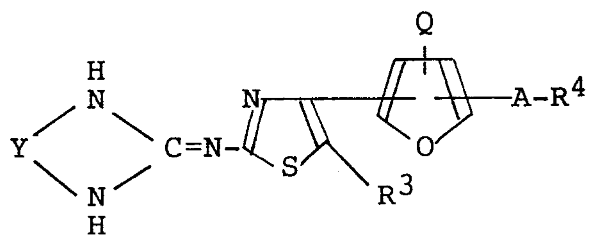

wherein

$R^3$, $R^4$, A, Q and Y are each as defined above,

or a salt thereof, or

(13) subjecting a compound of the formula :

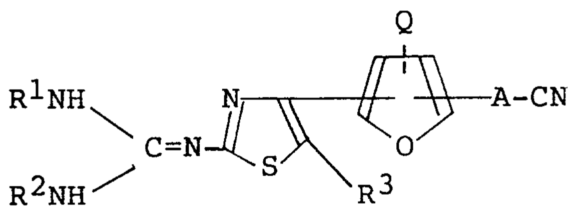

wherein

$R^1$, $R^2$, $R^3$, A and Q are each as defined above,

or a salt thereof, to reduction, to give a compound of the formula :

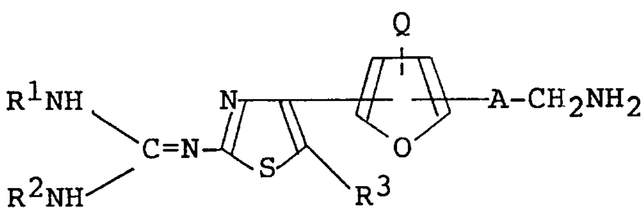

wherein

$R^1$, $R^2$, $R^3$, A and Q are each as defined above,
or a salt thereof, or
(14) reacting a compound of the formula :

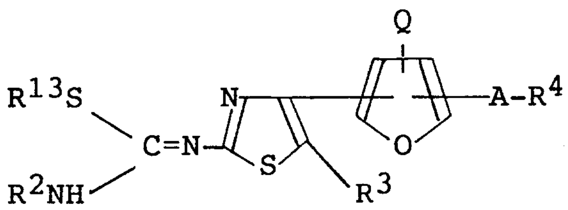

wherein
$R^2$, $R^3$, $R^4$, $R^{13}$, A and Q are each as defined above,
or a salt thereof, with a compound of the formula :

$NH_2R^1_a$

wherein
$R^1_a$ is $C_1$-$C_6$ alkyl which may have halogen,
or a salt thereof, to give a compound of the formula:

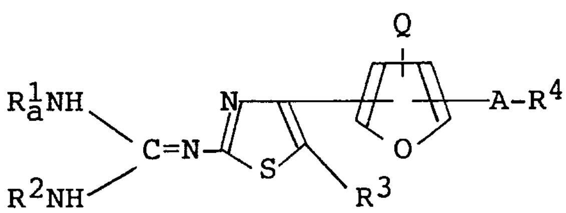

wherein
$R^1_a$, $R^2$, $R^3$, $R^4$, A and Q are each as defined above,
or a salt thereof, or
(15) subjecting a compound of the formula :

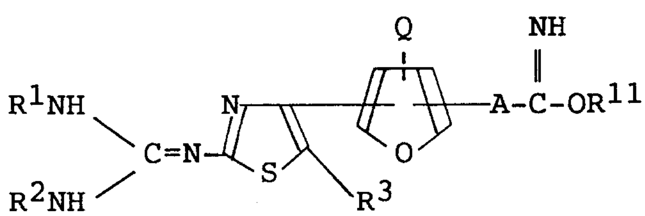

wherein
$R^1$, $R^2$, $R^3$, $R^{11}$, A and Q are each as defined above,
or a salt thereof, to hydrolysis, to give a compound of the formula :

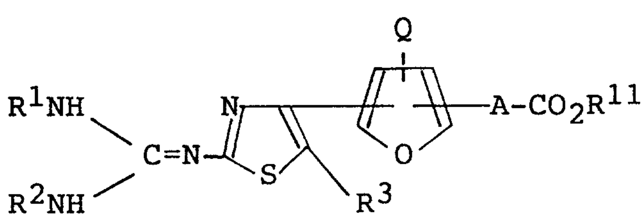

wherein

$R^1$, $R^2$, $R^3$, $R^{11}$, A and Q are each as defined above,

or a salt thereof, or

(16) subjecting a compound of the formula :

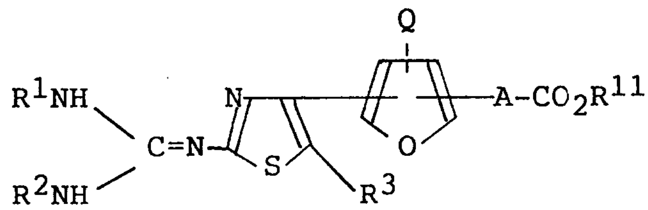

wherein

$R^1$, $R^2$, $R^3$, $R^{11}$, A and Q are each as defined above,

or a salt thereof, to amidation reaction, to give a compound of the formula :

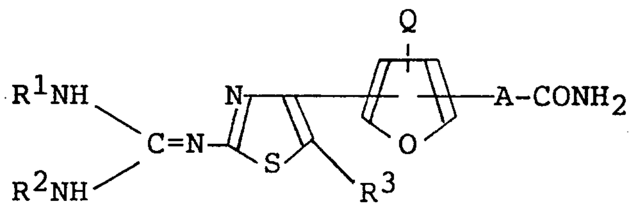

wherein

$R^1$, $R^2$, $R^3$, A and Q are each as defined above,

or a salt thereof, or

(17) reacting a compound of the formula :

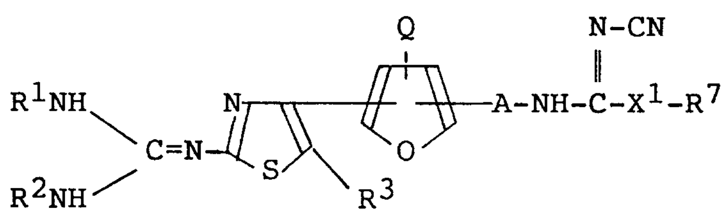

wherein

$R^1$, $R^2$, $R^3$, $R^7$, $X^1$, A and Q are each as defined above,

or a salt thereof, with a compound of the formula :

$R^{14}NHNH_2$

wherein

$R^{14}$ is hydrogen or $C_1$-$C_6$ alkyl,

to give a compound of the formula :

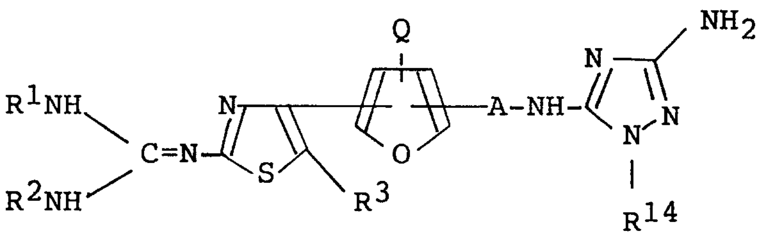

wherein

R1, $R^2$, $R^3$, $R^{14}$, A and Q are each as defined above,

or a salt thereof, or

(18) reacting a compound of the formula :

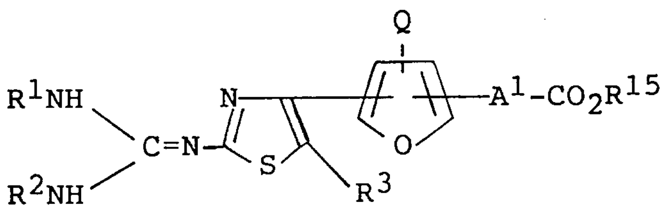

wherein

$R^1$, $R^2$, $R^3$ and Q are each as defined above,

$R^{15}$ is $C_1$-$C_6$ alkyl, and

$A^1$ is $C_1$-$C_6$ alkylene or bond,

or a salt thereof, with hydrazine, to give a compound of the formula :

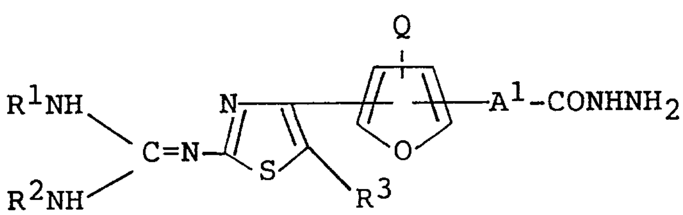

wherein

$R^1$, $R^2$, $R^3$, $A^1$ and Q are each as defined above,

or a salt thereof, or

(19) reacting a compound of the formula :

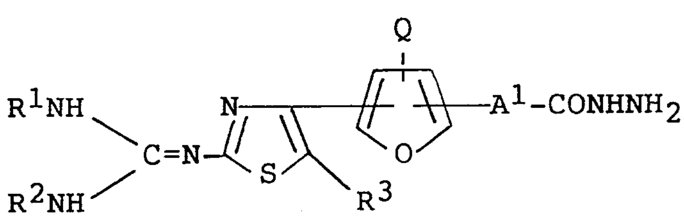

wherein

R1, $R^2$, $R^3$, $A^1$ and Q are each as defined above,

or a salt thereof, with S-$C_1$-$C_6$ alkylisothiourea

or a salt thereof, to give a compound of the formula:

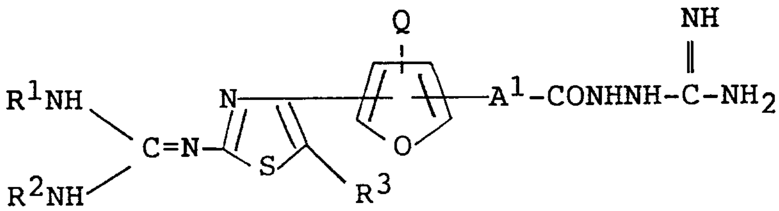

wherein
$R^1$, $R^2$, $R^3$, $A^1$ and Q are each as defined above,
or a salt thereof, or
(20) subjecting a compound of the formula :

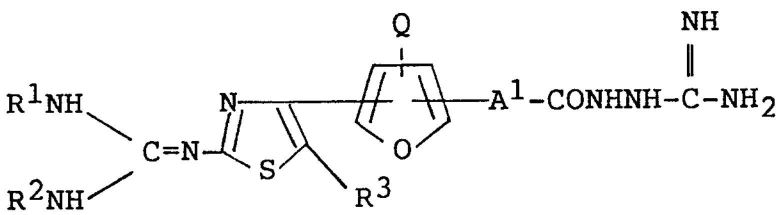

wherein
$R^1$, $R^2$, $R^3$, $A^1$ and Q are each as defined above,
or a salt thereof, to ring closure, to give a compound of the formula :

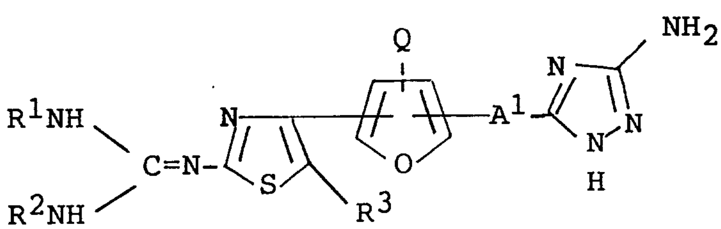

wherein
$R^1$, $R^2$, $R^3$, $A^1$ and Q are each as defined above,
or a salt thereof, or
(21) reacting a compound of the formula :

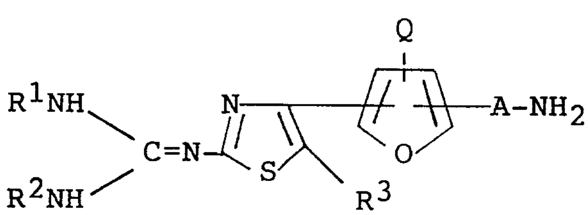

wherein
$R^1$, $R^2$, $R^3$, A and Q are each as defined above,
or a salt thereof, with a compound of the formula :

$R^{16}$-$Z^4$

wherein

$R^{16}$ is thiazolyl substituted with $C_1$-$C_6$ alkyl, triazolyl substituted with amino, triazolyl substituted with amino and $C_1$-$C_6$ alkyl, benzoisothiazolyl substituted with oxo, or benzothiadiazinyl substituted with oxo, and

$Z^4$ is halogen,

or a salt thereof, to give a compound of the formula:

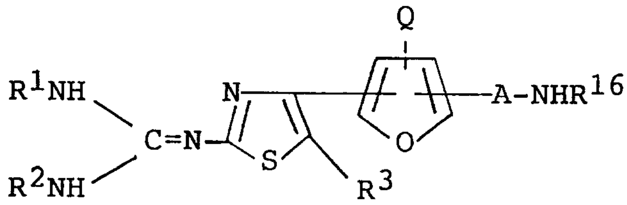

wherein

$R^1$, $R^2$, $R^3$, $R^{16}$, A and Q are each as defined above,

or a salt thereof, or

(22) subjecting a compound of the formula :

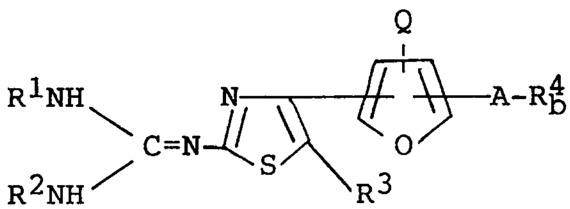

wherein

$R^1$, $R^2$, $R^3$, A and Q are each as defined above, and

$R^4_b$ is furyl($C_1$-$C_6$)alkylthio($C_1$-$C_6$)alkanoylamino,

or a salt thereof, to oxidation reaction, to give a compound of the formula :

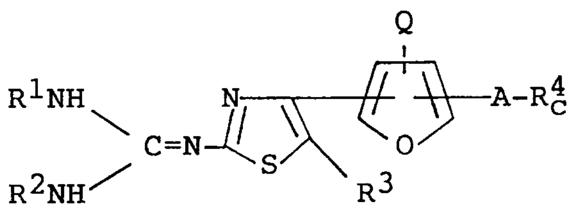

wherein

$R^1$, $R^2$, $R^3$, A and Q are each as defined above, and

$R^4_c$ is furyl($C_1$-$C_6$)alkylsulfinyl($C_1$-$C_6$)alkanoylamino,

or a salt thereof, or

(23) reacting a compound of the formula :

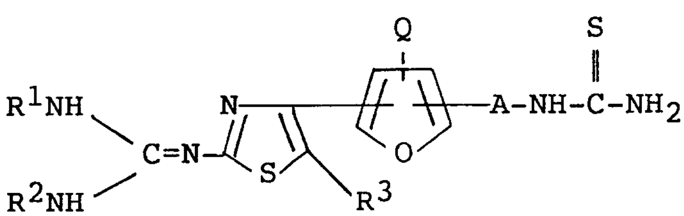

wherein

R1, $R^2$, $R^3$, A and Q are each as defined above,

or a salt thereof, with a compound of the formula :

$R^{17}$-$Z^5$

wherein

$R^{17}$ is $C_1$-$C_6$ alkyl, and

$Z^5$ is halogen,

or a salt thereof, to give a compound of the formula:

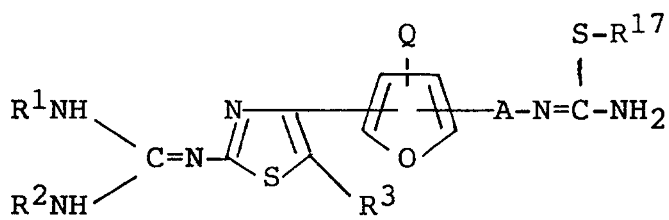

wherein

$R^1$, $R^2$, $R^3$, $R^{17}$, A and Q are each as defined above,

or a salt thereof, or

(24) reacting a compound of the formula :

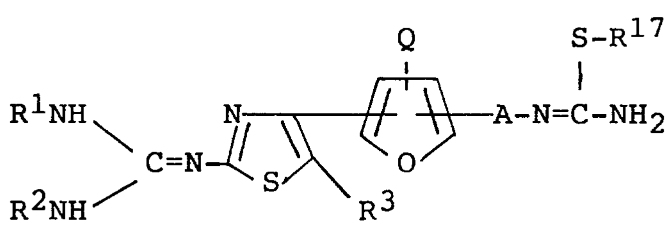

wherein

$R^1$, $R^2$, $R^3$, $R^{17}$, A and Q are each as defined above,

or a salt thereof, with a compound of the formula :

$H_2N$-R5

wherein

$R^5$ is as defined above,

or a salt thereof, to give a compound of the formula:

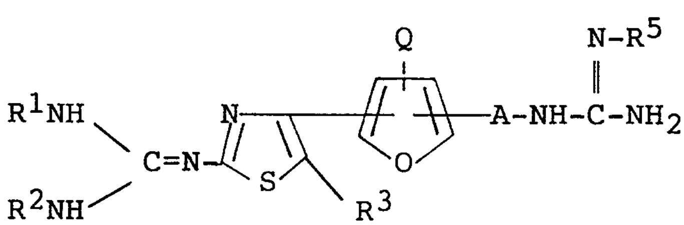

wherein

$R^1$, $R^2$, $R^3$, $R^5$, A and Q are each as defined above,

or a salt thereof, or

(25) subjecting a compound of the formula :

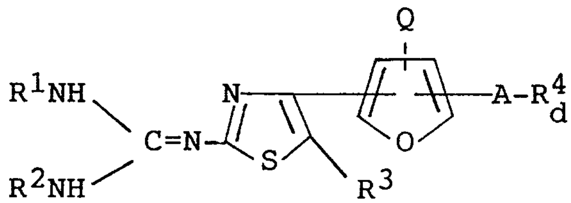

wherein
$R^1$, $R^2$, $R^3$, A and Q are each as defined above, and
$R^4_d$ is $C_1$-$C_6$ alkanoyloxy($C_1$-$C_6$)alkylureido,
or a salt thereof, to elimination reaction of $C_1$-$C_6$ alkanoyl, to give a compound of the formula :

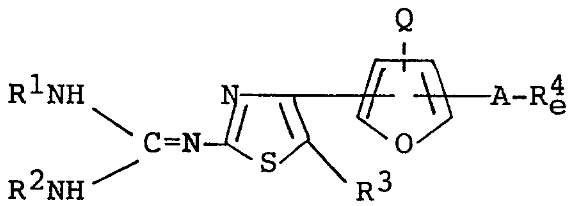

wherein
R1, $R^2$, $R^3$, A and Q are each as defined above, and
$R^4_e$ is hydroxy($C_1$-$C_6$)alkylureido,
or a salt thereof, or
(26) reacting a compound of the formula :

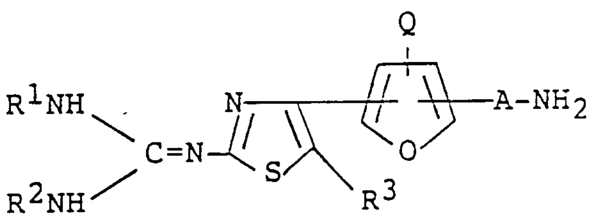

wherein
$R^1$, $R^2$, $R^3$, A and Q are each as defined above,
or a salt thereof, with a compound of the formula :

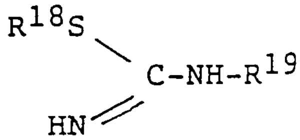

wherein
$R^{18}$ is $C_1$-$C_6$ alkyl, and
$R^{19}$ is carbamoyl, $C_1$-$C_6$ alkoxycarbonyl, sulfamoyl, $C_1$-$C_6$ alkylsulfonyl, benzenesulfonyl, which is substituted with one or more substituent(s) selected from $C_1$-$C_6$ alkyl, $C_1$-$C_6$ alkoxy, halogen and amino, or mono or di($C_1$-$C_6$)alkylsulfamoyl,
or a salt thereof, to give a compound of the formula:

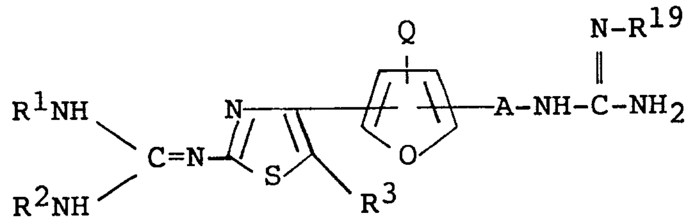

wherein

$R^1$, $R^2$, $R^3$, $R^{19}$, A and Q are each as defined above,

or a salt thereof, or

(27) subjecting a compound of the formula :

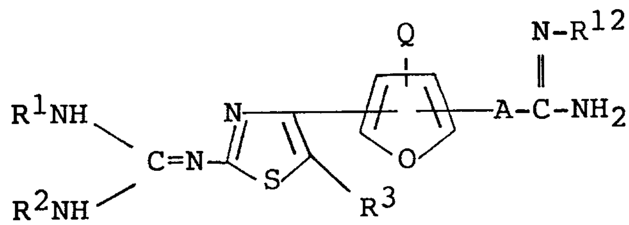

wherein

$R^1$, $R^2$, $R^3$, $R^{12}$, A and Q are each as defined above,

or a salt thereof, to hydrolysis, to give a compound of the formula :

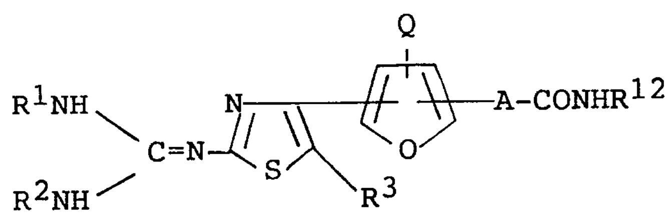

wherein

$R^1$, $R^2$, $R^3$, $R^{12}$, A and Q are each as defined above,

or a salt thereof.

**2.** A process according to claim 1, wherein

$R^1$ and $R^2$ are each hydrogen, $C_1$-$C_6$ alkyl or trihalo($C_1$-$C_6$)alkyl; or

$R^1$ and $R^2$ are linked together to form $C_1$-$C_6$ alkylene,

$R^4$ is amino, carbamoyl, aminocarbamoyl, guanidinocarbamoyl, $C_1$-$C_6$ alkylcarbamoyl, sulfamoylaminocarbonyl, $C_1$-$C_6$ alkoxycarbonyl, ureido, thioureido, sulfamoylamino, $C_1$-$C_6$ alkanoylamino, $C_1$-$C_6$ alkoxycarbonylamino, $C_1$-$C_6$ alkylsulfonylamino, $C_1$-$C_6$ alkoxy($C_1$-$C_6$)alkanoylamino, $C_1$-$C_6$ alkanoyloxy($C_1$-$C_6$)alkanoylamino, $C_1$-$C_6$ alkylthio($C_1$-$C_6$)alkanoylamino, nitrobenzoylamino, furoylamino, thenoylamino, nicotinoylamino, 1-oxonicotinoylamino, morpholinocarbonylamino, 3-$C_1$-$C_6$ alkylureido, 3-$C_1$-$C_6$ alkylthioureido, 3-$C_1$-$C_6$ alkanoylureido, 3-allylureido, 3-benzoylthioureido, furyl($C_1$-$C_6$)alkylthio($C_1$-$C_6$)alkanoylamino, furyl($C_1$-$C_6$)alkylsulfinyl($C_1$-$C_6$)alkanoylamino, mono or di($C_1$-$C_6$)alkylsulfamoylamino, 3-hydroxy($C_1$-$C_6$)-alkylureido, 3-$C_1$-$C_6$ alkanoyloxy($C_1$-$C_6$)alkylureido, 2-$C_1$-$C_6$ alkylisothioureido, thiazolylamino substituted with $C_1$-$C_6$ alkyl, triazolylamino substituted with amino, triazolylamino substituted with amino and $C_1$-$C_6$ alkyl, benzoisothiazolylamino substituted with oxo, benzothiadiazinylamino substituted with oxo and halogen, pyrimidinyl

substituted with oxo and $C_1$-$C_6$ alkyl, triazolyl substituted with amino, or a group of the formula :

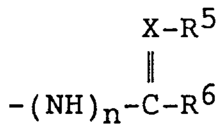

wherein

n, X and $R^6$ are as defined in claim 1,

$R^5$ is hydrogen, cyano, nitro, carbamoyl, $C_1$-$C_6$ alkoxycarbonyl, sulfamoyl, $C_1$-$C_6$ alkylsulfonyl, phenylsulfonyl substituted with $C_1$-$C_6$ alkyl, phenylsulfonyl substituted with $C_1$-$C_6$ alkoxy, phenylsulfonyl substituted with halogen, phenylsulfonyl substituted with amino, mono or di($C_1$-$C_6$)alkylsulfamoyl,

A is as defined in claim 1 ; or

A-$R^4$ is as defined in claim 1, and

$R^3$ and Q are each as defined in claim 1.

**3.** A process according to claim 2, wherein

$R^1$ and $R^2$ are each hydrogen,

$R^3$ is hydrogen,

$R^4$ is a group of the formula :

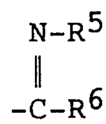

wherein

$R^5$ is sulfamoyl, and

$R^6$ is amino,

A is $C_1$-$C_6$ alkylene, and

Q is hydrogen.

**4.** A process according to claim 2, wherein

$R^1$ and $R^2$ are each hydrogen,

$R^3$ is hydrogen,

$R^4$ is ureido or 3-$C_1$-$C_6$ alkylureido,

A is $C_1$-$C_6$ alkylene, and

Q is hydrogen.

**5.** A process for the manufacture of a pharmaceutical composition comprising as an active ingredient a compound of formula I according to claim 1 characterized by mixing a compound of formula I with at least one pharmaceutically acceptable non-toxic carrier or excipient.

**6.** Modification of the process claimed in claim 1 which is characterized by bringing a compound of the formula I or a non-toxic salt thereof produced by a process claimed in claim 1, into pharmaceutically acceptable form by admixture or presentation of said compound with a pharmaceutically acceptable diluent or carrier.

**7.** Use of a compound I according to claim 1 for the manufacture of a medicament for therapeutic treatment of ulcer in human beings or animals.

**Patentansprüche**

**Patentansprüche für folgende Vertragsstaaten : AT, BE, CH, DE, FR, GB, IT, LI, LU, NL, SE**

1. Verbindung der Formel:

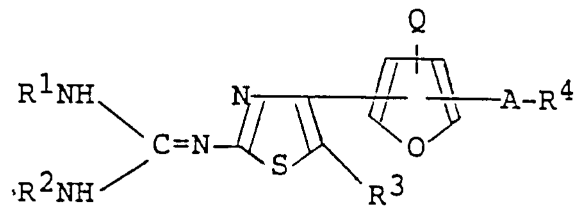

worin $R^1$ und $R^2$ jeweils Wasserstoff, Furoyl oder $(C_1-C_6)$Alkyl, das Halogen aufweisen kann, sind; oder $R^1$ und $R^2$ miteinander verbunden sind, um $(C_1-C_6)$Alkylen zu bilden, $R^3$ Wasserstoff oder $(C_1-C_6)$Alkyl ist, $R^4$ ist Amino, Carbamoyl, Aminocarbamoyl, Guanidinocarbamoyl, $(C_1-C_6)$Alkylcarbamoyl, Sulfamoylaminocarbonyl, $(C_1-C_6)$Alkoxycarbonyl, Ureido, Thioureido, Sulfamoylamino, $(C_1-C_6)$Alkanoylamino, $(C_1-C_6)$Alkoxycarbonylamino, $(C_1-C_6)$Alkylsulfonylamino, $(C_1-C_6)$Alkoxy$(C_1-C_6)$alkanoylamino, $(C_1-C_6)$-Alkanoyloxy$(C_1-C_6)$alkanoylamino, $(C_1-C_6)$Alkylthio$(C_1-C_6)$alkanoylamino, Benzoylamino wahlweise substituiert durch Nitro, Furoylamino, Thenoylamino, Nicotinoylamino, 1-Oxonicotinoylamino, Morpholinocarbonylamino, $(C_1-C_6)$Alkylureido, $(C_1-C_6)$Alkylthioureido, $(C_1-C_6)$Alkanoylureido, Allylureido, Benzoylthioureido, Furyl$(C_1-C_6)$alkylthio$(C_1-C_6)$alkanoylamino, Furyl$(C_1-C_6)$alkylsulfinyl$(C_1-C_6)$alkanoylamino, Mono- oder Di$(C_1-C_6)$Alkylsulfamoylamino, Hydroxy$(C_1-C_6)$alkylureido, $(C_1-C_6)$Alkanoyloxy$(C_1-C_6)$-alkylureido, $(C_1-C_6)$Alkylisothioureido, Thiazolylamino substituiert mit $(C_1-C_6)$Alkyl, Triazolylamino substituiert mit Amino, Triazolylamino substituiert mit Amino und $(C_1-C_6)$Alkyl, Benzoisothiazolylamino substituiert mit Oxo, Benzothiadiazinylamino substituiert mit Oxo und Halogen, Pyrimidinyl substituiert mit Oxo und $(C_1-C_6)$Alkyl, Triazolyl substituiert mit Amino oder einer Gruppe der Formel:

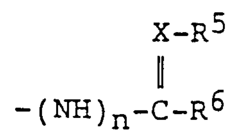

worin n 0 oder 1 ist, X =CH- oder =N- ist, $R^5$ Wasserstoff, Cyano, Nitro, Carbamoyl, $(C_1-C_6)$-Alkoxycarbonyl, Sulfamoyl, $(C_1-C_6)$Alkylsulfonyl, Benzolsulfonyl, das mit einem oder mehreren Substiuenten substituiert ist, ausgewählt aus $(C_1-C_6)$Alkyl, $(C_1-C_6)$Alkoxy, Halogen und Amino, oder Mono- oder Di$(C_1-C_6)$Alkylsulfamoyl ist, $R^6$ Wasserstoff, $(C_1-C_6)$Alkyl, $(C_1-C_6)$Alkylthio, $(C_1-C_6)$Alkoxy, Amino, Mono- oder Di$(C_1-C_6)$Alkylamino, Allylamino, Propargylamino, Hydroxy$(C_1-C_6)$Alkylamino, $(C_1-C_6)$-Alkoxy$(C_1-C_6)$alkylamino, oder Mono- oder Di$(C_1-C_6)$Alkylamino$(C_1-C_6)$alkylamino ist; A $(C_1-C_6)$Alkylen oder -CONH- ist; oder A-$R^4$ Imidazolyl substituiert mit $(C_1-C_6)$Alkyl, Triazolyl substituiert mit Amino ist, und Q Wasserstoff oder $(C_1-C_6)$Alkyl ist, und pharmazeutisch verträgliche Salze davon.

2. Verbindung nach Anspruch 1, worin $R^1$ und $R^2$ jeweils Wasserstoff, $(C_1-C_6)$Alkyl oder Trihalogen$(C_1-C_6)$alkyl sind; oder $R^1$ und $R^2$ miteinander verbunden sind, um $(C_1-C_6)$Alkylen zu bilden, $R^4$ Amino, Carbamoyl, Aminocarbamoyl, Guanidinocarbamoyl, $(C_1-C_6)$Alkylcarbamoyl, Sulfamoylaminocarbonyl, $(C_1-C_6)$-Alkoxycarbonyl, Ureido, Thioureido, Sulfamoylamino, $(C_1-C_6)$Alkanoylamino, $(C_1-C_6)$-Alkoxycarbonylamino, $(C_1-C_6)$Alkylsulfonylamino, $(C_1-C_6)$Alkoxy$(C_1-C_6)$alkanoylamino, $(C_1-C_6)$-Alkanoyloxy$(C_1-C_6)$alkanoylamino, $(C_1-C_6)$Alkylthio$(C_1-C_6)$alkanoylamino, Nitrobenzoylamino, Furoylamino, Thenoylamino, Nicotinoylamino, 1-Oxonicotinoylamino, Morpholinocarbonylamino, 3-$(C_1-C_6)$-Alkylureido, 3-$(C_1-C_6)$Alkylthioureido, 3-$(C_1-C_6)$Alkanoylureido, 3-Allylureido, 3-Benzoylthioureido, Furyl-$(C_1-C_6)$alkylthio$(C_1-C_6)$alkanoylamino, Furyl$(C_1-C_6)$alkylsulfinyl$(C_1-C_6)$alkanoylamino, Mono- oder Di-$(C_1-C_6)$Alkylsulfamoylamino, 3-Hydroxy$(C_1-C_6)$alkylureido, 3-$(C_1-C_6)$Alkanoyloxy$(C_1-C_6)$alkylureido, 2-$(C_1-C_6)$Alkylisothioureido, Thiazolylamino substituiert mit $(C_1-C_6)$Alkyl, Triazolylamino substituiert mit Amino, Triazolylamino substituiert mit Amino und $(C_1-C_6)$Alkyl, Benzoisothiazolylamino substituiert mit Oxo, Benzothiadiazinylamino substituiert mit Oxo und Halogen, Pyrimidinyl substituiert mit Oxo und $(C_1-C_6)$Alkyl, Triazolyl substituiert mit Amino, oder eine Gruppe der Formel:

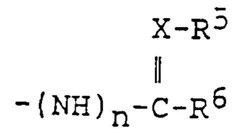

worin n, X und $R^6$ wie in Anspruch 1 definiert sind, ist, $R^5$ Wasserstoff, Cyano, Nitro, Carbamoyl, ($C_1$-$C_6$)Alkoxycarbonyl, Sulfamoyl, ($C_1$-$C_6$)Alkylsulfonyl, Phenylsulfonyl substituiert mit ($C_1$-$C_6$)Alkyl, Phenylsulfonyl substituiert mit ($C_1$-$C_6$)Alkoxy, Phenylsulfonyl substituiert mit Halogen, Phenylsulfonyl substituiert mit Amino, Mono- oder Di($C_1$-$C_6$)Alkylsulfamoyl ist, A wie in Anspruch 1 definiert ist; oder A-$R^4$ wie in Anspruch 1 definiert ist, und $R^3$ und Q jeweils wie in Anspruch 1 definiert sind.

**3.** Verbindung nach Anspruch 2, worin $R^1$ und $R^2$ jeweils Wasserstoff sind, $R^3$ Wasserstoff ist, $R^4$ eine Gruppe der Formel ist:

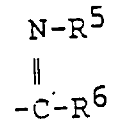

worin $R^5$ Sulfamoyl ist, und $R^6$ Amino ist, A ($C_1$-$C_6$)Alkylen und Q Wasserstoff ist.

**4.** Verbindung nach Anspruch 3, die 4-[5-(2-Amino-2-sulfamoyliminoethyl)furan-2-yl]-2-(diaminomethylenamino)thiazol oder ihr Hydrochlorid oder ihr Methansulfonat ist.

**5.** Verbindung nach Anspruch 2, worin $R^1$ und $R^2$ jeweils Wasserstoff sind, $R^3$ Wasserstoff ist, $R^4$ Ureido oder 3-($C_1$-$C_6$)Alkylureido ist, A ($C_1$-$C_6$)Alkylen ist und Q Wasserstoff ist.

**6.** Verbindung nach Anspruch 5, die aus der Gruppe ausgewählt ist, die aus 2-(Diaminomethylenamino)-4-(5-ureidomethylfuran-2-yl)thiazol oder ihrem Hydrochlorid, und 2-(Diaminomethylenamino)-4-[5-(3-methylureido)methylfuran-2-yl]thiazol oder ihrem Hydrochlorid besteht.

**7.** Verfahren zur Herstellung einer Verbindung der Formel:

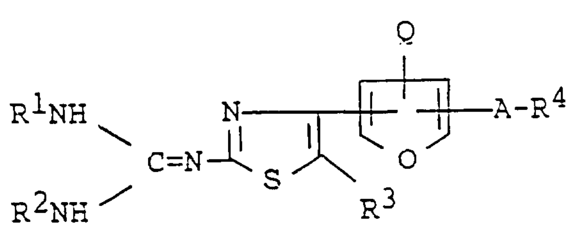

worin $R^1$, $R^2$, $R^3$, $R^4$, $R^5$, $R^6$, A, A-$R^4$, Q, X und n jeweils wie in Anspruch 1 definiert sind, oder eines Salz davon, das umfaßt:

(1) Reagieren einer Verbindung der Formel:

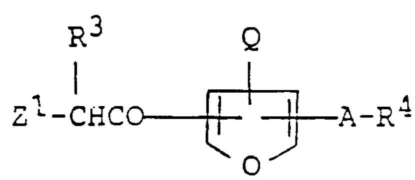

worin $R^3$, $R^4$, A und Q jeweils wie oben definiert sind, und $Z^1$ Halogen ist, oder eines Salz davon mit einer Verbindung der Formel:

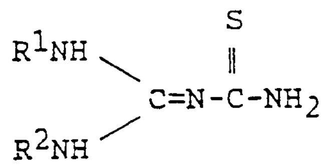

worin $R^1$ und $R^2$ jeweils wie oben definiert sind, um eine Verbindung der Formel:

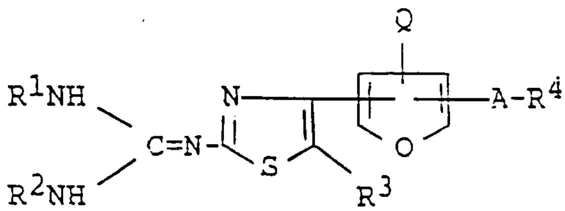

worin $R^1$, $R^2$, $R^3$, $R^4$, A und Q jeweils wie oben definiert sind, oder ein Salz davon zu ergeben oder
(2) Unterwerfen einer Verbindung der Formel:

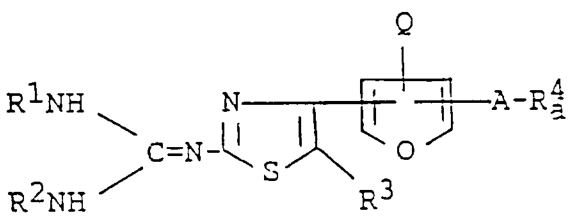

worin $R^1$, $R^2$, $R^3$, A und Q jeweils wie oben definiert sind, und $R^4_a$ Ureido, Thioureido, Sulfamoylamino, ($C_1$-$C_6$)Alkanoylamino, ($C_1$-$C_6$)Alkoxycarbonylamino, ($C_1$-$C_6$)Alkylsulfonylamino, ($C_1$-$C_6$)Alkoxy-($C_1$-$C_6$)alkanoylamino, ($C_1$-$C_6$)Alkanoyloxy($C_1$-$C_6$)alkanoylamino, ($C_1$-$C_6$)Alkylthio($C_1$-$C_6$)-alkanoylamino, Benzoylamino wahlweise substituiert mit Nitro, Furoylamino, Thenoylamino, Nicotinoylamino, 1-Oxonicotinoylamino, Morpholinocarbonylamino, ($C_1$-$C_6$)Alkylureido, ($C_1$-$C_6$)-Alkylthioureido, ($C_1$-$C_6$)Alkanoylureido, Allylureido, Benzoylthioureido, Furyl($C_1$-$C_6$)alkylthio($C_1$-$C_6$)-alkanoylamino, Mono- oder Di($C_1$-$C_6$)Alkylsulfamoylamino, Hydroxy($C_1$-$C_6$)alkylureido oder ($C_1$-$C_6$)-Alkanoyloxy($C_1$-$C_6$)alkylureido ist, oder eines Salzes davon der Deacylierungsreaktion, um eine Verbindung der Formel:

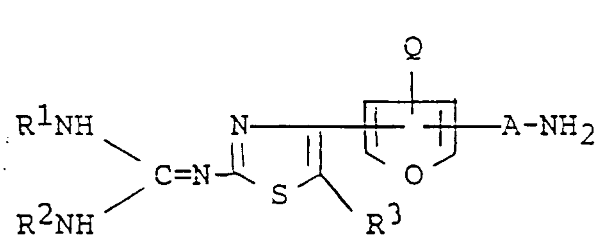

worin $R^1$, $R^2$, $R^3$, A und Q jeweils wie oben definiert sind, oder ein Salz davon zu ergeben oder

(3) Unterwerfen einer Verbindung der Formel:

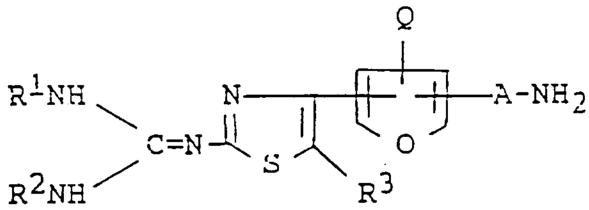

worin $R^1$, $R^2$, $R^3$, A und Q jeweils wie oben definiert sind, oder eines Salzes davon der Acylierungsreaktion, um eine Verbindung der Formel:

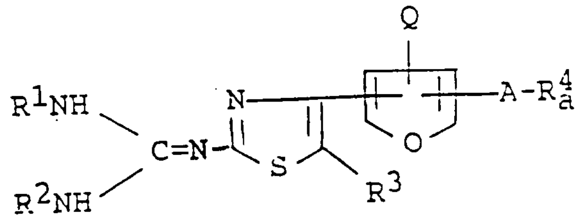

worin $R^1$, $R^2$, $R^3$, $R^4{}_a$, A und Q jeweils wie oben definiert sind, oder ein Salz davon zu ergeben oder

(4) Reagieren einer Verbindung der Formel:

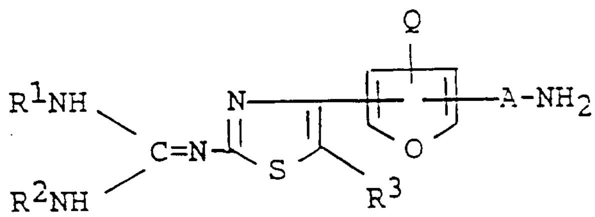

worin $R^1$, $R^2$, $R^3$, A und Q jeweils wie oben definiert sind, oder eines Salzes davon mit einer Verbindung der Formel:

$(R^7\text{-}X^1)_2C=X\text{-}R^5$

worin $R^5$ und X jeweils wie oben definiert sind, R7 $(C_1\text{-}C_6)$Alkyl oder Phenyl ist, und $X^1$ S oder O ist, um eine Verbindung der Formel:

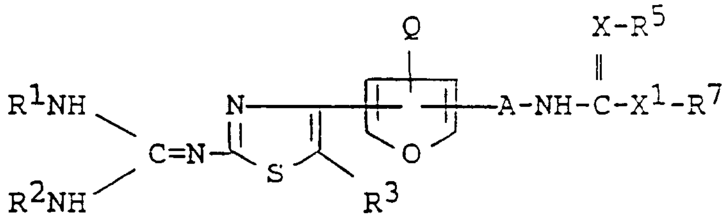

worin $R^1$, $R^2$, $R^3$, $R^5$, $R^7$, X, $X^1$, A und Q jeweils wie oben definiert sind, oder ein Salz davon zu ergeben oder

(5) Reagieren einer Verbindung der Formel:

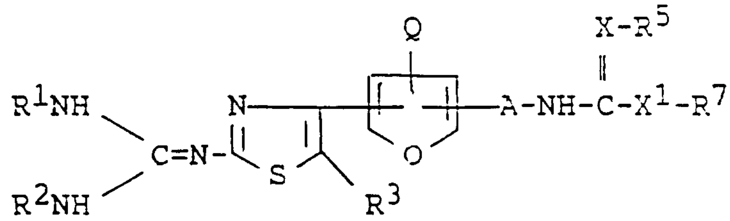

worin $R^1$, $R^2$, $R^3$, $R^5$, $R^7$, X, $X^1$, A und Q jeweils wie oben definiert sind, oder eines Salzes davon mit einer Verbindung der Formel:

$H\text{-}R^6{}_a$

worin $R^6{}_a$ Amino ist, das Mono- oder Di($C_1$-$C_6$)Alkyl, Allyl, Propargyl. Hydroxy($C_1$-$C_6$)Alkyl oder Mono- oder Di($C_1$-$C_6$)Alkylamino($C_1$-$C_6$)alkyl, oder ($C_1$-$C_6$)Alkoxy aufweisen kann, um eine Verbindung der Formel:

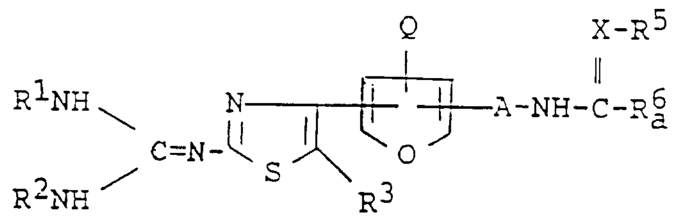

worin $R^1$, $R^2$, $R^3$, $R^5$, $R^6{}_a$, X, A und Q jeweils wie oben definiert sind, oder ein Salz davon zu ergeben oder

(6) Unterwerfen einer Verbindung der Formel:

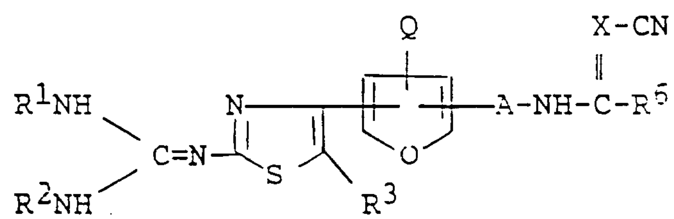

worin $R^1$, $R^2$, $R^3$, $R^6$, X, A und Q jeweils wie oben definiert sind, oder eines Salzes davon der Hydrolyse, um eine Verbindung der Formel:

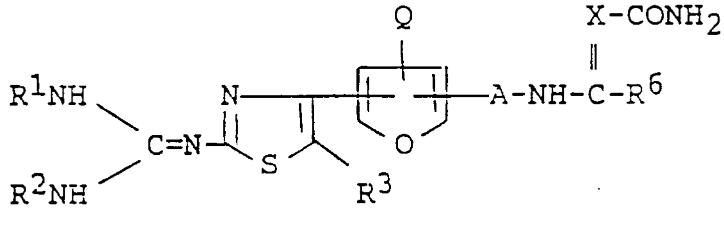

worin $R^1$, $R^2$, $R^3$, $R^6$, X, A und Q jeweils wie oben definiert sind, oder ein Salz davon zu ergeben oder

(7) Unterwerfen einer Verbindung der Formel:

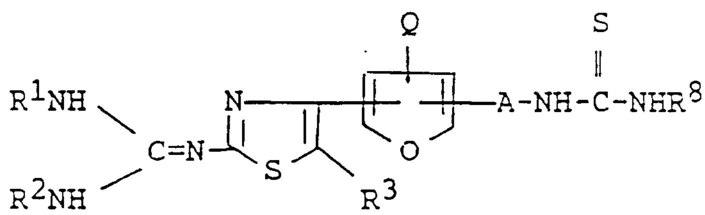

worin $R^1$, $R^2$, $R^3$, A und Q jeweils wie oben definiert sind, und $R^8$ eine Amino-Schutzgruppe ist, die aus ($C_1$-$C_6$)Alkanoyl und Benzoyl besteht, oder eines Salzes davon der Eliminierungsreaktion der Amino-Schutzgruppe, um eine Verbindung der Formel:

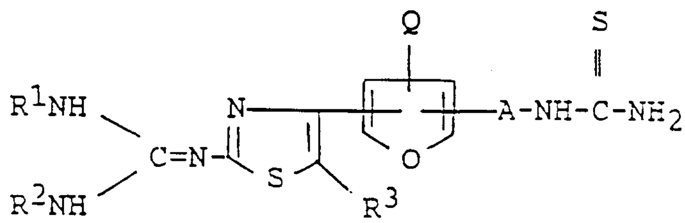

worin $R^1$, $R^2$, $R^3$, A und Q jeweils wie oben definiert sind, oder ein Salz davon zu ergeben, oder
(8) Reagieren einer Verbindung der Formel:

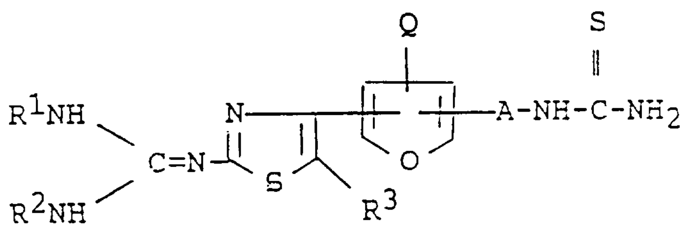

worin $R^1$, $R^2$, $R^3$, A und Q jeweils wie oben definiert sind, oder eines Salzes davon mit einer Verbindung der Formel:

$Z^2$-$CH_2$-CO-$R^9$

worin $R^9$ ($C_1$-$C_6$)Alkyl ist und $Z^2$ Halogen ist, um eine Verbindung der Formel:

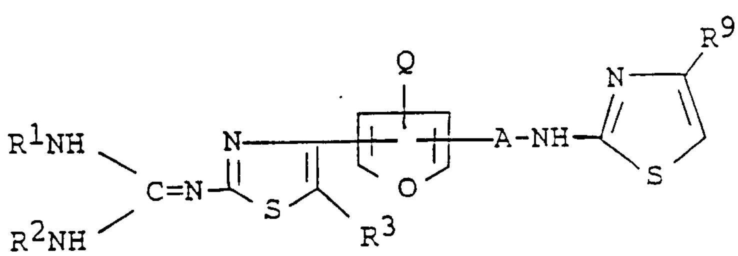

worin $R^1$, $R^2$, $R^3$, $R^9$, A und Q jeweils wie oben definiert sind, oder ein Salz davon zu ergeben oder

(9) Reagieren einer Verbindung der Formel:

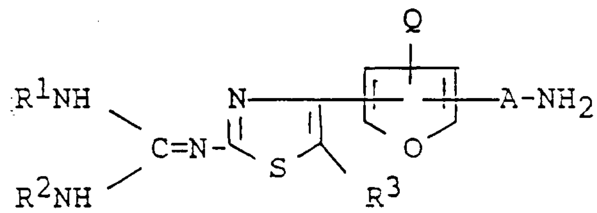

worin $R^1$, $R^2$, $R^3$, A und Q jeweils wie oben definiert sind, oder eines Salzes davon mit einer Verbindung der Formel:

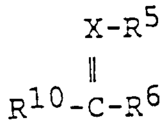

worin $R^5$, $R^6$ und X jeweils wie oben definiert sind und $R^{10}$ $(C_1\text{-}C_6)$Alkanoyloxy, Phenyl$(C_1\text{-}C_6)$-alkoxy, $(C_1\text{-}C_6)$Alkoxy$(C_1\text{-}C_6)$alkoxy, Tetrahydropyranyloxy oder $(C_1\text{-}C_6)$Alkoxy ist, um eine Verbindung der Formel:

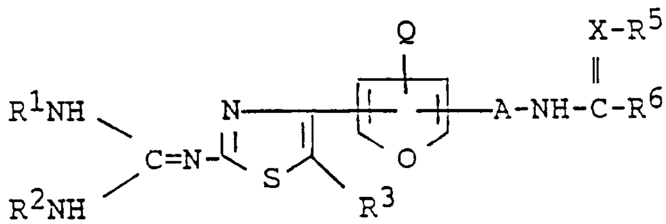

worin $R^1$, $R^2$, $R^3$, $R^5$, $R^6$, X, A und Q jeweils wie oben definiert sind, oder ein Salz davon zu ergeben oder

(10) Reagieren einer Verbindung der Formel:

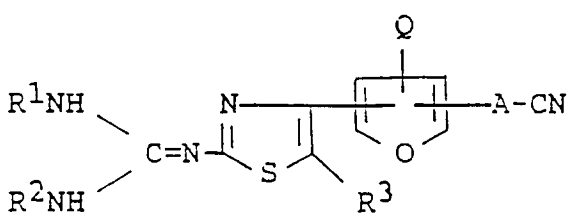

worin $R^1$, $R^2$, $R^3$, A und Q jeweils wie oben definiert sind, oder eines Salzes davon mit einer Verbindung der Formel:

$R^{11}OH$

worin $R^{11}$ $(C_1\text{-}C_6)$Alkyl ist, um eine Verbindung der Formel:

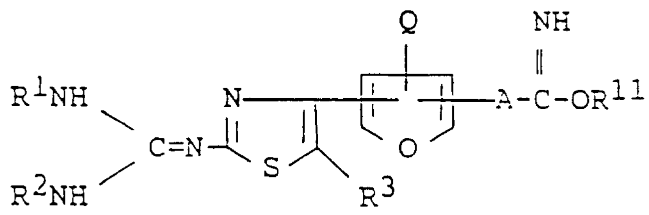

worin $R^1$, $R^2$, $R^3$, $R^{11}$, A und Q jeweils wie oben definiert sind, oder ein Salz davon zu ergeben oder
(11) Reagieren einer Verbindung der Formel:

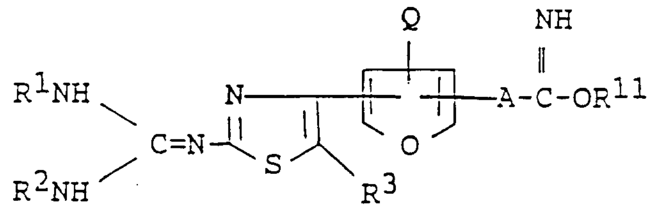

worin $R^1$, $R^2$, $R^3$, $R^{11}$, A und Q jeweils wie oben definiert sind, oder eines Salzes davon mit einer Verbindung der Formel:

$NH_2R^{12}$

worin $R^{12}$ Acyl ist, um eine Verbindung der Formel:

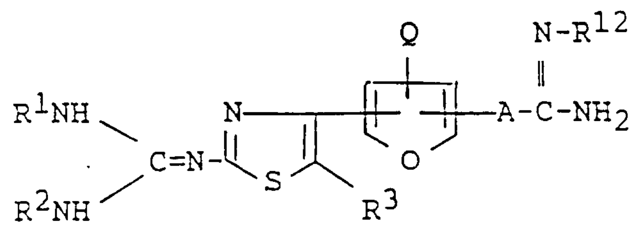

worin $R^1$, $R^2$, $R^3$, $R^{12}$, A und Q jeweils wie oben definiert sind, oder ein Salz davon zu ergeben oder
(12) Reagieren einer Verbindung der Formel:

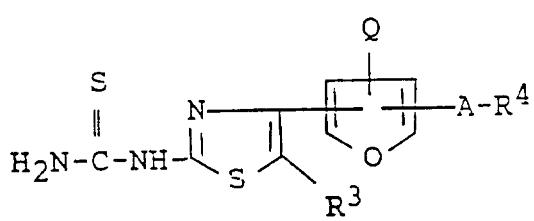

worin $R^3$, $R^4$, A und Q jeweils wie oben definiert sind, oder eines Salzes davon mit einer Verbindung der Formel:

$R^{13}$-$Z^3$

worin $R^{13}$ $(C_1$-$C_6)$Alkyl ist und $Z^3$ Halogen ist, oder eines Salzes davon, um eine Verbindung der Formel:

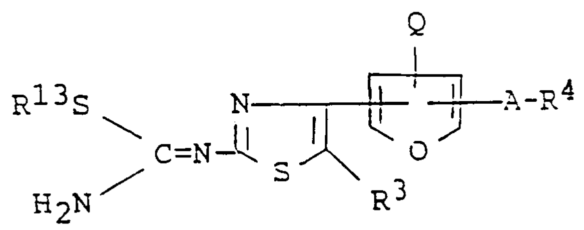

worin $R^3$, $R^4$, $R^{13}$, A und Q jeweils wie oben definiert sind, oder ein Salz davon zu ergeben, und ihr durchgehendes Reagierenlassen mit einer Verbindung der Formel:

$H_2N\text{-}Y\text{-}NH_2$

worin Y $(C_1\text{-}C_6)$Alkylen ist, oder ein Salz davon, um eine Verbindung der Formel:

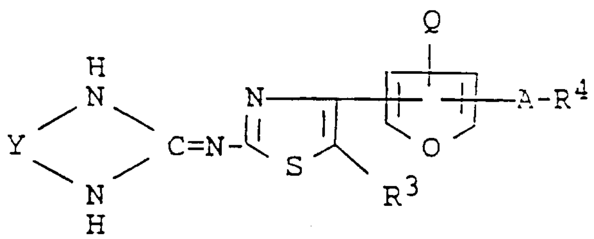

worin $R^3$, $R^4$, A, Q und Y jeweils wie oben definiert sind, oder ein Salz davon zu ergeben oder
(13) Unterwerfen einer Verbindung der Formel:

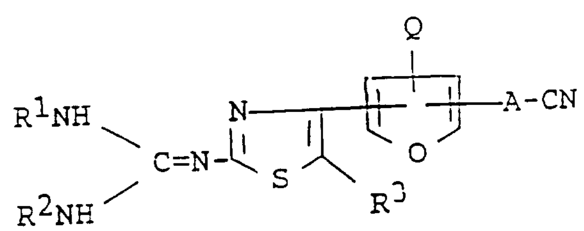

worin $R^1$, $R^2$, $R^3$, A und Q jeweils wie oben definiert sind, oder eines Salzes davon der Reduktion, um eine Verbindung der Formel:

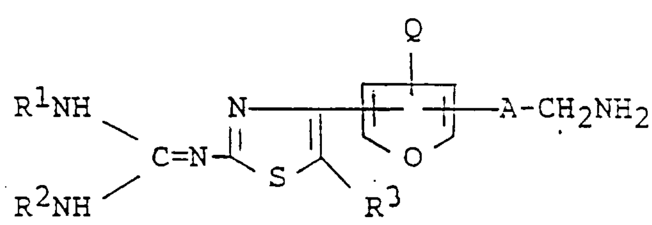

worin $R^1$, $R^2$, $R^3$, A und Q jeweils wie oben definiert sind, oder ein Salz davon zu ergeben oder

(14) Reagieren einer Verbindung der Formel:

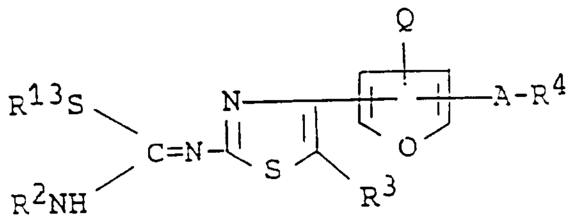

worin $R^2$, $R^3$, $R^4$, $R^{13}$, A und Q jeweils wie oben definiert sind, oder eines Salzes davon mit einer Verbindung der Formel:

$NH_2R^1_a$

worin $R^1_a$ $(C_1\text{-}C_6)$Alkyl ist, das Halogen aufweisen kann, oder einem Salz davon, um eine Verbindung der Formel:

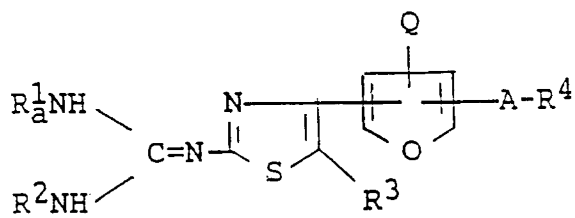

worin $R^1_a$, $R^2$, $R^3$, $R^4$, A und Q jeweils wie oben definiert sind, oder ein Salz davon zu ergeben oder
(15) Unterwerfen einer Verbindung der Formel:

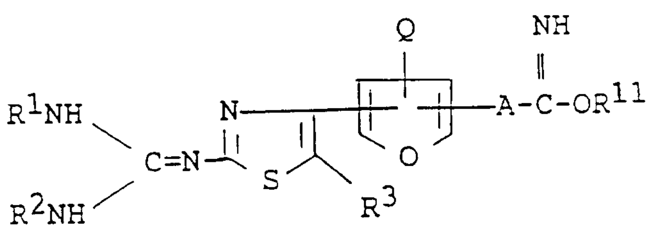

worin $R^1$, $R^2$, $R^3$, $R^{11}$, A und Q jeweils wie oben definiert sind, oder eines Salzes davon der Hydrolyse, um eine Verbindung der Formel:

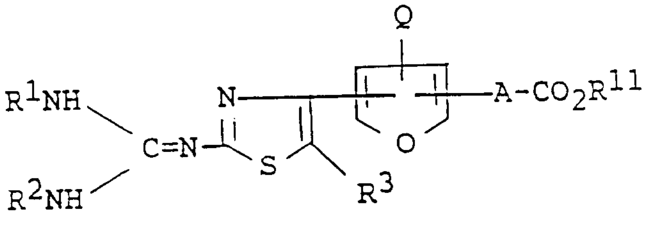

worin $R^1$, $R^2$, $R^3$, $R^{11}$, A und Q jeweils wie oben definiert sind, oder ein Salz davon zu ergeben oder

(16) Unterwerfen einer Verbindung der Formel:

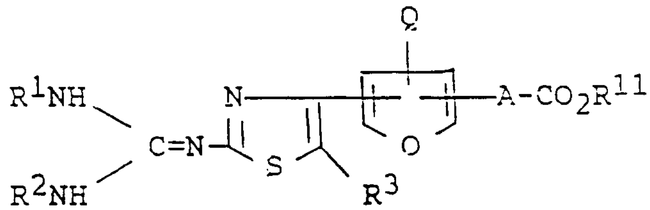

worin $R^1$, $R^2$, $R^3$, $R^{11}$, A und Q jeweils wie oben definiert sind, oder eines Salzes davon der Amidierungsreaktion, um eine Verbindung der Formel:

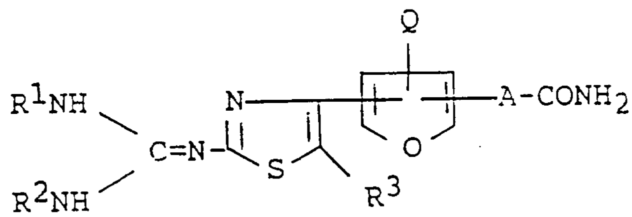

worin $R^1$, $R^2$, $R^3$, A und Q jeweils wie oben definiert sind, oder ein Salz davon zu ergeben oder

(17) Reagieren einer Verbindung der Formel:

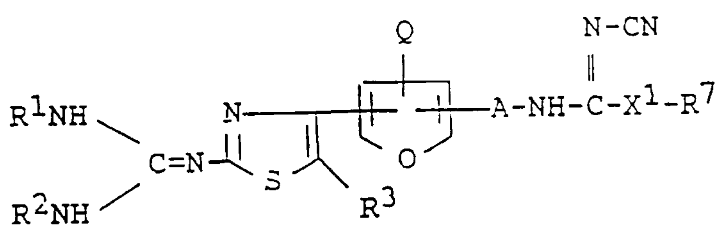

worin $R^1$, $R^2$, $R^3$, $R^7$, $X^1$, A und Q jeweils wie oben definiert sind, oder eines Salzes davon mit einer Verbindung der Formel:

$R^{14}NHNH_2$

worin $R^{14}$ Wasserstoff oder $(C_1-C_6)$Alkyl ist, um eine Verbindung der Formel:

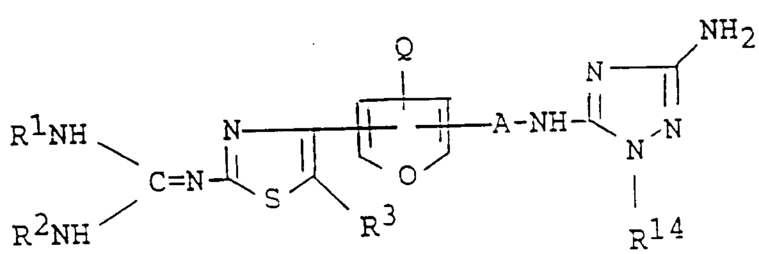

worin $R^1$, $R^2$, $R^3$, $R^{14}$, A und Q jeweils wie oben definiert sind, oder ein Salz davon zu ergeben oder

(18) Reagieren einer Verbindung der Formel:

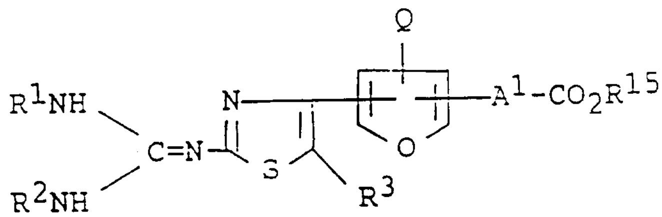

worin $R^1$, $R^2$, $R^3$ und Q jeweils wie oben definiert sind, $R^{15}$ $(C_1-C_6)$Alkyl ist und $A^1$ $(C_1-C_6)$Alkylen oder eine Bindung ist, oder eines Salzes davon mit Hydrazin, um eine Verbindung der Formel:

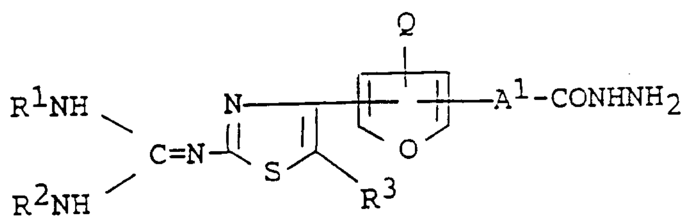

worin $R^1$, $R^2$, $R^3$, $A^1$ und Q jeweils wie oben definiert sind, oder ein Salz davon zu ergeben oder
(19) Reagieren einer Verbindung der Formel:

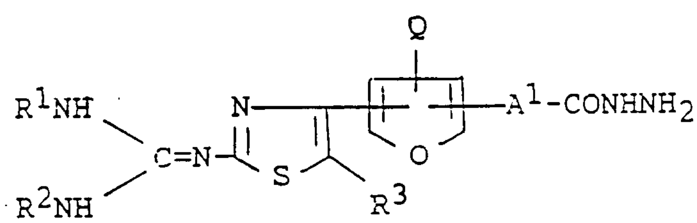

worin $R^1$, $R^2$, $R^3$, $A^1$ und Q jeweils wie oben definiert sind, oder eines Salzes davon mit S-$(C_1-C_6)$-Alkylisothioharnstoff oder einem Salz davon, um eine Verbindung der Formel:

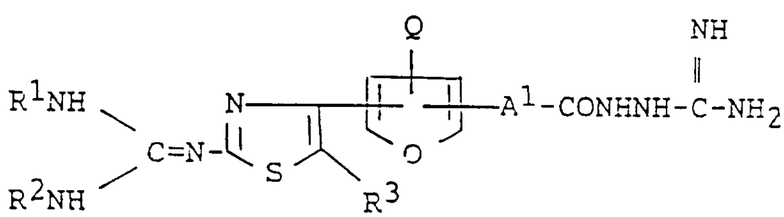

worin $R^1$, $R^2$, $R^3$, $A^1$ und Q jeweils wie oben definiert sind, oder ein Salz davon zu ergeben oder

(20) Unterwerfen einer Verbindung der Formel:

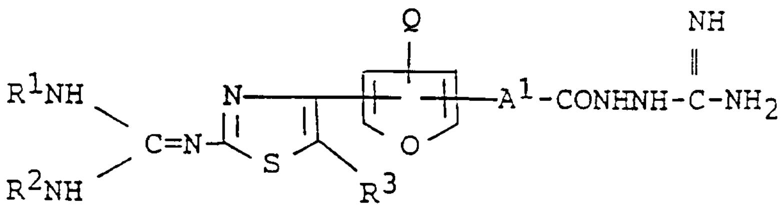

worin $R^1$, $R^2$, $R^3$, $A^1$ und Q jeweils wie oben definiert sind, oder eines Salzes davon dem Ringschluß, um eine Verbindung der Formel:

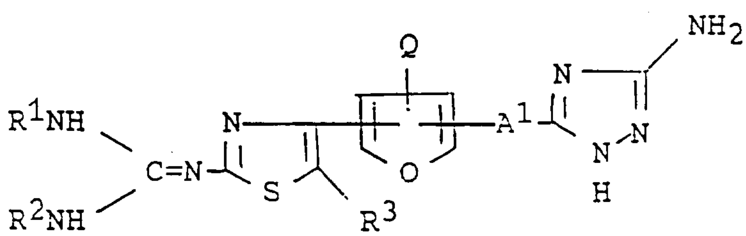

worin $R^1$, $R^2$, $R^3$, $A^1$ und Q jeweils wie oben definiert sind, oder ein Salz davon zu ergeben oder
(21) Reagieren einer Verbindung der Formel:

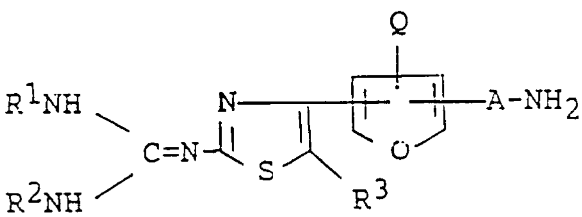

worin $R^1$, $R^2$, $R^3$, A und Q jeweils wie oben definiert sind, oder eines Salzes davon mit einer Verbindung der Formel:

$R^{16}$-$Z^4$

worin $R^{16}$ Thiazolyl substituiert mit ($C_1$-$C_6$)Alkyl, Triazolyl substituiert mit Amino, Triazolyl substituiert mit Amino und ($C_1$-$C_6$)Alkyl, Benzoisothiazolyl substituiert mit Oxo oder Benzothiadiazinyl substituiert mit Oxo ist, und $Z^4$ Halogen ist, oder einem Salz davon, um eine Verbindung der Formel:

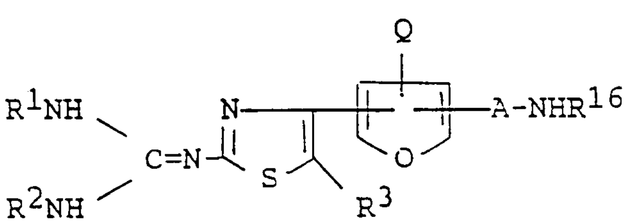

worin $R^1$, $R^2$, $R^3$, $R^{16}$, A und Q jeweils wie oben definiert sind, oder ein Salz davon zu ergeben oder
(22) Unterwerfen einer Verbindung der Formel:

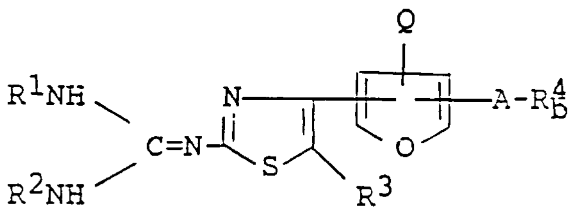

worin $R^1$, $R^2$, $R^3$, A und Q jeweils wie oben definiert sind, und $R^4_b$ Furyl($C_1$-$C_6$)alkylthio($C_1$-$C_6$)-Alkanoylamino ist, oder eines Salzes davon der Oxidationsreaktion, um eine Verbindung der Formel:

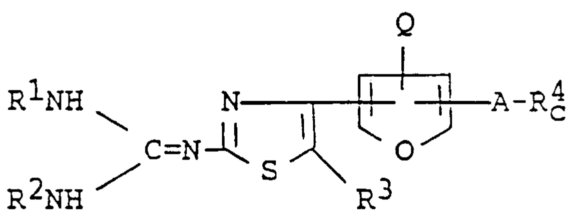

worin $R^1$, $R^2$, $R^3$, A und Q jeweils wie oben definiert sind, und $R^4_c$ Furyl($C_1$-$C_6$)alkylsulfinyl($C_1$-$C_6$)-alkanoylamino ist, oder ein Salz davon zu ergeben oder
(23) Reagieren einer Verbindung der Formel:

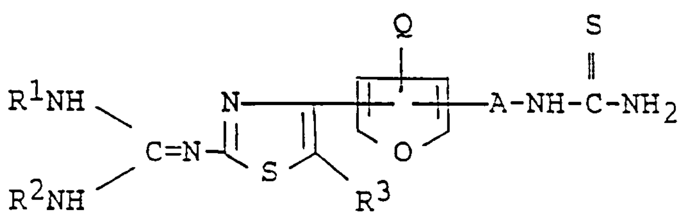

worin $R^1$, $R^2$, $R^3$, A und Q jeweils wie oben definiert sind, oder eines Salzes davon mit einer Verbindung der Formel:

$R^{17}$-$Z^5$

worin $R^{17}$ ($C_1$-$C_6$)Alkyl ist, und $Z^5$ Halogen ist, oder einem Salz davon, um eine Verbindung der Formel:

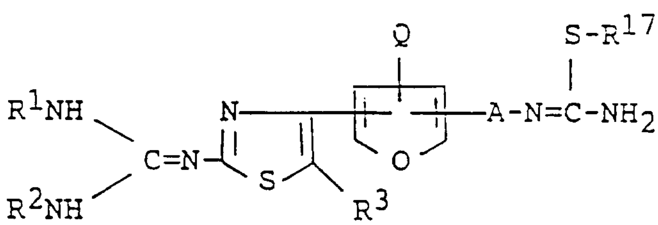

worin $R^1$, $R^2$, $R^3$, $R^{17}$, A und Q jeweils wie oben definiert sind, oder ein Salz davon zu ergeben oder

(24) Reagieren einer Verbindung der Formel:

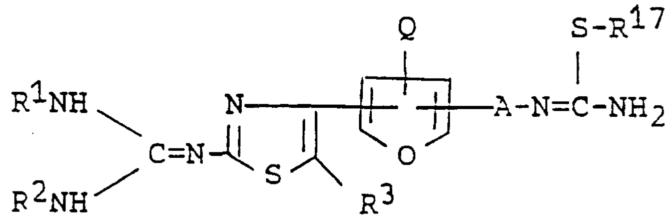

worin $R^1$, $R^2$, $R^3$, $R^{17}$, A und Q jeweils wie oben definiert sind, oder eines Salzes davon mit einer Verbindung der Formel:

$H_2N\text{-}R^5$

worin $R^5$ wie oben definiert ist, oder einem Salz davon, um eine Verbindung der Formel:

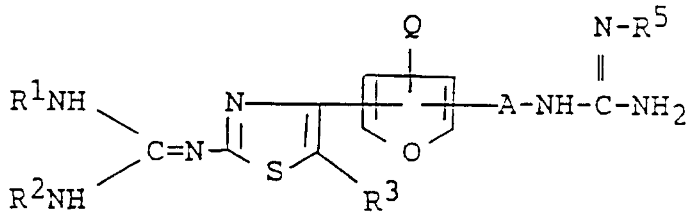

worin $R^1$, $R^2$, $R^3$, $R^5$, A und Q jeweils wie oben definiert sind, oder ein Salz davon zu ergeben oder
(25) Unterwerfen einer Verbindung der Formel:

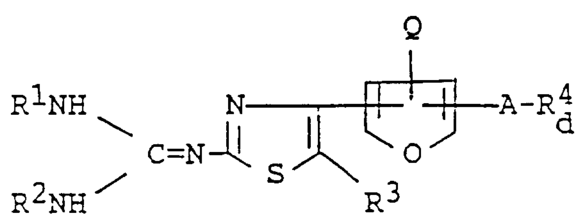

worin $R^1$, $R^2$, $R^3$, A und Q jeweils wie oben definiert sind, und $R^4{}_d$ $(C_1\text{-}C_6)$Alkanoyloxy$(C_1\text{-}C_6)$-alkylureido ist, oder eines Salzes davon der Eliminierungsreaktion von $(C_1\text{-}C_6)$Alkanoyl, um eine Verbindung der Formel:

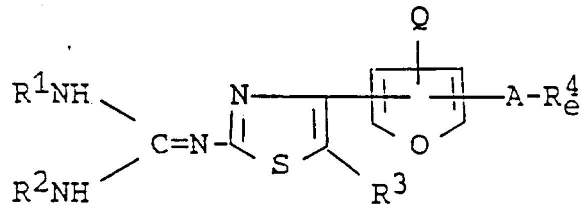

worin $R^1$, $R^2$, $R^3$, A und Q jeweils wie oben definiert sind, und $R^4{}_e$ Hydroxy$(C_1\text{-}C_6)$alkylureido ist, oder ein Salz davon zu ergeben oder

(26) Reagieren einer Verbindung der Formel:

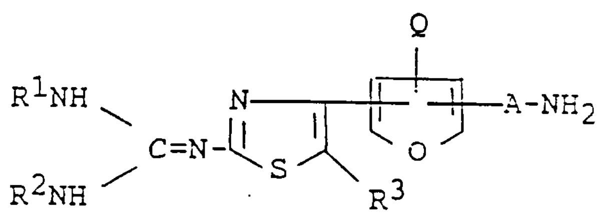

worin $R^1$, $R^2$, $R^3$, A und Q jeweils wie oben definiert sind, oder eines Salzes davon mit einer Verbindung der Formel:

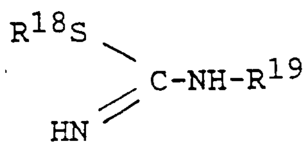

worin $R^{18}$ $(C_1\text{-}C_6)$Alkyl ist, und $R^{19}$ Carbamoyl, $(C_1\text{-}C_6)$Alkoxycarbonyl, Sulfamoyl, $(C_1\text{-}C_6)$-Alkylsulfonyl, Benzolsulfonyl, das mit einem oder mehreren Substituenten substituiert ist, ausgewählt aus $(C_1\text{-}C_6)$Alkyl, $(C_1\text{-}C_6)$Alkoxy, Halogen und Amino, oder Mono- oder Di$(C_1\text{-}C_6)$Alkylsulfamoyl ist, oder einem Salz davon, um eine Verbindung der Formel:

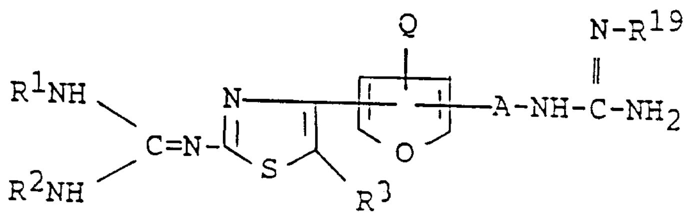

worin $R^1$, $R^2$, $R^3$, $R^{19}$, A und Q jeweils wie oben definiert sind, oder ein Salz davon zu ergeben oder
(27) Unterwerfen einer Verbindung der Formel:

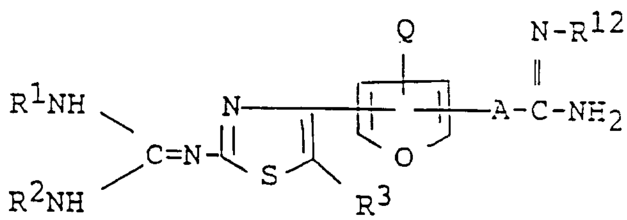

worin $R^1$, $R^2$, $R^3$, $R^{12}$, A und Q jeweils wie oben definiert sind, oder eines Salzes davon der Hydrolyse, um eine Verbindung der Formel:

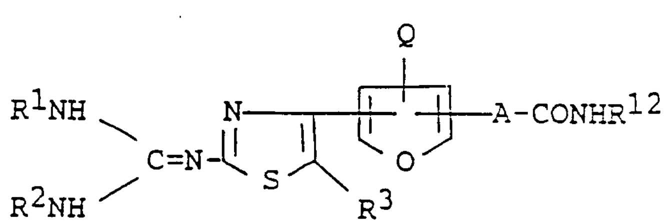

worin $R^1$, $R^2$, $R^3$, $R^{12}$, A und Q jeweils wie oben definiert sind, oder ein Salz davon zu ergeben.

8. Pharmazeutische Zusammensetzung, die als aktiven Bestandteil eine Verbindung nach Anspruch 1 oder ein pharmazeutisch verträgliches Salz davon in Zusammenmischung mit pharmazeutisch verträglichen Trägern umfaßt.

9. Verwendung einer Verbindung nach Anspruch 1 oder eines pharmazeutisch verträglichen Salzes davon zur Herstellung eines Medikamentes für die therapeutische Behandlung von Geschwüren in Menschen und Tieren.

10. Verbindung nach Anspruch 1 oder eines pharmazeutisch verträglichen Salzes davon zur Verwendung als Medikament.

**Patentansprüche für folgenden Vertragsstaat : ES**

1. Verfahren zur Herstellung einer Verbindung der Formel:

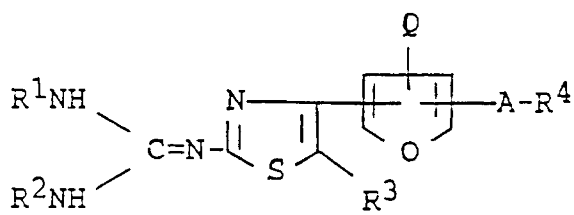

worin $R^1$ und $R^2$ jeweils Wasserstoff, Furoyl oder ($C_1$-$C_6$)Alkyl, das Halogen aufweisen kann, sind; oder $R^1$ und $R^2$ miteinander verbunden sind, um ($C_1$-$C_6$)Alkylen zu bilden, $R^3$ Wasserstoff oder ($C_1$-$C_6$)Alkyl ist, $R^4$ ist Amino, Carbamoyl, Aminocarbamoyl, Guanidinocarbamoyl, ($C_1$-$C_6$)Alkylcarbamoyl, Sulfamoylaminocarbonyl, ($C_1$-$C_6$)Alkoxycarbonyl, Ureido, Thioureido, Sulfamoylamino, ($C_1$-$C_6$)Alkanoylamino, ($C_1$-$C_6$)Alkoxycarbonylamino, ($C_1$-$C_6$)Alkylsulfonylamino, ($C_1$-$C_6$)Alkoxy($C_1$-$C_6$)alkanoylamino, ($C_1$-$C_6$)-Alkanoyloxy($C_1$-$C_6$)alkanoylamino, ($C_1$-$C_6$)Alkylthio($C_1$-$C_6$)alkanoylamino, Benzoylamino wahlweise substituiert durch Nitro, Furoylamino, Thenoylamino, Nicotinoylamino, 1-Oxonicotinoylamino, Morpholinocarbonylamino, ($C_1$-$C_6$)Alkylureido, ($C_1$-$C_6$)Alkylthioureido, ($C_1$-$C_6$)Alkanoylureido, Allylureido, Benzoylthioureido, Furyl($C_1$-$C_6$)alkylthio($C_1$-$C_6$)alkanoylamino, Furyl($C_1$-$C_6$)alkylsulfinyl($C_1$-$C_6$)alkanoylamino, Mono- oder Di($C_1$-$C_6$)Alkylsulfamoylamino, Hydroxy($C_1$-$C_6$)alkylureido, ($C_1$-$C_6$)Alkanoyloxy($C_1$-$C_6$)-alkylureido, ($C_1$-$C_6$)Alkylisothioureido, Thiazolylamino substituiert mit ($C_1$-$C_6$)Alkyl, Triazolylamino substituiert mit Amino, Triazolylamino substituiert mit Amino und ($C_1$-$C_6$)Alkyl, Benzoisothiazolylamino substituiert mit Oxo, Benzothiadiazinylamino substituiert mit Oxo und Halogen, Pyrimidinyl substituiert mit Oxo und ($C_1$-$C_6$)Alkyl, Triazolyl substituiert mit Amino oder einer Gruppe der Formel:

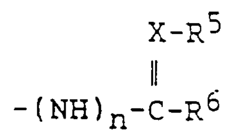

worin n 0 oder 1 ist, X =CH- oder =N- ist, $R^5$ Wasserstoff, Cyano, Nitro, Carbamoyl, ($C_1$-$C_6$)-Alkoxycarbonyl, Sulfamoyl, ($C_1$-$C_6$)Alkylsulfonyl, Benzolsulfonyl, das mit einem oder mehreren Substituenten substituiert ist, ausgewählt aus ($C_1$-$C_6$)Alkyl, ($C_1$-$C_6$)Alkoxy, Halogen und Amino, oder Mono- oder Di($C_1$-$C_6$)Alkylsulfamoyl ist, $R^6$ Wasserstoff, ($C_1$-$C_6$)Alkyl, ($C_1$-$C_6$)Alkylthio, ($C_1$-$C_6$)Alkoxy, Amino, Mono- oder Di($C_1$-$C_6$)Alkylamino, Allylamino, Propargylamino, Hydroxy($C_1$-$C_6$)Alkylamino, ($C_1$-$C_6$)-Alkoxy($C_1$-$C_6$)alkylamino, oder Mono- oder Di($C_1$-$C_6$)Alkylamino($C_1$-$C_6$)alkylamino ist; A ($C_1$-$C_6$)Alkylen oder -CONH- ist; oder A-$R^4$ Imidazolyl substituiert mit ($C_1$-$C_6$)Alkyl, Triazolyl substituiert mit Amino ist, und Q Wasserstoff oder ($C_1$-$C_6$)Alkyl ist, oder eines pharmazeutisch verträglichen Salzes davon, das umfaßt:

(1) Reagieren einer Verbindung der Formel:

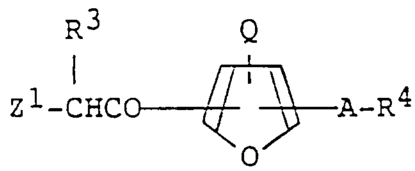

worin $R^3$, $R^4$, A und Q jeweils wie oben definiert sind, und $Z^1$ Halogen ist, oder eines Salz davon mit einer Verbindung der Formel:

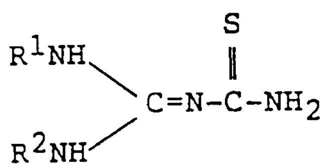

worin $R^1$ und $R^2$ jeweils wie oben definiert sind, um eine Verbindung der Formel:

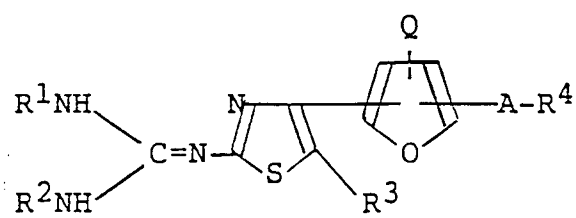

worin $R^1$, $R^2$, $R^3$, $R^4$, A und Q jeweils wie oben definiert sind, oder ein Salz davon zu ergeben oder
(2) Unterwerfen einer Verbindung der Formel:

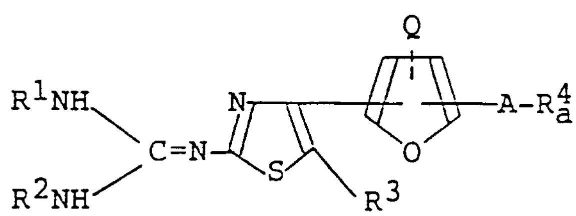

worin $R^1$, $R^2$, $R^3$, A und Q jeweils wie oben definiert sind, und $R^4{}_a$ Ureido, Thioureido, Sulfamoylamino, $(C_1\text{-}C_6)$Alkanoylamino, $(C_1\text{-}C_6)$Alkoxycarbonylamino, $(C_1\text{-}C_6)$Alkylsulfonylamino, $(C_1\text{-}C_6)$Alkoxy-$(C_1\text{-}C_6)$alkanoylamino, $(C_1\text{-}C_6)$Alkanoyloxy$(C_1\text{-}C_6)$alkanoylamino, $(C_1\text{-}C_6)$Alkylthio$(C_1\text{-}C_6)$-alkanoylamino, Benzoylamino wahlweise substituiert mit Nitro, Furoylamino, Thenoylamino, Nicotinoylamino, 1-Oxonicotinoylamino, Morpholinocarbonylamino, $(C_1\text{-}C_6)$Alkylureido, $(C_1\text{-}C_6)$-Alkylthioureido, $(C_1\text{-}C_6)$Alkanoylureido, Allylureido, Benzoylthioureido, Furyl$(C_1\text{-}C_6)$alkylthio$(C_1\text{-}C_6)$-alkanoylamino, Mono- oder Di$(C_1\text{-}C_6)$Alkylsulfamoylamino, Hydroxy$(C_1\text{-}C_6)$alkylureido oder $(C_1\text{-}C_6)$-Alkanoyloxy$(C_1\text{-}C_6)$alkylureido ist, oder eines Salzes davon der Deacylierungsreaktion, um eine Verbindung der Formel:

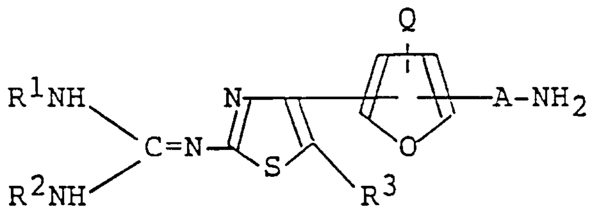

worin $R^1$, $R^2$, $R^3$, A und Q jeweils wie oben definiert sind, oder ein Salz davonzu ergeben oder
(3) Unterwerfen einer Verbindung der Formel:

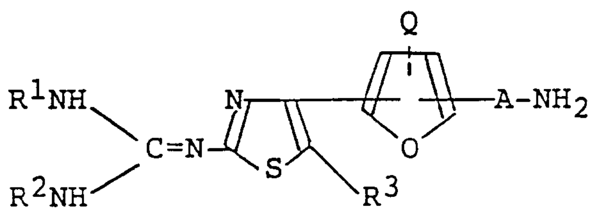

worin $R^1$, $R^2$, $R^3$, A und Q jeweils wie oben definiert sind, oder eines Salzes davon der Acylierungsreaktion, um eine Verbindung der Formel:

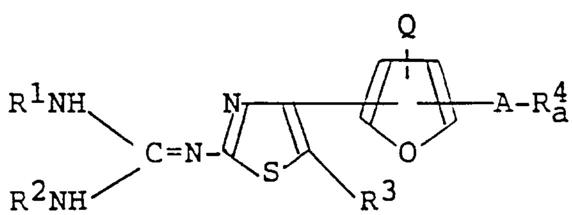

worin $R^1$, $R^2$, $R^3$, $R^4{}_a$, A und Q jeweils wie oben definiert sind, oder ein Salz davon zu ergeben oder
(4) Reagieren einer Verbindung der Formel:

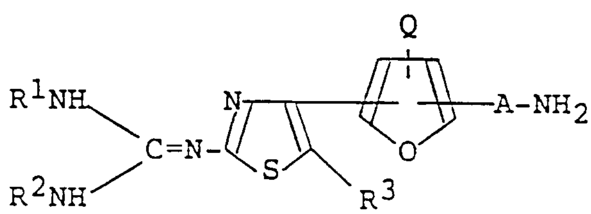

worin $R^1$, $R^2$, $R^3$, A und Q jeweils wie oben definiert sind, oder eines Salzes davon mit einer Verbindung der Formel:

$(R^7\text{-}X^1)_2C=X\text{-}R^5$

worin $R^5$ und X jeweils wie oben definiert sind, R7 $(C_1\text{-}C_6)$Alkyl oder Phenyl ist, und $X^1$ S oder O ist, um eine Verbindung der Formel:

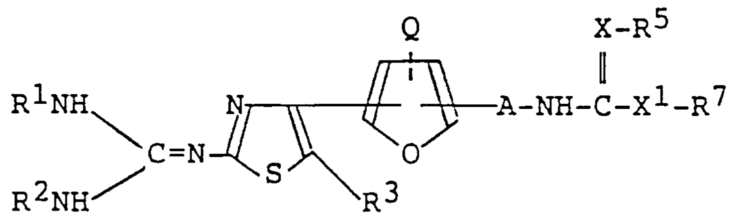

worin $R^1$, $R^2$, $R^3$, $R^5$, $R^7$, X, $X^1$, A und Q jeweils wie oben definiert sind, oder ein Salz davon zu ergeben oder

(5) Reagieren einer Verbindung der Formel:

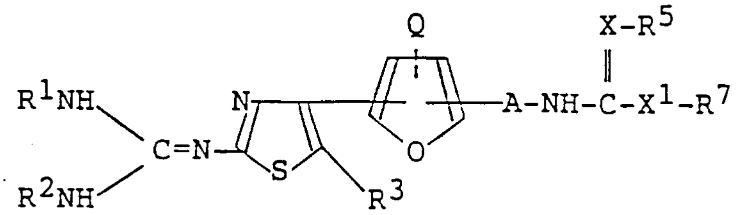

worin $R^1$, $R^2$, $R^3$, $R^5$, $R^7$, X, $X^1$, A und Q jeweils wie oben definiert sind, oder eines Salzes davon mit einer Verbindung der Formel:

$H\text{-}R^6{}_a$

worin $R^6{}_a$ Amino ist, das Mono- oder Di($C_1$-$C_6$)Alkyl, Allyl, Propargyl. Hydroxy($C_1$-$C_6$)Alkyl oder Mono- oder Di($C_1$-$C_6$)Alkylamino($C_1$-$C_6$)alkyl, oder ($C_1$-$C_6$)Alkoxy aufweisen kann, um eine Verbindung der Formel:

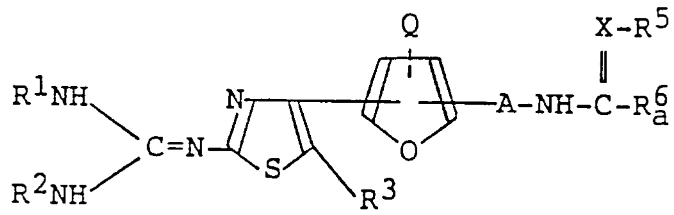

worin $R^1$, $R^2$, $R^3$, $R^5$, $R^6{}_a$, X, A und Q jeweils wie oben definiert sind, oder ein Salz davon zu ergeben oder

(6) Unterwerfen einer Verbindung der Formel:

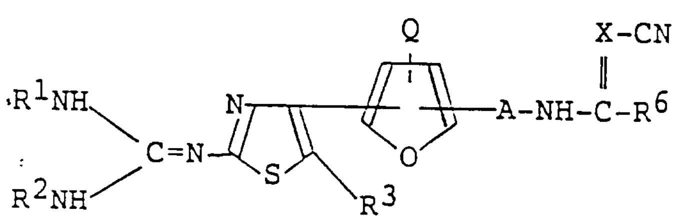

worin $R^1$, $R^2$, $R^3$, $R^6$, X, A und Q jeweils wie oben definiert sind, oder eines Salzes davon der Hydrolyse, um eine Verbindung der Formel:

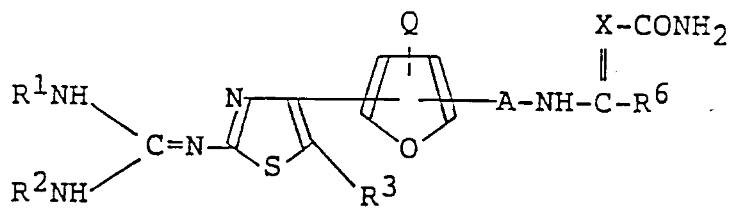

worin $R^1$, $R^2$, $R^3$, $R^6$, X, A und Q jeweils wie oben definiert sind, oder ein Salz davon zu ergeben oder

(7) Unterwerfen einer Verbindung der Formel:

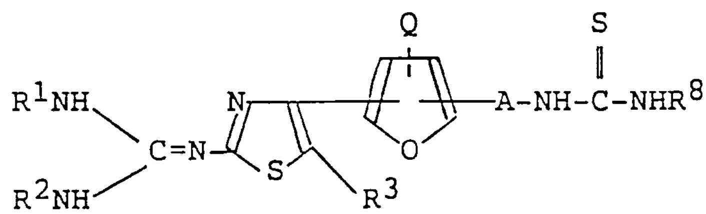

worin $R^1$, $R^2$, $R^3$, A und Q jeweils wie oben definiert sind, und $R^8$ eine Amino-Schutzgruppe ist, die aus $(C_1\text{-}C_6)$Alkanoyl und Benzoyl besteht, oder eines Salzes davon der Eliminierungsreaktion der Amino-Schutzgruppe, um eine Verbindung der Formel:

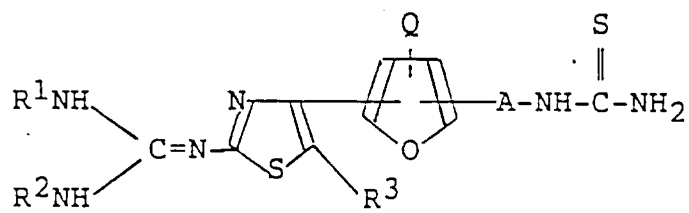

worin $R^1$, $R^2$, $R^3$, A und Q jeweils wie oben definiert sind, oder ein Salz davon zu ergeben, oder

(8) Reagieren einer Verbindung der Formel:

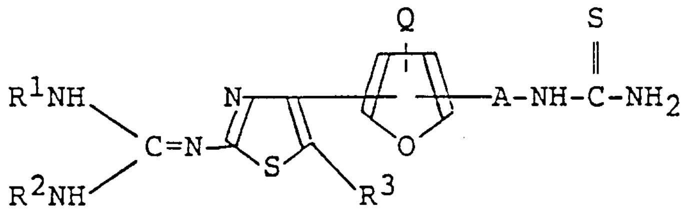

worin $R^1$, $R^2$, $R^3$, A und Q jeweils wie oben definiert sind, oder eines Salzes davon mit einer Verbindung der Formel:

$Z^2\text{-}CH_2\text{-}CO\text{-}R^9$

worin $R^9$ $(C_1\text{-}C_6)$Alkyl ist und $Z^2$ Halogen ist, um eine Verbindung der Formel:

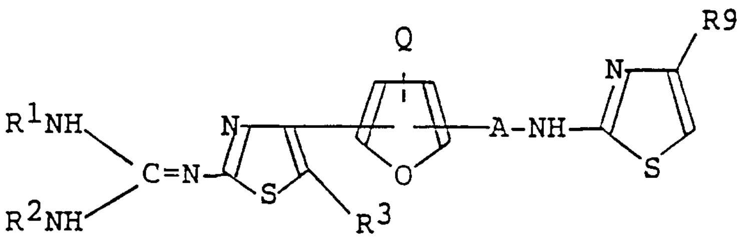

worin $R^1$, $R^2$, $R^3$, $R^9$, A und Q jeweils wie oben definiert sind, oder ein Salz davon zu ergeben oder

(9) Reagieren einer Verbindung der Formel:

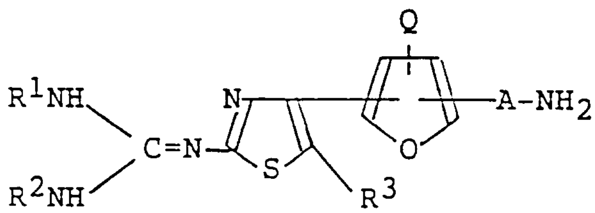

worin $R^1$, $R^2$, $R^3$, A und Q jeweils wie oben definiert sind, oder eines Salzes davon mit einer Verbindung der Formel:

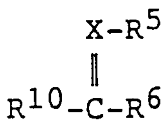

worin $R^5$, $R^6$ und X jeweils wie oben definiert sind und $R^{10}$ $(C_1\text{-}C_6)$Alkanoyloxy, Phenyl$(C_1\text{-}C_6)$-alkoxy, $(C_1\text{-}C_6)$Alkoxy$(C_1\text{-}C_6)$alkoxy, Tetrahydropyranyloxy oder $(C_1\text{-}C_6)$Alkoxy ist, um eine Verbindung der Formel:

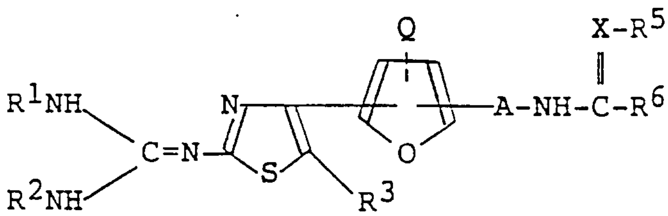

worin $R^1$, $R^2$, $R^3$, $R^5$, $R^6$, X, A und Q jeweils wie oben definiert sind, oder ein Salz davon zu ergeben oder

(10) Reagieren einer Verbindung der Formel:

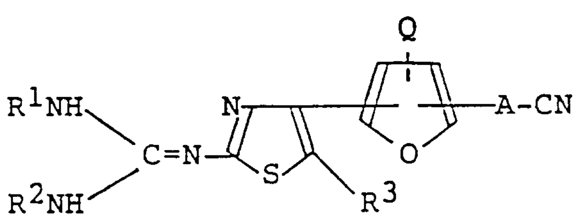

worin $R^1$, $R^2$, $R^3$, A und Q jeweils wie oben definiert sind, oder eines Salzes davon mit einer Verbindung der Formel:

$R^{11}OH$

worin $R^{11}$ $(C_1\text{-}C_6)$Alkyl ist, um eine Verbindung der Formel:

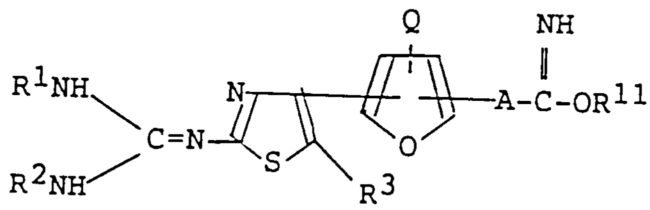

worin $R^1$, $R^2$, $R^3$, $R^{11}$, A und Q jeweils wie oben definiert sind, oder ein Salz davon zu ergeben oder
(11) Reagieren einer Verbindung der Formel:

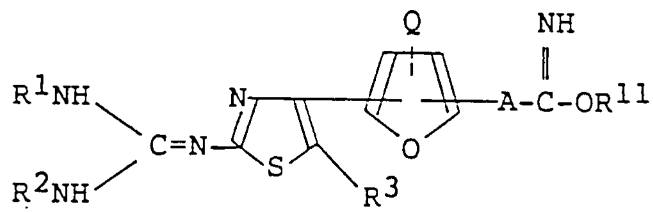

worin $R^1$, $R^2$, $R^3$, $R^{11}$, A und Q jeweils wie oben definiert sind, oder eines Salzes davon mit einer Verbindung der Formel:

$NH_2R^{12}$

worin $R^{12}$ Acyl ist, um eine Verbindung der Formel:

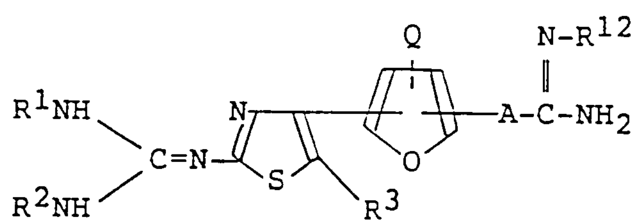

worin $R^1$, $R^2$, $R^3$, $R^{12}$, A und Q jeweils wie oben definiert sind, oder ein Salz davon zu ergeben oder
(12) Reagieren einer Verbindung der Formel:

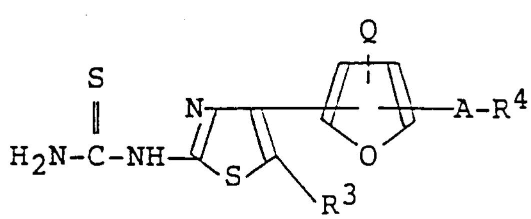

worin $R^3$, $R^4$, A und Q jeweils wie oben definiert sind, oder eines Salzes davon mit einer Verbindung der Formel:

$R^{13}\text{-}Z^3$

worin $R^{13}$ $(C_1\text{-}C_6)$Alkyl ist und $Z^3$ Halogen ist, oder eines Salzes davon, um eine Verbindung der Formel:

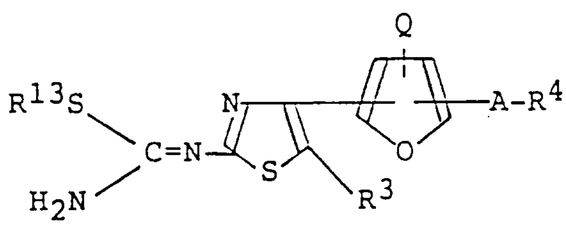

worin $R^3$, $R^4$, $R^{13}$, A und Q jeweils wie oben definiert sind, oder ein Salz davon zu ergeben, und ihr durchgehendes Reagierenlassen mit einer Verbindung der Formel:

$H_2N\text{-}Y\text{-}NH_2$

worin Y $(C_1\text{-}C_6)$Alkylen ist, oder ein Salz davon, um eine Verbindung der Formel:

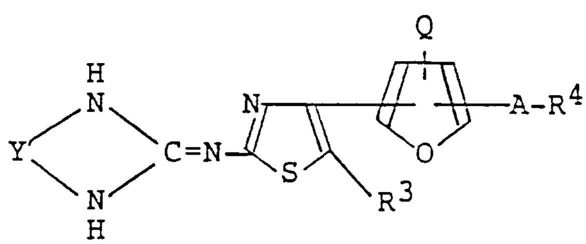

worin $R^3$, $R^4$, A, Q und Y jeweils wie oben definiert sind, oder ein Salz davon zu ergeben oder
(13) Unterwerfen einer Verbindung der Formel:

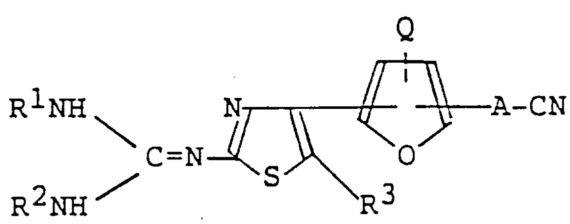

worin $R^1$, $R^2$, $R^3$, A und Q jeweils wie oben definiert sind, oder eines Salzes davon der Reduktion, um eine Verbindung der Formel:

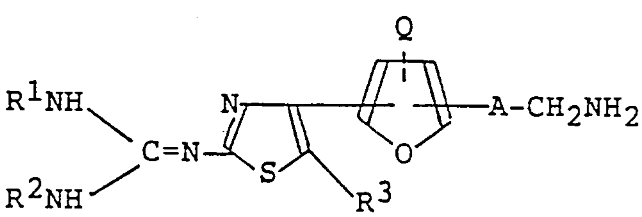

worin $R^1$, $R^2$, $R^3$, A und Q jeweils wie oben definiert sind, oder ein Salz davon zu ergeben oder

(14) Reagieren einer Verbindung der Formel:

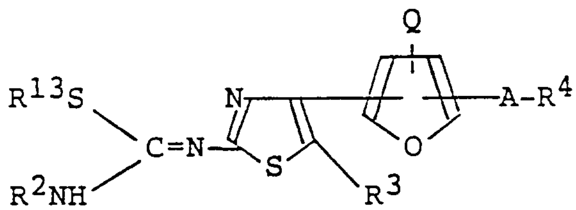

worin $R^2$, $R^3$, $R^4$, $R^{13}$, A und Q jeweils wie oben definiert sind, oder eines Salzes davon mit einer Verbindung der Formel:

$NH_2R^1_a$

worin $R^1_a$ $(C_1-C_6)$Alkyl ist, das Halogen aufweisen kann, oder einem Salz davon, um eine Verbindung der Formel:

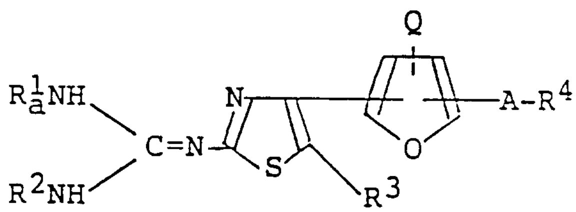

worin $R^1_a$, $R^2$, $R^3$, $R^4$, A und Q jeweils wie oben definiert sind, oder ein Salz davon zu ergeben oder

(15) Unterwerfen einer Verbindung der Formel:

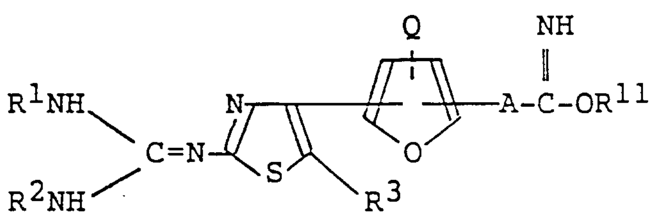

worin $R^1$, $R^2$, $R^3$, $R^{11}$, A und Q jeweils wie oben definiert sind, oder eines Salzes davon der Hydrolyse, um eine Verbindung der Formel:

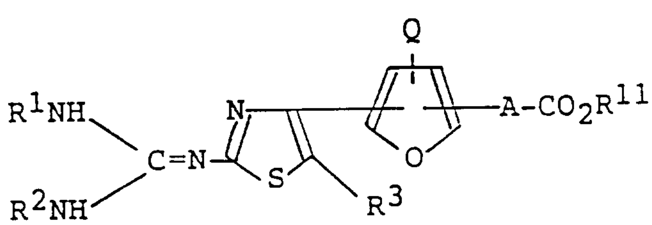

worin $R^1$, $R^2$, $R^3$, $R^{11}$, A und Q jeweils wie oben definiert sind, oder ein Salz davon zu ergeben oder

(16) Unterwerfen einer Verbindung der Formel:

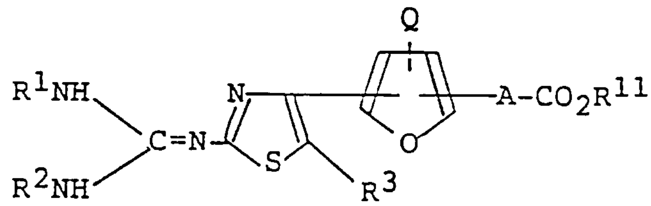

worin $R^1$, $R^2$, $R^3$, $R^{11}$, A und Q jeweils wie oben definiert sind, oder eines Salzes davon der Amidierungsreaktion, um eine Verbindung der Formel:

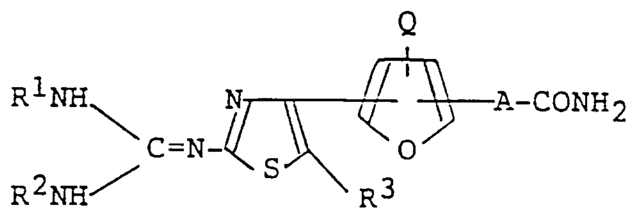

worin $R^1$, $R^2$, $R^3$, A und Q jeweils wie oben definiert sind, oder ein Salz davon zu ergeben oder

(17) Reagieren einer Verbindung der Formel:

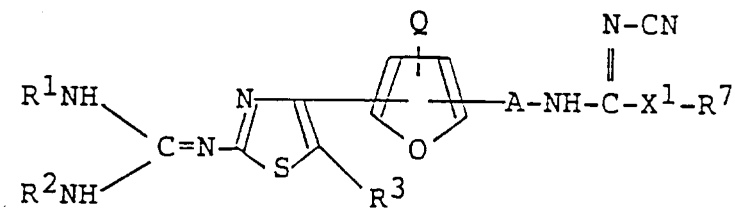

worin $R^1$, $R^2$, $R^3$, $R^7$, $X^1$, A und Q jeweils wie oben definiert sind, oder eines Salzes davon mit einer Verbindung der Formel:

$R^{14}NHNH_2$

worin $R^{14}$ Wasserstoff oder $(C_1$-$C_6)$Alkyl ist, um eine Verbindung der Formel:

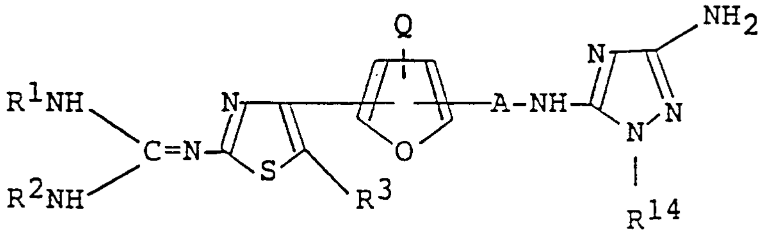

worin $R^1$, $R^2$, $R^3$, $R^{14}$, A und Q jeweils wie oben definiert sind, oder ein Salz davon zu ergeben oder

(18) Reagieren einer Verbindung der Formel:

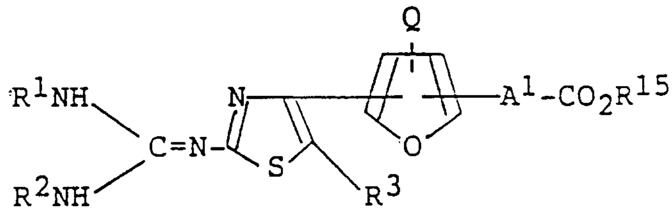

worin $R^1$, $R^2$, $R^3$ und Q jeweils wie oben definiert sind, $R^{15}$ $(C_1-C_6)$Alkyl ist und $A^1$ $(C_1-C_6)$Alkylen oder eine Bindung ist, oder eines Salzes davon mit Hydrazin, um eine Verbindung der Formel:

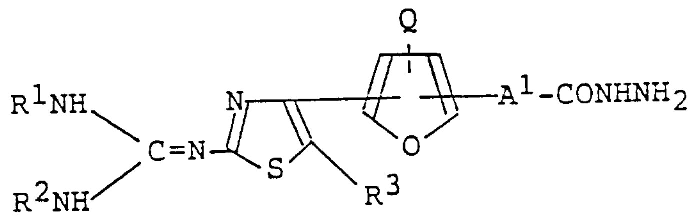

worin $R^1$, $R^2$, $R^3$, $A^1$ und Q jeweils wie oben definiert sind, oder ein Salz davon zu ergeben oder
(19) Reagieren einer Verbindung der Formel:

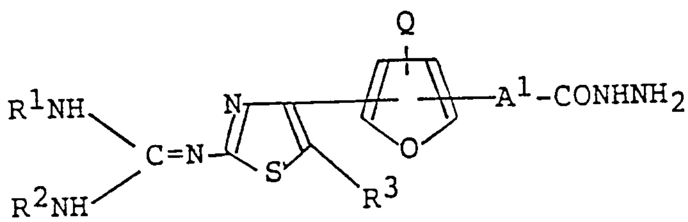

worin $R^1$, $R^2$, $R^3$, $A^1$ und Q jeweils wie oben definiert sind, oder eines Salzes davon mit S-$(C_1-C_6)$-Alkylisothioharnstoff oder einem Salz davon, um eine Verbindung der Formel:

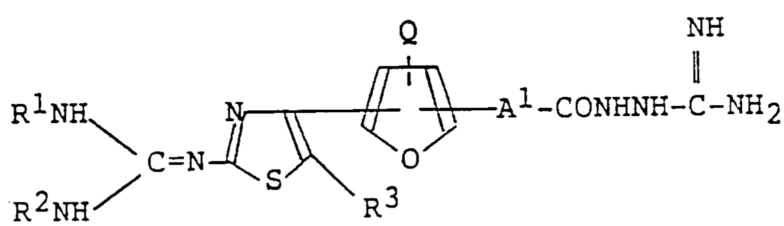

worin $R^1$, $R^2$, $R^3$, $A^1$ und Q jeweils wie oben definiert sind, oder ein Salz davon zu ergeben oder
(20) Unterwerfen einer Verbindung der Formel:

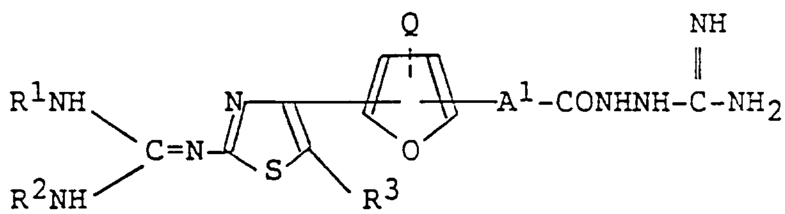

worin $R^1$, $R^2$, $R^3$, $A^1$ und Q jeweils wie oben definiert sind, oder eines Salzes davon dem Ringschluß, um eine Verbindung der Formel:

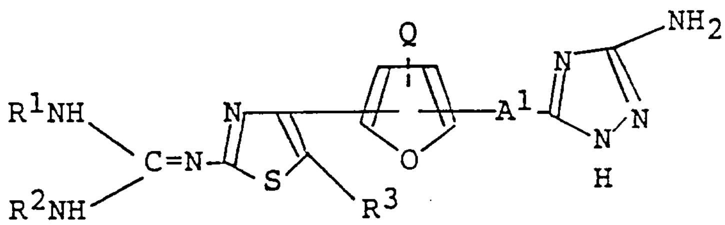

worin $R^1$, $R^2$, $R^3$, $A^1$ und Q jeweils wie oben definiert sind, oder ein Salz davon zu ergeben oder (21) Reagieren einer Verbindung der Formel:

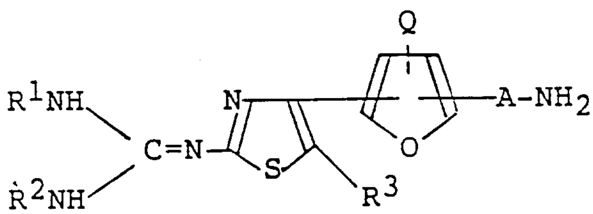

worin $R^1$, $R^2$, $R^3$, A und Q jeweils wie oben definiert sind, oder eines Salzes davon mit einer Verbindung der Formel:

$R^{16}$-$Z^4$

worin $R^{16}$ Thiazolyl substituiert mit $(C_1$-$C_6)$Alkyl, Triazolyl substituiert mit Amino, Triazolyl substituiert mit Amino und $(C_1$-$C_6)$Alkyl, Benzoisothiazolyl substituiert mit Oxo oder Benzothiadiazinyl substituiert mit Oxo ist, und $Z^4$ Halogen ist, oder einem Salz davon, um eine Verbindung der Formel:

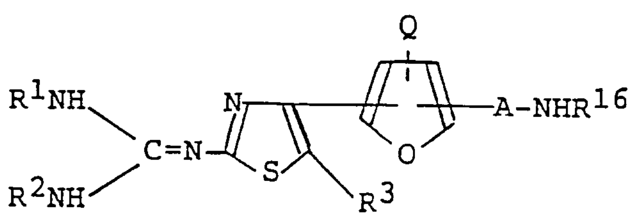

worin $R^1$, $R^2$, $R^3$, $R^{16}$, A und Q jeweils wie oben definiert sind, oder ein Salz davon zu ergeben oder (22) Unterwerfen einer Verbindung der Formel:

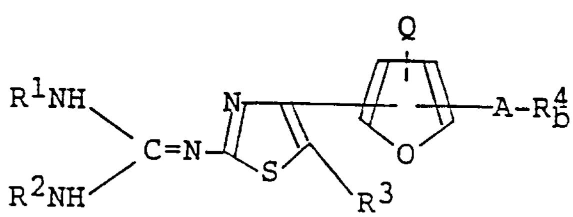

worin $R^1$, $R^2$, $R^3$, A und Q jeweils wie oben definiert sind, und $R^4{}_b$ Furyl$(C_1$-$C_6)$alkylthio$(C_1$-$C_6)$-Alkanoylamino ist, oder eines Salzes davon der Oxidationsreaktion, um eine Verbindung der Formel:

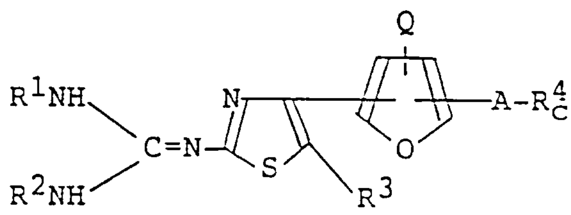

worin $R^1$, $R^2$, $R^3$, A und Q jeweils wie oben definiert sind, und $R^4_c$ Furyl($C_1$-$C_6$)alkylsulfinyl($C_1$-$C_6$)-alkanoylamino ist, oder ein Salz davon zu ergeben oder
(23) Reagieren einer Verbindung der Formel:

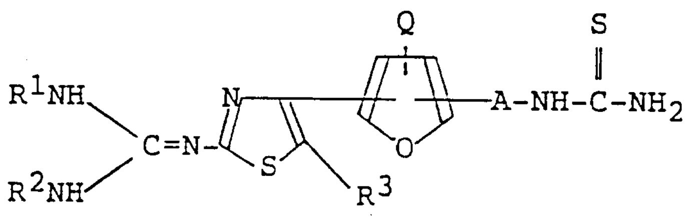

worin $R^1$, $R^2$, $R^3$, A und Q jeweils wie oben definiert sind, oder eines Salzes davon mit einer Verbindung der Formel:

$R^{17}$-$Z^5$

worin $R^{17}$ ($C_1$-$C_6$)Alkyl ist, und $Z^5$ Halogen ist, oder einem Salz davon, um eine Verbindung der Formel:

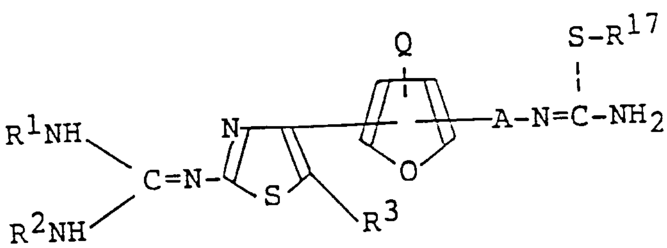

worin $R^1$, $R^2$, $R^3$, $R^{17}$, A und Q jeweils wie oben definiert sind, oder ein Salz davon zu ergeben oder
(24) Reagieren einer Verbindung der Formel:

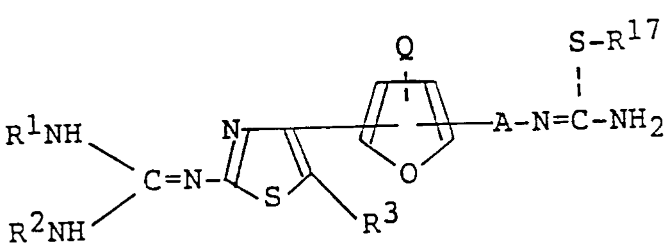

worin $R^1$, $R^2$, $R^3$, $R^{17}$, A und Q jeweils wie oben definiert sind, oder eines Salzes davon mit einer Verbindung der Formel:

$H_2N$-$R^5$

worin $R^5$ wie oben definiert ist, oder einem Salz davon, um eine Verbindung der Formel:

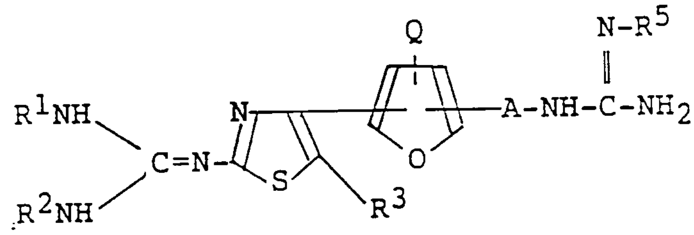

worin $R^1$, $R^2$, $R^3$, $R^5$, A und Q jeweils wie oben definiert sind, oder ein Salz davon zu ergeben oder
(25) Unterwerfen einer Verbindung der Formel:

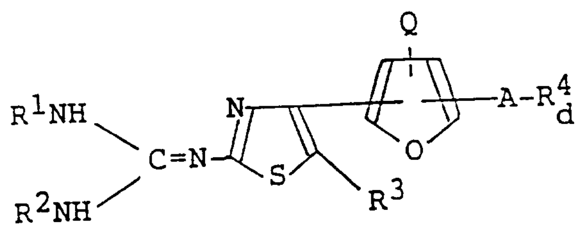

worin $R^1$, $R^2$, $R^3$, A und Q jeweils wie oben definiert sind, und $R^4{}_d$ $(C_1\text{-}C_6)$Alkanoyloxy$(C_1\text{-}C_6)$-alkylureido ist, oder eines Salzes davon der Eliminierungsreaktion von $(C_1\text{-}C_6)$Alkanoyl, um eine Verbindung der Formel:

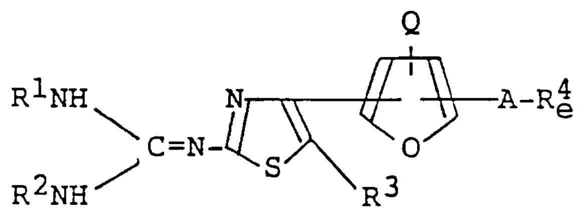

worin $R^1$, $R^2$, $R^3$, A und Q jeweils wie oben definiert sind, und $R^4{}_e$ Hydroxy$(C_1\text{-}C_6)$alkylureido ist, oder ein Salz davon zu ergeben oder
(26) Reagieren einer Verbindung der Formel:

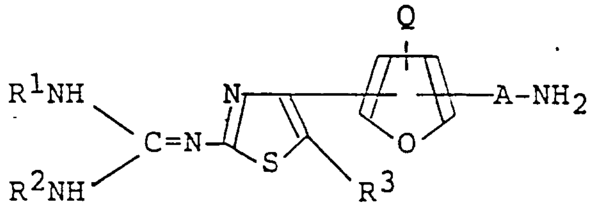

worin $R^1$, $R^2$, $R^3$, A und Q jeweils wie oben definiert sind, oder eines Salzes davon mit einer Verbindung der Formel:

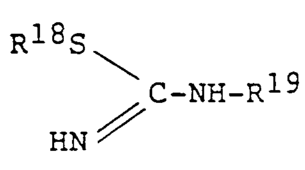

worin $R^{18}$ $(C_1\text{-}C_6)$Alkyl ist, und $R^{19}$ Carbamoyl, $(C_1\text{-}C_6)$Alkoxycarbonyl, Sulfamoyl, $(C_1\text{-}C_6)$-Alkylsulfonyl, Benzolsulfonyl, das mit einem oder mehreren Substituenten substituiert ist, ausgewählt

aus $(C_1-C_6)$Alkyl, $(C_1-C_6)$Alkoxy, Halogen und Amino, oder Mono- oder Di$(C_1-C_6)$Alkylsulfamoyl ist, oder einem Salz davon, um eine Verbindung der Formel:

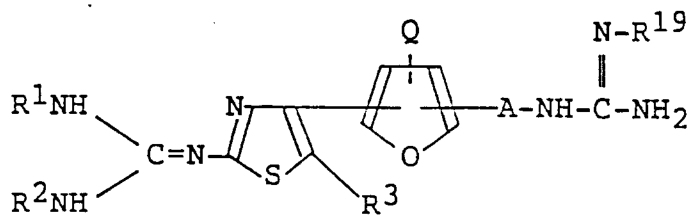

worin $R^1$, $R^2$, $R^3$, $R^{19}$, A und Q jeweils wie oben definiert sind, oder ein Salz davon zu ergeben oder (27) Unterwerfen einer Verbindung der Formel:

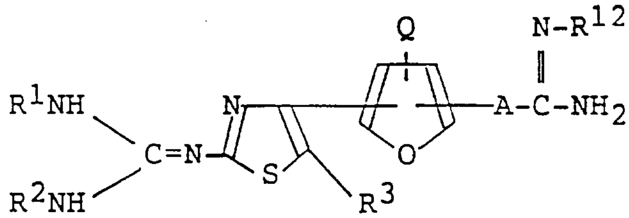

worin $R^1$, $R^2$, $R^3$, $R^{12}$, A und Q jeweils wie oben definiert sind, oder eines Salzes davon der Hydrolyse, um eine Verbindung der Formel:

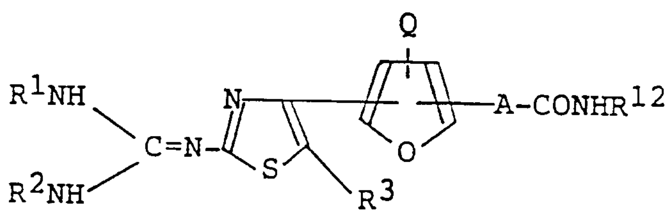

worin $R^1$, $R^2$, $R^3$, $R^{12}$, A und Q jeweils wie oben definiert sind, oder ein Salz davon zu ergeben.

**2.** Verfahren nach Anspruch 1, worin $R^1$ und $R^2$ jeweils Wasserstoff, $(C_1-C_6)$Alkyl oder Trihalogen$(C_1-C_6)$-alkyl sind; oder $R^1$ und $R^2$ miteinander verbunden sind, um $(C_1-C_6)$Alkylen zu bilden, $R^4$ Amino, Carbamoyl, Aminocarbamoyl, Guanidinocarbamoyl, $(C_1-C_6)$Alkylcarbamoyl, Sulfamoylaminocarbonyl, $(C_1-C_6)$Alkoxycarbonyl, Ureido, Thioureido, Sulfamoylamino, $(C_1-C_6)$Alkanoylamino, $(C_1-C_6)$-Alkoxycarbonylamino, $(C_1-C_6)$Alkylsulfonylamino, $(C_1-C_6)$-Alkoxy$(C_1-C_6)$alkanoylamino, $(C_1-C_6)$-Alkanoyloxy$(C_1-C_6)$alkanoylamino, $(C_1-C_6)$Alkylthio$(C_1-C_6)$alkanoylamino, Nitrobenzoylamino, Furoylamino, Thenoylamino, Nicotinoylamino, 1-Oxonicotinoylamino, Morpholinocarbonylamino, 3-$(C_1-C_6)$-Alkylureido, 3-$(C_1-C_6)$Alkylthioureido, 3-$(C_1-C_6)$Alkanoylureido, 3-Allylureido, 3-Benzoylthioureido, Furyl-$(C_1-C_6)$alkylthio$(C_1-C_6)$alkanoylamino, Furyl$(C_1-C_6)$alkylsulfinyl$(C_1-C_6)$alkanoylamino, Monooder Di$(C_1-C_6)$Alkylsulfamoylamino, 3-Hydroxy$(C_1-C_6)$alkylureido, 3-$(C_1-C_6)$Alkanoyloxy$(C_1-C_6)$alkylureido, 2-$(C_1-C_6)$Alkylisothioureido, Thiazolylamino substituiert mit $(C_1-C_6)$Alkyl, Triazolylamino substituiert mit Amino, Triazolylamino substituiert mit Amino und $(C_1-C_6)$Alkyl, Benzoisothiazolylamino substituiert mit Oxo, Benzothiadiazinylamino substituiert mit Oxo und Halogen, Pyrimidinyl substituiert mit Oxo und $(C_1-C_6)$-Alkyl, Triazolyl substituiert mit Amino, oder eine Gruppe der Formel:

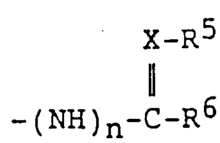

worin n, X und $R^6$ wie in Anspruch 1 definiert sind, ist, $R^5$ Wasserstoff, Cyano, Nitro, Carbamoyl, $(C_1$-$C_6)$Alkoxycarbonyl, Sulfamoyl, $(C_1$-$C_6)$Alkylsulfonyl, Phenylsulfonyl substituiert mit $(C_1$-$C_6)$Alkyl, Phenylsulfonyl substituiert mit $(C_1$-$C_6)$Alkoxy, Phenylsulfonyl substituiert mit Halogen, Phenylsulfonyl substituiert mit Amino, Mono- oder Di$(C_1$-$C_6)$Alkylsulfamoyl ist, A wie in Anspruch 1 definiert ist; oder A-$R^4$ wie in Anspruch 1 definiert ist, und $R^3$ und Q jeweils wie in Anspruch 1 definiert sind.

**3.** Verfahren nach Anspruch 2, worin $R^1$ und $R^2$ jeweils Wasserstoff sind, $R^3$ Wasserstoff ist, $R^4$ eine Gruppe der Formel ist:

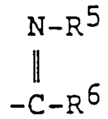

worin $R^5$ Sulfamoyl ist, und $R^6$ Amino ist, A $(C_1$-$C_6)$Alkylen und Q Wasserstoff ist.

**4.** Verfahren nach Anspruch 2, worin $R^1$ und $R^2$ jeweils Wasserstoff sind, $R^3$ Wasserstoff ist, $R^4$ Ureido oder 3-$(C_1$-$C_6)$Alkylureido ist, A $(C_1$-$C_6)$Alkylen ist und Q Wasserstoff ist.

**5.** Verfahren zur Herstellung einer pharmazeutischen Zusammensetzung, die als aktiven Bestandteil eine Verbindung nach Anspruch 1 oder ein pharmazeutisch verträgliches Salz davon umfaßt, dadurch gekennzeichnet, daß eine Verbindung nach Anspruch 1 mit mindestens einem pharmazeutisch verträglichen Träger oder Exzipienten gemischt wird.

**6.** Verfahren zur Herstellung einer Zusammensetzung nach Anspruch 5 für die therapeutische Behandlung von Geschwüren in Menschen oder Tieren.

**Patentansprüche für folgenden Vertragsstaat : GR**

**1.** Verfahren zur Herstellung einer Verbindung der Formel:

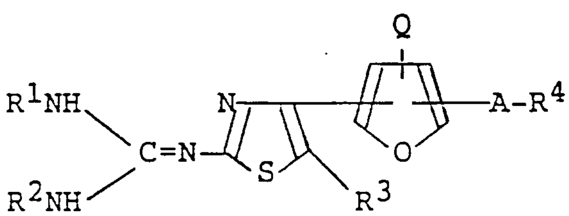

(I)

worin $R^1$ und $R^2$ jeweils Wasserstoff, Furoyl oder $(C_1$-$C_6)$Alkyl, das Halogen aufweisen kann, sind; oder $R^1$ und $R^2$ miteinander verbunden sind, um $(C_1$-$C_6)$Alkylen zu bilden, $R^3$ Wasserstoff oder $(C_1$-$C_6)$Alkyl ist, $R^4$ ist Amino, Carbamoyl, Aminocarbamoyl, Guanidinocarbamoyl, $(C_1$-$C_6)$Alkylcarbamoyl, Sulfamoylaminocarbonyl, $(C_1$-$C_6)$Alkoxycarbonyl, Ureido, Thioureido, Sulfamoylamino, $(C_1$-$C_6)$Alkanoylamino, $(C_1$-$C_6)$Alkorycarbonylamino, $(C_1$-$C_6)$Alkylsulfonylamino, $(C_1$-$C_6)$Alkoxy$(C_1$-$C_6)$alkanoylamino, $(C_1$-$C_6)$-Alkanoyloxy$(C_1$-$C_6)$alkanoylamino, $(C_1$-$C_6)$Alkylthio$(C_1$-$C_6)$alkanoylamino, Benzoylamino wahlweise substituiert durch Nitro, Furoylamino, Thenoylamino, Nicotinoylamino, 1-Oxonicotinoylamino, Morpholinocarbonylamino, $(C_1$-$C_6)$Alkylureido, $(C_1$-$C_6)$Alkylthioureido, $(C_1$-$C_6)$Alkanoylureido, Allylureido, Benzoylthioureido, Furyl$(C_1$-$C_6)$alkylthio$(C_1$-$C_6)$alkanoylamino, Furyl$(C_1$-$C_6)$alkylsulfinyl$(C_1$-$C_6)$alkanoylamino, Mono- oder Di$(C_1$-$C_6)$Alkylsulfamoylamino, Hydroxy$(C_1$-$C_6)$alkylureido, $(C_1$-$C_6)$Alkanoyloxy$(C_1$-$C_6)$-alkylureido, $(C_1$-$C_6)$Alkylisothioureido, Thiazolylamino substituiert mit $(C_1$-$C_6)$Alkyl, Triazolylamino substituiert mit Amino, Triazolylamino substituiert mit Amino und $(C_1$-$C_6)$Alkyl, Benzoisothiazolylamino substituiert mit Oxo, Benzothiadiazinylamino substituiert mit Oxo und Halogen, Pyrimidinyl substituiert mit Oxo und $(C_1$-$C_6)$Alkyl, Triazolyl substituiert mit Amino oder einer Gruppe der Formel:

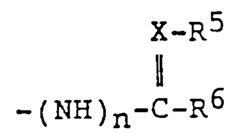

worin n 0 oder 1 ist, X =CH- oder =N- ist, $R^5$ Wasserstoff, Cyano, Nitro, Carbamoyl, $(C_1-C_6)$-Alkoxycarbonyl, Sulfamoyl, $(C_1-C_6)$Alkylsulfonyl, Benzolsulfonyl, das mit einem oder mehreren Substiuenten substituiert ist, ausgewählt aus $(C_1-C_6)$Alkyl, $(C_1-C_6)$Alkoxy, Halogen und Amino, oder Mono- oder Di$(C_1-C_6)$Alkylsulfamoyl ist, $R^6$ Wasserstoff, $(C_1-C_6)$Alkyl, $(C_1-C_6)$Alkylthio, $(C_1-C_6)$Alkoxy, Amino, Mono- oder Di$(C_1-C_6)$Alkylamino, Allylamino, Propargylamino, Hydroxy$(C_1-C_6)$Alkylamino, $(C_1-C_6)$-Alkoxy$(C_1-C_6)$alkylamino, oder Mono- oder Di$(C_1-C_6)$Alkylamino$(C_1-C_6)$alkylamino ist; A $(C_1-C_6)$Alkylen oder -CONH- ist; oder A-$R^4$ Imidazolyl substituiert mit $(C_1-C_6)$Alkyl, Triazolyl substituiert mit Amino ist, und Q Wasserstoff oder $(C_1-C_6)$Alkyl ist, oder eines pharmazeutisch verträglichen Salzes davon, das umfaßt:

(1) Reagieren einer Verbindung der Formel:

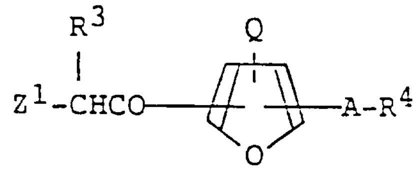

worin $R^3$, $R^4$, A und Q jeweils wie oben definiert sind, und $Z^1$ Halogen ist, oder eines Salz davon mit einer Verbindung der Formel:

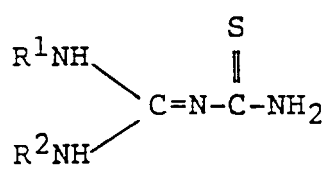

worin $R^1$ und $R^2$ jeweils wie oben definiert sind, um eine Verbindung der Formel:

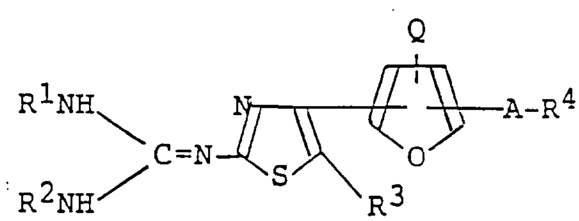

worin $R^1$, $R^2$, $R^3$, $R^4$, A und Q jeweils wie oben definiert sind, oder ein Salz davon zu ergeben oder

(2) Unterwerfen einer Verbindung der Formel:

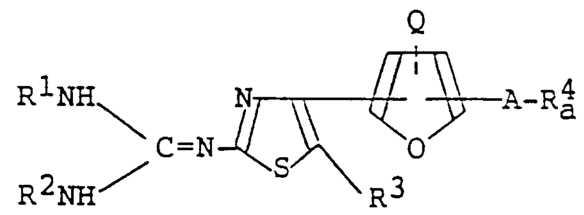

worin $R^1$, $R^2$, $R^3$, A und Q jeweils wie oben definiert sind, und $R^4{}_a$ Ureido, Thioureido, Sulfamoylami-

no, ($C_1$-$C_6$)Alkanoylamino, ($C_1$-$C_6$)Alkoxycarbonylamino, ($C_1$-$C_6$)Alkylsulfonylamino, ($C_1$-$C_6$)Alkoxy-($C_1$-$C_6$)alkanoylamino, ($C_1$-$C_6$)Alkanoyloxy($C_1$-$C_6$)alkanoylamino, ($C_1$-$C_6$)Alkylthio($C_1$-$C_6$)-alkanoylamino, Benzoylamino wahlweise substituiert mit Nitro, Furoylamino, Thenoylamino, Nicotinoylamino, 1-Oxonicotinoylamino, Morpholinocarbonylamino, ($C_1$-$C_6$)Alkylureido, ($C_1$-$C_6$)-Alkylthioureido, ($C_1$-$C_6$)Alkanoylureido, Allylureido, Benzoylthioureido, Furyl($C_1$-$C_6$)alkylthio($C_1$-$C_6$)-alkanoylamino, Mono- oder Di($C_1$-$C_6$)Alkylsulfamoylamino, Hydroxy($C_1$-$C_6$)alkylureido oder ($C_1$-$C_6$)-Alkanoyloxy($C_1$-$C_6$)alkylureido ist, oder eines Salzes davon der Deacylierungsreaktion, um eine Verbindung der Formel:

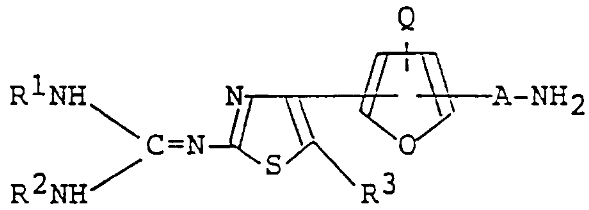

worin $R^1$, $R^2$, $R^3$, A und Q jeweils wie oben definiert sind, oder ein Salz davonzu ergeben oder
(3) Unterwerfen einer Verbindung der Formel:

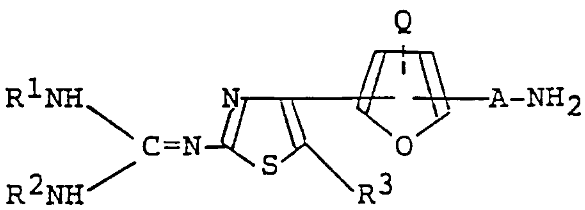

worin $R^1$, $R^2$, $R^3$, A und Q jeweils wie oben definiert sind, oder eines Salzes davon der Acylierungsreaktion, um eine Verbindung der Formel:

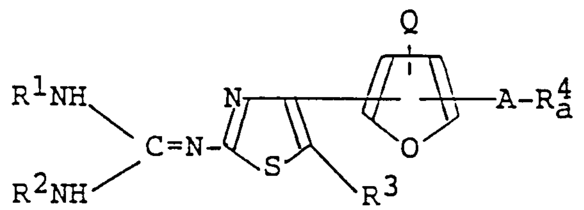

worin $R^1$, $R^2$, $R^3$, $R^4{}_a$, A und Q jeweils wie oben definiert sind, oder ein Salz davon zu ergeben oder
(4) Reagieren einer Verbindung der Formel:

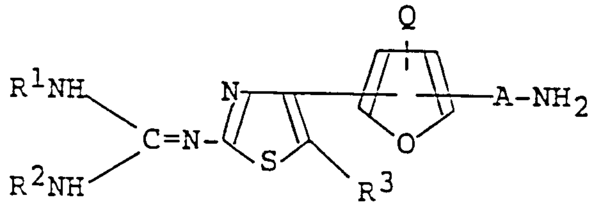

worin $R^1$, $R^2$, $R^3$, A und Q jeweils wie oben definiert sind, oder eines Salzes davon mit einer Verbindung der Formel:

$(R^7\text{-}X^1)_2C=X\text{-}R^5$

worin $R^5$ und X jeweils wie oben definiert sind, R7 $(C_1\text{-}C_6)$Alkyl oder Phenyl ist, und $X^1$ S oder O ist, um eine Verbindung der Formel:

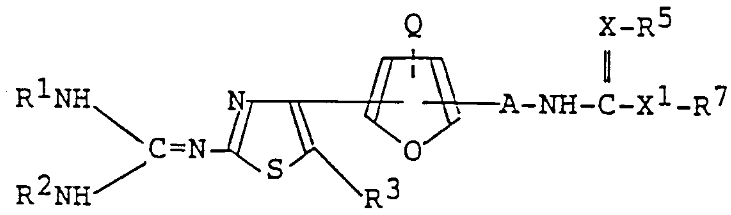

worin $R^1$, $R^2$, $R^3$, $R^5$, $R^7$, X, $X^1$, A und Q jeweils wie oben definiert sind, oder ein Salz davon zu ergeben oder

(5) Reagieren einer Verbindung der Formel:

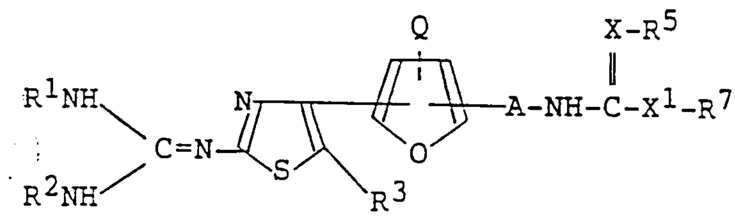

worin $R^1$, $R^2$, $R^3$, $R^5$, $R^7$, X, $X^1$, A und Q jeweils wie oben definiert sind, oder eines Salzes davon mit einer Verbindung der Formel:

$H\text{-}R^6{}_a$

worin $R^6{}_a$ Amino ist, das Mono- oder Di$(C_1\text{-}C_6)$Alkyl, Allyl, Propargyl. Hydroxy$(C_1\text{-}C_6)$Alkyl oder Mono- oder Di$(C_1\text{-}C_6)$Alkylamino$(C_1\text{-}C_6)$alkyl, oder $(C_1\text{-}C_6)$Alkoxy aufweisen kann, um eine Verbindung der Formel:

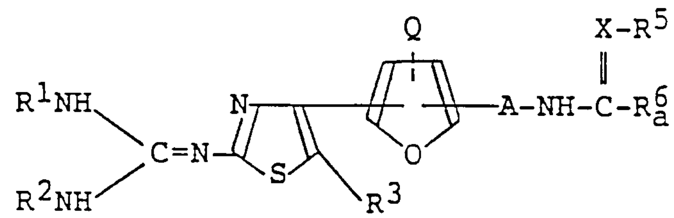

worin $R^1$, $R^2$, $R^3$, $R^5$, $R^6{}_a$, X, A und Q jeweils wie oben definiert sind, oder ein Salz davon zu ergeben oder

(6) Unterwerfen einer Verbindung der Formel:

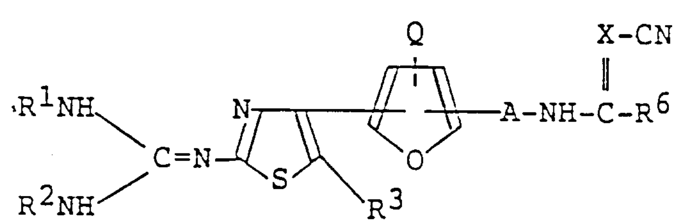

worin $R^1$, $R^2$, $R^3$, $R^6$, X, A und Q jeweils wie oben definiert sind, oder eines Salzes davon der Hydrolyse, um eine Verbindung der Formel:

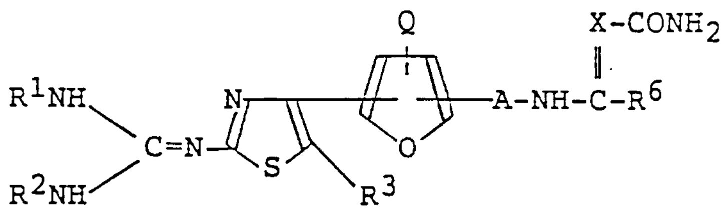

worin $R^1$, $R^2$, $R^3$, $R^6$, X, A und Q jeweils wie oben definiert sind, oder ein Salz davon zu ergeben oder

(7) Unterwerfen einer Verbindung der Formel:

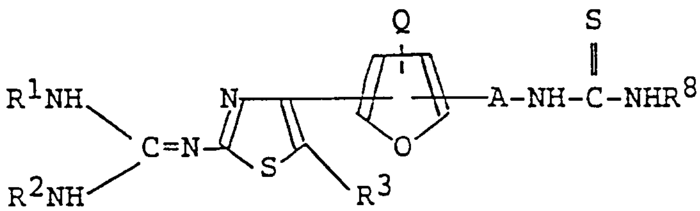

worin $R^1$, $R^2$, $R^3$, A und Q jeweils wie oben definiert sind, und $R^8$ eine Amino-Schutzgruppe ist, die aus $(C_1\text{-}C_6)$Alkanoyl und Benzoyl besteht, oder eines Salzes davon der Eliminierungsreaktion der Amino-Schutzgruppe, um eine Verbindung der Formel:

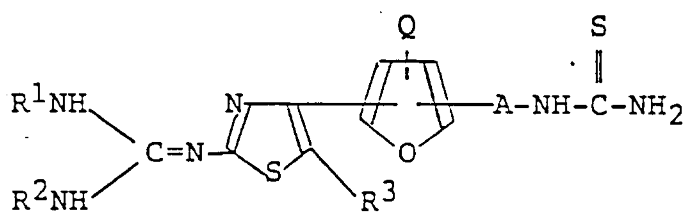

worin $R^1$, $R^2$, $R^3$, A und Q jeweils wie oben definiert sind, oder ein Salz davon zu ergeben, oder

(8) Reagieren einer Verbindung der Formel:

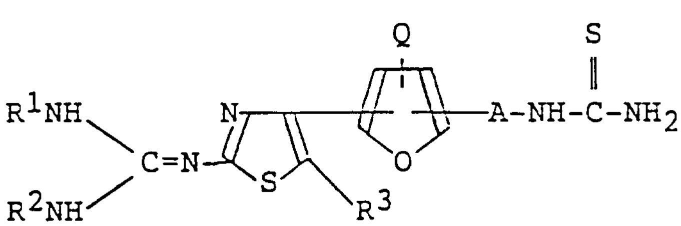

worin $R^1$, $R^2$, $R^3$, A und Q jeweils wie oben definiert sind, oder eines Salzes davon mit einer Verbindung der Formel:

$Z^2\text{-}CH_2\text{-}CO\text{-}R^9$

worin $R^9$ $(C_1\text{-}C_6)$Alkyl ist und $Z^2$ Halogen ist, um eine Verbindung der Formel:

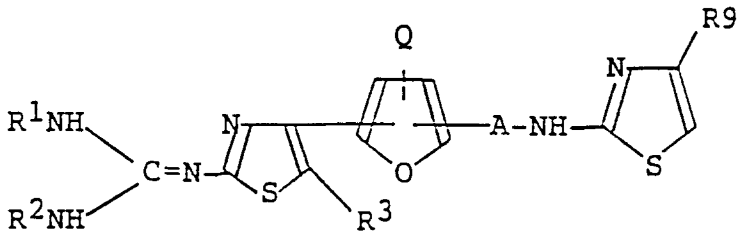

worin $R^1$, $R^2$, $R^3$, $R^9$, A und Q jeweils wie oben definiert sind, oder ein Salz davon zu ergeben oder
(9) Reagieren einer Verbindung der Formel:

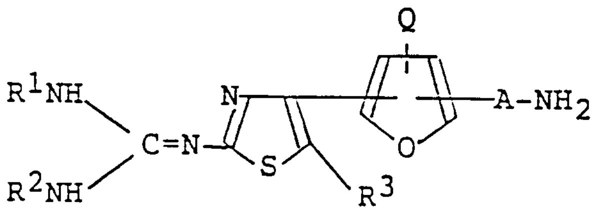

worin $R^1$, $R^2$, $R^3$, A und Q jeweils wie oben definiert sind, oder eines Salzes davon mit einer Verbindung der Formel:

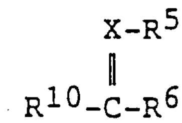

worin $R^5$, $R^6$ und X jeweils wie oben definiert sind und $R^{10}$ ($C_1$-$C_6$)Alkanoyloxy, Phenyl($C_1$-$C_6$)-alkoxy, ($C_1$-$C_6$)Alkoxy($C_1$-$C_6$)alkoxy, Tetrahydropyranyloxy oder ($C_1$-$C_6$)Alkoxy ist, um eine Verbindung der Formel:

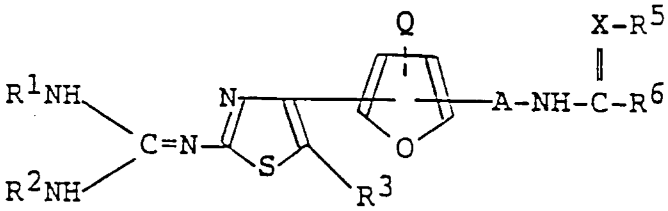

worin $R^1$, $R^2$, $R^3$, $R^5$, $R^6$, X, A und Q jeweils wie oben definiert sind, oder ein Salz davon zu ergeben oder
(10) Reagieren einer Verbindung der Formel:

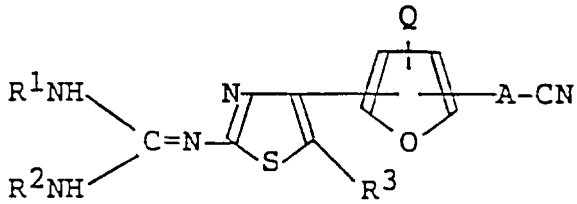

worin $R^1$, $R^2$, $R^3$, A und Q jeweils wie oben definiert sind, oder eines Salzes davon mit einer Verbindung der Formel:

$R^{11}OH$

worin $R^{11}$ $(C_1-C_6)$Alkyl ist, um eine Verbindung der Formel:

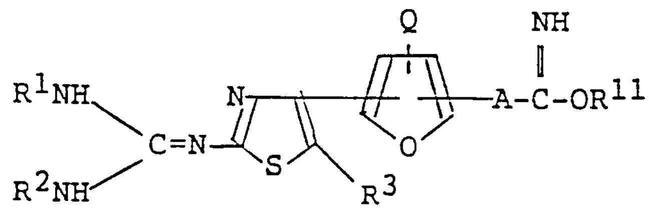

worin $R^1$, $R^2$, $R^3$, $R^{11}$, A und Q jeweils wie oben definiert sind, oder ein Salz davon zu ergeben oder (11) Reagieren einer Verbindung der Formel:

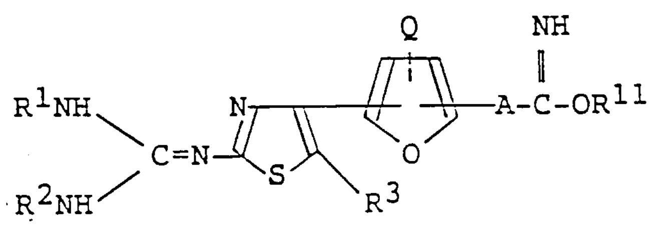

worin $R^1$, $R^2$, $R^3$, $R^{11}$, A und Q jeweils wie oben definiert sind, oder eines Salzes davon mit einer Verbindung der Formel:

$NH_2R^{12}$

worin $R^{12}$ Acyl ist, um eine Verbindung der Formel:

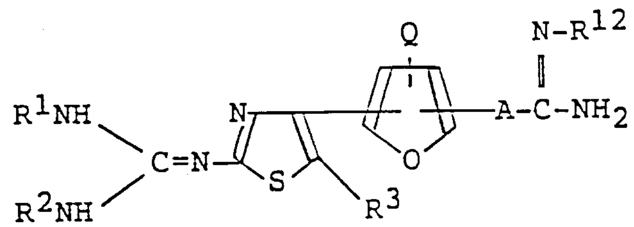

worin $R^1$, $R^2$, $R^3$, $R^{12}$, A und Q jeweils wie oben definiert sind, oder ein Salz davon zu ergeben oder (12) Reagieren einer Verbindung der Formel:

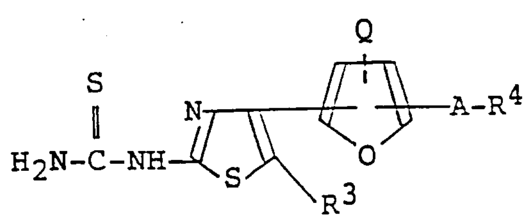

worin $R^3$, $R^4$, A und Q jeweils wie oben definiert sind, oder eines Salzes davon mit einer Verbindung der Formel:

$R^{13}-Z^3$

worin $R^{13}$ $(C_1\text{-}C_6)$Alkyl ist und $Z^3$ Halogen ist, oder eines Salzes davon, um eine Verbindung der Formel:

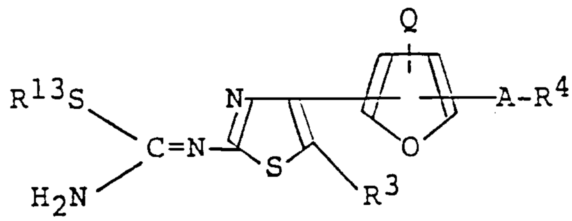

worin $R^3$, $R^4$, $R^{13}$, A und Q jeweils wie oben definiert sind, oder ein Salz davon zu ergeben, und ihr durchgehendes Reagierenlassen mit einer Verbindung der Formel:

$H_2N\text{-}Y\text{-}NH_2$

worin Y $(C_1\text{-}C_6)$Alkylen ist, oder ein Salz davon, um eine Verbindung der Formel:

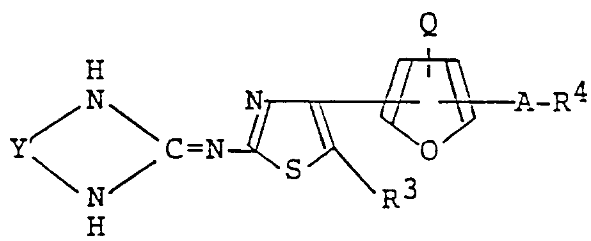

worin $R^3$, $R^4$, A, Q und Y jeweils wie oben definiert sind, oder ein Salz davon zu ergeben oder
(13) Unterwerfen einer Verbindung der Formel:

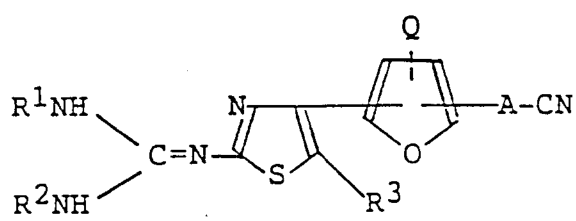

worin $R^1$, $R^2$, $R^3$, A und Q jeweils wie oben definiert sind, oder eines Salzes davon der Reduktion, um eine Verbindung der Formel:

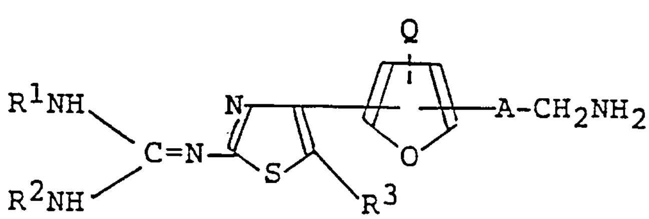

worin $R^1$, $R^2$, $R^3$, A und Q jeweils wie oben definiert sind, oder ein Salz davon zu ergeben oder

(14) Reagieren einer Verbindung der Formel:

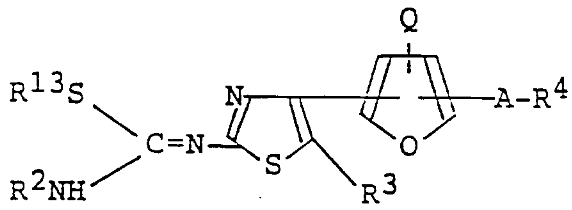

worin $R^2$, $R^3$, $R^4$, $R^{13}$, A und Q jeweils wie oben definiert sind, oder eines Salzes davon mit einer Verbindung der Formel:

$NH_2R^1_a$

worin $R^1_a$ $(C_1-C_6)$Alkyl ist, das Halogen aufweisen kann, oder einem Salz davon, um eine Verbindung der Formel:

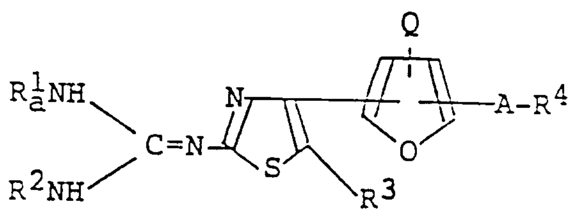

worin $R^1_a$, $R^2$, $R^3$, $R^4$, A und Q jeweils wie oben definiert sind, oder ein Salz davon zu ergeben oder
(15) Unterwerfen einer Verbindung der Formel:

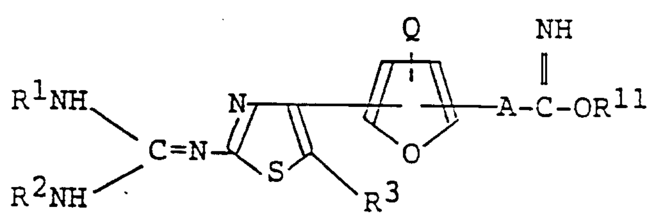

worin $R^1$, $R^2$, $R^3$, $R^{11}$, A und Q jeweils wie oben definiert sind, oder eines Salzes davon der Hydrolyse, um eine Verbindung der Formel:

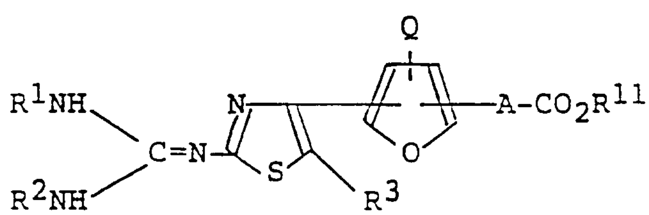

worin $R^1$, $R^2$, $R^3$, $R^{11}$, A und Q jeweils wie oben definiert sind, oder ein Salz davon zu ergeben oder

(16) Unterwerfen einer Verbindung der Formel:

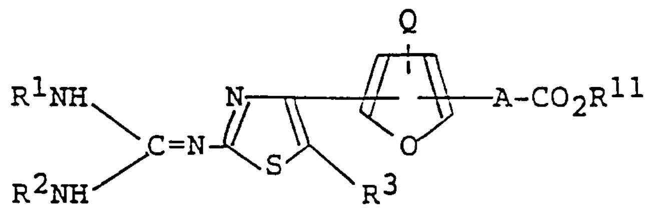

worin $R^1$, $R^2$, $R^3$, $R^{11}$, A und Q jeweils wie oben definiert sind, oder eines Salzes davon der Amidierungsreaktion, um eine Verbindung der Formel:

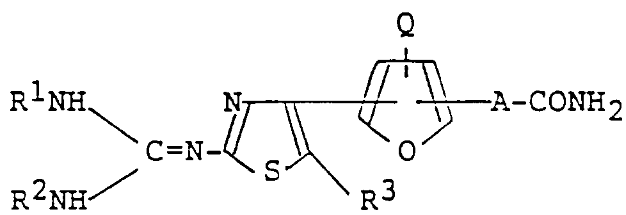

worin $R^1$, $R^2$, $R^3$, A und Q jeweils wie oben definiert sind, oder ein Salz davon zu ergeben oder
(17) Reagieren einer Verbindung der Formel:

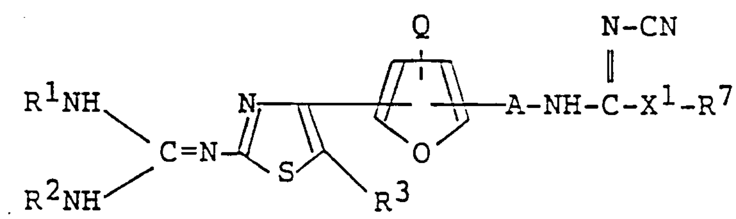

worin $R^1$, $R^2$, $R^3$, $R^7$, $X^1$, A und Q jeweils wie oben definiert sind, oder eines Salzes davon mit einer Verbindung der Formel:

$R^{14}NHNH_2$

worin $R^{14}$ Wasserstoff oder $(C_1\text{-}C_6)$Alkyl ist, um eine Verbindung der Formel:

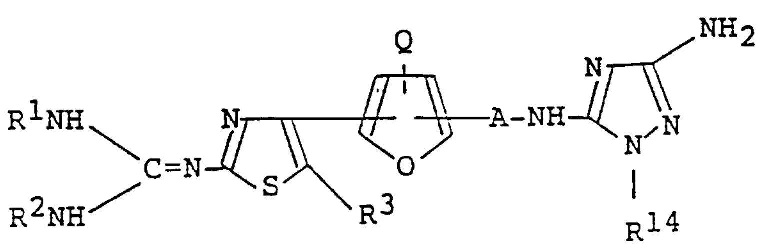

worin $R^1$, $R^2$, $R^3$, $R^{14}$, A und Q jeweils wie oben definiert sind, oder ein Salz davon zu ergeben oder

(18) Reagieren einer Verbindung der Formel:

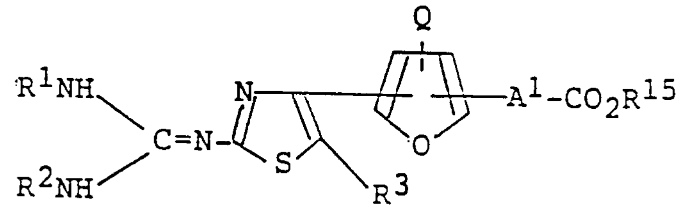

worin $R^1$, $R^2$, $R^3$ und Q jeweils wie oben definiert sind, $R^{15}$ ($C_1$-$C_6$)Alkyl ist und $A^1$ ($C_1$-$C_6$)Alkylen oder eine Bindung ist, oder eines Salzes davon mit Hydrazin, um eine Verbindung der Formel:

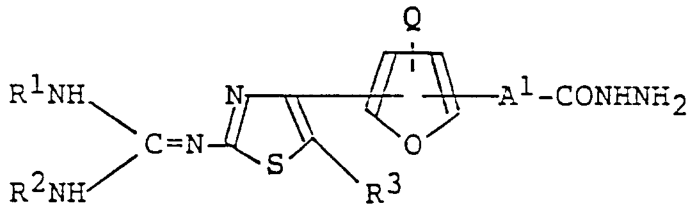

worin $R^1$, $R^2$, $R^3$, $A^1$ und Q jeweils wie oben definiert sind, oder ein Salz davon zu ergeben oder
(19) Reagieren einer Verbindung der Formel:

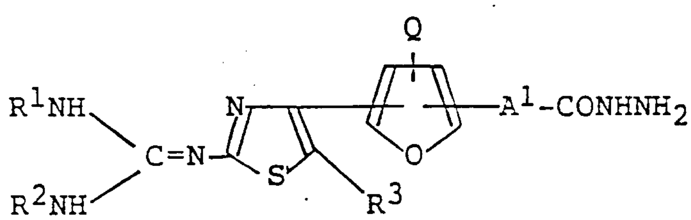

worin $R^1$, $R^2$, $R^3$, $A^1$ und Q jeweils wie oben definiert sind, oder eines Salzes davon mit S-($C_1$-$C_6$)-Alkylisothioharnstoff oder einem Salz davon, um eine Verbindung der Formel:

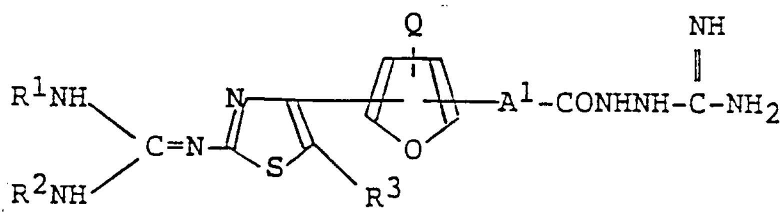

worin $R^1$, $R^2$, $R^3$, $A^1$ und Q jeweils wie oben definiert sind, oder ein Salz davon zu ergeben oder
(20) Unterwerfen einer Verbindung der Formel:

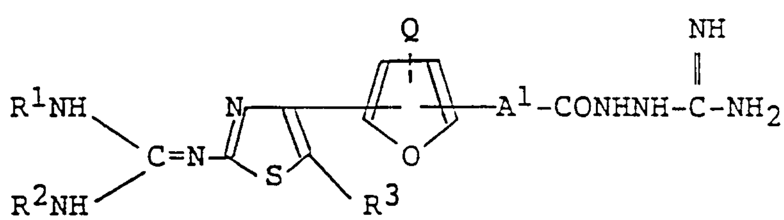

worin $R^1$, $R^2$, $R^3$, $A^1$ und Q jeweils wie oben definiert sind, oder eines Salzes davon dem Ringschluß,

um eine Verbindung der Formel:

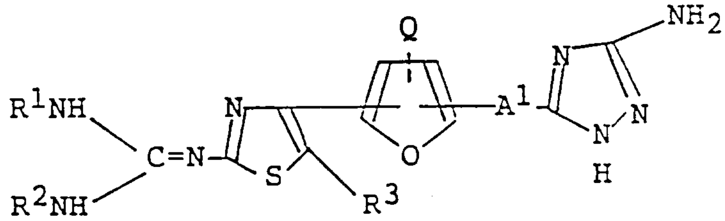

worin $R^1$, $R^2$, $R^3$, $A^1$ und Q jeweils wie oben definiert sind, oder ein Salz davon zu ergeben oder
(21) Reagieren einer Verbindung der Formel:

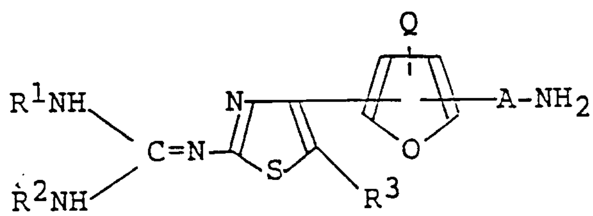

worin $R^1$, $R^2$, $R^3$, A und Q jeweils wie oben definiert sind, oder eines Salzes davon mit einer Verbindung der Formel:

$R^{16}$-$Z^4$

worin $R^{16}$ Thiazolyl substituiert mit ($C_1$-$C_6$)Alkyl, Triazolyl substituiert mit Amino, Triazolyl substituiert mit Amino und ($C_1$-$C_6$)Alkyl, Benzoisothiazolyl substituiert mit Oxo oder Benzothiadiazinyl substituiert mit Oxo ist, und $Z^4$ Halogen ist, oder einem Salz davon, um eine Verbindung der Formel:

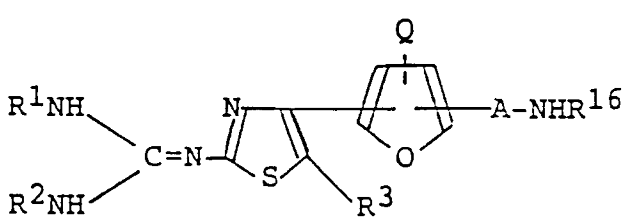

worin $R^1$, $R^2$, $R^3$, $R^{16}$, A und Q jeweils wie oben definiert sind, oder ein Salz davon zu ergeben oder
(22) Unterwerfen einer Verbindung der Formel:

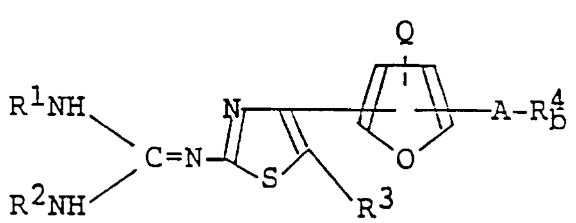

worin $R^1$, $R^2$, $R^3$, A und Q jeweils wie oben definiert sind, und $R^4_b$ Furyl($C_1$-$C_6$)alkylthio($C_1$-$C_6$)-Alkanoylamino ist, oder eines Salzes davon der Oxidationsreaktion, um eine Verbindung der Formel:

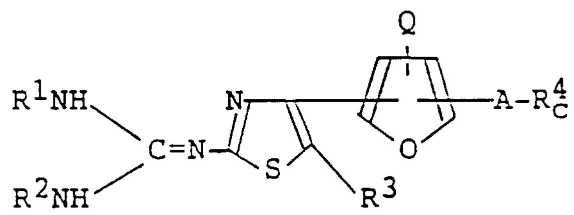

worin $R^1$, $R^2$, $R^3$, A und Q jeweils wie oben definiert sind, und $R^4_c$ Furyl($C_1$-$C_6$)alkylsulfinyl($C_1$-$C_6$)-alkanoylamino ist, oder ein Salz davon zu ergeben oder
(23) Reagieren einer Verbindung der Formel:

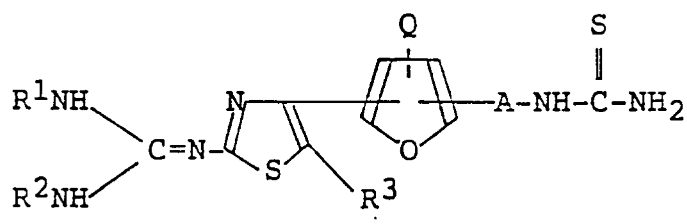

worin $R^1$, $R^2$, $R^3$, A und Q jeweils wie oben definiert sind, oder eines Salzes davon mit einer Verbindung der Formel:

$R^{17}$-$Z^5$

worin $R^{17}$ ($C_1$-$C_6$)Alkyl ist, und $Z^5$ Halogen ist, oder einem Salz davon, um eine Verbindung der Formel:

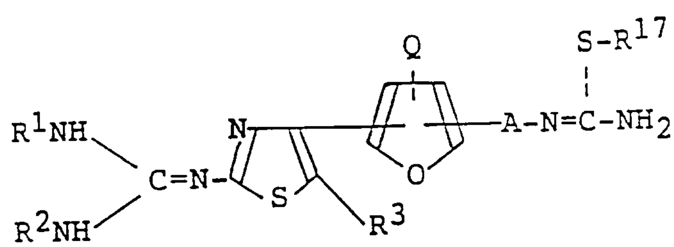

worin $R^1$, $R^2$, $R^3$, $R^{17}$, A und Q jeweils wie oben definiert sind, oder ein Salz davon zu ergeben oder
(24) Reagieren einer Verbindung der Formel:

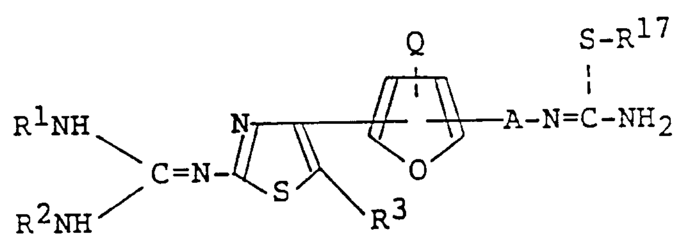

worin $R^1$, $R^2$, $R^3$, $R^{17}$, A und Q jeweils wie oben definiert sind, oder eines Salzes davon mit einer Verbindung der Formel:

$H_2N$-$R^5$

worin $R^5$ wie oben definiert ist, oder einem Salz davon, um eine Verbindung der Formel:

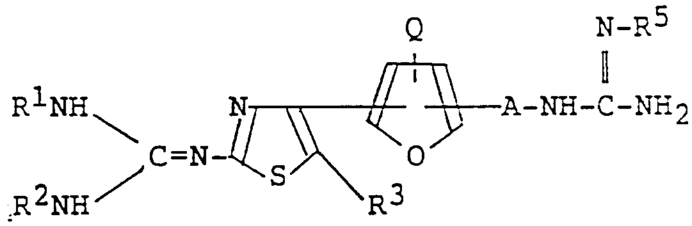

worin $R^1$, $R^2$, $R^3$, $R^5$, A und Q jeweils wie oben definiert sind, oder ein Salz davon zu ergeben oder
(25) Unterwerfen einer Verbindung der Formel:

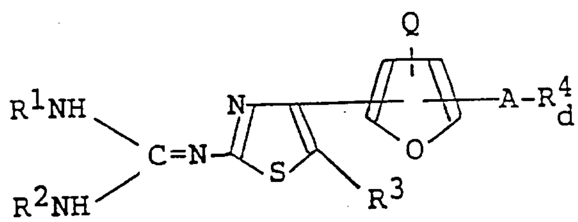

worin $R^1$, $R^2$, $R^3$, A und Q jeweils wie oben definiert sind, und $R^4{}_d$ $(C_1-C_6)$Alkanoyloxy$(C_1-C_6)$-alkylureido ist, oder eines Salzes davon der Eliminierungsreaktion von $(C_1-C_6)$Alkanoyl, um eine Verbindung der Formel:

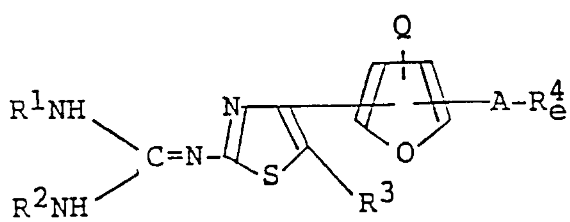

worin $R^1$, $R^2$, $R^3$, A und Q jeweils wie oben definiert sind, und $R^4{}_e$ Hydroxy$(C_1-C_6)$alkylureido ist, oder ein Salz davon zu ergeben oder
(26) Reagieren einer Verbindung der Formel:

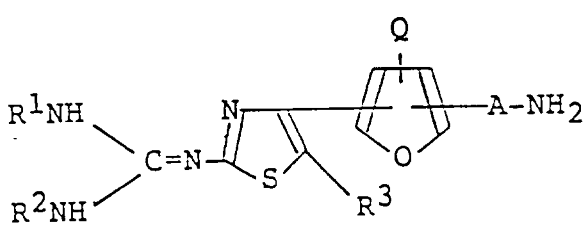

worin $R^1$, $R^2$, $R^3$, A und Q jeweils wie oben definiert sind, oder eines Salzes davon mit einer Verbindung der Formel:

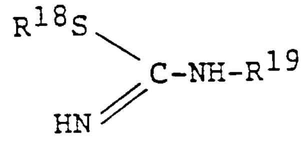

worin $R^{18}$ $(C_1\text{-}C_6)$Alkyl ist, und $R^{19}$ Carbamoyl, $(C_1\text{-}C_6)$Alkoxycarbonyl, Sulfamoyl, $(C_1\text{-}C_6)$-Alkylsulfonyl, Benzolsulfonyl, das mit einem oder mehreren Substituenten substituiert ist, ausgewählt aus $(C_1\text{-}C_6)$Alkyl, $(C_1\text{-}C_6)$Alkoxy, Halogen und Amino, oder Mono- oder Di$(C_1\text{-}C_6)$Alkylsulfamoyl ist, oder einem Salz davon, um eine Verbindung der Formel:

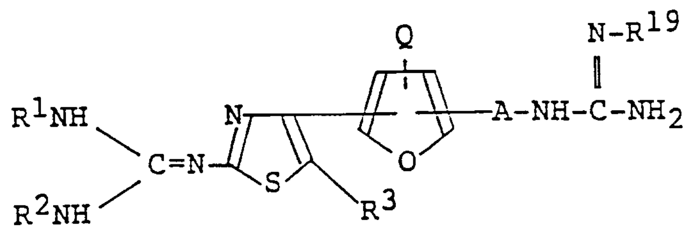

worin $R^1$, $R^2$, $R^3$, $R^{19}$, A und Q jeweils wie oben definiert sind, oder ein Salz davon zu ergeben oder (27) Unterwerfen einer Verbindung der Formel:

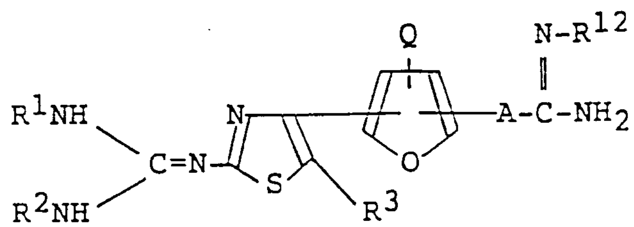

worin $R^1$, $R^2$, $R^3$, $R^{12}$, A und Q jeweils wie oben definiert sind, oder eines Salzes davon der Hydrolyse, um eine Verbindung der Formel:

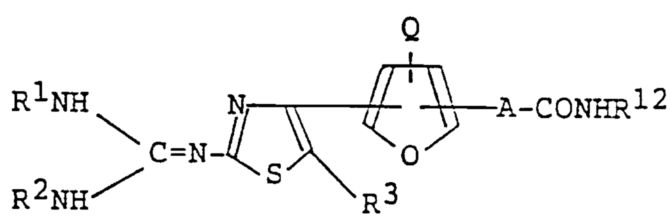

worin $R^1$, $R^2$, $R^3$, $R^{12}$, A und Q jeweils wie oben definiert sind, oder ein Salz davon zu ergeben.

**2.** Verfahren nach Anspruch 1, worin $R^1$ und $R^2$ jeweils Wasserstoff, $(C_1\text{-}C_6)$Alkyl oder Trihalogen$(C_1\text{-}C_6)$-alkyl sind; oder $R^1$ und $R^2$ miteinander verbunden sind, um $(C_1\text{-}C_6)$Alkylen zu bilden, $R^4$ Amino, Carbamoyl, Aminocarbamoyl, Guanidinocarbamoyl, $(C_1\text{-}C_6)$Alkylcarbamoyl, Sulfamoylaminocarbonyl, $(C_1\text{-}C_6)$Alkoxycarbonyl, Ureido, Thioureido, Sulfamoylamino, $(C_1\text{-}C_6)$Alkanoylamino, $(C_1\text{-}C_6)$-Alkoxycarbonylamino, $(C_1\text{-}C_6)$Alkylsulfonylamino, $(C_1\text{-}C_6)$-Alkoxy$(C_1\text{-}C_6)$alkanoylamino, $(C_1\text{-}C_6)$-Alkanoyloxy$(C_1\text{-}C_6)$alkanoylamino, $(C_1\text{-}C_6)$Alkylthio$(C_1\text{-}C_6)$alkanoylamino, Nitrobenzoylamino, Furoylamino, Thenoylamino, Nicotinoylamino, 1-Oxonicotinoylamino, Morpholinocarbonylamino, 3-$(C_1\text{-}C_6)$-Alkylureido, 3-$(C_1\text{-}C_6)$Alkylthioureido, 3-$(C_1\text{-}C_6)$Alkanoylureido, 3-Allylureido, 3-Benzoylthioureido, Furyl-$(C_1\text{-}C_6)$alkylthio$(C_1\text{-}C_6)$alkanoylamino, Furyl$(C_1\text{-}C_6)$alkylsulfinyl$(C_1\text{-}C_6)$alkanoylamino, Monooder Di$(C_1\text{-}C_6)$Alkylsulfamoylamino, 3-Hydroxy$(C_1\text{-}C_6)$alkylureido, 3-$(C_1\text{-}C_6)$Alkanoyloxy$(C_1\text{-}C_6)$alkylureido, 2-$(C_1\text{-}C_6)$Alkylisothioureido, Thiazolylamino substituiert mit $(C_1\text{-}C_6)$Alkyl, Triazolylamino substituiert mit Amino, Triazolylamino substituiert mit Amino und $(C_1\text{-}C_6)$Alkyl, Benzoisothiazolylamino substituiert mit Oxo, Benzothiadiazinylamino substituiert mit Oxo und Halogen, Pyrimidinyl substituiert mit Oxo und $(C_1\text{-}C_6)$-Alkyl, Triazolyl substituiert mit Amino, oder eine Gruppe der Formel:

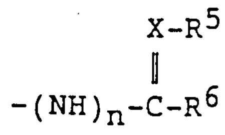

worin n, X und $R^6$ wie in Anspruch 1 definiert sind, ist, $R^5$ Wasserstoff, Cyano, Nitro, Carbamoyl, ($C_1$-$C_6$)Alkoxycarbonyl, Sulfamoyl, ($C_1$-$C_6$)Alkylsulfonyl, Phenylsulfonyl substituiert mit ($C_1$-$C_6$)Alkyl, Phenylsulfonyl substituiert mit ($C_1$-$C_6$)Alkoxy, Phenylsulfonyl substituiert mit Halogen, Phenylsulfonyl substituiert mit Amino, Mono- oder Di($C_1$-$C_6$)Alkylsulfamoyl ist, A wie in Anspruch 1 definiert ist; oder A-$R^4$ wie in Anspruch 1 definiert ist, und $R^3$ und Q jeweils wie in Anspruch 1 definiert sind.

**3.** Verfahren nach Anspruch 2, worin $R^1$ und $R^2$ jeweils Wasserstoff sind, $R^3$ Wasserstoff ist, $R^4$ eine Gruppe der Formel ist:

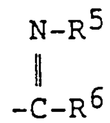

worin $R^5$ Sulfamoyl ist, und $R^6$ Amino ist, A ($C_1$-$C_6$)Alkylen und Q Wasserstoff ist.

**4.** Verfahren nach Anspruch 2, worin $R^1$ und $R^2$ jeweils Wasserstoff sind, $R^3$ Wasserstoff ist, $R^4$ Ureido oder 3-($C_1$-$C_6$)Alkylureido ist, A ($C_1$-$C_6$)Alkylen ist und Q Wasserstoff ist.

**5.** Verfahren zur Herstellung einer pharmazeutischen Zusammensetzung, die als aktiven Bestandteil eine Verbindung der Formel I nach Anspruch 1 umfaßt, dadurch gekennzeichnet, daß eine Verbindung der Formel I mit mindestens einem pharmazeutisch verträglichen Träger oder Exzipienten gemischt wird.

**6.** Modifizierung des Verfahrens nach Anspruch 1, dadurch gekennzeichnet, daß eine Verbindung der Formel I oder ein nichttoxisches Salz davon, hergestellt nach einem Verfahren nach Anspruch 1, durch Zusammenmischen oder Präsentation der genannten Verbindung mit einem pharmazeutisch verträglichen Verdünnungsmittel oder Träger in eine pharmazeutisch verträgliche Form gebracht wird.

**7.** Verwendung einer Verbindung I nach Anspruch 1 zur Herstellung eines Medikamentes zur therapeutische Behandlung von Geschwüren in Menschen oder Tieren.

**Revendications**

**Revendications pour les Etats contractants suivants : AT, BE, CH, DE, FR, GB, IT, LI, LU, NL, SE**

**1.** Composé de formule:

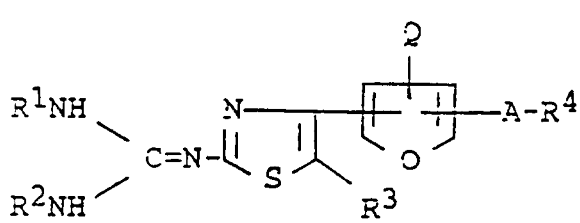

dans lequel

| | |
|---|---|
| $R^1$ et $R^2$ | sont chacun un hydrogène, un furoyle ou un alkyle en $C_1$-$C_6$ qui peut avoir un halogène; ou |
| $R^1$ et $R^2$ | sont liés ensemble pour former un alkylène en $C_1$-$C_6$, |
| $R^3$ est | un hydrogène ou un alkyle en $C_1$-$C_6$, |
| $R^4$ est | un amino, un carbamoyle, |

un aminocarbamoyle, un guanidinocarbamoyle,
un alkyl(en $C_1$-$C_6$)carbamoyle,
un sulfamoylaminocarbonyle,
un alcoxy(en $C_1$-$C_6$)carbonyle,
un uréido, un thiouréido, un sulfamoylamino,
un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,
un alcanoyl(en $C_1$-$C_6$)oxyalcanoyl(en $C_1$-$C_6$)amino,
un alkyl(en $C_1$-$C_6$)thioalcanoyl(en $C_1$-$C_6$)amino,
un benzoylamino éventuellement substitué avec
un nitro, un furoylamino, un thénoylamino,
un nicotinoylamino, un 1-oxonicotinoylamino,
un morpholinocarbonylamino,
un alkyl(en $C_1$-$C_6$)uréido,
un alkyl(en $C_1$-$C_6$)thiouréido,
un alcanoyl(en $C_1$-$C_6$)uréido, un allyluréido,
un benzoylthiouréido,
un furylalkyl(en $C_1$-$C_6$)thioalcanoyl(en $C_1$-$C_6$)amino,
un furylalkyl(en $C_1$-$C_6$)sulfinylalcanoyl(en $C_1$-$C_6$)amino,
un mono ou dialkyl(en $C_1$-$C_6$)sulfamoylamino,
un hydroxyalkyl(en $C_1$-$C_6$)uréido,
un alcanoyl(en $C_1$-$C_6$)oxyalkyl(en $C_1$-$C_6$)uréido,
un alkyl(en $C_1$-$C_6$)isothiouréido,
un thiazolylamino substitué avec un alkyle en $C_1$-$C_6$, un triazolylamino substitué avec un amino, un triazolylamino substitué avec un amino et un alkyle en $C_1$-$C_6$,
un benzoisothiazolylamino substitué avec un oxo, un benzothiadiazinylamino substitué avec un oxo et un halogène, un pyrimidinyle substitué avec un oxo et un alkyle en $C_1$-$C_6$,
un triazolyle substitué avec un amino, ou un groupe de formule:

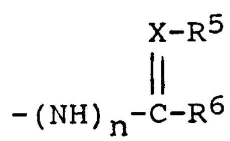

dans lequel

n = 0 ou 1,

X est =CH- ou =N-,

$R^5$ est un hydrogène, un cyano, un nitro,
un carbamoyle, un alcoxy(en $C_1$-$C_6$)carbonyle,
un sulfamoyle, un alkyl(en $C_1$-$C_6$)sulfonyle,
un benzènesulfonyle, qui est substitué avec un ou plusieurs substituants choisis parmi un alkyle en $C_1$-$C_6$, un alcoxy en $C_1$-$C_6$,
un halogène, et un amino, ou un mono ou dialkyl(en $C_1$-$C_6$)sulfamoyle,

$R^6$ est un hydrogène, un alkyle en $C_1$-$C_6$,
un alkyl(en $C_1$-$C_6$)thio, un alcoxy en $C_1$-$C_6$,
un amino, un mono ou dialkyl(en $C_1$-$C_6$)amino,
un allylamino, un propargylamino,
un hydroxyalkyl(en $C_1$-$C_6$)amino,
un alcoxy(en $C_1$-$C_6$)alkyl(en $C_1$-$C_6$)amino,
ou un mono ou dialkyl(en $C_1$-$C_6$)aminoalkyl(en $C_1$-$C_6$)amino;

A est un alkylène en $C_1$-$C_6$ ou -CONH-; ou

A-$R^4$ est un imidazolyle substitué avec un alkyle en $C_1$-$C_6$, un triazolyle substitué avec un amino, et

Q est un hydrogène, ou un alkyle en $C_1$-$C_6$,

ou les sels pharmaceutiquement acceptables de celui-ci.

2. Composé selon la revendication 1, dans lequel

$R^1$ et $R^2$ sont chacun un hydrogène, un alkyle en $C_1$-$C_6$ ou un trihaloalkyle en $C_1$-$C_6$; ou

$R^1$ et $R^2$ sont liés ensemble pour former un alkylène en $C_1$-$C_6$,

$R^4$ est un amino, un carbamoyle, un aminocarbamoyle,
un guanidinocarbamoyle, un alkyl(en $C_1$-$C_6$)carbamoyle,
un sulfamoylaminocarbonyle,
un alcoxy(en $C_1$-$C_6$)carbonyle, un uréido,
un thiouréido, un sulfamoylamino,
un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,
un alcanoyl(en $C_1$-$C_6$)oxyalcanoyl(en $C_1$-$C_6$)amino,
un alkyl(en $C_1$-$C_6$)thioalcanoyl(en $C_1$-$C_6$)amino,
un nitrobenzoylamino, un furoylamino, un thénoylamino,
un nicotinoylamino,
un 1-oxonicotinoylamino, un morpholinocarbonylamino,
un 3-alkyl(en $C_1$-$C_6$)uréido,
un 3-alkyl(en $C_1$-$C_6$)thiouréido,
un 3-alcanoyl(en $C_1$-$C_6$)uréido, un 3-allyluréido,
un 3-benzoylthiouréido,
un furylalkyl(en $C_1$-$C_6$)thioalcanoyl(en $C_1$-$C_6$)amino,
un furylalkyl(en $C_1$-$C_6$)sulfinylalcanoyl(en $C_1$-$C_6$)amino,
un mono ou dialkyl(en $C_1$-$C_6$)sulfamoylamino,
un 3-hydroxyalkyl(en $C_1$-$C_6$)uréido,
un 3-alcanoyl(en $C_1$-$C_6$)oxyalkyl(en $C_1$-$C_6$)uréido,
un 2-alkyl(en $C_1$-$C_6$)isothiouréido,
un thiazolylamino substitué avec un alkyle en $C_1$-$C_6$,
un triazolylamino substitué avec un amino,
un triazolylamino substitué avec un amino et un alkyle en $C_1$-$C_6$,
un benzoisothiazolylamino substitué avec un oxo,
un benzothiadiazinylamino substitué avec un oxo et un halogène,
un pyrimidinyle substitué avec un oxo et un alkyle en $C_1$-$C_6$,
un triazolyle substitué avec un amino, ou
un groupe de formule:

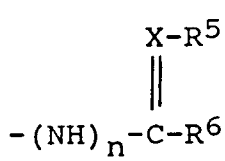

dans lequel

n, X et $R^6$ sont tels que définis dans la revendication 1,

$R^5$ est un hydrogène, un cyano, un nitro,
un carbamoyle, un alcoxy(en $C_1$-$C_6$)carbonyle,
un sulfamoyle, un alkyl(en $C_1$-$C_6$)sulfonyle,
un phénylsulfonyle substitué avec un alkyle en $C_1$-$C_6$, un phénylsulfonyle substitué avec un alcoxy en $C_1$-$C_6$, un phénylsulfonyle substitué avec un halogène, un phénylsulfonyle substitué avec un amino,
un mono ou dialkyl(en $C_1$-$C_6$)sulfamoyle,

A est tel que défini dans la revendication 1; ou

A-$R^4$ est tel que défini dans la revendication 1, et

$R^3$ et Q sont tels que définis dans la revendication 1.

**3.** Composé selon la revendication 2, dans lequel

$R^1$ et $R^2$ sont chacun un hydrogène,
$R^3$ est un hydrogène,
$R^4$ est un groupe de formule:

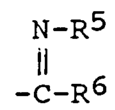

dans lequel
$R^5$ est un sulfamoyle, et
$R^6$ est un amino,
A est un alkylène en $C_1$-$C_6$, et
Q est un hydrogène.

**4.** Composé selon la revendication 3, qui est le 4-[5-(2-amino-2-sulfamoyliminoéthyl)furan-2-yl]-2-(diaminométhylèneamino)thiazole ou son chlorhydrate ou son méthanesulfonate.

**5.** Composé selon la revendication 2, dans lequel

$R^1$ et $R^2$ sont chacun un hydrogène,
$R^3$ est un hydrogène,
$R^4$ est un uréido ou un 3-alkyl(en $C_1$-$C_6$)uréido,
A est un alkylène en $C_1$-$C_6$, et
Q est un hydrogène.

**6.** Composé selon la revendication 5, qui est choisi parmi le groupe constitué de 2-(diaminométhylèneamino)-4-(5-uréidométhylfuran-2-yl)thiazole ou son chlorhydrate, et de 2-(diaminométhylèneamino)-4-[5-(3-méthyluréido)méthylfuran-2-yl]thiazole ou son chlorhydrate.

**7.** Procédé pour la préparation d'un composé de formule:

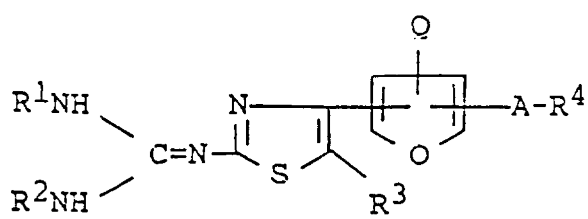

dans lequel
$R^1$, $R^2$, $R^3$, $R^4$, $R^5$, $R^6$, A, A-$R^4$, Q, X, et n sont tels que définis dans la revendication 1,
ou un sel de celui-ci,
qui consiste à
(1) faire réagir un composé de formule :

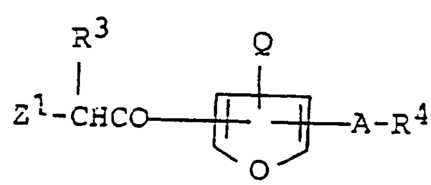

dans lequel
$R^3$, $R^4$, A et Q sont chacun tels que définis ci-dessus, et

$Z^1$ est un halogène,
ou un sel de celui-ci, avec un composé de formule :

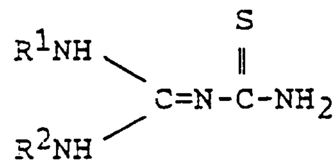

dans lequel
$R^1$ et $R^2$ sont chacun tels que définis ci-dessus,
pour donner un composé de formule:

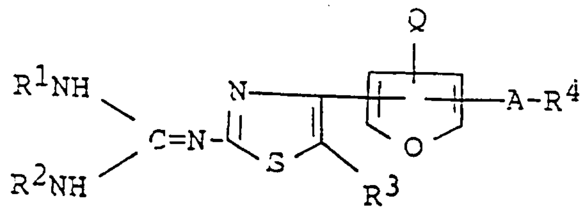

dans lequel
$R^1$, $R^2$, $R^3$, $R^4$, A et Q sont chacun tels que définis ci-dessus,
ou un sel de celui-ci, ou
(2) soumettre un composé de formule

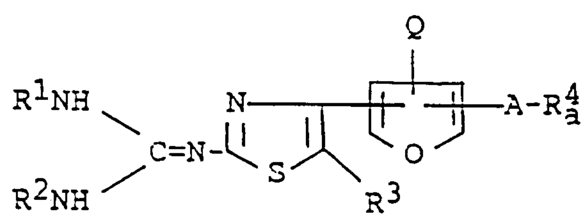

dans lequel
$R^1$, $R^2$, $R^3$, A et Q sont chacun tels que définis ci-dessus, et
$R^4_a$ est un uréido, un thiouréido, un sulfamoylamino, un alcanoyl(en $C_1$-$C_6$)amino, un alcoxy(en $C_1$-$C_6$)carbonylamino, un alkyl(en $C_1$-$C_6$)sulfonylamino, un alcoxy(en $C_1$-$C_6$)alcanoyl(en $C_1$-$C_6$)amino, un alcanoyl(en $C_1$-$C_6$)oxyalcanoyl(en $C_1$-$C_6$)amino, un alkyl(en $C_1$-$C_6$)thioalcanoyl(en $C_1$-$C_6$)amino, un benzoylamino éventuellement substitué par un nitro, un furoylamino, un thénoylamino, un nicotinoylamino, un 1-oxonicotinoylamino, un morpholinocarbonylamino, un alkyl(en $C_1$-$C_6$)uréido, un alkyl(en $C_1$-$C_6$)thiouréido, un alcanoyl(en $C_1$-$C_6$)uréido, un allyluréido, un benzoylthiouréido, un furylalkyl(en $C_1$-$C_6$)thioalcanoyl(en $C_1$-$C_6$)amino, un mono ou dialkyl(en $C_1$-$C_6$)sulfamoylamino,

un hydroxyalkyl(en $C_1$-$C_6$)uréido, ou
un alcanoyl(en $C_1$-$C_6$)oxyalkyl(en $C_1$-$C_6$)uréido, ou un sel de celui-ci, à une réaction de déacylation, pour donner un composé de formule:

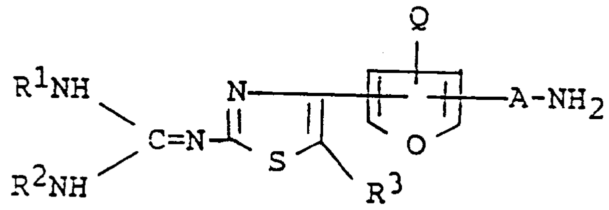

dans lequel
$R^1$, $R^2$, $R^3$, A et Q sont tels que définis ci-dessus,
ou un sel de celui-ci, ou
(3) soumettre un composé de formule:

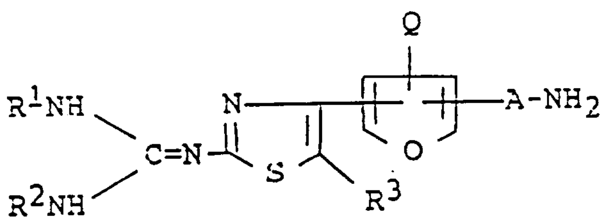

dans lequel
$R^1$, $R^2$, $R^3$, A et Q sont chacun tels que définis ci-dessus,
ou un sel de celui-ci, à une réaction d'acylation, pour donner un composé de formule:

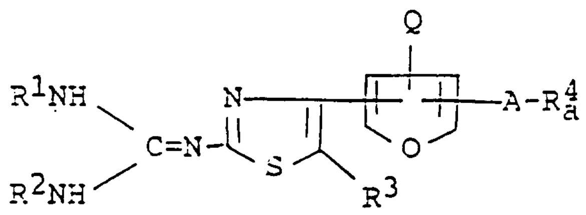

dans lequel
$R^1$, $R^2$, $R^3$, $R^4_a$, A et Q sont chacun tels que définis ci-dessus,
ou un sel de celui-ci, ou
(4) faire réagir un composé de formule:

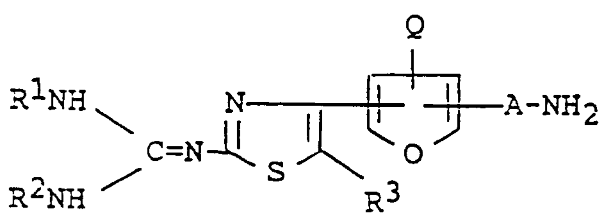

dans lequel
$R^1$, $R^2$, $R^3$, A et Q sont chacun tels que définis ci-dessus,
ou un sel de celui-ci, avec un composé de formule:

$(R^7\text{-}X^1)_2C=X\text{-}R^5$

dans lequel

$R^5$ et X sont chacun tels que définis ci-dessus,
$R^7$ est un alkyle en $C_1$-$C_6$ ou un phényle, et
$X^1$ est S ou O,

pour donner un composé de formule:

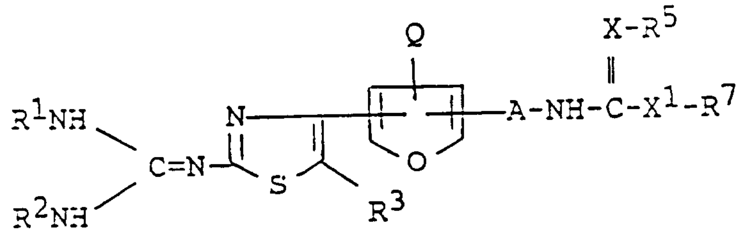

dans lequel

$R^1$, $R^2$, $R^3$, $R^5$, $R^7$, X, $X^1$, A et Q sont chacun tels que défini ci-dessus,

ou un sel de celui-ci, ou

(5) faire réagir un composé de formule:

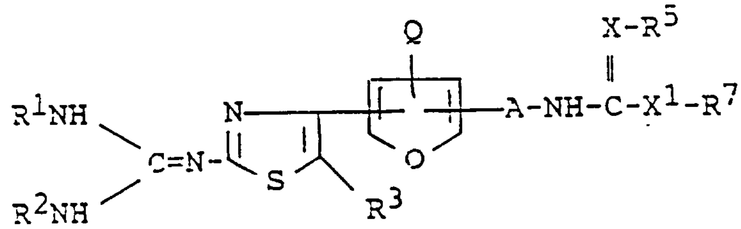

dans lequel

$R^1$, $R^2$, $R^3$, $R^5$, $R^7$, X, $X^1$, A et Q sont chacun tels que définis ci-dessus,

ou un sel de celui-ci avec un composé de formule:

$H\text{-}R^6_a$

dans lequel

$R^6_a$ est un amino qui peut avoir un mono ou dialkyle en $C_1$-$C_6$, un allyle, un propargyle, un hydroxyalkyle en $C_1$-$C_6$ ou un mono ou dialkyl(en $C_1$-$C_6$)aminoalkyle en $C_1$-$C_6$, ou
un alcoxy en $C_1$-$C_6$,

pour donner un composé de formule:

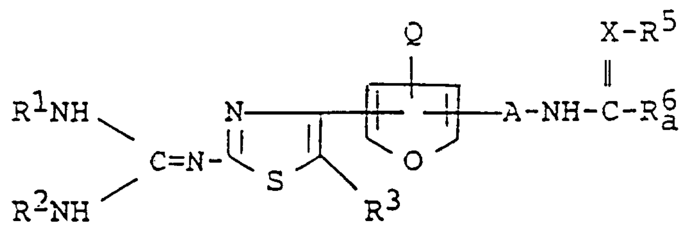

dans lequel

$R^1$, $R^2$, $R^3$, $R^5$, $R^6_a$, X, A et Q sont chacun tels que définis ci-dessus,

ou un sel de celui-ci, ou

(6) soumettre un composé de formule:

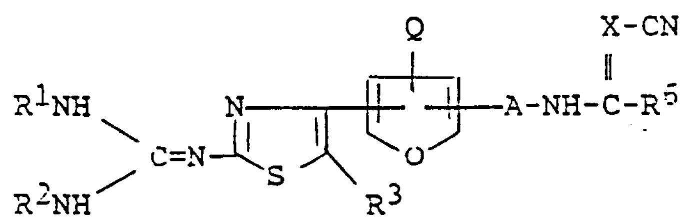

dans lequel

$R^1$, $R^2$, $R^3$, $R^6$, X, A et Q sont chacun tels définis ci-dessus,

ou un sel de celui-ci, à une hydrolyse, pour donner un composé de formule:

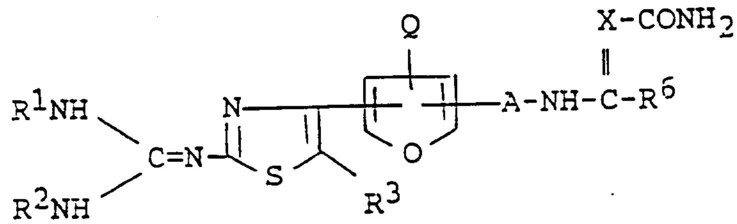

dans lequel

$R^1$, $R^2$, $R^3$, $R^6$, X, A et Q sont chacun tels que définis ci-dessus,

ou un sel de celui-ci, ou

(7) soumettre un composé de formule

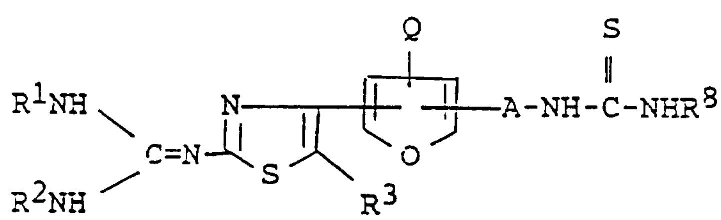

dans lequel

$R^1$, $R^2$, $R^3$, A et Q sont chacun tels que définis ci-dessus, et

$R^8$ est un groupe protecteur de l'amino constitué d'un alcanoyle en $C_1$-$C_6$ et d'un benzoyle,

ou un sel de celui-ci, à une réaction d'élimination du groupe protecteur de l'amino, pour donner un composé de formule:

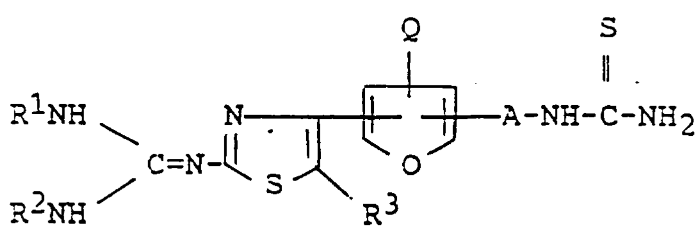

dans lequel

$R^1$, $R^2$, $R^3$, A et Q sont chacun tels que définis ci-dessus, ou un sel de celui-ci, ou

(8) faire réagir un composé de formule:

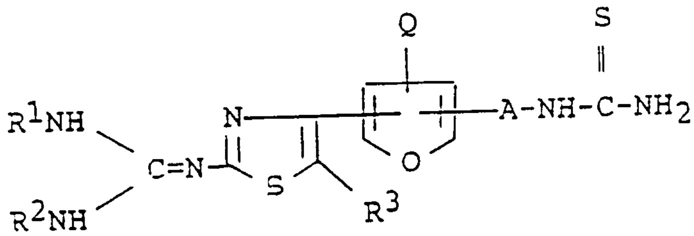

dans lequel

$R^1$, $R^2$, $R^3$, A et Q sont chacun tels que définis ci-dessus, ou un sel de celui-ci, avec un composé de formule:

$Z^2-CH_2-CO-R^9$

dans lequel

$R^9$ est un alkyle en $C_1$-$C_6$, et

$Z^2$ est un halogène,

pour donner un composé de formule:

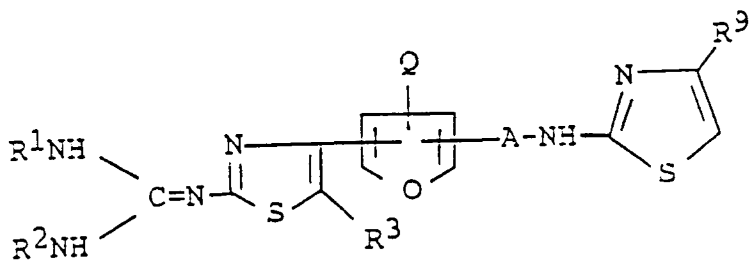

dans lequel

$R^1$, $R^2$, $R^3$, $R^9$, A et Q sont chacun tels que définis ci-dessus, ou un sel de celui-ci, ou

(9) faire réagir un composé de formule:

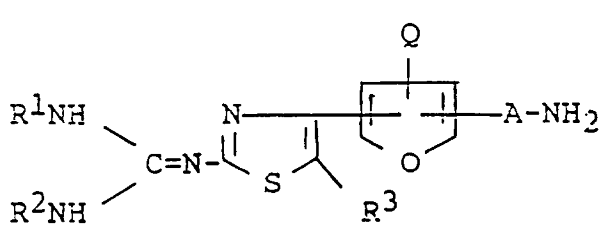

dans lequel

$R^1$, $R^2$, $R^3$, A et Q sont chacun tels que définis ci-dessus, ou un sel de celui-ci, avec un composé de formule:

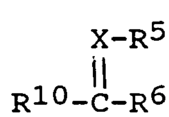

dans lequel

$R^5$, $R^6$ et X sont chacun tels que définis ci-dessus, et

$R^{10}$ est un alcanoyl(en $C_1$-$C_6$)oxy, un phénylalcoxy en $C_1$-$C_6$, un alcoxy(en $C_1$-$C_6$)alcoxy en $C_1$-$C_6$, un tétrahydropyranyloxy ou un alcoxy en $C_1$-$C_6$,

pour donner un composé de formule:

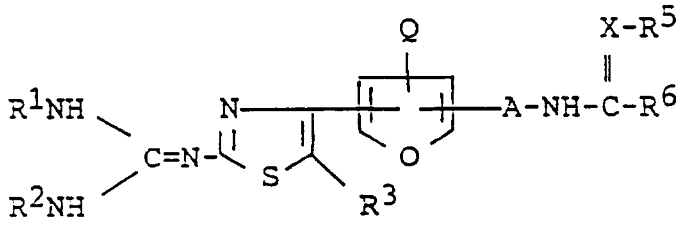

dans lequel

$R^1$, $R^2$, $R^3$, $R^5$, $R^6$, X, A et Q sont chacun tels que définis ci-dessus,

ou un sel de celui-ci, ou

(10) faire réagir un composé de formule:

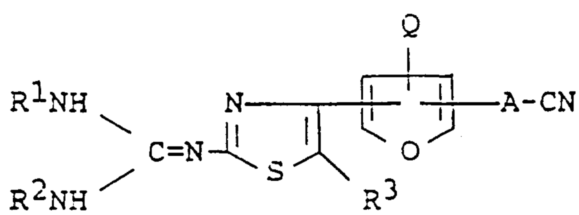

dans lequel

$R^1$, $R^2$, $R^3$, A et Q sont chacun tels que définis ci-dessus,

ou un sel de celui-ci, avec un composé de formule:

$R^{11}OH$

dans lequel

$R^{11}$ est un alkyle en $C_1$-$C_6$,

pour donner un composé de formule:

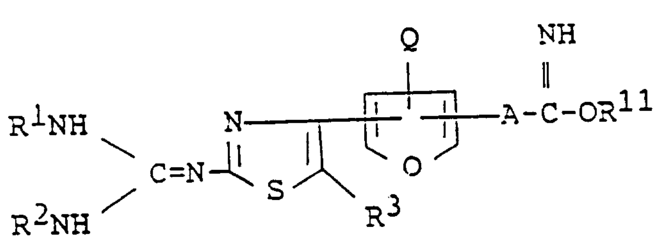

dans lequel

$R^1$, $R^2$, $R^3$, $R^{11}$, A et Q sont chacun tels que définis ci-dessus,

ou un sel de celui-ci, ou

(11) faire réagir un composé de formule:

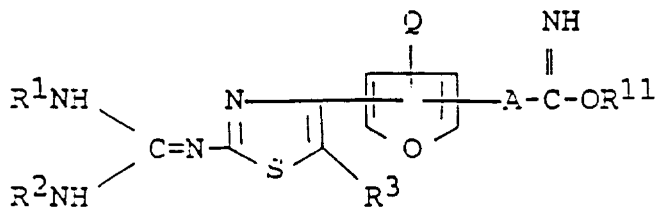

dans lequel
$R^1$, $R^2$, $R^3$, $R^{11}$, A et Q sont chacun tels que définis ci-dessus,
ou un sel de celui-ci, avec un composé de formule:

$NH_2R^{12}$

dans lequel
$R^{12}$ est un acyle,
pour donner un composé de formule:

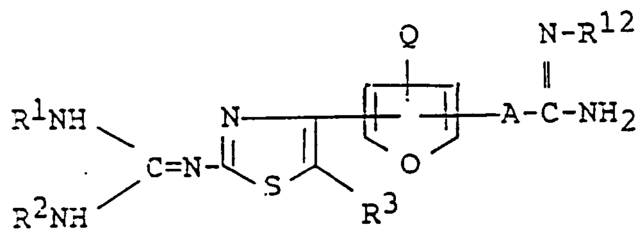

dans lequel
$R^1$, $R^2$, $R^3$, $R^{12}$, A et Q sont chacun tels que définis ci-dessus,
ou un sel de celui-ci, ou
(12) faire réagir un composé de formule:

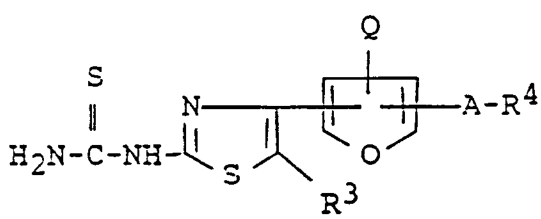

dans lequel
$R^3$, $R^4$, A et Q sont chacun tels que définis ci-dessus,
ou un sel de celui-ci, avec un composé de formule:

$R^{13}$-$Z^3$

dans lequel
$R^{13}$ est un alkyle en $C_1$-$C_6$, et
$Z^3$ est un halogène,
ou un sel de celui-ci, pour donner un composé de formule:

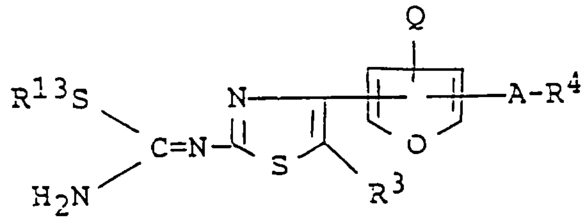

dans lequel

$R^3$, $R^4$, $R^{13}$, A et Q sont tels que définis ci-dessus,

ou un sel de celui-ci, et le faire réagir de manière continue avec un composé de formule:

$H_2N-Y-NH_2$

dans lequel

Y est un alkylène en $C_1$-$C_6$,

ou un sel de celui-ci,

pour donner un composé de formule:

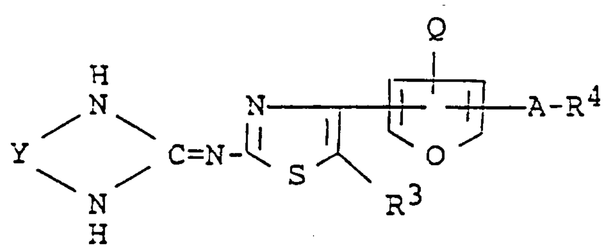

dans lequel

$R^3$, $R^4$, A, Q et Y sont chacun tels que définis ci-dessus,

ou un sel de celui-ci, ou

(13) soumettre un composé de formule:

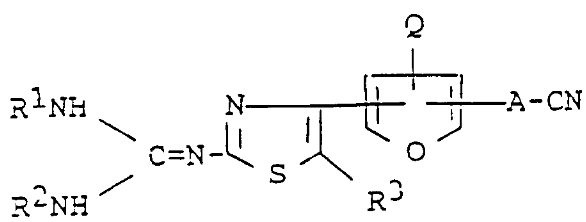

dans lequel

$R^1$, $R^2$, $R^3$, A et Q sont chacun tels que définis ci-dessus,

ou un sel de celui-ci, à une réduction, pour donner un composé de formule:

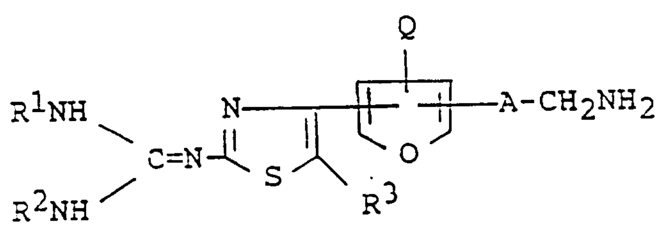

dans lequel

$R^1$, $R^2$, $R^3$, A et Q sont chacun tels que définis ci-dessus,
ou un sel de celui-ci, ou
(14) faire réagir un composé de formule:

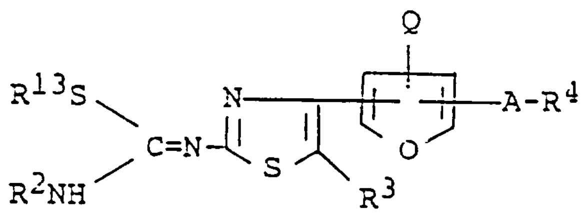

dans lequel
$R^2$, $R^3$, $R^4$, $R^{13}$, A et Q sont chacun tels que définis ci-dessus,
ou un sel de celui-ci, avec un composé de formule:

$NH_2R^1_a$

dans lequel
$R^1_a$ est un alkyle en $C_1$-$C_6$ qui peut avoir un halogène,
ou un sel de celui-ci, pour donner un composé de formule:

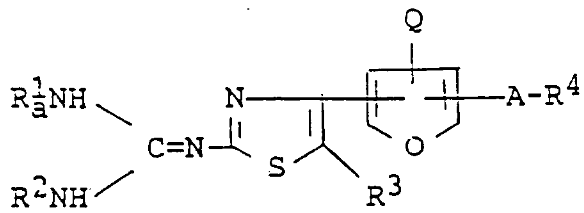

dans lequel,
$R^1_a$ , $R^2$, $R^3$, $R^4$, A et Q sont chacun tels que définis ci-dessus,
ou un sel de celui-ci, ou
(15) soumettre un composé de formule:

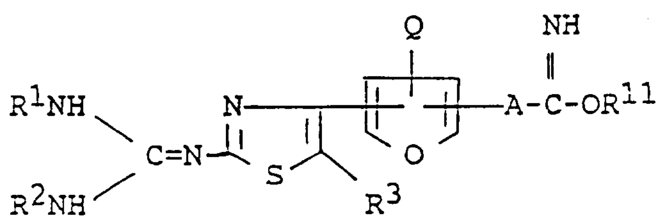

dans lequel
$R^1$, $R^2$, $R^3$, $R^{11}$, A et Q sont chacun tels que définis ci-dessus,
ou un sel de celui-ci, à une hydrolyse, pour donner un composé de formule:

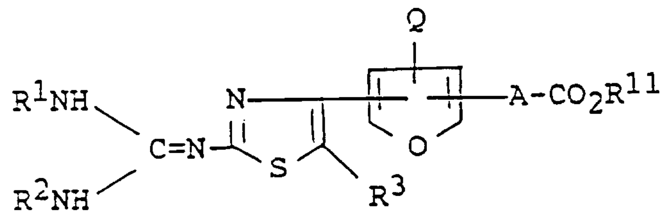

dans lequel

$R^1$, $R^2$, $R^3$, $R^{11}$, A et Q sont chacun tels que définis ci-dessus,

ou un sel de celui-ci, ou

(16) soumettre un composé de formule:

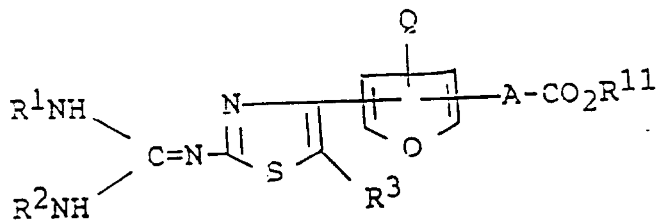

dans lequel

$R^1$, $R^2$, $R^3$, $R^{11}$, A et Q sont chacun tels que définis ci-dessus,

ou un sel de celui-ci, à une réaction d'amidation, pour donner un composé de formule:

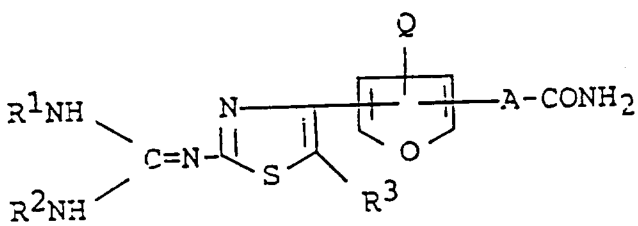

dans lequel

$R^1$, $R^2$, $R^3$, A et Q sont chacun tels que définis ci-dessus,

ou un sel de celui-ci, ou

(17) faire réagir un composé de formule:

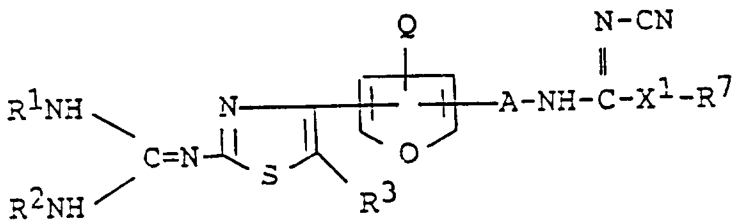

dans lequel

$R^1$, $R^2$, $R^3$, $R^7$, $X^1$, A et Q sont chacun tels que définis ci-dessus,

ou un sel de celui-ci, avec un composé de formule:

$R^{14}NHNH_2$

dans lequel

$R^{14}$ est un hydrogène ou un alkyle en $C_1$-$C_6$,

pour donner un composé de formule:

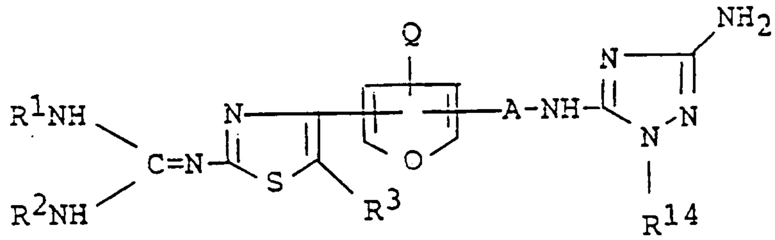

dans lequel

$R^1$, $R^2$, $R^3$, $R^{14}$, A et Q sont chacun tels que définis ci-dessus,

ou un sel de celui-ci, ou

(18) faire réagir un composé de formule:

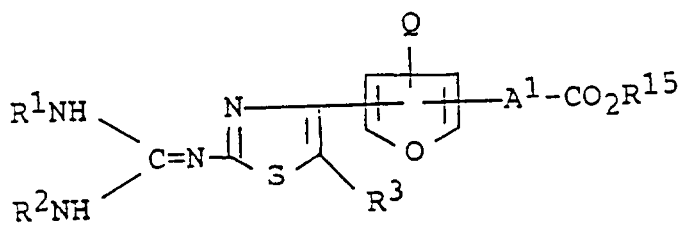

dans lequel

$R^1$, $R^2$, $R^3$ et Q sont chacun tels que définis ci-dessus,

$R^{15}$ est un alkyle en $C_1$-$C_6$, et

$A^1$ est un alkylène en $C_1$-$C_6$ ou une liaison,

ou un sel de celui-ci, avec de l'hydrazine, pour donner un composé de formule:

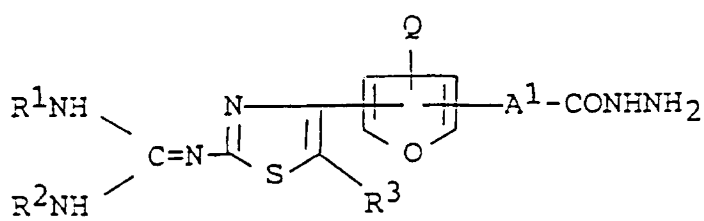

dans lequel

$R^1$, $R^2$, $R^3$, $A^1$ et Q sont chacun tels que définis ci-dessus,

ou un sel de celui-ci, ou

(19) faire réagir un composé de formule:

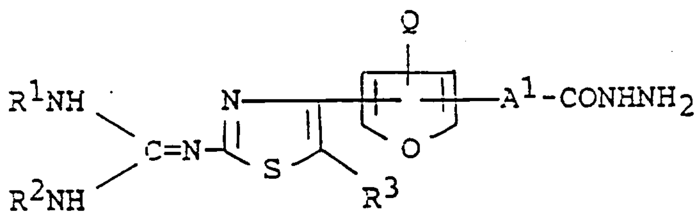

dans lequel

$R^1$, $R^2$, $R^3$, $A^1$ et Q  sont chacun tels que définis ci-dessus,

ou un sel de celui-ci, avec un S-alkyl(en $C_1$-$C_6$)isothiourée, ou un sel de celui-ci, pour donner un composé de formule:

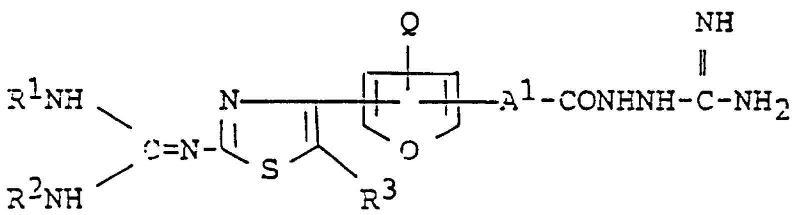

dans lequel

$R^1$, $R^2$, $R^3$, $A^1$ et Q  sont chacun tels que définis ci-dessus,

ou un sel de celui-ci, ou

(20) soumettre un composé de formule:

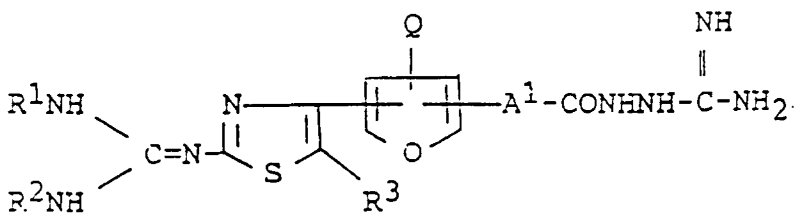

dans lequel

$R^1$, $R^2$, $R^3$, $A^1$ et Q  sont chacun tels que définis ci-dessus,

ou un sel de celui-ci, à une fermeture de cycle, pour donner un composé de formule:

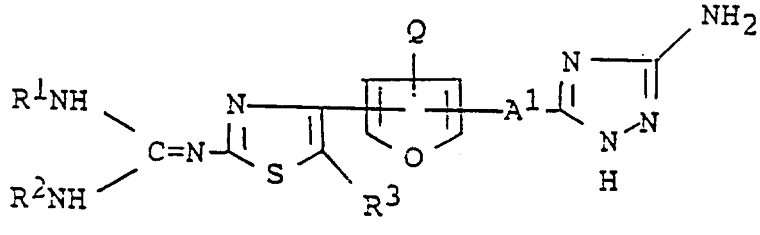

dans lequel

$R^1$, $R^2$, $R^3$, $A^1$ et Q  sont chacun tels que définis ci-dessus,

ou un sel de celui-ci, ou

(21) faire réagir un composé de formule:

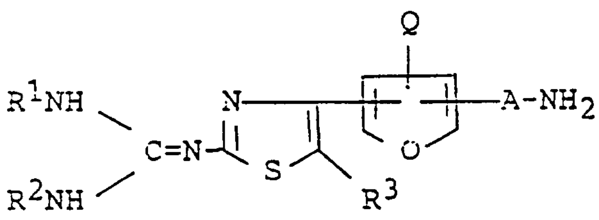

dans lequel

$R^1$, $R^2$, $R^3$, A et Q sont chacun tels que définis ci-dessus,

ou un sel de celui-ci, avec un composé de formule:

$R^{16}$-$Z^4$

dans lequel

$R^{16}$ est un thiazolyle substitué avec un alkyle en $C_1$-$C_6$, un triazolyle substitué avec un amino, un triazolyle substitué avec un amino et un alkyle en $C_1$-$C_6$, un benzoisothiazolyle substitué avec un oxo, ou un benzothiadiazinyle substitué avec un oxo, et

$Z_4$ est un halogène,

ou un sel de celui-ci, pour donner un composé de formule:

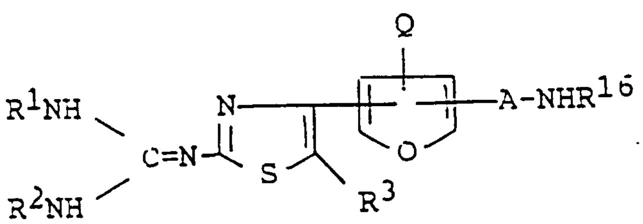

dans lequel

$R^1$, $R^2$, $R^3$, $R^{16}$, A et Q sont chacun tels que définis ci-dessus,

ou un sel de celui-ci, ou

(22) soumettre un composé de formule:

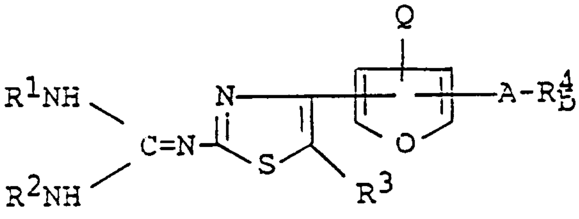

dans lequel

$R^1$, $R^2$, $R^3$, A et Q sont chacun tels que définis ci-dessus, et

$R^4_b$ est un furylalkyl(en $C_1$-$C_6$)thioalcanoyl(en $C_1$-$C_6$)amino,

ou un sel de celui-ci, à une réaction d'oxydation, pour donner un composé de formule:

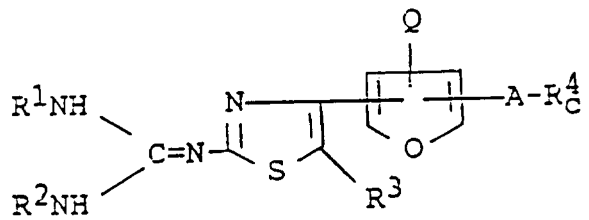

dans lequel
R¹, R², R³, A et Q sont chacun tels que définis ci-dessus, et
$R^4_c$ est un furylalkyl(en $C_1$-$C_6$)sulfinylalcanoyl(en $C_1$-$C_6$)amino,
ou un sel de celui-ci, ou
(23) faire réagir un composé de formule:

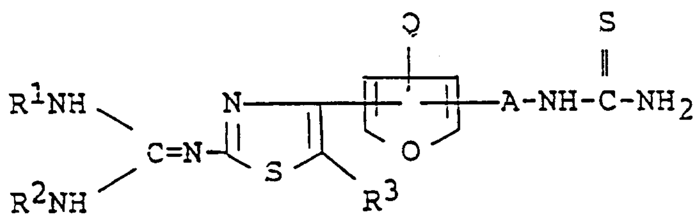

dans lequel
$R^1$, $R^2$, $R^3$, A et Q sont chacun tels que définis ci-dessus,
ou un sel de celui-ci, avec un composé de formule:

$R^{17}$-$Z^5$

dans lequel
$R^{17}$ est un alkyle en $C_1$-$C_6$, et
$Z^5$ est un halogène,
ou un sel de celui-ci, pour donner un composé de formule:

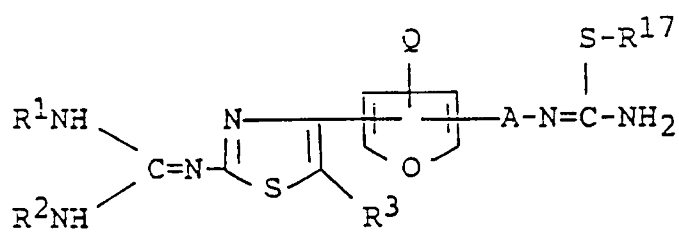

dans lequel
$R^1$, $R^2$, $R^3$, $R^{17}$, A et Q sont chacun tels que définis ci-dessus,
ou un sel de celui-ci, ou
(24) faire réagir un composé de formule:

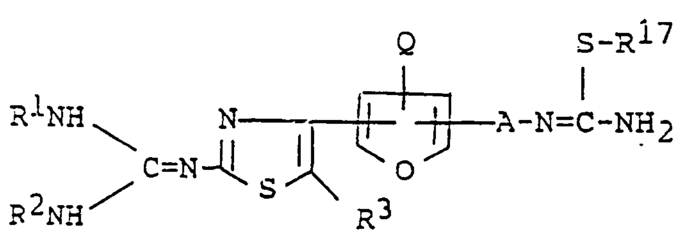

dans lequel
$R^1$, $R^2$, $R^3$, $R^{17}$, A et Q sont chacun tels que définis ci-dessus,
ou un sel de celui-ci, avec un composé de formule:

$H_2N$-$R^5$

dans lequel
$R^5$ est tel que défini ci-dessus,
ou un sel de celui-ci, pour donner un composé de formule:

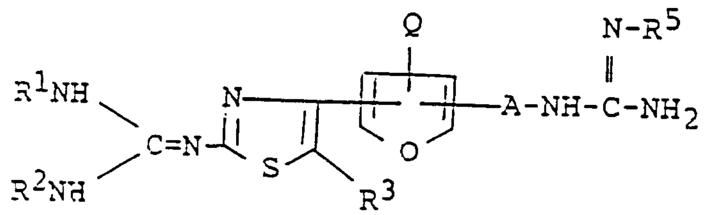

dans lequel
$R^1$, $R^2$, $R^3$, $R^5$, A et Q sont chacun tels que définis ci-dessus,
ou un sel de celui-ci, ou
(25) soumettre un composé de formule:

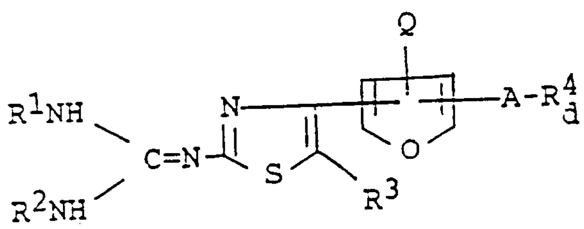

dans lequel
$R^1$, $R^2$, $R^3$, A et Q sont chacun tels que définis ci-dessus, et
$R^4_d$ est un alcanoyl(en $C_1$-$C_6$)oxyalkyl(en $C_1$-$C_6$)uréido,
ou un sel de celui-ci, à une réaction d'élimination de l'alcanoyle en $C_1$-$C_6$, pour donner un composé de formule:

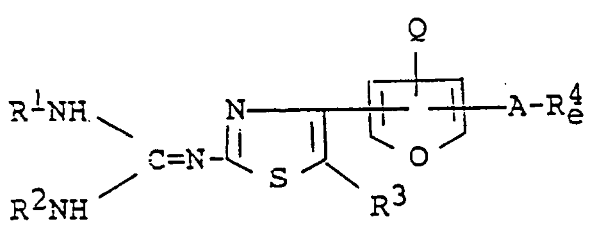

dans lequel
$R^1$, $R^2$, $R^3$, A et Q sont chacun tels que définis ci-dessus, et
$R^4_e$ est un hydroxyalkyl(en $C_1$-$C_6$)uréido,
ou un sel de celui-ci, ou

(26) faire réagir un composé de formule:

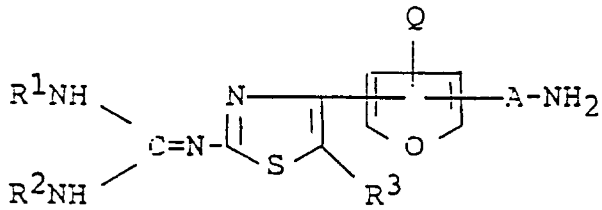

dans lequel

$R^1$, $R^2$, $R^3$, A et Q sont chacun tels que définis ci-dessus,

ou un sel de celui-ci, avec un composé de formule

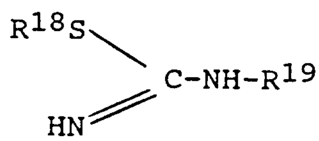

dans lequel

$R^{18}$ est un alkyle en $C_1$-$C_6$, et

$R^{19}$ est un carbamoyle,
un alcoxy(en $C_1$-$C_6$)carbonyle,
un sulfamoyle,
un alkyl(en $C_1$-$C_6$)sulfonyle,
un benzènesulfonyle, qui est substitué avec un ou plusieurs substituents choisis parmi un alkyle en $C_1$-$C_6$, un alcoxy en $C_1$-$C_6$, un halogène et un amino, ou un mono ou dialkyl(en $C_1$-$C_6$)sulfamoyle,

ou un sel de celui-ci, pour donner un composé de formule:

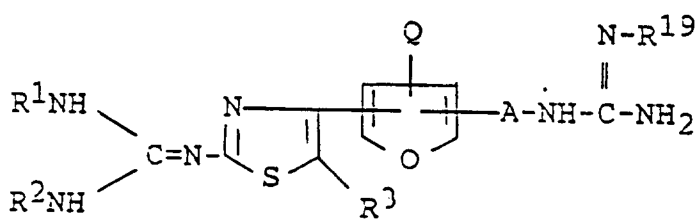

dans lequel

$R^1$, $R^2$, $R^3$, $R^{19}$, A et Q sont chacun tels que définis ci-dessus,

ou un sel de celui-ci, ou

(27) soumettre un composé de formule:

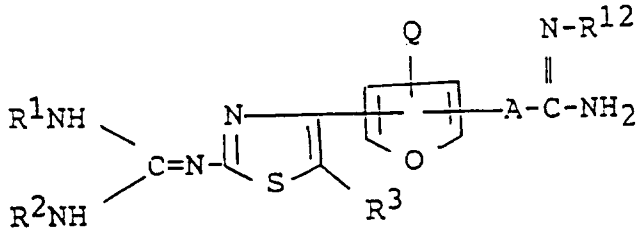

dans lequel

$R^1$, $R^2$, $R^3$, $R^{12}$, A et Q  sont chacun tels que définis ci-dessus,

ou un sel de celui-ci, à une hydrolyse, pour donner un composé de formule:

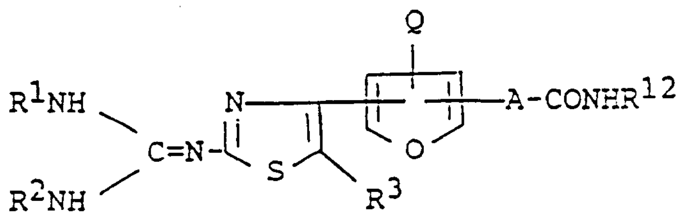

dans lequel

$R^1$, $R^2$, $R^3$, $R^{12}$, A et Q  sont chacun tels que définis ci-dessus,

ou un sel de celui-ci.

**8.** Composition pharmaceutique qui comprend, en tant qu'ingrédient actif, un composé de la revendication 1 ou un sel pharmaceutiquement acceptable de celui-ci, en mélange avec des supports pharmaceutiquement acceptables.

**9.** Utilisation d'un composé de la revendication 1 ou d'un sel pharmaceutiquement acceptable de celui-ci pour la préparation d'un médicament pour le traitement thérapeutique d'ulcère chez les êtres humains ou les animaux.

**10.** Composé selon la revendication 1 ou un sel pharmaceutiquement acceptable de celui-ci pour l'utilisation en tant que médicament.

**Revendications pour l'Etat contractant suivant : ES**

**1.** Procédé pour la préparation d'un composé de formule :

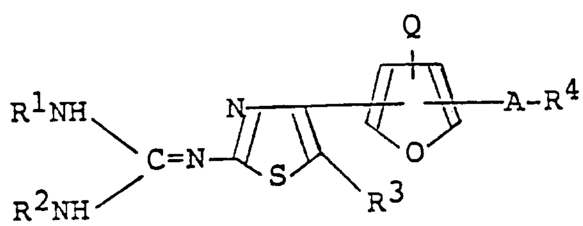

dans lequel

$R^1$ et $R^2$ sont chacun un hydrogène, un furoyle ou un alkyle en $C_1$-$C_6$ qui peut avoir un halogène; ou

$R^1$ et $R^2$ sont liés ensemble pour former un alkylène en $C_1$-$C_6$,

$R^3$ est un hydrogène ou un alkyle en $C_1$-$C_6$,

$R^4$ est un amino, un carbamoyle,
un aminocarbamoyle, un guanidinocarbamoyle,
un alkyl(en $C_1$-$C_6$)carbamoyle,
un sulfamoylaminocarbonyle,
un alcoxy(en $C_1$-$C_6$)carbonyle,
un uréido, un thiouréido, un sulfamoylamino,
un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,
un alcanoyl(en $C_1$-$C_6$)oxyalcanoyl(en $C_1$-$C_6$)amino,

un alkyl(en $C_1$-$C_6$)thioalcanoyl(en $C_1$-$C_6$)amino, un benzoylamino éventuellement substitué avec un nitro, un furoylamino, un thénoylamino, un nicotinoylamino, un 1-oxonicotinoylamino, un morpholinocarbonylamino, un alkyl(en $C_1$-$C_6$)uréido, un alkyl(en $C_1$-$C_6$)thiouréido, un alcanoyl(en $C_1$-$C_6$)uréido, un allyluréido, un benzoylthiouréido, un furylalkyl(en $C_1$-$C_6$)thioalcanoyl(en $C_1$-$C_6$)amino, un furylalkyl(en $C_1$-$C_6$)sulfinylalcanoyl(en $C_1$-$C_6$)amino, un mono ou dialkyl(en $C_1$-$C_6$)sulfamoylamino, un hydroxyalkyl(en $C_1$-$C_6$)uréido, un alcanoyl(en $C_1$-$C_6$)oxyalkyl(en $C_1$-$C_6$)uréido, un alkyl(en $C_1$-$C_6$)isothiouréido, un thiazolylamino substitué avec un alkyle en $C_1$-$C_6$, un triazolylamino substitué avec un amino, un triazolylamino substitué avec un amino et un alkyle en $C_1$-$C_6$, un benzoisothiazolylamino substitué avec un oxo, un benzothiadiazinylamino substitué avec un oxo et un halogène, un pyrimidinyle substitué avec un oxo et un alkyle en $C_1$-$C_6$, un triazolyle substitué avec un amino, ou un groupe de formule:

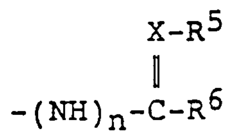

dans lequel

- n = 0 ou 1,
- X est =CH- ou =N-,
- $R^5$ est un hydrogène, un cyano, un nitro, un carbamoyle, un alcoxy(en $C_1$-$C_6$)carbonyle, un sulfamoyle, un alkyl(en $C_1$-$C_6$)sulfonyle, un benzènesulfonyle, qui est substitué avec un ou plusieurs substituants choisis parmi un alkyle en $C_1$-$C_6$, un alcoxy en $C_1$-$C_6$, un halogène, et un amino, ou un mono ou dialkyl(en $C_1$-$C_6$)sulfamoyle,
- $R^6$ est un hydrogène, un alkyle en $C_1$-$C_6$, un alkyl(en $C_1$-$C_6$)thio, un alcoxy en $C_1$-$C_6$, un amino, un mono ou dialkyl(en $C_1$-$C_6$)amino, un allylamino, un propargylamino, un hydroxyalkyl(en $C_1$-$C_6$)amino, un alcoxy(en $C_1$-$C_6$)alkyl(en $C_1$-$C_6$)amino, ou un mono ou dialkyl(en $C_1$-$C_6$)aminoalkyl(en $C_1$-$C_6$)amino;
- A est un alkylène en $C_1$-$C_6$ ou -CONH-; ou
- A-$R^4$ est un imidazolyle substitué avec un alkyle en $C_1$-$C_6$, un triazolyle substitué avec un amino, et
- Q est un hydrogène, ou un alkyle en $C_1$-$C_6$,

ou un sel de celui-ci, qui consiste à

(1) faire réagir un composé de formule :

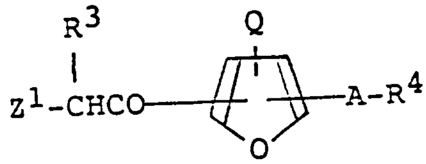

dans lequel
$R^3$, $R^4$, A et Q sont chacun tels que définis ci-dessus, et
$Z^1$ est un halogène,
ou un sel de celui-ci, avec un composé de formule :

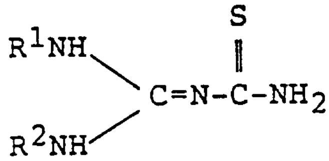

dans lequel
$R^1$ et $R^2$ sont chacun tels que définis ci-dessus,
pour donner un composé de formule:

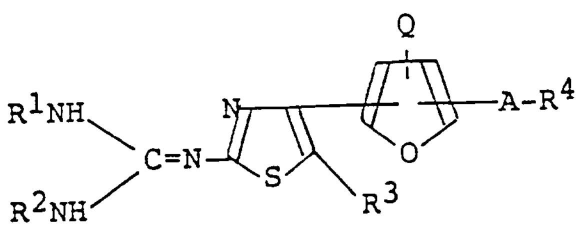

dans lequel
$R^1$, $R^2$, $R^3$, $R^4$, A et Q sont chacun tels que définis ci-dessus,
ou un sel de celui-ci, ou
(2) soumettre un composé de formule

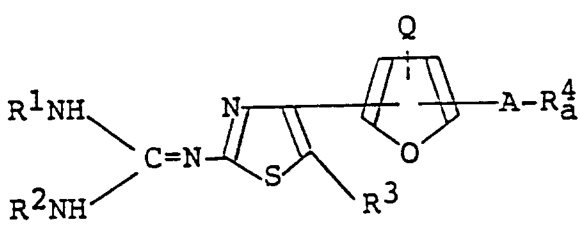

dans lequel
$R^1$, $R^2$, $R^3$, A et Q sont chacun tels que définis ci-dessus, et
$R^4_a$ est un uréido, un thiouréido,
un sulfamoylamino, un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,

un alcanoyl(en $C_1$-$C_6$)oxyalcanoyl(en $C_1$-$C_6$)amino,
un alkyl(en $C_1$-$C_6$)thioalcanoyl(en $C_1$-$C_6$)amino,
un benzoylamino éventuellement substitué par un nitro, un furoylamino, un thénoylamino,
un nicotinoylamino, un 1-oxonicotinoylamino,
un morpholinocarbonylamino,
un alkyl(en $C_1$-$C_6$)uréido,
un alkyl(en $C_1$-$C_6$)thiouréido,
un alcanoyl(en $C_1$-$C_6$)uréido,
un allyluréido, un benzoylthiouréido,
un furylalkyl(en $C_1$-$C_6$)thioalcanoyl(en $C_1$-$C_6$)amino,
un mono ou dialkyl(en $C_1$-$C_6$)sulfamoylamino,
un hydroxyalkyl(en $C_1$-$C_6$)uréido, ou
un alcanoyl(en $C_1$-$C_6$)oxyalkyl(en $C_1$-$C_6$)uréido,

ou un sel de celui-ci, à une réaction de déacylation, pour donner un composé de formule:

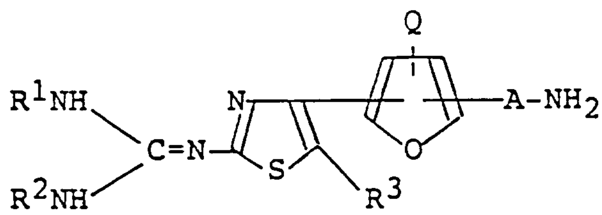

dans lequel
$R^1$, $R^2$, $R^3$, A et Q sont tels que définis ci-dessus,
ou un sel de celui-ci, ou
(3) soumettre un composé de formule:

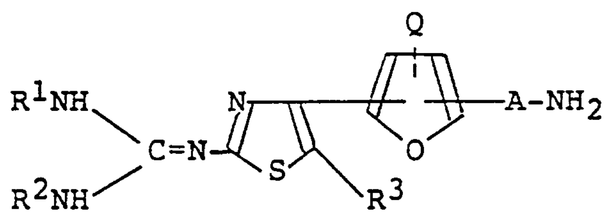

dans lequel
$R^1$, $R^2$, $R^3$, A et Q sont chacun tels que définis ci-dessus,
ou un sel de celui-ci, à une réaction d'acylation, pour donner un composé de formule:

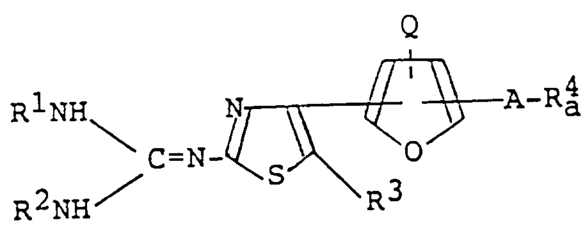

dans lequel
$R^1$, $R^2$, $R^3$, $R^4_a$ , A et Q sont chacun tels que définis ci-dessus,
ou un sel de celui-ci, ou

(4) faire réagir un composé de formule:

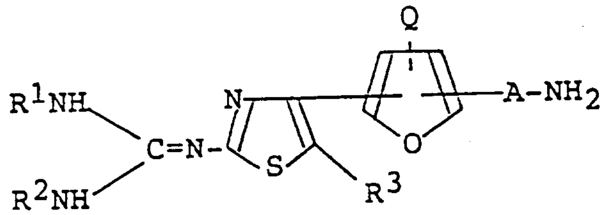

dans lequel
$R^1$, $R^2$, $R^3$, A et Q sont chacun tels que définis ci-dessus,
ou un sel de celui-ci, avec un composé de formule:

$(R^7\text{-}X^1)_2C=X\text{-}R^5$

dans lequel
$R^5$ et X sont chacun tels que définis ci-dessus,
$R^7$ est un alkyle en $C_1$-$C_6$ ou un phényle, et
$X^1$ est S ou O,
pour donner un composé de formule:

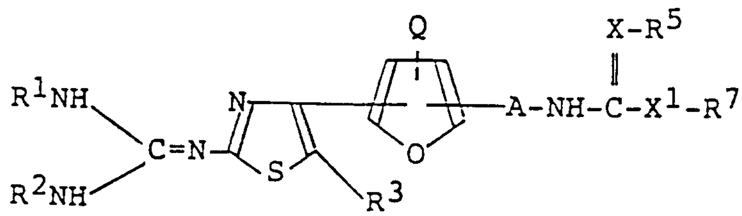

dans lequel
$R^1$, $R^2$, $R^3$, $R^5$, $R^7$, X, $X^1$, A et Q sont chacun tels que défini ci-dessus,
ou un sel de celui-ci, ou
(5) faire réagir un composé de formule:

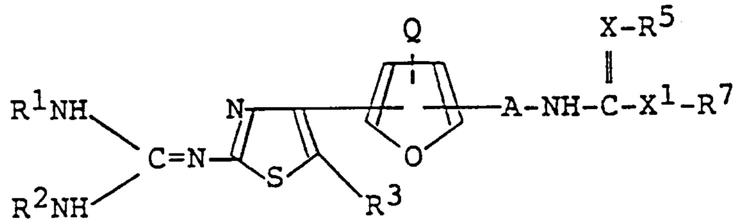

dans lequel
$R^1$, $R^2$, $R^3$, $R^5$, $R^7$, X, $X^1$, A et Q sont chacun tels que définis ci-dessus,
ou un sel de celui-ci avec un composé de formule:

$H\text{-}R^6_a$

dans lequel
$R^6_a$ est un amino qui peut avoir un mono ou dialkyle en $C_1$-$C_6$, un allyle, un propargyle, un hydroxyalkyle en $C_1$-$C_6$ ou un mono ou dialkyl(en $C_1$-$C_6$)aminoalkyle en $C_1$-$C_6$,

ou un alcoxy en $C_1$-$C_6$,
pour donner un composé de formule:

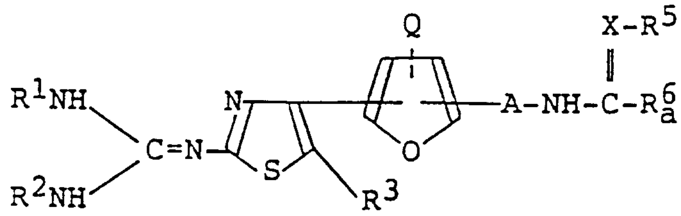

dans lequel
$R^1$, $R^2$, $R^3$, $R^5$, $R_a^6$ X, A et Q sont chacun tels que définis ci-dessus,
ou un sel de celui-ci, ou
(6) soumettre un composé de formule:

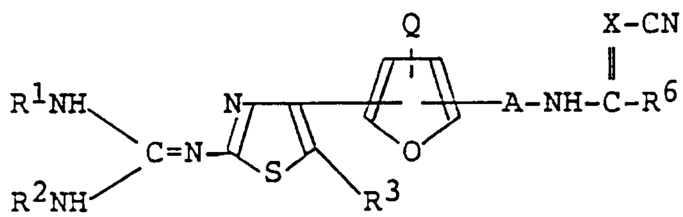

dans lequel
$R^1$, $R^2$, $R^3$, $R^6$, X, A et Q sont chacun tels définis ci-dessus,
ou un sel de celui-ci, à une hydrolyse, pour donner un composé de formule:

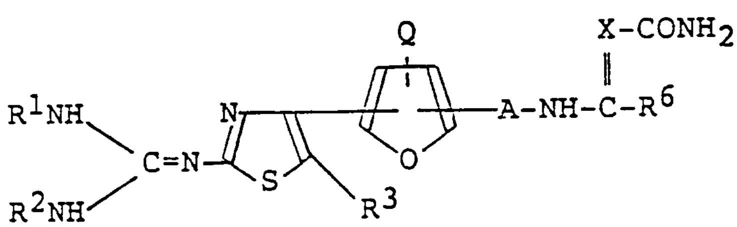

dans lequel
$R^1$, $R^2$, $R^3$, $R^6$, X, A et Q sont chacun tels que définis ci-dessus,
ou un sel de celui-ci, ou
(7) soumettre un composé de formule

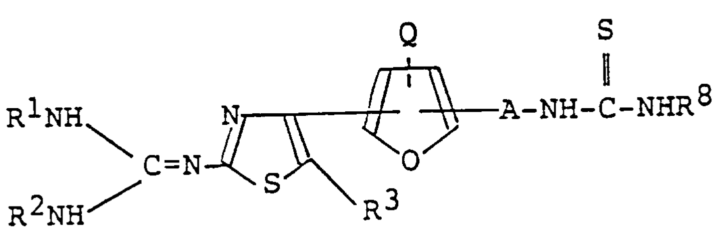

dans lequel
$R^1$, $R^2$, $R^3$, A et Q sont chacun tels que définis ci-dessus, et
$R^8$ est un groupe protecteur de l'amino constitué d'un alcanoyle en $C_1$-$C_6$ et d'un

benzoyle,
ou un sel de celui-ci, à une réaction d'élimination du groupe protecteur de l'amino, pour donner un composé de formule:

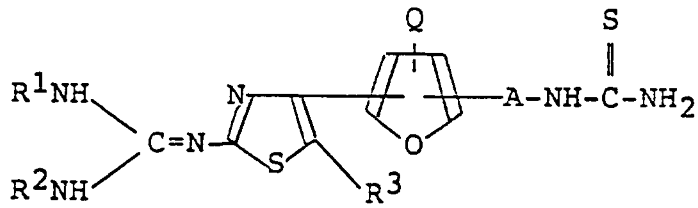

dans lequel
$R^1$, $R^2$, $R^3$, A et Q sont chacun tels que définis ci-dessus,
ou un sel de celui-ci, ou
(8) faire réagir un composé de formule:

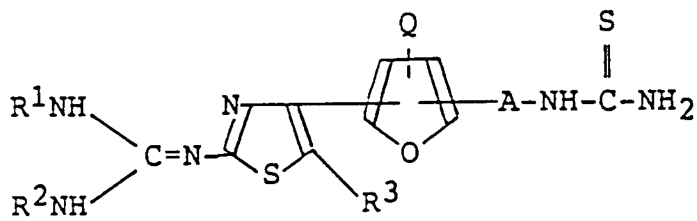

dans lequel
$R^1$, $R^2$, $R^3$, A et Q sont chacun tels que définis ci-dessus,
ou un sel de celui-ci, avec un composé de formule:

$Z^2$-$CH_2$-CO-$R^9$

dans lequel
$R^9$ est un alkyle en $C_1$-$C_6$, et
$Z^2$ est un halogène,
pour donner un composé de formule:

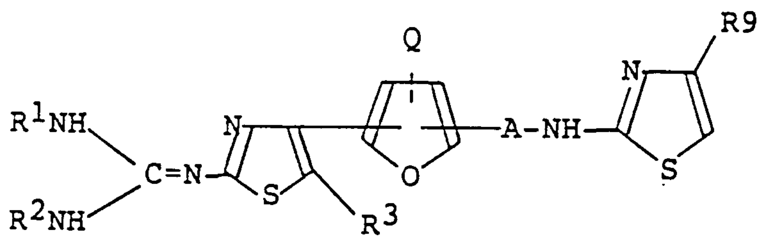

dans lequel
$R^1$, $R^2$, $R^3$, $R^9$, A et Q sont chacun tels que définis ci-dessus,
ou un sel de celui-ci, ou

(9) faire réagir un composé de formule:

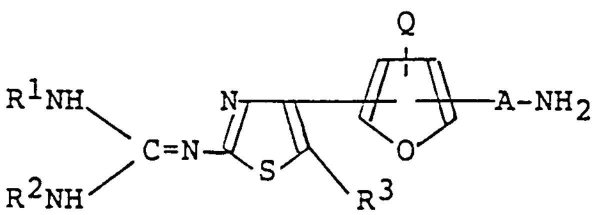

dans lequel
$R^1$, $R^2$, $R^3$, A et Q sont chacun tels que définis ci-dessus,
ou un sel de celui-ci, avec un composé de formule:

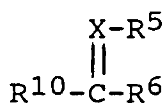

dans lequel
$R^5$, $R^6$ et X sont chacun tels que définis ci-dessus, et
$R^{10}$ est un alcanoyl(en $C_1$-$C_6$)oxy,
un phénylalcoxy en $C_1$-$C_6$,
un alcoxy(en $C_1$-$C_6$)alcoxy en $C_1$-$C_6$,
un tétrahydropyranyloxy ou
un alcoxy en $C_1$-$C_6$,
pour donner un composé de formule:

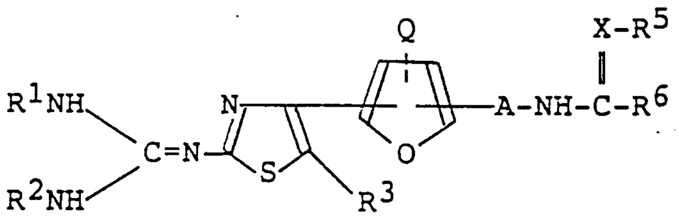

dans lequel
$R^1$, $R^2$, $R^3$, $R^5$, $R^6$, X, A et Q sont chacun tels que définis ci-dessus,
ou un sel de celui-ci, ou
(10) faire réagir un composé de formule:

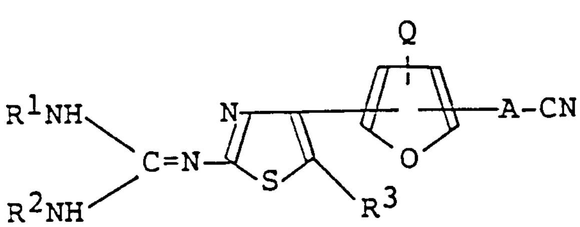

dans lequel
$R^1$, $R^2$, $R^3$, A et Q sont chacun tels que définis ci-dessus,
ou un sel de celui-ci, avec un composé de formule:

$R^{11}OH$

dans lequel

$R^{11}$ est un alkyle en $C_1$-$C_6$,

pour donner un composé de formule:

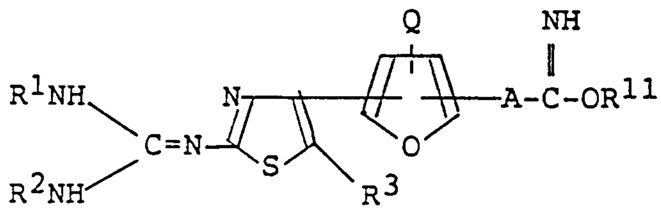

dans lequel

$R^1$, $R^2$, $R^3$, $R^{11}$, A et Q sont chacun tels que définis ci-dessus,

ou un sel de celui-ci, ou

(11) faire réagir un composé de formule:

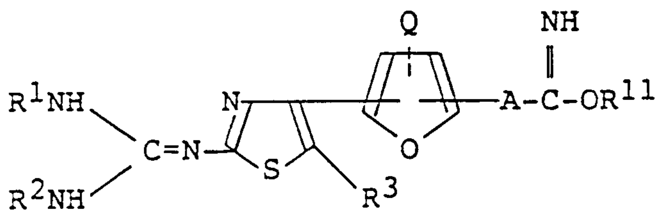

dans lequel

$R^1$, $R^2$, $R^3$, $R^{11}$, A et Q sont chacun tels que définis ci-dessus,

ou un sel de celui-ci, avec un composé de formule:

$NH_2R^{12}$

dans lequel

$R^{12}$ est un acyle,

pour donner un composé de formule:

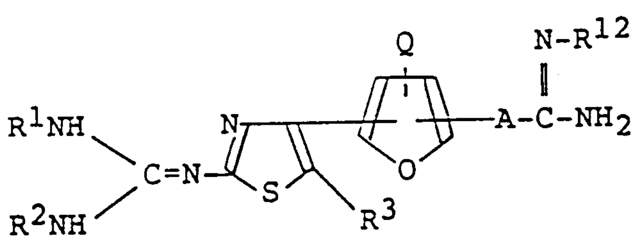

dans lequel

$R^1$, $R^2$, $R^3$, $R^{12}$, A et Q sont chacun tels que définis ci-dessus,

ou un sel de celui-ci, ou

(12) faire réagir un composé de formule:

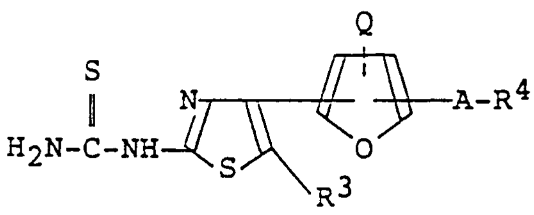

dans lequel
$R^3$, $R^4$, A et Q sont chacun tels que définis ci-dessus,
ou un sel de celui-ci, avec un composé de formule:

$R^{13}$-$Z^3$

dans lequel
$R^{13}$ est un alkyle en $C_1$-$C_6$, et
$Z^3$ est un halogène,
ou un sel de celui-ci, pour donner un composé de formule:

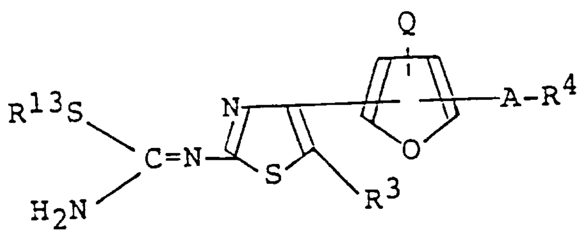

dans lequel
$R^3$, $R^4$, $R^{13}$, A et Q sont tels que définis ci-dessus,
ou un sel de celui-ci, et le faire réagir de manière continue avec un composé de formule:

$H_2N$-Y-$NH_2$

dans lequel
Y est un alkylène en $C_1$-$C_6$,
ou un sel de celui-ci,
pour donner un composé de formule:

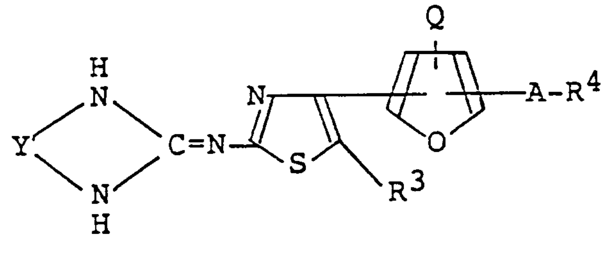

dans lequel
$R^3$, $R^4$, A, Q et Y sont chacun tels que définis ci-dessus,
ou un sel de celui-ci, ou

(13) soumettre un composé de formule:

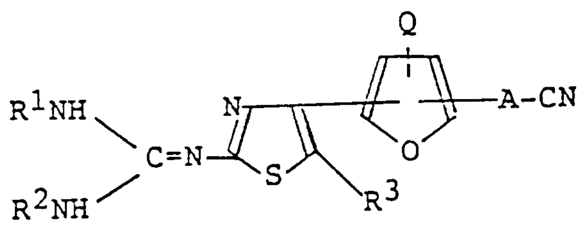

dans lequel
$R^1$, $R^2$, $R^3$, A et Q sont chacun tels que définis ci-dessus,
ou un sel de celui-ci, à une réduction, pour donner un composé de formule:

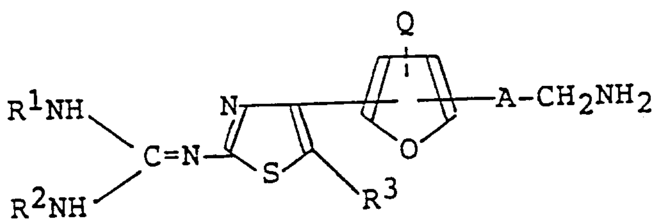

dans lequel
$R^1$, $R^2$, $R^3$, A et Q sont chacun tels que définis ci-dessus,
ou un sel de celui-ci, ou
(14) faire réagir un composé de formule:

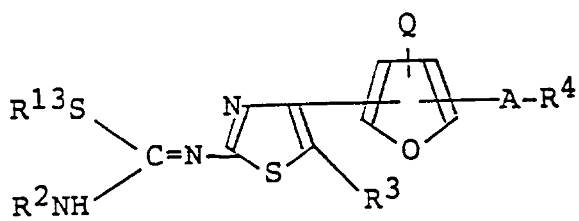

dans lequel
$R^2$, $R^3$, $R^4$, $R^{13}$, A et Q sont chacun tels que définis ci-dessus,
ou un sel de celui-ci, avec un composé de formule:

$NH_2R_a^1$

dans lequel
$R_a^1$ est un alkyle en $C_1$-$C_6$ qui peut avoir un halogène,
ou un sel de celui-ci, pour donner un composé de formule:

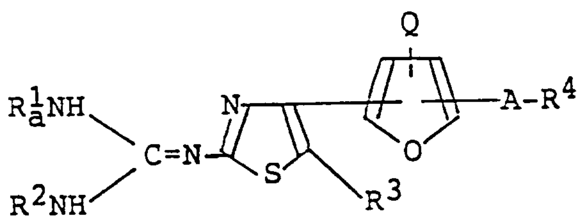

dans lequel,

$R^1_a$, $R^2$, $R^3$, $R^4$, A et Q sont chacun tels que définis ci-dessus,
ou un sel de celui-ci, ou
(15) soumettre un composé de formule:

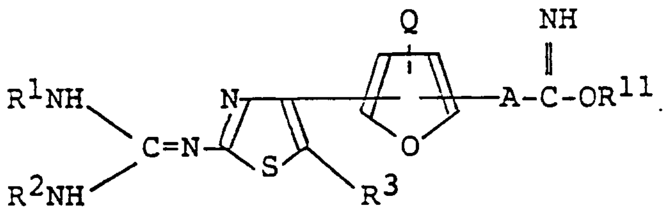

dans lequel
$R^1$, $R^2$, $R^3$, $R^{11}$, A et Q sont chacun tels que définis ci-dessus,
ou un sel de celui-ci, à une hydrolyse, pour donner un composé de formule:

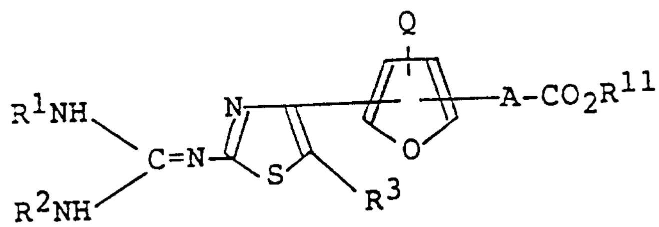

dans lequel
$R^1$, $R^2$, $R^3$, $R^{11}$, A et Q sont chacun tels que définis ci-dessus,
ou un sel de celui-ci, ou
(16) soumettre un composé de formule:

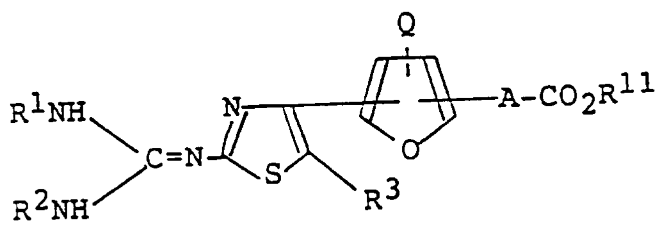

dans lequel
$R^1$, $R^2$, $R^3$, $R^{11}$, A et Q sont chacun tels que définis ci-dessus,
ou un sel de celui-ci, à une réaction d'amidation, pour donner un composé de formule:

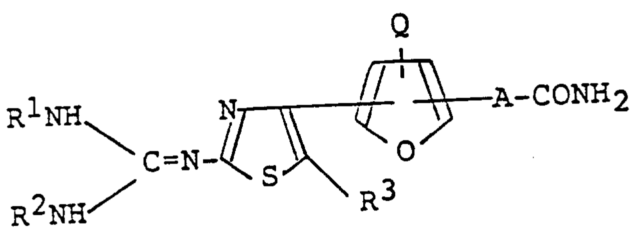

dans lequel
$R^1$, $R^2$, $R^3$, A et Q sont chacun tels que définis ci-dessus,

ou un sel de celui-ci, ou
(17) faire réagir un composé de formule:

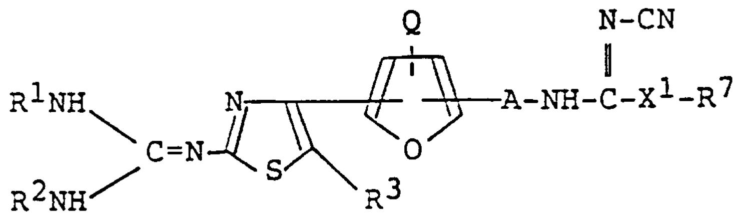

dans lequel
$R^1$, $R^2$, $R^3$, $R^7$, $X^1$, A et Q sont chacun tels que définis ci-dessus,
ou un sel de celui-ci, avec un composé de formule:

$R^{14}NHNH_2$

dans lequel
$R^{14}$ est un hydrogène ou un alkyle en $C_1$-$C_6$,
pour donner un composé de formule:

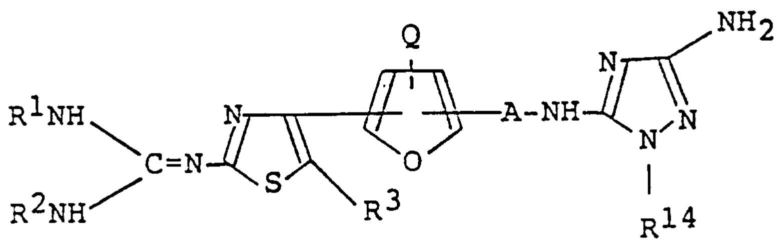

dans lequel
$R^1$, $R^2$, $R^3$, $R^{14}$, A et Q sont chacun tels que définis ci-dessus,
ou un sel de celui-ci, ou
(18) faire réagir un composé de formule:

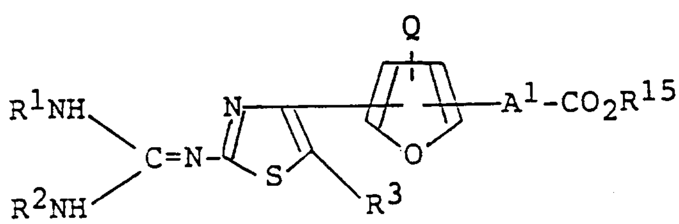

dans lequel
$R^1$, $R^2$, $R^3$ et Q sont chacun tels que définis ci-dessus,
$R^{15}$ est un alkyle en $C_1$-$C_6$, et
$A^1$ est un alkylène en $C_1$-$C_6$ ou une liaison,
ou un sel de celui-ci, avec de l'hydrazine, pour donner un composé de formule:

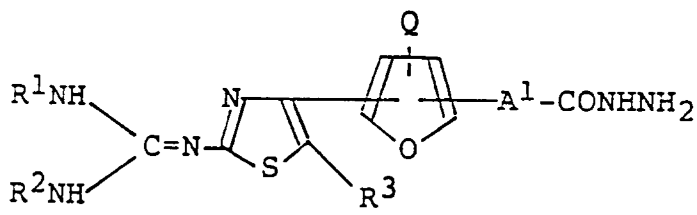

dans lequel
$R^1$, $R^2$, $R^3$, $A^1$ et Q sont chacun tels que définis ci-dessus,
ou un sel de celui-ci, ou
(19) faire réagir un composé de formule:

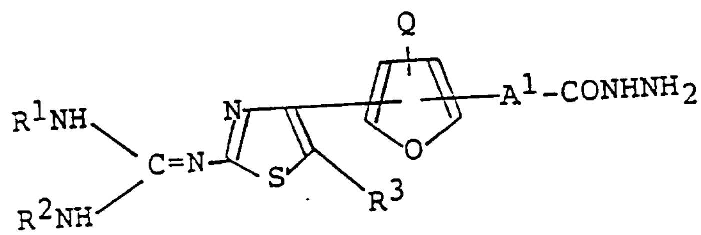

dans lequel
$R^1$, $R^2$, $R^3$, $A^1$ et Q sont chacun tels que définis ci-dessus,
ou un sel de celui-ci, avec un S-alkyl(en $C_1$-$C_6$)isothiourée, ou un sel de celui-ci, pour donner un composé de formule:

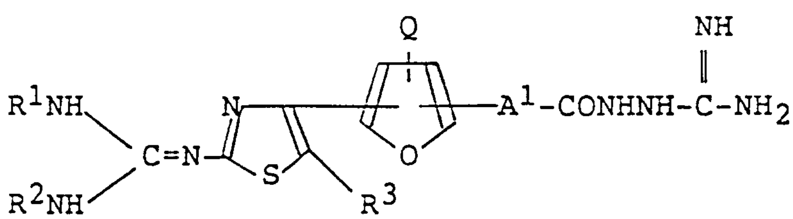

dans lequel
$R^1$, $R^2$, $R^3$, $A^1$ et Q sont chacun tels que définis ci-dessus,
ou un sel de celui-ci, ou
(20) soumettre un composé de formule:

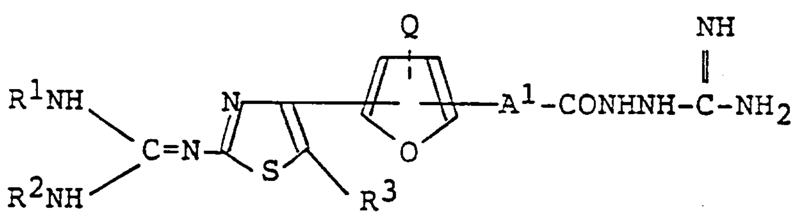

dans lequel
$R^1$, $R^2$, $R^3$, $A^1$ et Q sont chacun tels que définis ci-dessus,
ou un sel de celui-ci, à une fermeture de cycle, pour donner un composé de formule:

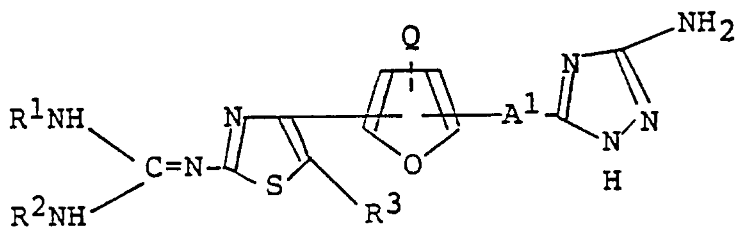

dans lequel

$R^1$, $R^2$, $R^3$, $A^1$ et Q sont chacun tels que définis ci-dessus,

ou un sel de celui-ci, ou

(21) faire réagir un composé de formule:

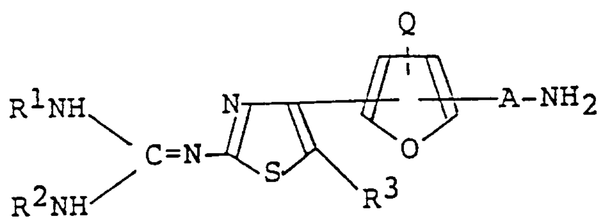

dans lequel

$R^1$, $R^2$, $R^3$, A et Q sont chacun tels que définis ci-dessus,

ou un sel de celui-ci, avec un composé de formule:

$R^{16}$-$Z^4$

dans lequel

$R^{16}$ est un thiazolyle substitué avec un alkyle en $C_1$-$C_6$, un triazolyle substitué avec un amino, un triazolyle substitué avec un amino et un alkyle en $C_1$-$C_6$, un benzoisothiazolyle substitué avec un oxo, ou un benzothiadiazinyle substitué avec un oxo, et

$Z_4$ est un halogène,

ou un sel de celui-ci, pour donner un composé de formule:

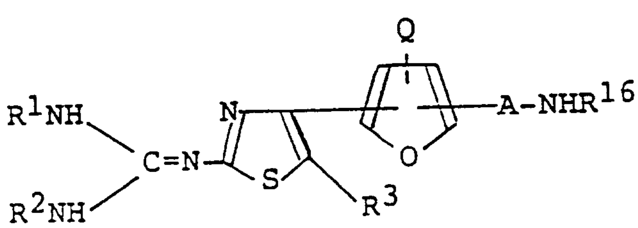

dans lequel

$R^1$, $R^2$, $R^3$, $R^{16}$, A et Q sont chacun tels que définis ci-dessus,

ou un sel de celui-ci, ou

(22) soumettre un composé de formule:

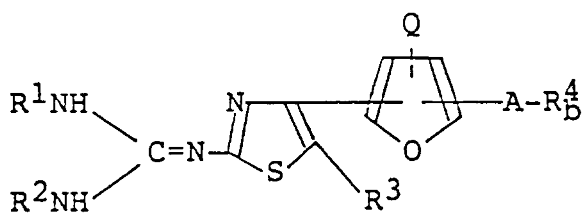

dans lequel

$R^1$, $R^2$, $R^3$, A et Q sont chacun tels que définis ci-dessus, et

$R^4_b$ est un furylalkyl(en $C_1$-$C_6$)thioalcanoyl(en $C_1$-$C_6$)amino,

ou un sel de celui-ci, à une réaction d'oxydation, pour donner un composé de formule:

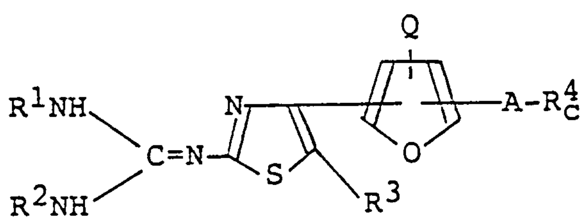

dans lequel

$R^1$, $R^2$, $R^3$, A et Q sont chacun tels que définis ci-dessus, et

$R^4_c$ est un furylalkyl(en $C_1$-$C_6$)sulfinylalcanoyl(en $C_1$-$C_6$)amino,

ou un sel de celui-ci, ou

(23) faire réagir un composé de formule:

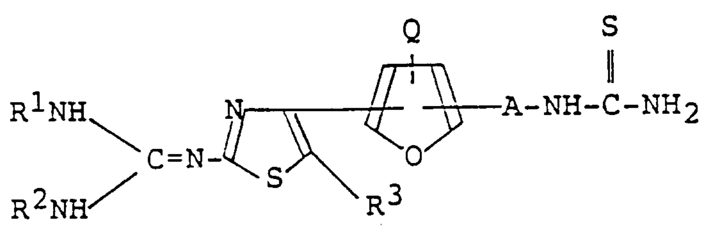

dans lequel

$R^1$, $R^2$, $R^3$, A et Q sont chacun tels que définis ci-dessus,

ou un sel de celui-ci, avec un composé de formule:

$R^{17}$-$Z^5$

dans lequel

$R^{17}$ est un alkyle en $C_1$-$C_6$, et

$Z^5$ est un halogène,

ou un sel de celui-ci, pour donner un composé de formule:

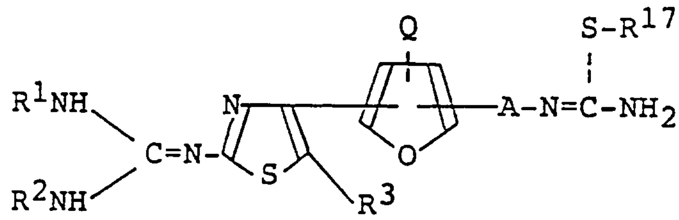

dans lequel

$R^1$, $R^2$, $R^3$, $R^{17}$, A et Q sont chacun tels que définis ci-dessus,

ou un sel de celui-ci, ou

(24) faire réagir un composé de formule:

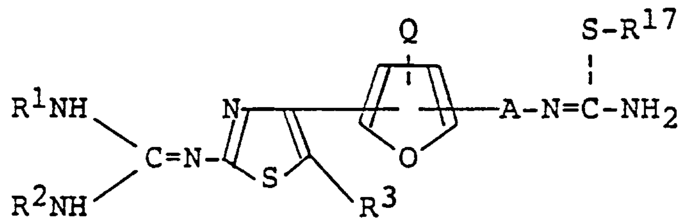

dans lequel

$R^1$, $R^2$, $R^3$, $R^{17}$, A et Q sont chacun tels que définis ci-dessus,

ou un sel de celui-ci, avec un composé de formule:

$H_2N\text{-}R^5$

dans lequel $R^5$ est tel que défini ci-dessus,

ou un sel de celui-ci, pour donner un composé de formule:

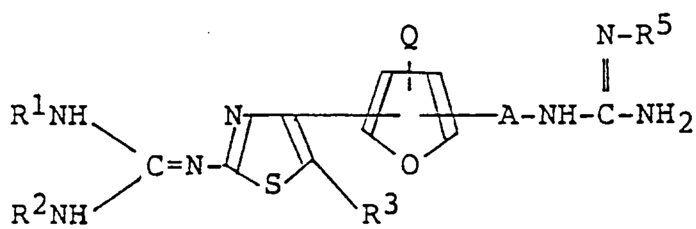

dans lequel

$R^1$, $R^2$, $R^3$, $R^5$, A et Q sont chacun tels que définis ci-dessus,

ou un sel de celui-ci, ou

(25) soumettre un composé de formule:

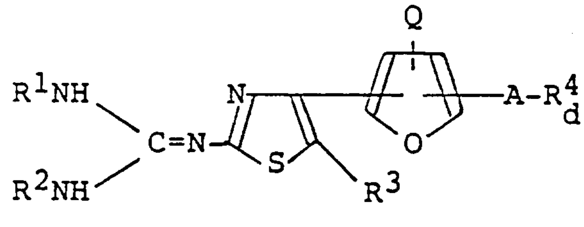

dans lequel

$R^1$, $R^2$, $R^3$, A et Q sont chacun tels que définis ci-dessus, et

$R^4_d$ est un alcanoyl(en $C_1$-$C_6$)oxyalkyl(en $C_1$-$C_6$)uréido,

ou un sel de celui-ci, à une réaction d'élimination de l'alcanoyle en $C_1$-$C_6$, pour donner un composé de formule:

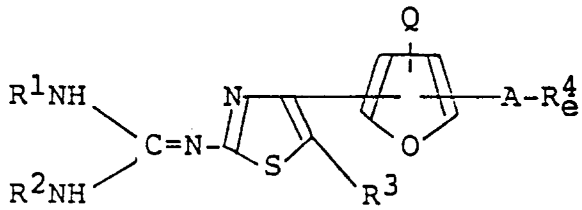

dans lequel

$R^1$, $R^2$, $R^3$, A et Q sont chacun tels que définis ci-dessus, et

$R^4_e$ est un hydroxyalkyl(en $C_1$-$C_6$)uréido,

ou un sel de celui-ci, ou

(26) faire réagir un composé de formule:

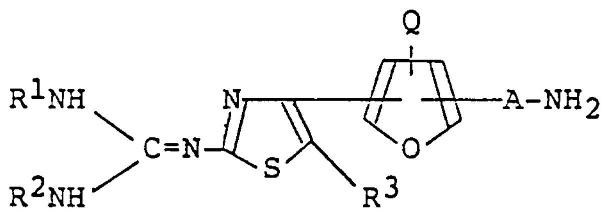

dans lequel

$R^1$, $R^2$, $R^3$, A et Q sont chacun tels que définis ci-dessus,

ou un sel de celui-ci, avec un composé de formule

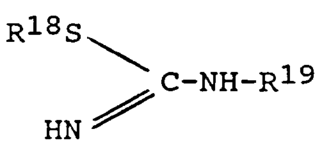

dans lequel

$R^{18}$ est un alkyle en $C_1$-$C_6$, et

$R^{19}$ est un carbamoyle,
un alcoxy(en $C_1$-$C_6$)carbonyle,
un sulfamoyle,
un alkyl(en $C_1$-$C_6$)sulfonyle,
un benzènesulfonyle, qui est substitué avec un ou plusieurs substituants choisis parmi un alkyle en $C_1$-$C_6$, un alcoxy en $C_1$-$C_6$, un halogène et un amino, ou un mono ou dialkyl(en $C_1$-$C_6$)sulfamoyle,

ou un sel de celui-ci, pour donner un composé de formule:

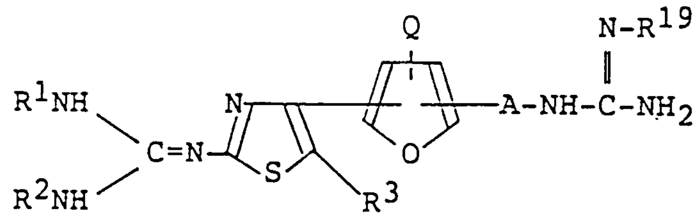

dans lequel

$R^1$, $R^2$, $R^3$, $R^{19}$, A et Q sont chacun tels que définis ci-dessus,

ou un sel de celui-ci, ou

(27) soumettre un composé de formule:

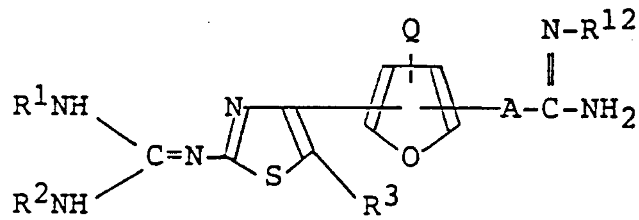

dans lequel

$R^1$, $R^2$, $R^3$, $R^{12}$, A et Q sont chacun tels que définis ci-dessus,

ou un sel de celui-ci, à une hydrolyse, pour donner un composé de formule:

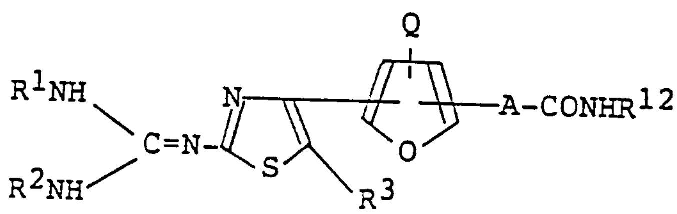

dans lequel

$R^1$, $R^2$, $R^3$, $R^{12}$, A et Q sont chacun tels que définis ci-dessus,

ou un sel de celui-ci.

**2.** Procédé selon la revendication 1, dans lequel

$R^1$ et $R^2$ sont chacun un hydrogène, un alkyle en $C_1$-$C_6$ ou un trihaloalkyle en $C_1$-$C_6$; ou

$R^1$ et $R^2$ sont liés ensemble pour former un alkylène en $C_1$-$C_6$,

$R^4$ est un amino, un carbamoyle, un aminocarbamoyle,
un guanidinocarbamoyle, un alkyl(en $C_1$-$C_6$)carbamoyle,
un sulfamoylaminocarbonyle,
un alcoxy(en $C_1$-$C_6$)carbonyle, un uréido,
un thiouréido, un sulfamoylamino,
un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,
un alcanoyl(en $C_1$-$C_6$)oxyalcanoyl(en $C_1$-$C_6$)amino,
un alkyl(en $C_1$-$C_6$)thioalcanoyl(en $C_1$-$C_6$)amino,
un nitrobenzoylamino, un furoylamino, un thénoylamino,
un nicotinoylamino,
un 1-oxonicotinoylamino, un morpholinocarbonylamino,

un 3-alkyl(en $C_1$-$C_6$)uréido,
un 3-alkyl(en $C_1$-$C_6$)thiouréido,
un 3-alcanoyl(en $C_1$-$C_6$)uréido, un 3-allyluréido,
un 3-benzoylthiouréido,
un furylalkyl(en $C_1$-$C_6$)thioalcanoyl(en $C_1$-$C_6$)amino,
un furylalkyl(en $C_1$-$C_6$)sulfinylalcanoyl(en $C_1$-$C_6$)amino,
un mono ou dialkyl(en $C_1$-$C_6$)sulfamoylamino,
un 3-hydroxyalkyl(en $C_1$-$C_6$)uréido,
un 3-alcanoyl(en $C_1$-$C_6$)oxyalkyl(en $C_1$-$C_6$)uréido,
un 2-alkyl(en $C_1$-$C_6$)isothiouréido,
un thiazolylamino substitué avec un alkyle en $C_1$-$C_6$,
un triazolylamino substitué avec un amino,
un triazolylamino substitué avec un amino et un alkyle en $C_1$-$C_6$,
un benzoisothiazolylamino substitué avec un oxo,
un benzothiadiazinylamino substitué avec un oxo et un halogène,
un pyrimidinyle substitué avec un oxo et un alkyle en $C_1$-$C_6$,
un triazolyle substitué avec un amino, ou
un groupe de formule:

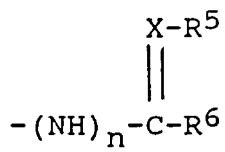

dans lequel

| | |
|---|---|
| n, X et $R^6$ | sont tels que définis dans la revendication 1, |
| $R^5$ est | un hydrogène, un cyano, un nitro, un carbamoyle, un alcoxy(en $C_1$-$C_6$)carbonyle, un sulfamoyle, un alkyl(en $C_1$-$C_6$)sulfonyle, un phénylsulfonyle substitué avec un alkyle en $C_1$-$C_6$, un phénylsulfonyle substitué avec un alcoxy en $C_1$-$C_6$, un phénylsulfonyle substitué avec un halogène, un phénylsulfonyle substitué avec un amino, un mono ou dialkyl(en $C_1$-$C_6$)sulfamoyle, |
| A est | tel que défini dans la revendication 1; ou |
| A-$R^4$ est | tel que défini dans la revendication 1, et |
| $R^3$ et Q | sont tels que définis dans la revendication 1. |

**3.** Procédé selon la revendication 2, dans lequel

| | |
|---|---|
| $R^1$ et $R^2$ | sont chacun un hydrogène, |
| $R^3$ est | un hydrogène, |
| $R^4$ est | un groupe de formule: |

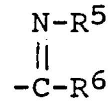

dans lequel

| | |
|---|---|
| $R^5$ est | un sulfamoyle, et |
| $R^6$ est | un amino, |
| A est | un alkylène en $C_1$-$C_6$, et |
| Q est | un hydrogène. |

**4.** Procédé selon la revendication 2, dans lequel

| | |
|---|---|
| $R^1$ et $R^2$ | sont chacun un hydrogène, |

| | |
|---|---|
| $R^3$ est | un hydrogène, |
| $R^4$ est | un uréido ou un 3-alkyl(en $C_1$-$C_6$)uréido, |
| A est | un alkylène en $C_1$-$C_6$, et |
| Q est | un hydrogène. |

**5.** Procédé pour la préparation d'une composition pharmaceutique qui comprend, en tant qu'ingrédient actif, un composé de la revendication 1 ou un sel pharmaceutiquement acceptable de celui-ci, caractérisé par le mélange d'un composé de la revendication 1 avec au moins un support ou un excipient pharmaceutiquement acceptable.

**6.** Procédé pour la préparation d'une composition selon la revendication 5, pour le traitement thérapeutique d'ulcère chez les êtres humains ou les animaux.

**Revendications pour l'Etat contractant : GR**

**1.** Procédé pour la préparation d'un composé de formule :

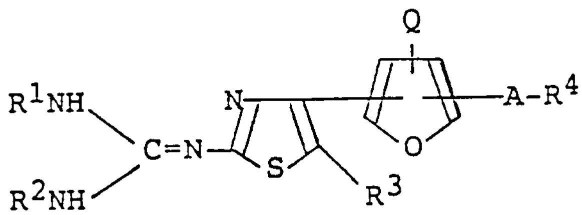

(I)

dans lequel

| | |
|---|---|
| $R^1$ et $R^2$ | sont chacun un hydrogène, un furoyle ou un alkyle en $C_1$-$C_6$ qui peut avoir un halogène; ou |
| $R^1$ et $R^2$ | sont liés ensemble pour former un alkylène en $C_1$-$C_6$, |
| $R^3$ est | un hydrogène ou un alkyle en $C_1$-$C_6$, |
| $R^4$ est | un amino, un carbamoyle,<br>un aminocarbamoyle, un guanidinocarbamoyle,<br>un alkyl(en $C_1$-$C_6$)carbamoyle,<br>un sulfamoylaminocarbonyle,<br>un alcoxy(en $C_1$-$C_6$)carbonyle,<br>un uréido, un thiouréido, un sulfamoylamino,<br>un alcanoyl(en $C_1$-$C_6$)amino,<br>un alcoxy(en $C_1$-$C_6$)carbonylamino,<br>un alkyl(en $C_1$-$C_6$)sulfonylamino,<br>un alcoxy(en $C_1$-$C_6$)alcanoyl(en $C_1$-$C_6$)amino,<br>un alcanoyl(en $C_1$-$C_6$)oxyalcanoyl(en $C_1$-$C_6$)amino,<br>un alkyl(en $C_1$-$C_6$)thioalcanoyl(en $C_1$-$C_6$)amino,<br>un benzoylamino éventuellement substitué avec<br>un nitro, un furoylamino, un thénoylamino,<br>un nicotinoylamino, un 1-oxonicotinoylamino,<br>un morpholinocarbonylamino,<br>un alkyl(en $C_1$-$C_6$)uréido,<br>un alkyl(en $C_1$-$C_6$)thiouréido,<br>un alcanoyl(en $C_1$-$C_6$)uréido, un allyluréido,<br>un benzoylthiouréido,<br>un furylalkyl(en $C_1$-$C_6$)thioalcanoyl(en $C_1$-$C_6$)amino,<br>un furylalkyl(en $C_1$-$C_6$)sulfinylalcanoyl(en $C_1$-$C_6$)amino,<br>un mono ou dialkyl(en $C_1$-$C_6$)sulfamoylamino,<br>un hydroxyalkyl(en $C_1$-$C_6$)uréido,<br>un alcanoyl(en $C_1$-$C_6$)oxyalkyl(en $C_1$-$C_6$)uréido,<br>un alkyl(en $C_1$-$C_6$)isothiouréido,<br>un thiazolylamino substitué avec un alkyle en $C_1$-$C_6$, un triazolylamino substitué avec |

un amino, un triazolylamino substitué avec un amino et un alkyle en $C_1$-$C_6$, un benzoisothiazolylamino substitué avec un oxo, un benzothiadiazinylamino substitué avec un oxo et un halogène, un pyrimidinyle substitué avec un oxo et un alkyle en $C_1$-$C_6$, un triazolyle substitué avec un amino, ou un groupe de formule:

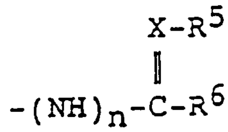

dans lequel

n = 0 ou 1,

X est =CH- ou =N-,

$R^5$ est un hydrogène, un cyano, un nitro, un carbamoyle, un alcoxy(en $C_1$-$C_6$)carbonyle, un sulfamoyle, un alkyl(en $C_1$-$C_6$)sulfonyle, un benzènesulfonyle, qui est substitué avec un ou plusieurs substituants choisis parmi un alkyle en $C_1$-$C_6$, un alcoxy en $C_1$-$C_6$, un halogène, et un amino, ou un mono ou dialkyl(en $C_1$-$C_6$)sulfamoyle,

$R^6$ est un hydrogène, un alkyle en $C_1$-$C_6$, un alkyl(en $C_1$-$C_6$)thio, un alcoxy en $C_1$-$C_6$, un amino, un mono ou dialkyl(en $C_1$-$C_6$)amino, un allylamino, un propargylamino, un hydroxyalkyl(en $C_1$-$C_6$)amino, un alcoxy(en $C_1$-$C_6$)alkyl(en $C_1$-$C_6$)amino, ou un mono ou dialkyl(en $C_1$-$C_6$)aminoalkyl(en $C_1$-$C_6$)amino;

A est un alkylène en $C_1$-$C_6$ ou -CONH-; ou

A-$R^4$ est un imidazolyle substitué avec un alkyle en $C_1$-$C_6$, un triazolyle substitué avec un amino, et

Q est un hydrogène, ou un alkyle en $C_1$-$C_6$,

ou un sel de celui-ci,
qui consiste à

(1) faire réagir un composé de formule :

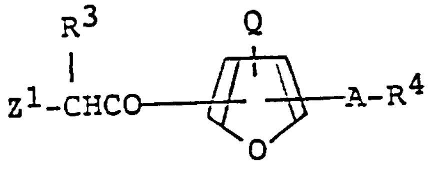

dans lequel

$R^3$, $R^4$, A et Q sont chacun tels que définis ci-dessus, et

$Z^1$ est un halogène,

ou un sel de celui-ci, avec un composé de formule :

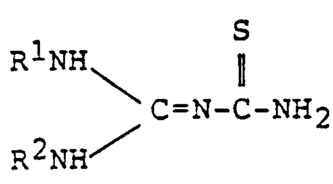

dans lequel

$R^1$ et $R^2$ sont chacun tels que définis ci-dessus,

pour donner un composé de formule:

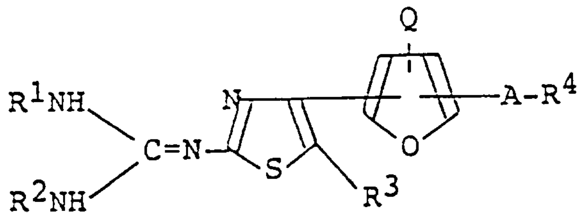

dans lequel

$R^1$, $R^2$, $R^3$, $R^4$, A et Q sont chacun tels que définis ci-dessus,

ou un sel de celui-ci, ou

(2) soumettre un composé de formule

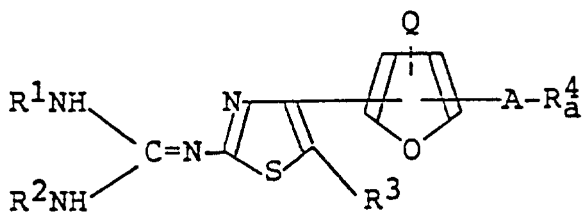

dans lequel

$R^1$, $R^2$, $R^3$, A et Q sont chacun tels que définis ci-dessus, et

$R^4_a$ est un uréido, un thiouréido, un sulfamoylamino, un alcanoyl(en $C_1$-$C_6$)amino, un alcoxy(en $C_1$-$C_6$)carbonylamino, un alkyl(en $C_1$-$C_6$)sulfonylamino, un alcoxy(en $C_1$-$C_6$)alcanoyl(en $C_1$-$C_6$)amino, un alcanoyl(en $C_1$-$C_6$)oxyalcanoyl(en $C_1$-$C_6$)amino, un alkyl(en $C_1$-$C_6$)thioalcanoyl(en $C_1$-$C_6$)amino, un benzoylamino éventuellement substitué par un nitro, un furoylamino, un thénoylamino, un nicotinoylamino, un 1-oxonicotinoylamino, un morpholinocarbonylamino, un alkyl(en $C_1$-$C_6$)uréido, un alkyl(en $C_1$-$C_6$)thiouréido, un alcanoyl(en $C_1$-$C_6$)uréido, un allyluréido, un benzoylthiouréido, un furylalkyl(en $C_1$-$C_6$)thioalcanoyl(en $C_1$-$C_6$)amino, un mono ou dialkyl(en $C_1$-$C_6$)sulfamoylamino, un hydroxyalkyl(en $C_1$-$C_6$)uréido, ou un alcanoyl(en $C_1$-$C_6$)oxyalkyl(en $C_1$-$C_6$)uréido, ou un sel de celui-ci, à une réaction de déacylation, pour donner un composé de formule:

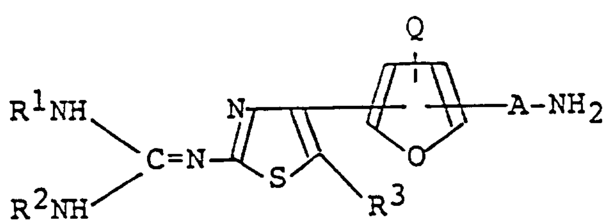

dans lequel

$R^1$, $R^2$, $R^3$, A et Q sont tels que définis ci-dessus,

ou un sel de celui-ci, ou

(3) soumettre un composé de formule:

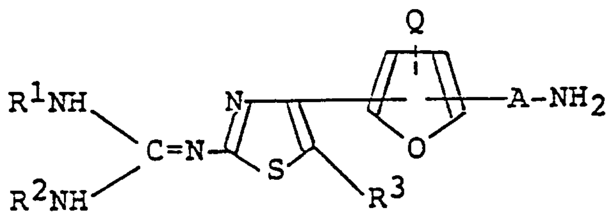

dans lequel

$R^1$, $R^2$, $R^3$, A et Q sont chacun tels que définis ci-dessus,

ou un sel de celui-ci, à une réaction d'acylation, pour donner un composé de formule:

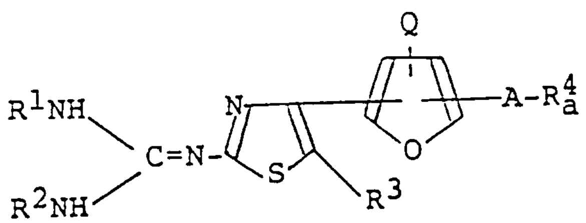

dans lequel

$R^1$, $R^2$, $R^3$, $R^4_a$ , A et Q sont chacun tels que définis ci-dessus,

ou un sel de celui-ci, ou

(4) faire réagir un composé de formule:

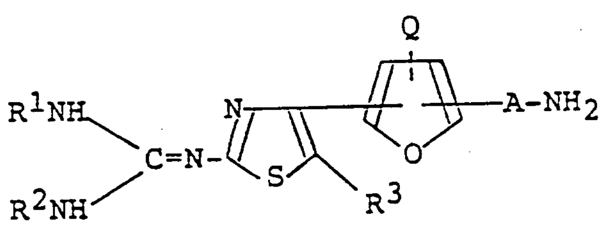

dans lequel

$R^1$, $R^2$, $R^3$, A et Q sont chacun tels que définis ci-dessus,

ou un sel de celui-ci, avec un composé de formule:

$(R^7\text{-}X^1)_2C=X\text{-}R^5$

dans lequel

$R^5$ et X sont chacun tels que définis ci-dessus,

$R^7$ est un alkyle en $C_1$-$C_6$ ou un phényle, et

$X^1$ est S ou O,

pour donner un composé de formule:

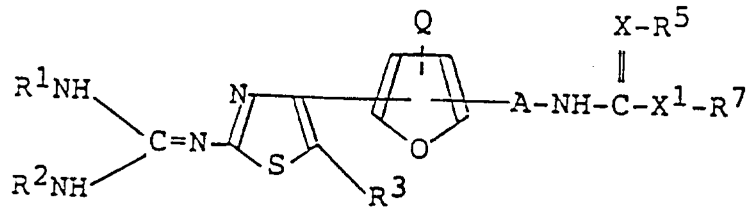

dans lequel

R¹, R², R³, R⁵, R⁷, X, X¹, A et Q sont chacun tels que défini ci-dessus,

ou un sel de celui-ci, ou

(5) faire réagir un composé de formule:

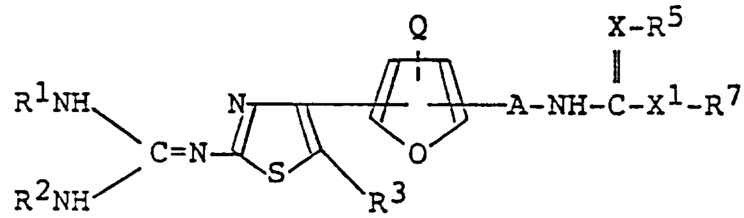

dans lequel

$R^1$, $R^2$, $R^3$, $R^5$, $R^7$, X, $X^1$, A et Q sont chacun tels que définis ci-dessus,

ou un sel de celui-ci avec un composé de formule:

$H\text{-}R^6_a$

dans lequel

$R^6_a$ est un amino qui peut avoir un mono ou dialkyle en $C_1$-$C_6$, un allyle, un propargyle, un hydroxyalkyle en $C_1$-$C_6$ ou un mono ou dialkyl(en $C_1$-$C_6$)aminoalkyle en $C_1$-$C_6$, ou un alcoxy en $C_1$-$C_6$,

pour donner un composé de formule:

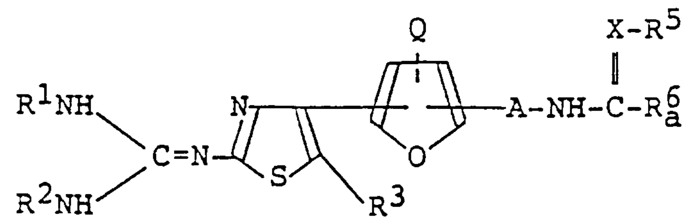

dans lequel

$R^1$, $R^2$, $R^3$, $R^5$, $R^6_a$ X, A et Q sont chacun tels que définis ci-dessus,

ou un sel de celui-ci, ou

(6) soumettre un composé de formule:

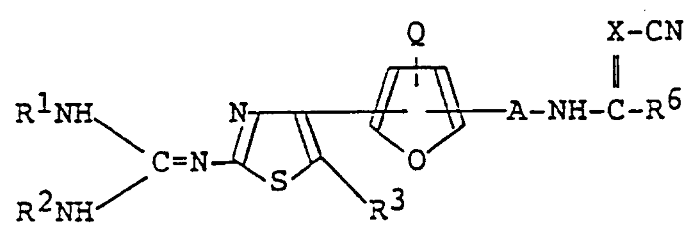

dans lequel

R¹, R², R³, R⁶, X, A et Q sont chacun tels définis ci-dessus,

ou un sel de celui-ci, à une hydrolyse, pour donner un composé de formule:

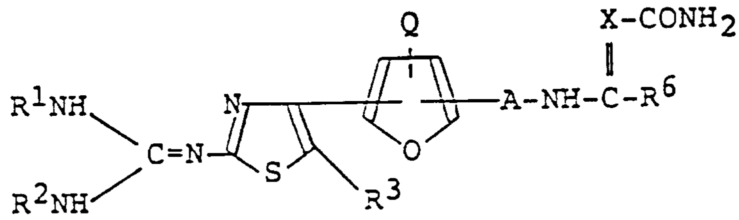

dans lequel

$R^1$, $R^2$, $R^3$, $R^6$, X, A et Q sont chacun tels que définis ci-dessus,

ou un sel de celui-ci, ou

(7) soumettre un composé de formule

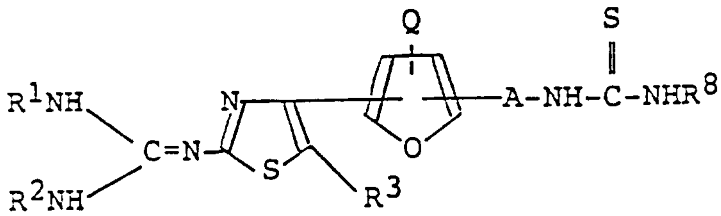

dans lequel

$R^1$, $R^2$, $R^3$, A et Q sont chacun tels que définis ci-dessus, et

$R^8$ est un groupe protecteur de l'amino constitué d'un alcanoyle en $C_1$-$C_6$ et d'un benzoyle,

ou un sel de celui-ci, à une réaction d'élimination du groupe protecteur de l'amino, pour donner un composé de formule:

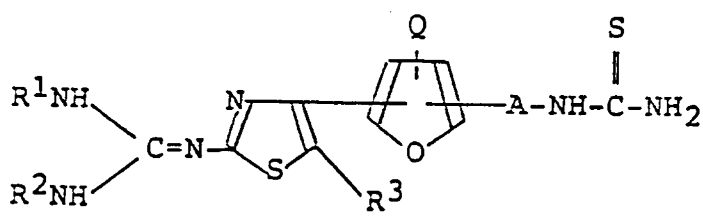

dans lequel

$R^1$, $R^2$, $R^3$, A et Q sont chacun tels que définis ci-dessus,

ou un sel de celui-ci, ou

(8) faire réagir un composé de formule:

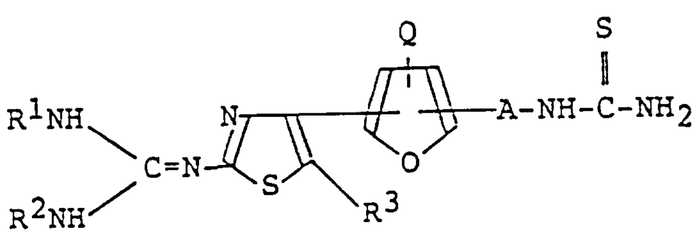

dans lequel
$R^1$, $R^2$, $R^3$, A et Q sont chacun tels que définis ci-dessus,
ou un sel de celui-ci, avec un composé de formule:

$Z^2$-$CH_2$-CO-$R^9$

dans lequel
$R^9$ est un alkyle en $C_1$-$C_6$, et
$Z^2$ est un halogène,
pour donner un composé de formule:

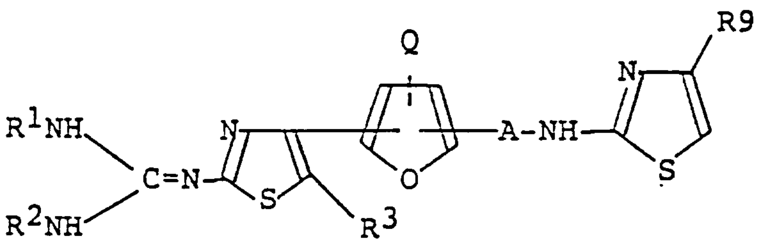

dans lequel
$R^1$, $R^2$, $R^3$, $R^9$, A et Q sont chacun tels que définis ci-dessus,
ou un sel de celui-ci, ou
(9) faire réagir un composé de formule:

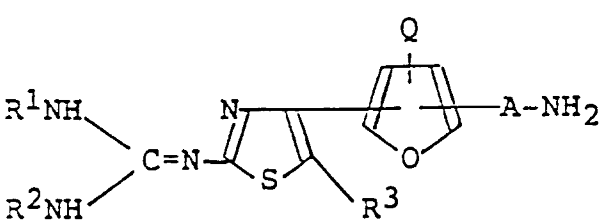

dans lequel
$R^1$, $R^2$, $R^3$, A et Q sont chacun tels que définis ci-dessus,
ou un sel de celui-ci, avec un composé de formule:

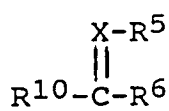

dans lequel
$R^5$, $R^6$ et X sont chacun tels que définis ci-dessus, et
$R^{10}$ est un alcanoyl(en $C_1$-$C_6$)oxy,
un phénylalcoxy en $C_1$-$C_6$,
un alcoxy(en $C_1$-$C_6$)alcoxy en $C_1$-$C_6$,
un tétrahydropyranyloxy ou
un alcoxy en $C_1$-$C_6$,
pour donner un composé de formule:

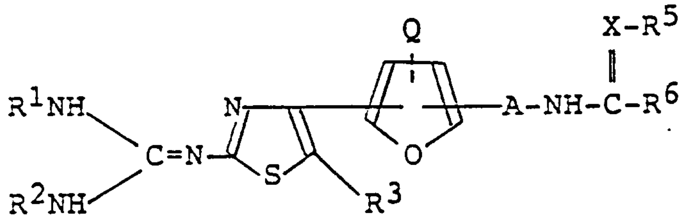

dans lequel

$R^1$, $R^2$, $R^3$, $R^5$, $R^6$, X, A et Q sont chacun tels que définis ci-dessus,

ou un sel de celui-ci, ou

(10) faire réagir un composé de formule:

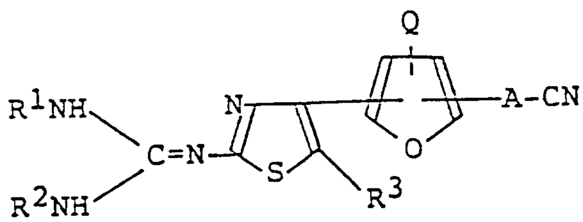

dans lequel

$R^1$, $R^2$, $R^3$, A et Q sont chacun tels que définis ci-dessus,

ou un sel de celui-ci, avec un composé de formule:

$R^{11}OH$

dans lequel

$R^{11}$ est un alkyle en $C_1$-$C_6$,

pour donner un composé de formule:

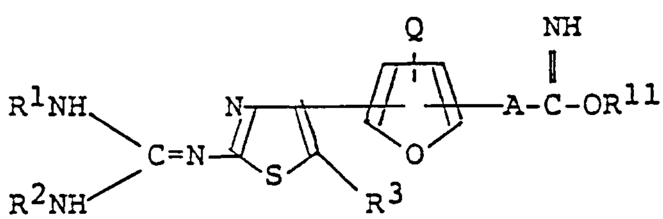

dans lequel

$R^1$, $R^2$, $R^3$, $R^{11}$, A et Q sont chacun tels que définis ci-dessus,

ou un sel de celui-ci, ou

(11) faire réagir un composé de formule:

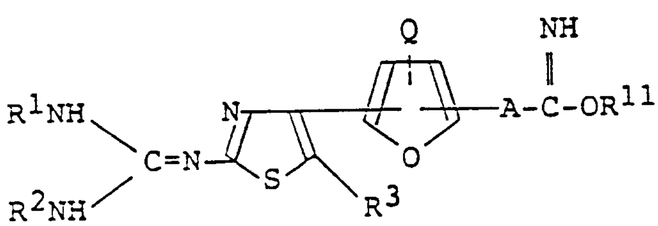

dans lequel

$R^1$, $R^2$, $R^3$, $R^{11}$, A et Q sont chacun tels que définis ci-dessus,
ou un sel de celui-ci, avec un composé de formule:

$NH_2R^{12}$

dans lequel
$R^{12}$ est un acyle,
pour donner un composé de formule:

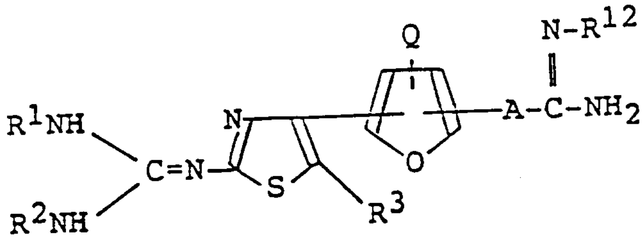

dans lequel
$R^1$, $R^2$, $R^3$, $R^{12}$, A et Q sont chacun tels que définis ci-dessus,
ou un sel de celui-ci, ou
(12) faire réagir un composé de formule:

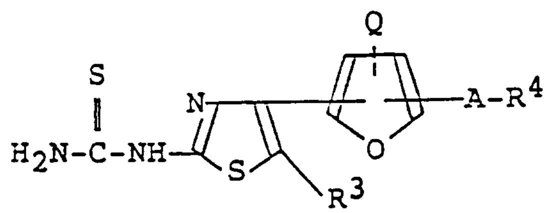

dans lequel
$R^3$, $R^4$, A et Q sont chacun tels que définis ci-dessus,
ou un sel de celui-ci, avec un composé de formule:

$R^{13}$-$Z^3$

dans lequel
$R^{13}$ est un alkyle en $C_1$-$C_6$, et
$Z^3$ est un halogène,
ou un sel de celui-ci, pour donner un composé de formule:

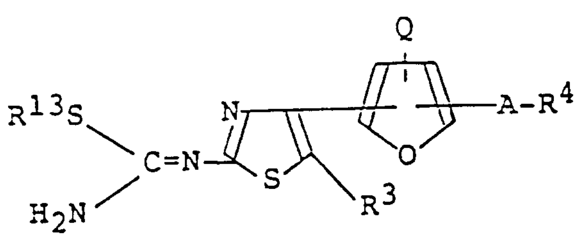

dans lequel
$R^3$, $R^4$, $R^{13}$, A et Q sont tels que définis ci-dessus,
ou un sel de celui-ci, et le faire réagir de manière continue avec un composé de formule:

$H_2N$-Y-$NH_2$

dans lequel

Y est un alkylène en $C_1$-$C_6$,

ou un sel de celui-ci,

pour donner un composé de formule:

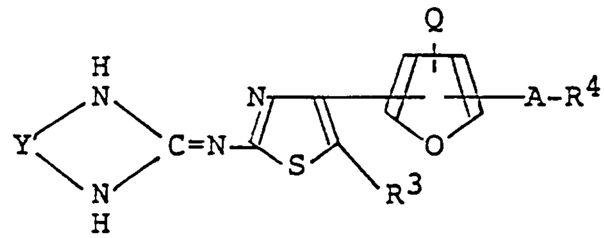

dans lequel

$R^3$, $R^4$, A, Q et Y sont chacun tels que définis ci-dessus,

ou un sel de celui-ci, ou

(13) soumettre un composé de formule:

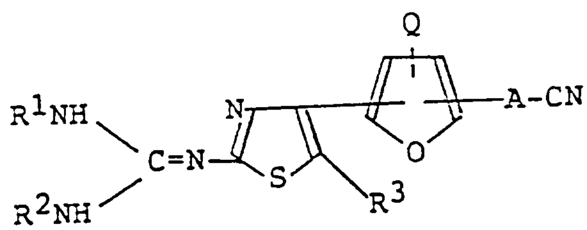

dans lequel

$R^1$, $R^2$, $R^3$, A et Q sont chacun tels que définis ci-dessus,

ou un sel de celui-ci, à une réduction, pour donner un composé de formule:

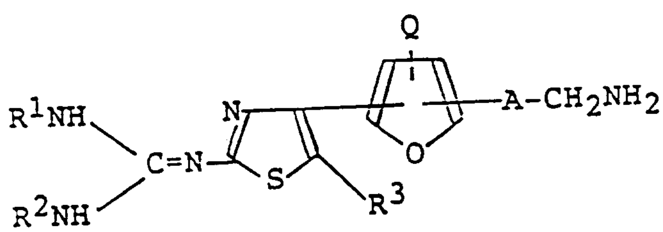

dans lequel

$R^1$, $R^2$, $R^3$, A et Q sont chacun tels que définis ci-dessus,

ou un sel de celui-ci, ou

(14) faire réagir un composé de formule:

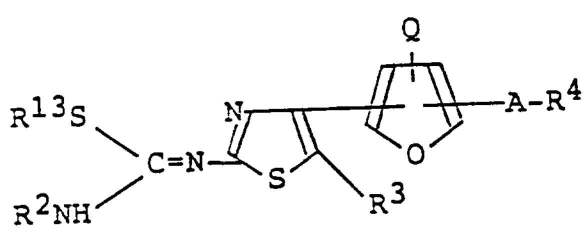

dans lequel

$R^2$, $R^3$, $R^4$, $R^{13}$, A et Q sont chacun tels que définis ci-dessus,

ou un sel de celui-ci, avec un composé de formule:

$NH_2R^1_a$

dans lequel

$R^1_a$ est un alkyle en $C_1$-$C_6$ qui peut avoir un halogène,

ou un sel de celui-ci, pour donner un composé de formule:

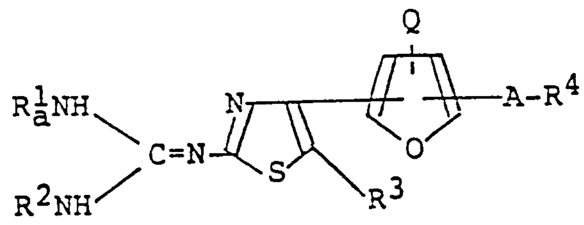

dans lequel,

$R^1_a$, $R^2$, $R^3$, $R^4$, A et Q sont chacun tels que définis ci-dessus,

ou un sel de celui-ci, ou

(15) soumettre un composé de formule:

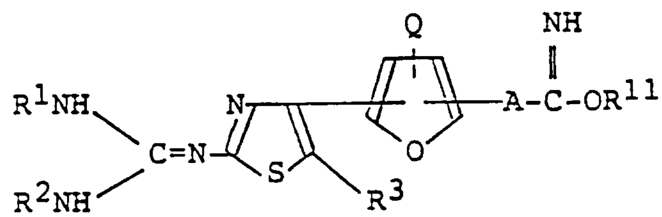

dans lequel

$R^1$, $R^2$, $R^3$, $R^{11}$, A et Q sont chacun tels que définis ci-dessus,

ou un sel de celui-ci, à une hydrolyse, pour donner un composé de formule:

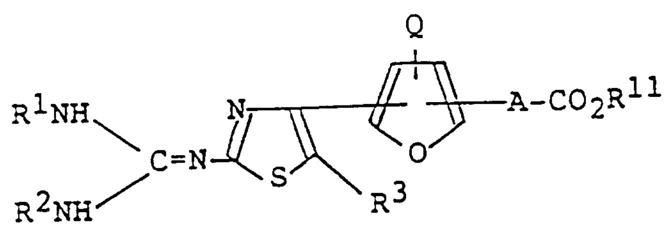

dans lequel

$R^1$, $R^2$, $R^3$, $R^{11}$, A et Q sont chacun tels que définis ci-dessus,

ou un sel de celui-ci, ou

(16) soumettre un composé de formule:

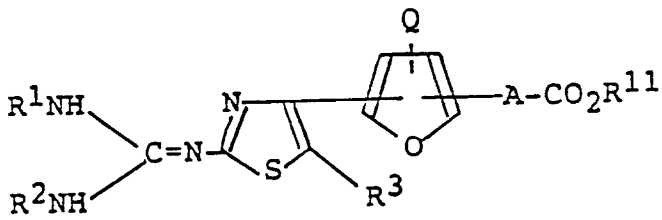

dans lequel

$R^1$, $R^2$, $R^3$, $R^{11}$, A et Q sont chacun tels que définis ci-dessus,

ou un sel de celui-ci, à une réaction d'amidation, pour donner un composé de formule:

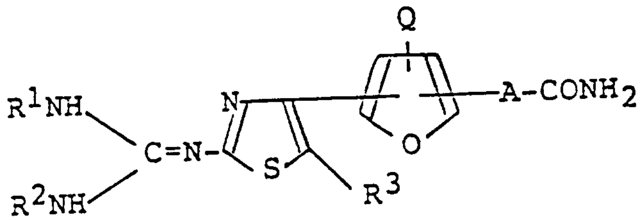

dans lequel

$R^1$, $R^2$, $R^3$, A et Q sont chacun tels que définis ci-dessus,

ou un sel de celui-ci, ou

(17) faire réagir un composé de formule:

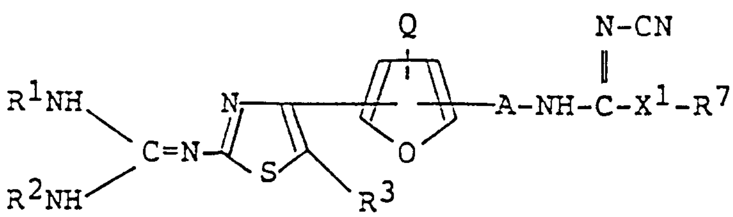

dans lequel

$R^1$, $R^2$, $R^3$, $R^7$, $X^1$, A et Q sont chacun tels que définis ci-dessus,

ou un sel de celui-ci, avec un composé de formule:

$R^{14}NHNH_2$

dans lequel

$R^{14}$ est un hydrogène ou un alkyle en $C_1$-$C_6$,

pour donner un composé de formule:

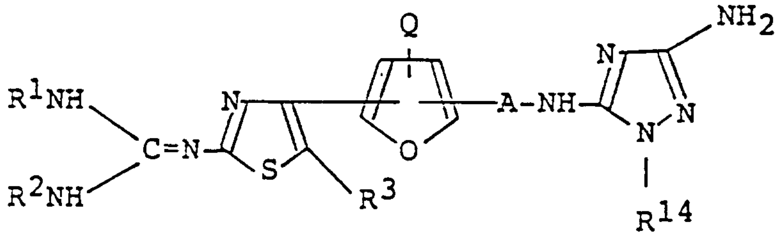

dans lequel
R¹, R², R³, R¹⁴, A et Q sont chacun tels que définis ci-dessus,
ou un sel de celui-ci, ou
(18) faire réagir un composé de formule:

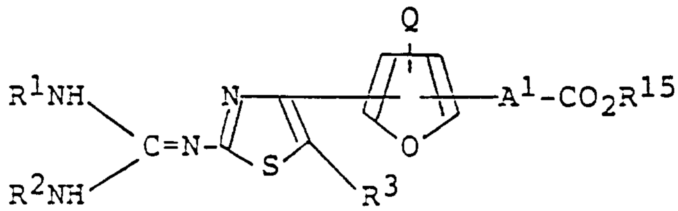

dans lequel
$R^1$, $R^2$, $R^3$ et Q sont chacun tels que définis ci-dessus,
$R^{15}$ est un alkyle en $C_1$-$C_6$, et
$A^1$ est un alkylène en $C_1$-$C_6$ ou une liaison,
ou un sel de celui-ci, avec de l'hydrazine, pour donner un composé de formule:

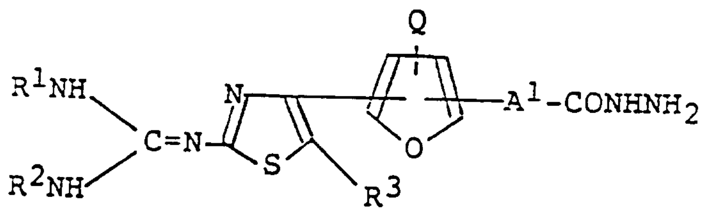

dans lequel
$R^1$, $R^2$, $R^3$, $A^1$ et Q sont chacun tels que définis ci-dessus,
ou un sel de celui-ci, ou
(19) faire réagir un composé de formule:

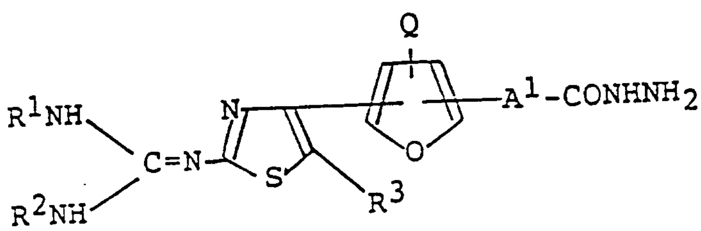

dans lequel
$R^1$, $R^2$, $R^3$, $A^1$ et Q sont chacun tels que définis ci-dessus,
ou un sel de celui-ci, avec un S-alkyl(en $C_1$-$C_6$)isothiourée,

ou un sel de celui-ci, pour donner un composé de formule:

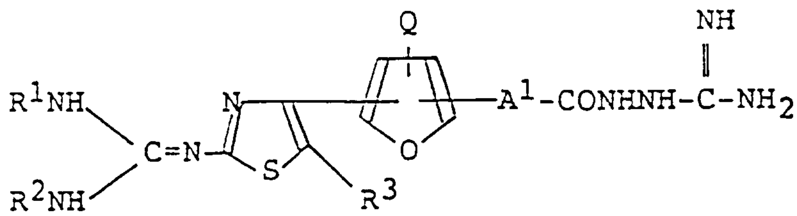

dans lequel

$R^1$, $R^2$, $R^3$, $A^1$ et Q sont chacun tels que définis ci-dessus,

ou un sel de celui-ci, ou

(20) soumettre un composé de formule:

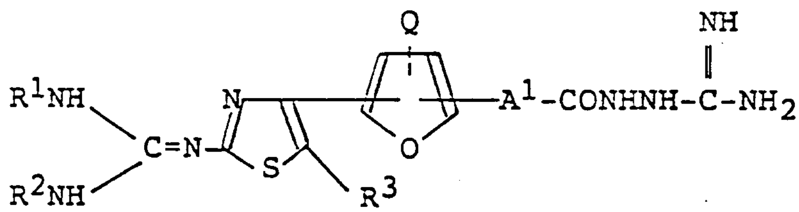

dans lequel

$R^1$, $R^2$, $R^3$, $A^1$ et Q sont chacun tels que définis ci-dessus,

ou un sel de celui-ci, à une fermeture de cycle, pour donner un composé de formule:

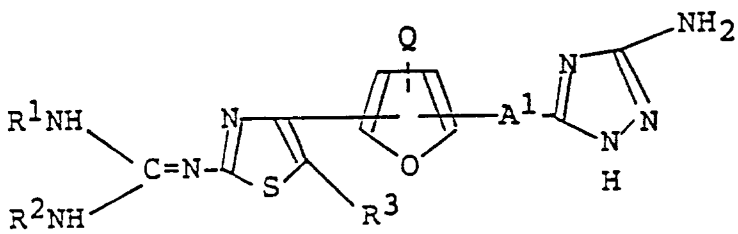

dans lequel

$R^1$, $R^2$, $R^3$, $A^1$ et Q sont chacun tels que définis ci-dessus,

ou un sel de celui-ci, ou

(21) faire réagir un composé de formule:

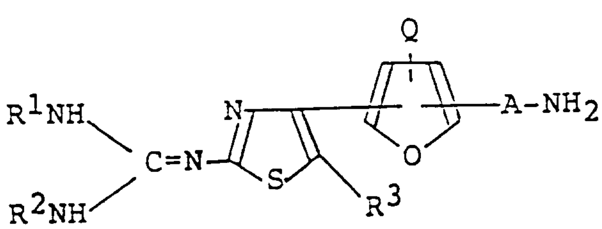

dans lequel

$R^1$, $R^2$, $R^3$, A et Q sont chacun tels que définis ci-dessus,

ou un sel de celui-ci, avec un composé de formule:

$R^{16}$-$Z^4$

dans lequel

$R^{16}$ est un thiazolyle substitué avec un alkyle en $C_1$-$C_6$, un triazolyle substitué avec un amino, un triazolyle substitué avec un amino et un alkyle en $C_1$-$C_6$, un benzoisothiazolyle substitué avec un oxo, ou un benzothiadiazinyle substitué avec un oxo, et

$Z_4$ est un halogène,

ou un sel de celui-ci, pour donner un composé de formule:

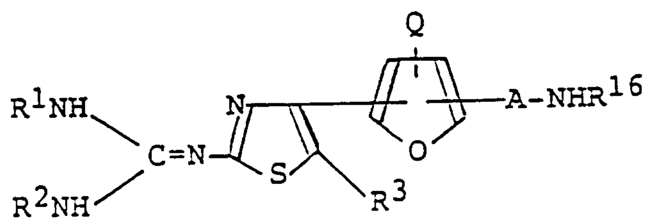

dans lequel

$R^1$, $R^2$, $R^3$, $R^{16}$, A et Q sont chacun tels que définis ci-dessus,

ou un sel de celui-ci, ou

(22) soumettre un composé de formule:

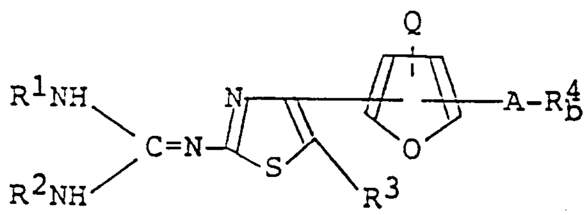

dans lequel

$R^1$, $R^2$, $R^3$, A et Q sont chacun tels que définis ci-dessus, et

$R^4_b$ est un furylalkyl(en $C_1$-$C_6$)thioalcanoyl(en $C_1$-$C_6$)amino,

ou un sel de celui-ci, à une réaction d'oxydation, pour donner un composé de formule:

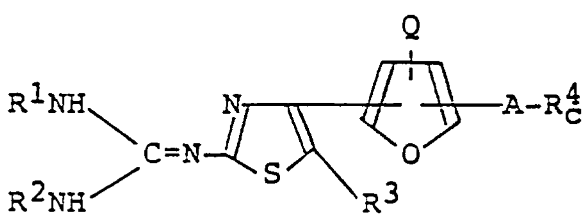

dans lequel

$R^1$, $R^2$, $R^3$, A et Q sont chacun tels que définis ci-dessus, et

$R^4_c$ est un furylalkyl(en $C_1$-$C_6$)sulfinylalcanoyl(en $C_1$-$C_6$)amino,

ou un sel de celui-ci, ou

(23) faire réagir un composé de formule:

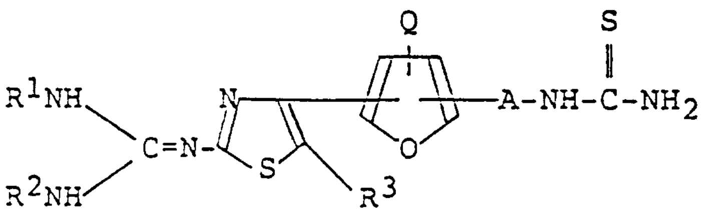

dans lequel

$R^1$, $R^2$, $R^3$, A et Q sont chacun tels que définis ci-dessus,

ou un sel de celui-ci, avec un composé de formule:

$R^{17}$-$Z^5$

dans lequel

$R^{17}$ est un alkyle en $C_1$-$C_6$, et

$Z^5$ est un halogène,

ou un sel de celui-ci, pour donner un composé de formule:

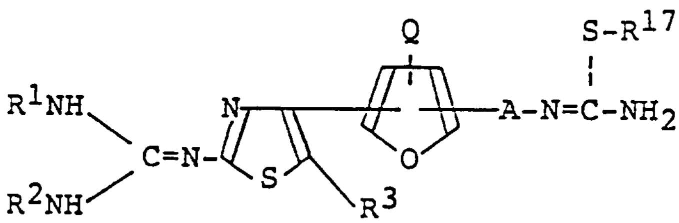

dans lequel

$R^1$, $R^2$, $R^3$, $R^{17}$, A et Q sont chacun tels que définis ci-dessus,

ou un sel de celui-ci, ou

(24) faire réagir un composé de formule:

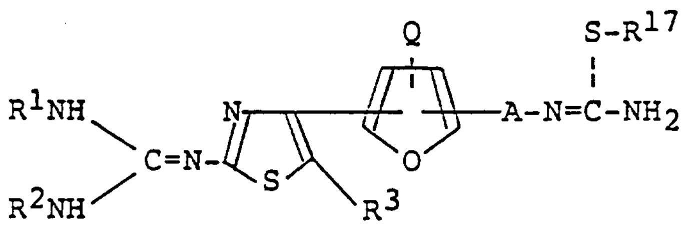

dans lequel

$R^1$, $R^2$, $R^3$, $R^{17}$, A et Q sont chacun tels que définis ci-dessus,

ou un sel de celui-ci, avec un composé de formule:

$H_2N$-$R^5$

dans lequel $R^5$ est tel que défini ci-dessus,

ou un sel de celui-ci, pour donner un composé de formule:

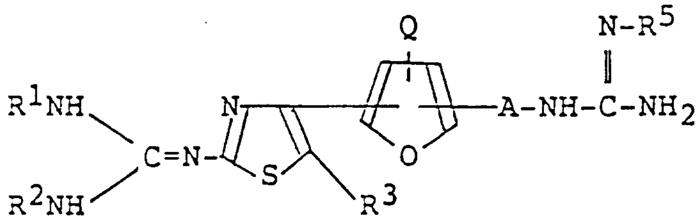

dans lequel

$R^1$, $R^2$, $R^3$, $R^5$, A et Q sont chacun tels que définis ci-dessus,

ou un sel de celui-ci, ou

(25) soumettre un composé de formule:

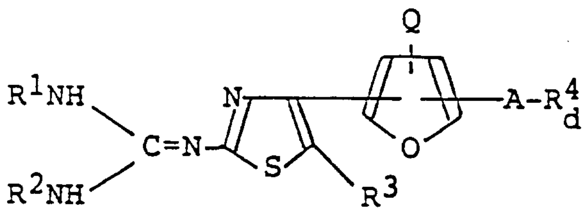

dans lequel

$R^1$, $R^2$, $R^3$, A et Q sont chacun tels que définis ci-dessus, et

$R^4_d$ est un alcanoyl(en $C_1$-$C_6$)oxyalkyl(en $C_1$-$C_6$)uréido,

ou un sel de celui-ci, à une réaction d'élimination de l'alcanoyle en $C_1$-$C_6$, pour donner un composé de formule:

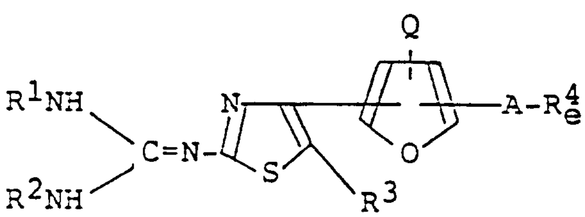

dans lequel

$R^1$, $R^2$, $R^3$, A et Q sont chacun tels que définis ci-dessus, et

$R^4_e$ est un hydroxyalkyl(en $C_1$-$C_6$)uréido,

ou un sel de celui-ci, ou

(26) faire réagir un composé de formule:

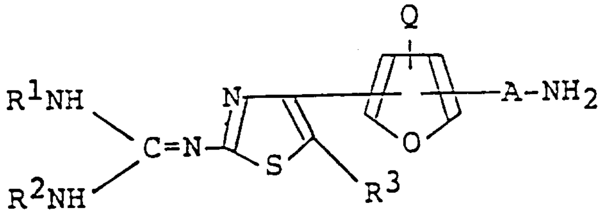

dans lequel

$R^1$, $R^2$, $R^3$, A et Q sont chacun tels que définis ci-dessus,

ou un sel de celui-ci, avec un composé de formule

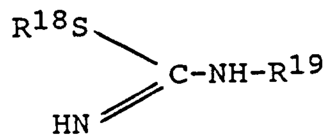

dans lequel

$R^{18}$ est un alkyle en $C_1$-$C_6$, et

$R^{19}$ est un carbamoyle,
un alcoxy(en $C_1$-$C_6$)carbonyle,
un sulfamoyle,
un alkyl(en $C_1$-$C_6$)sulfonyle,
un benzènesulfonyle, qui est substitué avec un ou plusieurs substituents choisis parmi un alkyle en $C_1$-$C_6$, un alcoxy en $C_1$-$C_6$, un halogène et un amino, ou un mono ou dialkyl(en $C_1$-$C_6$)sulfamoyle,

ou un sel de celui-ci, pour donner un composé de formule:

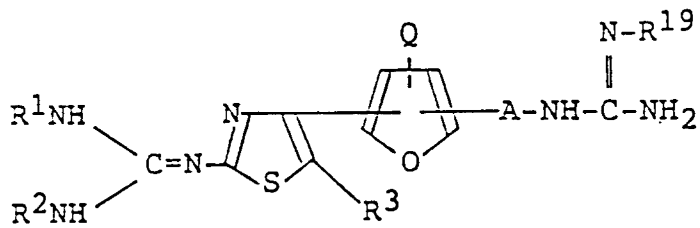

dans lequel

$R^1$, $R^2$, $R^3$, $R^{19}$, A et Q sont chacun tels que définis ci-dessus,

ou un sel de celui-ci, ou

(27) soumettre un composé de formule:

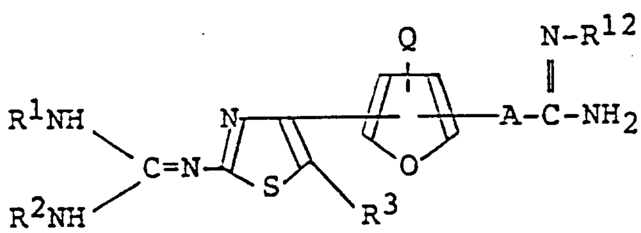

dans lequel

$R^1$, $R^2$, $R^3$, $R^{12}$, A et Q sont chacun tels que définis ci-dessus,

ou un sel de celui-ci, à une hydrolyse, pour donner un composé de formule:

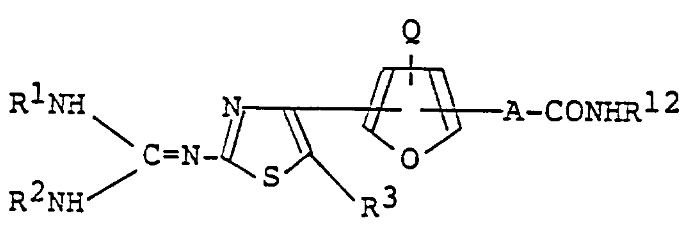

dans lequel

$R^1$, $R^2$, $R^3$, $R^{12}$, A et Q sont chacun tels que définis ci-dessus,

ou un sel de celui-ci.

**2.** Procédé selon la revendication 1, dans lequel

$R^1$ et $R^2$ sont chacun un hydrogène, un alkyle en $C_1$-$C_6$ ou un trihaloalkyle en $C_1$-$C_6$; ou

$R^1$ et $R^2$ sont liés ensemble pour former un alkylène en $C_1$-$C_6$,

$R^4$ est un amino, un carbamoyle, un aminocarbamoyle,
un guanidinocarbamoyle, un alkyl(en $C_1$-$C_6$)carbamoyle,
un sulfamoylaminocarbonyle,
un alcoxy(en $C_1$-$C_6$)carbonyle, un uréido,
un thiouréido, un sulfamoylamino,
un alcanoyl(en $C_1$-$C_6$)amino,
un alcoxy(en $C_1$-$C_6$)carbonylamino,
un alkyl(en $C_1$-$C_6$)sulfonylamino,
un alcoxy(en $C_1$-$C_6$)alcanoyl(en $C_1$-$C_6$)amino,
un alcanoyl(en $C_1$-$C_6$)oxyalcanoyl(en $C_1$-$C_6$)amino,
un alkyl(en $C_1$-$C_6$)thioalcanoyl(en $C_1$-$C_6$)amino,
un nitrobenzoylamino, un furoylamino, un thénoylamino,
un nicotinoylamino,
un 1-oxonicotinoylamino, un morpholinocarbonylamino,
un 3-alkyl(en $C_1$-$C_6$)uréido,
un 3-alkyl(en $C_1$-$C_6$)thiouréido,
un 3-alcanoyl(en $C_1$-$C_6$)uréido, un 3-allyluréido,
un 3-benzoylthiouréido,
un furylalkyl(en $C_1$-$C_6$)thioalcanoyl(en $C_1$-$C_6$)amino,
un furylalkyl(en $C_1$-$C_6$)sulfinylalcanoyl(en $C_1$-$C_6$)amino,
un mono ou dialkyl(en $C_1$-$C_6$)sulfamoylamino,
un 3-hydroxyalkyl(en $C_1$-$C_6$)uréido,
un 3-alcanoyl(en $C_1$-$C_6$)oxyalkyl(en $C_1$-$C_6$)uréido,
un 2-alkyl(en $C_1$-$C_6$)isothiouréido,
un thiazolylamino substitué avec un alkyle en $C_1$-$C_6$,
un triazolylamino substitué avec un amino,
un triazolylamino substitué avec un amino et un alkyle en $C_1$-$C_6$,
un benzoisothiazolylamino substitué avec un oxo,
un benzothiadiazinylamino substitué avec un oxo et un halogène,
un pyrimidinyle substitué avec un oxo et un alkyle en $C_1$-$C_6$,
un triazolyle substitué avec un amino, ou
un groupe de formule:

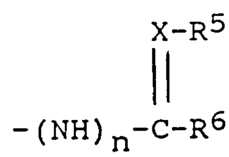

dans lequel

n, X et $R^6$ sont tels que définis dans la revendication 1,

$R^5$ est un hydrogène, un cyano, un nitro,
un carbamoyle, un alcoxy(en $C_1$-$C_6$)carbonyle,
un sulfamoyle, un alkyl(en $C_1$-$C_6$)sulfonyle,
un phénylsulfonyle substitué avec un alkyle en $C_1$-$C_6$, un phénylsulfonyle substitué avec un alcoxy en $C_1$-$C_6$, un phénylsulfonyle substitué avec un halogène, un phénylsulfonyle substitué avec un amino,
un mono ou dialkyl(en $C_1$-$C_6$)sulfamoyle,

A est tel que défini dans la revendication 1; ou

A-$R^4$ est tel que défini dans la revendication 1, et

$R^3$ et Q sont tels que définis dans la revendication 1.

**3.** Procédé selon la revendication 2, dans lequel

$R^1$ et $R^2$ sont chacun un hydrogène,

$R^3$ est un hydrogène,
$R^4$ est un groupe de formule:

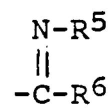

dans lequel
$R^5$ est un sulfamoyle, et
$R^6$ est un amino,
A est un alkylène en $C_1$-$C_6$, et
Q est un hydrogène.

4. Procédé selon la revendication 2, dans lequel
$R^1$ et $R^2$ sont chacun un hydrogène,
$R^3$ est un hydrogène,
$R^4$ est un uréido ou un 3-alkyl(en $C_1$-$C_6$)uréido,
A est un alkylène en $C_1$-$C_6$, et
Q est un hydrogène.

5. Procédé pour la préparation d'une composition pharmaceutique qui comprend en tant qu'ingrédient actif un composé de formule I selon la revendication 1, caractérisé par le mélange d'un composé de formule I avec au moins un support ou un excipient non-toxique pharmaceutiquement acceptable.

6. Modification du procédé selon la revendication 1, qui est caractérisé par la mise d'un composé de formule I ou d'un sel non-toxique de celui-ci produit par un procédé selon la revendication 1, dans une forme pharmaceutiquement acceptable, par mélange ou présentation dudit composé avec un diluant ou un support pharmaceutiquement acceptable.

7. Utilisation d'un composé de formule I selon la revendication 1, pour la préparation d'un médicament pour le traitement thérapeutique d'ulcère chez les êtres humains ou les animaux.